# EUROPEAN PATENT SPECIFICATION

(11) **EP 3 057 615 B1**
(45) Date of publication and mention of the grant of the patent: **24.02.2021**
(21) Application number: 14790974.1
(22) Date of filing: 17.10.2014
(51) Int. Cl.: A61P 1/04, A61P 11/00, A61P 35/00, A61P 43/00, C07K 16/18, A61K 39/00, C07K 16/28, C07K 16/30

(54) **ANTI-RSPO ANTIBODIES AND METHODS OF USE**
ANTI-RSPO-ANTIKÖRPER UND VERFAHREN ZUR VERWENDUNG
ANTICORPS ANTI-RSPO ET LEURS MÉTHODES D'UTILISATION

(30) Priority: 18.10.2013 US 201361893141 P; 26.09.2014 US 201462056324 P
(43) Date of publication of application: 24.08.2016
(60) Divisional application: 21151359.3
(73) Proprietor: F. Hoffmann-La Roche AG, 4070 Basel (CH)
(72) Inventor: STORM, Elaine, South San Francisco California 94080 (US); DE SAUVAGE, Frederic J., South San Francisco California 94080 (US); MURRAY, Jeremy M., South San Francisco California 94080 (US); NOLAND, Cameron L., South San Francisco California 94080 (US); WU, Yan, South San Francisco California 94080 (US); TAN, Christine, South San Francisco California 94080 (US); HONGO, Jo-Anne, South San Francisco California 94080 (US); CHEN, Yongmei, South San Francisco California 94080 (US)
(74) Representative: Brodbeck, Michel
(86) International application number: PCT/US2014/061215
(87) International publication number: WO 2015/058132

(56) References cited:
- WO-A1-2013/012747
- WO-A1-2013/012747
- WO-A1-2013/012747

### SEQUENCE LISTING

<110> GENENTECH, INC. ET AL.
<120> ANTI-RSPO ANTIBODIES AND METHODS OF USE
<130> P5719R1-WO
<140> 62/056,324
   <141> 2014-09-26
<150> 61/893,141
   <151> 2013-10-18
<160> 216
<170> PatentIn version 3.5
<210> 1
   <211> 243
   <212> PRT
   <213> Homo sapiens
<400> 1
   Met Gln Phe Arg Leu Phe Ser Phe Ala Leu Ile Ile Leu Asn Cys Met
   1               5                   10                  15
   Asp Tyr Ser His Cys Gln Gly Asn Arg Trp Arg Arg Ser Lys Arg Ala
               20                  25                  30
   Ser Tyr Val Ser Asn Pro Ile Cys Lys Gly Cys Leu Ser Cys Ser Lys
           35                  40                  45
   Asp Asn Gly Cys Ser Arg Cys Gln Gln Lys Leu Phe Phe Phe Leu Arg
       50                  55                  60
   Arg Glu Gly Met Arg Gln Tyr Gly Glu Cys Leu His Ser Cys Pro Ser
   65                  70                  75                  80
   Gly Tyr Tyr Gly His Arg Ala Pro Asp Met Asn Arg Cys Ala Arg Cys
                   85                  90                  95
   Arg Ile Glu Asn Cys Asp Ser Cys Phe Ser Lys Asp Phe Cys Thr Lys
               100                 105                 110
   Cys Lys Val Gly Phe Tyr Leu His Arg Gly Arg Cys Phe Asp Glu Cys
           115                 120                 125
   Pro Asp Gly Phe Ala Pro Leu Glu Glu Thr Met Glu Cys Val Glu Gly
       130                 135                 140
   Cys Glu Val Gly His Trp Ser Glu Trp Gly Thr Cys Ser Arg Asn Asn
   145                 150                 155                 160
   Arg Thr Cys Gly Phe Lys Trp Gly Leu Glu Thr Arg Thr Arg Gln Ile
   165 170 175
   Val Lys Lys Pro Val Lys Asp Thr Ile Leu Cys Pro Thr Ile Ala Glu
   180 185 190
   Ser Arg Arg Cys Lys Met Thr Met Arg His Cys Pro Gly Gly Lys Arg
   195 200 205
   Thr Pro Lys Ala Lys Glu Lys Arg Asn Lys Lys Lys Lys Arg Lys Leu
   210 215 220
   Ile Glu Arg Ala Gln Glu Gln His Ser Val Phe Leu Ala Thr Asp Arg
   225 230 235 240
   Ala Asn Gln
<210> 2
   <211> 272
   <212> PRT
   <213> Homo sapiens
<400> 2
   Met His Leu Arg Leu Ile Ser Trp Leu Phe Ile Ile Leu Asn Phe Met
   1 5 10 15
   Glu Tyr Ile Gly Ser Gln Asn Ala Ser Arg Gly Arg Arg Gln Arg Arg
   20 25 30
   Met His Pro Asn Val Ser Gln Gly Cys Gln Gly Gly Cys Ala Thr Cys
   35 40 45
   Ser Asp Tyr Asn Gly Cys Leu Ser Cys Lys Pro Arg Leu Phe Phe Ala
   50 55 60
   Leu Glu Arg Ile Gly Met Lys Gln Ile Gly Val Cys Leu Ser Ser Cys
   65 70 75 80
   Pro Ser Gly Tyr Tyr Gly Thr Arg Tyr Pro Asp Ile Asn Lys Cys Thr
   85 90 95
   Lys Cys Lys Ala Asp Cys Asp Thr Cys Phe Asn Lys Asn Phe Cys Thr
   100 105 110
   Lys Cys Lys Ser Gly Phe Tyr Leu His Leu Gly Lys Cys Leu Asp Asn
   115 120 125
   Cys Pro Glu Gly Leu Glu Ala Asn Asn His Thr Met Glu Cys Val Ser
   130 135 140
   Ile Val His Cys Glu Val Ser Glu Trp Asn Pro Trp Ser Pro Cys Thr
   145 150 155 160
   Lys Lys Gly Lys Thr Cys Gly Phe Lys Arg Gly Thr Glu Thr Arg Val
   165 170 175
   Arg Glu Ile Ile Gln His Pro Ser Ala Lys Gly Asn Leu Cys Pro Pro
   180 185 190
   Thr Asn Glu Thr Arg Lys Cys Thr Val Gln Arg Lys Lys Cys Gln Lys
   195 200 205
   Gly Glu Arg Gly Lys Lys Gly Arg Glu Arg Lys Arg Lys Lys Pro Asn
   210 215 220
   Lys Gly Glu Ser Lys Glu Ala Ile Pro Asp Ser Lys Ser Leu Glu Ser
   225 230 235 240
   Ser Lys Glu Ile Pro Glu Gln Arg Glu Asn Lys Gln Gln Gln Lys Lys
   245 250 255
   Arg Lys Val Gln Asp Lys Gln Lys Ser Val Ser Val Ser Thr Val His
   260 265 270
<210> 3
   <211> 263
   <212> PRT
   <213> Homo sapiens
<400> 3
   Met Arg Leu Gly Leu Cys Val Val Ala Leu Val Leu Ser Trp Thr His
   1               5                   10                  15
   Leu Thr Ile Ser Ser Arg Gly Ile Lys Gly Lys Arg Gln Arg Arg Ile
               20                  25                  30
   Ser Ala Glu Gly Ser Gln Ala Cys Ala Lys Gly Cys Glu Leu Cys Ser
           35                  40                  45
   Glu Val Asn Gly Cys Leu Lys Cys Ser Pro Lys Leu Phe Ile Leu Leu
       50                  55                  60
   Glu Arg Asn Asp Ile Arg Gln Val Gly Val Cys Leu Pro Ser Cys Pro
   65                  70                  75                  80
   Pro Gly Tyr Phe Asp Ala Arg Asn Pro Asp Met Asn Lys Cys Ile Lys
                   85                  90                  95
   Cys Lys Ile Glu His Cys Glu Ala Cys Phe Ser His Asn Phe Cys Thr
               100                 105                 110
   Lys Cys Lys Glu Gly Leu Tyr Leu His Lys Gly Arg Cys Tyr Pro Ala
           115                 120                 125
   Cys Pro Glu Gly Ser Ser Ala Ala Asn Gly Thr Met Glu Cys Ser Ser
       130                 135                 140
   Pro Ala Gln Cys Glu Met Ser Glu Trp Ser Pro Trp Gly Pro Cys Ser
   145                 150                 155                 160
   Lys Lys Gln Gln Leu Cys Gly Phe Arg Arg Gly Ser Glu Glu Arg Thr
                   165                 170                 175
   Arg Arg Val Leu His Ala Pro Val Gly Asp His Ala Ala Cys Ser Asp
               180                 185                 190
   Thr Lys Glu Thr Arg Arg Cys Thr Val Arg Arg Val Pro Cys Pro Glu
           195                 200                 205
   Gly Gln Lys Arg Arg Lys Gly Gly Gln Gly Arg Arg Glu Asn Ala Asn
       210                 215                 220
   Arg Asn Leu Ala Arg Lys Glu Ser Lys Glu Ala Gly Ala Gly Ser Arg
   225                 230                 235                 240
   Arg Arg Lys Gly Gln Gln Gln Gln Gln Gln Gln Gly Thr Val Gly Pro
                   245                 250                 255
   Leu Thr Ser Ala Gly Pro Ala
               260
<210> 4
   <211> 234
   <212> PRT
   <213> Homo sapiens
<400> 4
   Met Arg Ala Pro Leu Cys Leu Leu Leu Leu Val Ala His Ala Val Asp
   1               5                   10                  15
   Met Leu Ala Leu Asn Arg Arg Lys Lys Gln Val Gly Thr Gly Leu Gly
               20                  25                  30
   Gly Asn Cys Thr Gly Cys Ile Ile Cys Ser Glu Glu Asn Gly Cys Ser
           35                  40                  45
   Thr Cys Gln Gln Arg Leu Phe Leu Phe Ile Arg Arg Glu Gly Ile Arg
       50                  55                  60
   Gln Tyr Gly Lys Cys Leu His Asp Cys Pro Pro Gly Tyr Phe Gly Ile
   65                  70                  75                  80
   Arg Gly Gln Glu Val Asn Arg Cys Lys Lys Cys Gly Ala Thr Cys Glu
                   85                  90                  95
   Ser Cys Phe Ser Gln Asp Phe Cys Ile Arg Cys Lys Arg Gln Phe Tyr
               100                 105                 110
   Leu Tyr Lys Gly Lys Cys Leu Pro Thr Cys Pro Pro Gly Thr Leu Ala
           115                 120                 125
   His Gln Asn Thr Arg Glu Cys Gln Gly Glu Cys Glu Leu Gly Pro Trp
       130                 135                 140
   Gly Gly Trp Ser Pro Cys Thr His Asn Gly Lys Thr Cys Gly Ser Ala
   145                 150                 155                 160
   Trp Gly Leu Glu Ser Arg Val Arg Glu Ala Gly Arg Ala Gly His Glu
                   165                 170                 175
   Glu Ala Ala Thr Cys Gln Val Leu Ser Glu Ser Arg Lys Cys Pro Ile
               180                 185                 190
   Gln Arg Pro Cys Pro Gly Glu Arg Ser Pro Gly Gln Lys Lys Gly Arg
           195                 200                 205
   Lys Asp Arg Arg Pro Arg Lys Asp Arg Lys Leu Asp Arg Arg Leu Asp
       210                 215                 220
   Val Arg Pro Arg Gln Pro Gly Leu Gln Pro
   225                 230
<210> 5
   <211> 16
   <212> PRT
   <213> Artificial Sequence
<220>
   <221> source
   <223> /note="Description of Artificial Sequence: Synthetic peptide"
<400> 5
   Arg Ser Ser Gln Ser Ile Val His Ser Asn Gly Asn Thr Tyr Leu Glu
   1               5                   10                  15
<210> 6
   <211> 7
   <212> PRT
   <213> Artificial Sequence
<220>
   <221> source
   <223> /note="Description of Artificial Sequence: Synthetic peptide"
<400> 6
   Arg Ile Ser Asn Arg Phe Ser
   1               5
<210> 7
   <211> 9
   <212> PRT
   <213> Artificial Sequence
<220>
   <221> source
   <223> /note="Description of Artificial Sequence: Synthetic peptide"
<400> 7
   Phe Gln Gly Ser His Val Pro Tyr Thr
   1               5
<210> 8
   <211> 5
   <212> PRT
   <213> Artificial Sequence
<220>
   <221> source
   <223> /note="Description of Artificial Sequence: Synthetic peptide"
<400> 8
   Asn Phe Ala Met Ser
   1               5
<210> 9
   <211> 17
   <212> PRT
   <213> Artificial Sequence
<220>
   <221> source
   <223> /note="Description of Artificial Sequence: Synthetic peptide"
<400> 9
   Glu Ile Asn Asn Gly Gly Asn Tyr Ala Tyr Tyr Gln Asp Thr Val Thr
   1               5                   10                  15
   Gly
<210> 10
   <211> 12
   <212> PRT
   <213> Artificial Sequence
<220>
   <221> source
   <223> /note="Description of Artificial Sequence: Synthetic peptide"
<400> 10
   Glu Asp Tyr Val Asn Tyr Glu Ala Tyr Phe Ala Tyr
   1               5                   10
<210> 11
   <211> 16
   <212> PRT
   <213> Artificial Sequence
<220>
   <221> source
   <223> /note="Description of Artificial Sequence: Synthetic peptide"
<400> 11
   Arg Ser Ser Gln Ser Ile Val His Ser Asn Gly Asn Thr Tyr Leu Glu
   1               5                   10                  15
<210> 12
   <211> 7
   <212> PRT
   <213> Artificial Sequence
<220>
   <221> source
   <223> /note="Description of Artificial Sequence: Synthetic peptide"
<400> 12
   Arg Ile Ser Asn Arg Phe Ser
   1               5
<210> 13
   <211> 9
   <212> PRT
   <213> Artificial Sequence
<220>
   <221> source
   <223> /note="Description of Artificial Sequence: Synthetic peptide"
<400> 13
   Phe Gln Gly Ser His Val Pro Tyr Thr
   1 5
<210> 14
   <211> 5
   <212> PRT
   <213> Artificial Sequence
<220>
   <221> source
   <223> /note="Description of Artificial Sequence: Synthetic peptide"
<400> 14
   Asn Phe Ala Met Ser
   1 5
<210> 15
   <211> 17
   <212> PRT
   <213> Artificial Sequence
<220>
   <221> source
   <223> /note="Description of Artificial Sequence: Synthetic peptide"
<400> 15
   Glu Ile Asn Asn Gly Gly Asn Tyr Ala Tyr Tyr Gln Asp Thr Val Thr
   1 5 10 15
   Gly
<210> 16
   <211> 12
   <212> PRT
   <213> Artificial Sequence
<220>
   <221> source
   <223> /note="Description of Artificial Sequence: Synthetic peptide"
<400> 16
   Glu Asp Tyr Val Asn Tyr Glu Ala Tyr Phe Ala Tyr
   1 5 10
<210> 17
   <211> 11
   <212> PRT
   <213> Artificial Sequence
<220>
   <221> source
   <223> /note="Description of Artificial Sequence: Synthetic peptide"
<400> 17
   Arg Ala Ser Gln Asp Ile Ser Asn Tyr Leu Asn
   1               5                   10
<210> 18
   <211> 7
   <212> PRT
   <213> Artificial Sequence
<220>
   <221> source
   <223> /note="Description of Artificial Sequence: Synthetic peptide"
<400> 18
   Tyr Thr Ser Arg Leu His Ser
   1               5
<210> 19
   <211> 9
   <212> PRT
   <213> Artificial Sequence
<220>
   <221> source
   <223> /note="Description of Artificial Sequence: Synthetic peptide"
<400> 19
   Gln Gln Gly Asp Thr Leu Pro Pro Thr
   1               5
<210> 20
   <211> 5
   <212> PRT
   <213> Artificial Sequence
<220>
   <221> source
   <223> /note="Description of Artificial Sequence: Synthetic peptide"
<400> 20
   Ser Tyr Gly Val His
   1               5
<210> 21
   <211> 16
   <212> PRT
   <213> Artificial Sequence
<220>
   <221> source
   <223> /note="Description of Artificial Sequence: Synthetic peptide"
<400> 21
   Val Ile Trp Thr Gly Gly Ser Thr Asn Tyr Asn Ser Ala Leu Met Ser
   1 5 10 15
<210> 22
   <211> 9
   <212> PRT
   <213> Artificial Sequence
<220>
   <221> source
   <223> /note="Description of Artificial Sequence: Synthetic peptide"
<400> 22
   Val Asp Gly Tyr Tyr Tyr Phe Asp Tyr
   1 5
<210> 23
   <211> 11
   <212> PRT
   <213> Artificial Sequence
<220>
   <221> source
   <223> /note="Description of Artificial Sequence: Synthetic peptide"
<400> 23
   Lys Ala Ser Gln Asp Ile Asp Ser Tyr Leu Ser
   1 5 10
<210> 24
   <211> 7
   <212> PRT
   <213> Artificial Sequence
<220>
   <221> source
   <223> /note="Description of Artificial Sequence: Synthetic peptide"
<400> 24
   Leu Thr Asn Arg Leu Val Asp
   1 5
<210> 25
   <211> 9
   <212> PRT
   <213> Artificial Sequence
<220>
   <221> source
   <223> /note="Description of Artificial Sequence: Synthetic peptide"
<400> 25
   Leu His Tyr Asp Glu Phe Pro Leu Thr
   1 5
<210> 26
   <211> 5
   <212> PRT
   <213> Artificial Sequence
<220>
   <221> source
   <223> /note="Description of Artificial Sequence: Synthetic peptide"
<400> 26
   Ser Gly Tyr Trp Asn
   1 5
<210> 27
   <211> 16
   <212> PRT
   <213> Artificial Sequence
<220>
   <221> source
   <223> /note="Description of Artificial Sequence: Synthetic peptide"
<400> 27
   Tyr Ile Ser Tyr Ser Gly Lys Thr Tyr Gln Asn Pro Ser Leu Lys Ser
   1 5 10 15
<210> 28
   <211> 11
   <212> PRT
   <213> Artificial Sequence
<220>
   <221> source
   <223> /note="Description of Artificial Sequence: Synthetic peptide"
<400> 28
   Tyr Tyr Gly Tyr Gly Gly Pro Trp Phe Ala Tyr
   1 5 10
<210> 29
   <211> 11
   <212> PRT
   <213> Artificial Sequence
<220>
   <221> source
   <223> /note="Description of Artificial Sequence: Synthetic peptide"
<400> 29
   Arg Ala Ser Gln Asp Ile Ser Asn Tyr Leu Asn
   1 5 10
<210> 30
   <211> 7
   <212> PRT
   <213> Artificial Sequence
<220>
   <221> source
   <223> /note="Description of Artificial Sequence: Synthetic peptide"
<400> 30
   Tyr Thr Ser Arg Leu His Ser
   1 5
<210> 31
   <211> 9
   <212> PRT
   <213> Artificial Sequence
<220>
   <221> source
   <223> /note="Description of Artificial Sequence: Synthetic peptide"
<400> 31
   Gln His Gly Asp Thr Leu Pro Pro Thr
   1 5
<210> 32
   <211> 5
   <212> PRT
   <213> Artificial Sequence
<220>
   <221> source
   <223> /note="Description of Artificial Sequence: Synthetic peptide"
<400> 32
   Ser Tyr Ala Val His
   1 5
<210> 33
   <211> 16
   <212> PRT
   <213> Artificial Sequence
<220>
   <221> source
   <223> /note="Description of Artificial Sequence: Synthetic peptide"
<400> 33
   Val Ile Trp Ser Gly Gly Ser Thr Asp Tyr Asn Ala Ala Phe Ile Ser
   1 5 10 15
<210> 34
   <211> 9
   <212> PRT
   <213> Artificial Sequence
<220>
   <221> source
   <223> /note="Description of Artificial Sequence: Synthetic peptide"
<400> 34
   Asn Asp Gly Tyr Tyr Tyr Phe Asp Tyr
   1               5
<210> 35
   <211> 11
   <212> PRT
   <213> Artificial Sequence
<220>
   <221> source
   <223> /note="Description of Artificial Sequence: Synthetic peptide"
<400> 35
   Arg Ala Ser Gln Asp Ile Ser Asn Tyr Leu Asn
   1               5                   10
<210> 36
   <211> 7
   <212> PRT
   <213> Artificial Sequence
<220>
   <221> source
   <223> /note="Description of Artificial Sequence: Synthetic peptide"
<400> 36
   Tyr Thr Ser Arg Leu His Ser
   1               5
<210> 37
   <211> 9
   <212> PRT
   <213> Artificial Sequence
<220>
   <221> source
   <223> /note="Description of Artificial Sequence: Synthetic peptide"
<400> 37
   Gln Gln Gly Asp Thr Leu Pro Pro Ala
   1               5
<210> 38
   <211> 5
   <212> PRT
   <213> Artificial Sequence
<220>
   <221> source
   <223> /note="Description of Artificial Sequence: Synthetic peptide"
<400> 38
   Ser Tyr Gly Val His
   1 5
<210> 39
   <211> 16
   <212> PRT
   <213> Artificial Sequence
<220>
   <221> source
   <223> /note="Description of Artificial Sequence: Synthetic peptide"
<400> 39
   Val Ile Trp Ser Gly Gly Ser Thr Asp Tyr Asn Ala Ala Phe Ile Ser
   1 5 10 15
<210> 40
   <211> 9
   <212> PRT
   <213> Artificial Sequence
<220>
   <221> source
   <223> /note="Description of Artificial Sequence: Synthetic peptide"
<400> 40
   Asn Asp Gly Tyr Tyr Tyr Phe Asp Tyr
   1 5
<210> 41
   <211> 15
   <212> PRT
   <213> Artificial Sequence
<220>
   <221> source
   <223> /note="Description of Artificial Sequence: Synthetic peptide"
<400> 41
   Arg Ala Ser Glu Ser Val Asp Ser Tyr Gly Asn Thr Phe Met His
   1 5 10 15
<210> 42
   <211> 7
   <212> PRT
   <213> Artificial Sequence
<220>
   <221> source
   <223> /note="Description of Artificial Sequence: Synthetic peptide"
<400> 42
   Leu Ala Ser Asn Leu Glu Ser
   1               5
<210> 43
   <211> 9
   <212> PRT
   <213> Artificial Sequence
<220>
   <221> source
   <223> /note="Description of Artificial Sequence: Synthetic peptide"
<400> 43
   Gln Gln Asn Asn Glu Asp Pro Tyr Thr
   1               5
<210> 44
   <211> 5
   <212> PRT
   <213> Artificial Sequence
<220>
   <221> source
   <223> /note="Description of Artificial Sequence: Synthetic peptide"
<400> 44
   Asp Tyr Val Ile His
   1               5
<210> 45
   <211> 17
   <212> PRT
   <213> Artificial Sequence
<220>
   <221> source
   <223> /note="Description of Artificial Sequence: Synthetic peptide"
<400> 45
   Val Ile Thr Thr Tyr Tyr Gly Asp Ala Ser Tyr Asn Gln Lys Phe Lys
   1               5                   10                  15
   Gly
<210> 46
   <211> 14
   <212> PRT
   <213> Artificial Sequence
<220>
   <221> source
   <223> /note="Description of Artificial Sequence: Synthetic peptide"
<400> 46
   Gly Ala Tyr Gly Asn Ser Pro Ser Tyr Trp Tyr Phe Asp Val
   1               5                   10
<210> 47
   <211> 11
   <212> PRT
   <213> Artificial Sequence
<220>
   <221> source
   <223> /note="Description of Artificial Sequence: Synthetic peptide"
<400> 47
   Lys Ala Ser Gln Asp Ile Asp Ser Tyr Leu Ser
   1               5                   10
<210> 48
   <211> 7
   <212> PRT
   <213> Artificial Sequence
<220>
   <221> source
   <223> /note="Description of Artificial Sequence: Synthetic peptide"
<400> 48
   Leu Thr Asn Arg Leu Ile Asp
   1               5
<210> 49
   <211> 9
   <212> PRT
   <213> Artificial Sequence
<220>
   <221> source
   <223> /note="Description of Artificial Sequence: Synthetic peptide"
<400> 49
   Leu Gln Tyr Asp Glu Phe Pro Val Thr
   1               5
<210> 50
   <211> 5
   <212> PRT
   <213> Artificial Sequence
<220>
   <221> source
   <223> /note="Description of Artificial Sequence: Synthetic peptide"
<400> 50
   Ser Gly Tyr Trp Ser
   1               5
<210> 51
   <211> 16
   <212> PRT
   <213> Artificial Sequence
<220>
   <221> source
   <223> /note="Description of Artificial Sequence: Synthetic peptide"
<400> 51
   Tyr Ile Ser Phe Ser Gly Lys Thr Tyr Tyr Ile Pro Ser Leu Lys Ser
   1               5                   10                  15
<210> 52
   <211> 11
   <212> PRT
   <213> Artificial Sequence
<220>
   <221> source
   <223> /note="Description of Artificial Sequence: Synthetic peptide"
<400> 52
   Tyr His Gly Tyr Gly Gly Pro Trp Phe Ala Tyr
   1               5                   10
<210> 53
   <211> 12
   <212> PRT
   <213> Artificial Sequence
<220>
   <221> source
   <223> /note="Description of Artificial Sequence: Synthetic peptide"
<400> 53
   Thr Leu Ser Ser Gln His Ser Thr Asn Tyr Ile Glu
   1               5                   10
<210> 54
   <211> 10
   <212> PRT
   <213> Artificial Sequence
<220>
   <221> source
   <223> /note="Description of Artificial Sequence: Synthetic peptide"
<400> 54
   Val Arg Asp Gly Ser His Ser Lys Gly Asp
   1 5 10
<210> 55
   <211> 9
   <212> PRT
   <213> Artificial Sequence
<220>
   <221> source
   <223> /note="Description of Artificial Sequence: Synthetic peptide"
<400> 55
   Gly Leu Ser Asp Val Ser Leu Tyr Leu
   1 5
<210> 56
   <211> 5
   <212> PRT
   <213> Artificial Sequence
<220>
   <221> source
   <223> /note="Description of Artificial Sequence: Synthetic peptide"
<400> 56
   Asp Tyr Phe Met Ser
   1 5
<210> 57
   <211> 19
   <212> PRT
   <213> Artificial Sequence
<220>
   <221> source
   <223> /note="Description of Artificial Sequence: Synthetic peptide"
<400> 57
   His Ile Tyr Thr Lys Thr Tyr Asn Tyr Ala Thr Tyr Tyr Ser Gly Ser
   1 5 10 15
   Val Lys Gly
<210> 58
   <211> 8
   <212> PRT
   <213> Artificial Sequence
<220>
   <221> source
   <223> /note="Description of Artificial Sequence: Synthetic peptide"
<400> 58
   Asp Glu Asp Trp Tyr Phe Asp Phe
   1 5
<210> 59
   <211> 12
   <212> PRT
   <213> Artificial Sequence
<220>
   <221> source
   <223> /note="Description of Artificial Sequence: Synthetic peptide"
<400> 59
   Thr Leu Ser Ser Gln His Ser Ser Tyr Gly Ile Thr
   1 5 10
<210> 60
   <211> 11
   <212> PRT
   <213> Artificial Sequence
<220>
   <221> source
   <223> /note="Description of Artificial Sequence: Synthetic peptide"
<400> 60
   Leu Arg Ser Asp Gly Ser His Ser Lys Gly Asp
   1 5 10
<210> 61
   <211> 9
   <212> PRT
   <213> Artificial Sequence
<220>
   <221> source
   <223> /note="Description of Artificial Sequence: Synthetic peptide"
<400> 61
   Val Thr Tyr Asp Ser Thr Val Gly Val
   1 5
<210> 62
   <211> 5
   <212> PRT
   <213> Artificial Sequence
<220>
   <221> source
   <223> /note="Description of Artificial Sequence: Synthetic peptide"
<400> 62
   Glu Tyr Tyr Val Thr
   1 5
<210> 63
   <211> 17
   <212> PRT
   <213> Artificial Sequence
<220>
   <221> source
   <223> /note="Description of Artificial Sequence: Synthetic peptide"
<400> 63
   Asp Ile Asp Pro Glu Asn Gly Asp Thr Asp Tyr Asn Gln Lys Phe Gln
   1 5 10 15
   Gly
<210> 64
   <211> 8
   <212> PRT
   <213> Artificial Sequence
<220>
   <221> source
   <223> /note="Description of Artificial Sequence: Synthetic peptide"
<400> 64
   Gly Tyr Asp Tyr Ala Phe Asp Ser
   1 5
<210> 65
   <211> 13
   <212> PRT
   <213> Artificial Sequence
<220>
   <221> source
   <223> /note="Description of Artificial Sequence: Synthetic peptide"
<400> 65
   Thr Arg Ser Ser Gly Asn Ile Gly Ser Asn Tyr Val Ser
   1 5 10
<210> 66
   <211> 7
   <212> PRT
   <213> Artificial Sequence
<220>
   <221> source
   <223> /note="Description of Artificial Sequence: Synthetic peptide"
<400> 66
   Lys Phe Asp Gln Arg Pro Ser
   1 5
<210> 67
   <211> 8
   <212> PRT
   <213> Artificial Sequence
<220>
   <221> source
   <223> /note="Description of Artificial Sequence: Synthetic peptide"
<400> 67
   Leu Ser Gly Tyr Asp Lys Tyr Val
   1 5
<210> 68
   <211> 5
   <212> PRT
   <213> Artificial Sequence
<220>
   <221> source
   <223> /note="Description of Artificial Sequence: Synthetic peptide"
<400> 68
   Ser Ser Asp Trp Ser
   1 5
<210> 69
   <211> 16
   <212> PRT
   <213> Artificial Sequence
<220>
   <221> source
   <223> /note="Description of Artificial Sequence: Synthetic peptide"
<400> 69
   Tyr Met Asn Tyr Gly Gly Gly Thr Tyr Tyr Asn Pro Ser Leu Glu Asn
   1 5 10 15
<210> 70
   <211> 11
   <212> PRT
   <213> Artificial Sequence
<220>
   <221> source
   <223> /note="Description of Artificial Sequence: Synthetic peptide"
<400> 70
   Glu Arg Pro His Pro Tyr Ala Tyr Phe Asp Val
   1 5 10
<210> 71
   <211> 12
   <212> PRT
   <213> Artificial Sequence
<220>
   <221> source
   <223> /note="Description of Artificial Sequence: Synthetic peptide"
<400> 71
   Thr Leu Ser Ser Gln Tyr Asn Thr Tyr Tyr Ile Glu
   1 5 10
<210> 72
   <211> 10
   <212> PRT
   <213> Artificial Sequence
<220>
   <221> source
   <223> /note="Description of Artificial Sequence: Synthetic peptide"
<400> 72
   Leu Ser Asp Gly Ser His Ser Lys Gly Asp
   1 5 10
<210> 73
   <211> 9
   <212> PRT
   <213> Artificial Sequence
<220>
   <221> source
   <223> /note="Description of Artificial Sequence: Synthetic peptide"
<400> 73
   Gly Val Ser Asp Val Ser Leu Tyr Val
   1 5
<210> 74
   <211> 5
   <212> PRT
   <213> Artificial Sequence
<220>
   <221> source
   <223> /note="Description of Artificial Sequence: Synthetic peptide"
<400> 74
   Ser Tyr Asn Ile His
   1 5
<210> 75
   <211> 16
   <212> PRT
   <213> Artificial Sequence
<220>
   <221> source
   <223> /note="Description of Artificial Sequence: Synthetic peptide"
<400> 75
   Ala Val Trp Arg Gly Gly Gly Thr Tyr Tyr Asn Ser Asn Leu Lys Ser
   1 5 10 15
<210> 76
   <211> 10
   <212> PRT
   <213> Artificial Sequence
<220>
   <221> source
   <223> /note="Description of Artificial Sequence: Synthetic peptide"
<400> 76
   Glu Glu Leu Arg Tyr Val Tyr Phe Asp Val
   1 5 10
<210> 77
   <211> 11
   <212> PRT
   <213> Artificial Sequence
<220>
   <221> source
   <223> /note="Description of Artificial Sequence: Synthetic peptide"
<400> 77
   Lys Ala Ser Gln Asp Ile Asp Ser Tyr Leu Ser
   1 5 10
<210> 78
   <211> 7
   <212> PRT
   <213> Artificial Sequence
<220>
   <221> source
   <223> /note="Description of Artificial Sequence: Synthetic peptide"
<220>
   <221> VARIANT
   <222> (6)..(6)
   <223> /replace="Ile"
<220>
   <221> misc_feature
   <222> (1)..(7)
   <223> /note="Variant residues given in the sequence have no preference with respect to those in the annotations for variant positions"
<400> 78
   Leu Thr Asn Arg Leu Val Asp
   1 5
<210> 79
   <211> 9
   <212> PRT
   <213> Artificial Sequence
<220>
   <221> source
   <223> /note="Description of Artificial Sequence: Synthetic peptide"
<220>
   <221> VARIANT
   <222> (2)..(2)
   <223> /replace="Gln"
<220>
   <221> VARIANT
   <222> (8)..(8)
   <223> /replace="Val"
<220>
   <221> misc_feature
   <222> (1)..(9)
   <223> /note="Variant residues given in the sequence have no preference with respect to those in the annotations for variant positions"
<400> 79
   Leu His Tyr Asp Glu Phe Pro Leu Thr
   1 5
<210> 80
   <211> 5
   <212> PRT
   <213> Artificial Sequence
<220>
   <221> source
   <223> /note="Description of Artificial Sequence: Synthetic peptide"
<220>
   <221> VARIANT
   <222> (5)..(5)
   <223> /replace="Ser"
<220>
   <221> misc_feature
   <222> (1)..(5)
   <223> /note="Variant residues given in the sequence have no preference with respect to those in the annotations for variant positions"
<400> 80
   Ser Gly Tyr Trp Asn
   1 5
<210> 81
   <211> 16
   <212> PRT
   <213> Artificial Sequence
<220>
   <221> source
   <223> /note="Description of Artificial Sequence: Synthetic peptide"
<220>
   <221> VARIANT
   <222> (4)..(4)
   <223> /replace="Phe"
<220>
   <221> VARIANT
   <222> (10)..(10)
   <223> /replace="Tyr"
<220>
   <221> VARIANT
   <222> (11)..(11)
   <223> /replace="Ile"
<220>
   <221> misc_feature
   <222> (1)..(16)
   <223> /note="Variant residues given in the sequence have no preference with respect to those in the annotations for variant positions"
<400> 81
   Tyr Ile Ser Tyr Ser Gly Lys Thr Tyr Gln Asn Pro Ser Leu Lys Ser
   1 5 10 15
<210> 82
   <211> 11
   <212> PRT
   <213> Artificial Sequence
<220>
   <221> source
   <223> /note="Description of Artificial Sequence: Synthetic peptide"
<220>
   <221> VARIANT
   <222> (2)..(2)
   <223> /replace="His"
<220>
   <221> misc_feature
   <222> (1)..(11)
   <223> /note="Variant residues given in the sequence have no preference with respect to those in the annotations for variant positions"
<400> 82
   Tyr Tyr Gly Tyr Gly Gly Pro Trp Phe Ala Tyr
   1 5 10
<210> 83
   <211> 11
   <212> PRT
   <213> Artificial Sequence
<220>
   <221> source
   <223> /note="Description of Artificial Sequence: Synthetic peptide"
<400> 83
   Arg Ala Ser Gln Asp Ile Ser Asn Tyr Leu Asn
   1 5 10
<210> 84
   <211> 7
   <212> PRT
   <213> Artificial Sequence
<220>
   <221> source
   <223> /note="Description of Artificial Sequence: Synthetic peptide"
<400> 84
   Tyr Thr Ser Arg Leu His Ser
   1 5
<210> 85
   <211> 9
   <212> PRT
   <213> Artificial Sequence
<220>
   <221> source
   <223> /note="Description of Artificial Sequence: Synthetic peptide"
<220>
   <221> VARIANT
   <222> (2)..(2)
   <223> /replace="His"
<220>
   <221> VARIANT
   <222> (9)..(9)
   <223> /replace="Ala"
<220>
   <221> misc_feature
   <222> (1)..(9)
   <223> /note="Variant residues given in the sequence have no preference with respect to those in the annotations for variant positions"
<400> 85
   Gln Gln Gly Asp Thr Leu Pro Pro Thr
   1 5
<210> 86
   <211> 5
   <212> PRT
   <213> Artificial Sequence
<220>
   <221> source
   <223> /note="Description of Artificial Sequence: Synthetic peptide"
<220>
   <221> VARIANT
   <222> (3)..(3)
   <223> /replace="Gly"
<220>
   <221> misc_feature
   <222> (1)..(5)
   <223> /note="Variant residues given in the sequence have no preference with respect to those in the annotations for variant positions"
<400> 86
   Ser Tyr Ala Val His
   1 5
<210> 87
   <211> 16
   <212> PRT
   <213> Artificial Sequence
<220>
   <221> source
   <223> /note="Description of Artificial Sequence: Synthetic peptide"
<220>
   <221> VARIANT
   <222> (4)..(4)
   <223> /replace="Thr"
<220>
   <221> VARIANT
   <222> (9)..(9)
   <223> /replace="Asn"
<220>
   <221> VARIANT
   <222> (12)..(12)
   <223> /replace="Ser"
<220>
   <221> VARIANT
   <222> (14)..(14)
   <223> /replace="Phe"
<220>
   <221> VARIANT
   <222> (15)..(15)
   <223> /replace="Ile"
<220>
   <221> misc_feature
   <222> (1)..(16)
   <223> /note="Variant residues given in the sequence have no preference with respect to those in the annotations for variant positions"
<400> 87
   Val Ile Trp Ser Gly Gly Ser Thr Asp Tyr Asn Ala Ala Leu Met Ser
   1 5 10 15
<210> 88
   <211> 9
   <212> PRT
   <213> Artificial Sequence
<220>
   <221> source
   <223> /note="Description of Artificial Sequence: Synthetic peptide"
<220>
   <221> VARIANT
   <222> (1)..(1)
   <223> /replace="Val"
<220>
   <221> misc_feature
   <222> (1)..(9)
   <223> /note="Variant residues given in the sequence have no preference with respect to those in the annotations for variant positions"
<400> 88
   Asn Asp Gly Tyr Tyr Tyr Phe Asp Tyr
   1 5
<210> 89
   <211> 112
   <212> PRT
   <213> Artificial Sequence
<220>
   <221> source
   <223> /note="Description of Artificial Sequence: Synthetic polypeptide"
<400> 89
   Ser Ile Val Met Thr Gln Thr Pro Leu Ser Leu Pro Val Ser Leu Gly
   1 5 10 15
   Asp Gln Ala Ser Ile Ser Cys Arg Ser Ser Gln Ser Ile Val His Ser
   20 25 30
   Asn Gly Asn Thr Tyr Leu Glu Trp Tyr Leu Gln Lys Pro Gly Gln Ser
   35 40 45
   Pro Lys Leu Leu Ile Tyr Arg Ile Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Leu Gly Val Tyr Tyr Cys Phe Gln Gly
   85 90 95
   Ser His Val Pro Tyr Thr Phe Gly Gly Gly Thr Lys Leu Glu Ile Lys
   100 105 110
<210> 90
   <211> 121
   <212> PRT
   <213> Artificial Sequence
<220>
   <221> source
   <223> /note="Description of Artificial Sequence: Synthetic polypeptide"
<400> 90
   Glu Val Lys Leu Val Glu Ser Gly Gly Gly Phe Val Lys Pro Gly Gly
   1 5 10 15
   Ser Leu Lys Leu Ser Cys Ala Ala Ser Gly Phe Thr Phe Ser Asn Phe
   20 25 30
   Ala Met Ser Trp Val Arg Gln Ser Pro Glu Lys Arg Leu Glu Trp Val
   35 40 45
   Ala Glu Ile Asn Asn Gly Gly Asn Tyr Ala Tyr Tyr Gln Asp Thr Val
   50 55 60
   Thr Gly Arg Phe Thr Ile Ser Arg Asp Asn Ala Lys Asn Thr Leu Tyr
   65 70 75 80
   Leu Glu Met Ser Ser Leu Arg Ser Glu Asp Thr Ala Met Tyr Phe Cys
   85 90 95
   Ala Arg Glu Asp Tyr Val Asn Tyr Glu Ala Tyr Phe Ala Tyr Trp Gly
   100 105 110
   Gln Gly Thr Thr Leu Thr Val Ser Ser
   115 120
<210> 91
   <211> 106
   <212> PRT
   <213> Artificial Sequence
<220>
   <221> source
   <223> /note="Description of Artificial Sequence: Synthetic polypeptide"
<400> 91
   Asp Ile Gln Met Asn Gln Ser His Lys Phe Met Ser Thr Ser Val Gly
   1               5                   10                  15
   Asp Arg Val Ser Ile Thr Trp Lys Ala Ser Gln Asp Val Gly Thr Ala
               20                  25                  30
   Val Ala Trp Tyr Gln Gln Lys Pro Gly Gln Ser Pro Lys Leu Leu Ile
           35                  40                  45
   Tyr Trp Ala Ser Thr Arg His Thr Gly Val Pro Asp Arg Phe Thr Gly
       50                  55                  60
   Ser Gly Ser Gly Thr Asp Phe Thr Leu Thr Ile Ser Asn Val Gln Ser
   65                  70                  75                  80
   Glu Asp Leu Ala Asp Tyr Phe Cys Gln Gln Tyr Ser Ser Ser Ile Thr
                   85                  90                  95
   Phe Gly Ala Gly Thr Lys Leu Glu Leu Lys
               100                 105
<210> 92
   <211> 120
   <212> PRT
   <213> Artificial Sequence
<220>
   <221> source
   <223> /note="Description of Artificial Sequence: Synthetic polypeptide"
<400> 92
   Gln Val Gln Leu Gln Gln Ser Gly Pro Glu Leu Val Arg Pro Gly Glu
   1               5                   10                  15
   Ser Val Lys Ile Ser Cys Lys Gly Ser Gly Tyr Ser Phe Thr Asp Tyr
               20                  25                  30
   Ala Met His Trp Val Lys Gln Ser His Ala Lys Ser Leu Glu Trp Ile
           35                  40                  45
   Gly Ile Ile Ser Ile Tyr Tyr Asp Asn Thr Asn Tyr Asn Gln Lys Phe
       50                  55                  60
   Lys Gly Arg Ala Thr Met Thr Val Asp Lys Ser Ser Ser Thr Ala Tyr
   65                  70                  75                  80
   Met Glu Leu Ala Arg Leu Thr Ser Glu Asp Ser Ala Ile Tyr Tyr Cys
                   85                  90                  95
   Ala Arg Gly Gly Asn Gly Tyr Tyr Tyr Val Met Asp Tyr Trp Gly Gln
               100                 105                 110
   Gly Thr Ser Val Thr Val Ser Ser
           115                 120
<210> 93
   <211> 107
   <212> PRT
   <213> Artificial Sequence
<220>
   <221> source
   <223> /note="Description of Artificial Sequence: Synthetic polypeptide"
<400> 93
   Asp Ile Val Met Thr Gln Ser Thr Ser Ser Leu Ser Ala Ser Leu Gly
   1               5                   10                  15
   Asp Arg Val Thr Ile Ser Cys Arg Ala Ser Gln Asp Ile Ser Asn Tyr
               20                  25                  30
   Leu Asn Trp Tyr Gln Gln Lys Pro Asp Gly Thr Val Lys Leu Leu Ile
           35                  40                  45
   Tyr Tyr Thr Ser Arg Leu His Ser Gly Val Pro Ser Arg Phe Ser Gly
       50                  55                  60
   Ser Gly Ser Gly Thr Asp Tyr Ser Leu Thr Ile Ser Asn Leu Glu Pro
   65                  70                  75                  80
   Glu Asp Ile Ala Thr Tyr Phe Cys Gln Gln Gly Asp Thr Leu Pro Pro
                   85                  90                  95
   Thr Phe Gly Gly Gly Thr Lys Leu Glu Ile Lys
               100                 105
<210> 94
   <211> 117
   <212> PRT
   <213> Artificial Sequence
<220>
   <221> source
   <223> /note="Description of Artificial Sequence: Synthetic polypeptide"
<400> 94
   Glu Val Gln Leu Gln Glu Ser Gly Pro Gly Leu Val Ala Pro Ser Gln
   1               5                   10                  15
   Ser Leu Ser Ile Thr Cys Thr Val Ser Gly Phe Ser Leu Thr Ser Tyr
               20                  25                  30
   Gly Val His Trp Val Arg Gln Pro Pro Gly Lys Gly Leu Glu Trp Leu
           35                  40                  45
   Gly Val Ile Trp Thr Gly Gly Ser Thr Asn Tyr Asn Ser Ala Leu Met
       50                  55                  60
   Ser Arg Leu Ser Ile Ser Lys Asp Asn Ser Lys Ser Gln Val Phe Leu
   65                  70                  75                  80
   Lys Met Asn Ser Leu Gln Thr Asp Asp Thr Ala Met Tyr Tyr Cys Ala
                   85                  90                  95
   Arg Val Asp Gly Tyr Tyr Tyr Phe Asp Tyr Trp Gly Gln Gly Thr Thr
               100                 105                 110
   Leu Thr Val Ser Ser
           115
<210> 95
   <211> 107
   <212> PRT
   <213> Artificial Sequence
<220>
   <221> source
   <223> /note="Description of Artificial Sequence: Synthetic polypeptide"
<400> 95
   Asp Ile Val Leu Thr Gln Ser Pro Ser Ser Met Tyr Ala Ser Leu Gly
   1               5                   10                  15
   Glu Arg Val Thr Ile Thr Cys Lys Ala Ser Gln Asp Ile Asp Ser Tyr
               20                  25                  30
   Leu Ser Trp Phe Gln Gln Lys Pro Gly Lys Ser Pro Lys Thr Leu Ile
           35                  40                  45
   Tyr Leu Thr Asn Arg Leu Val Asp Gly Val Pro Ser Arg Phe Ser Gly
       50                  55                  60
   Ser Gly Ser Gly Gln Asp Tyr Ser Leu Thr Ile Ser Ser Leu Glu Tyr
   65                  70                  75                  80
   Glu Asp Met Gly Ile Tyr Tyr Cys Leu His Tyr Asp Glu Phe Pro Leu
   85 90 95
   Thr Phe Gly Ala Gly Thr Lys Leu Glu Ile Lys
   100 105
<210> 96
   <211> 119
   <212> PRT
   <213> Artificial Sequence
<220>
   <221> source
   <223> /note="Description of Artificial Sequence: Synthetic polypeptide"
<400> 96
   Glu Val Gln Leu Gln Glu Ser Gly Pro Ser Leu Val Lys Pro Ser Gln
   1 5 10 15
   Thr Leu Ser Leu Thr Cys Ser Val Thr Gly Asp Ser Ile Thr Ser Gly
   20 25 30
   Tyr Trp Asn Trp Ile Arg Lys Phe Pro Gly Asn Lys Phe Glu Tyr Met
   35 40 45
   Gly Tyr Ile Ser Tyr Ser Gly Lys Thr Tyr Gln Asn Pro Ser Leu Lys
   50 55 60
   Ser Arg Ile Ser Ile Thr Arg Asp Thr Ser Lys Asn Gln Tyr His Leu
   65 70 75 80
   Gln Leu Asn Ser Val Thr Thr Glu Asp Thr Ala Thr Tyr Tyr Cys Ala
   85 90 95
   Thr Tyr Tyr Gly Tyr Gly Gly Pro Trp Phe Ala Tyr Trp Gly Gln Gly
   100 105 110
   Thr Leu Val Thr Val Ser Ala
   115
<210> 97
   <211> 107
   <212> PRT
   <213> Artificial Sequence
<220>
   <221> source
   <223> /note="Description of Artificial Sequence: Synthetic polypeptide"
<400> 97
   Asp Ile Val Met Thr Gln Ser Thr Ser Ser Leu Ser Ala Ser Leu Gly
   1               5                   10                  15
   Asp Arg Val Thr Ile Ser Cys Arg Ala Ser Gln Asp Ile Ser Asn Tyr
               20                  25                  30
   Leu Asn Trp Tyr Gln Gln Lys Pro Asp Gly Thr Val Lys Leu Leu Ile
           35                  40                  45
   Tyr Tyr Thr Ser Arg Leu His Ser Gly Val Pro Ser Arg Phe Ser Gly
       50                  55                  60
   Ser Gly Ser Gly Thr Asp Tyr Ser Leu Thr Ile Ser Asn Leu Glu Lys
   65                  70                  75                  80
   Glu Asp Val Ala Thr Tyr Phe Cys Gln His Gly Asp Thr Leu Pro Pro
                   85                  90                  95
   Thr Phe Gly Gly Gly Thr Lys Leu Glu Ile Lys
               100                 105
<210> 98
   <211> 117
   <212> PRT
   <213> Artificial Sequence
<220>
   <221> source
   <223> /note="Description of Artificial Sequence: Synthetic polypeptide"
<400> 98
   Gln Val Gln Leu Lys Gln Ser Gly Pro Gly Leu Val Gln Pro Ser Gln
   1               5                   10                  15
   Ser Leu Ser Ile Thr Cys Thr Val Ser Gly Phe Ser Leu Ser Ser Tyr
               20                  25                  30
   Ala Val His Trp Val Arg Gln Ser Pro Gly Glu Gly Leu Glu Trp Leu
           35                  40                  45
   Gly Val Ile Trp Ser Gly Gly Ser Thr Asp Tyr Asn Ala Ala Phe Ile
       50                  55                  60
   Ser Arg Met Ser Ile Thr Lys Asp Asn Ser Lys Ser Gln Val Phe Phe
   65                  70                  75                  80
   Lys Met Asn Ser Leu Gln Ala Asp Asp Thr Ala Ile Tyr Phe Cys Ala
                   85                  90                  95
   Arg Asn Asp Gly Tyr Tyr Tyr Phe Asp Tyr Trp Gly Gln Gly Thr Thr
                   100                 105                 110
   Leu Thr Val Ser Ser
           115
<210> 99
   <211> 107
   <212> PRT
   <213> Artificial Sequence
<220>
   <221> source
   <223> /note="Description of Artificial Sequence: Synthetic polypeptide"
<400> 99
   Asp Ile Lys Met Thr Gln Ser Thr Ser Ser Leu Ser Ala Ser Leu Gly
   1               5                   10                  15
   Asp Arg Val Thr Ile Ser Cys Arg Ala Ser Gln Asp Ile Ser Asn Tyr
               20                  25                  30
   Leu Asn Trp Tyr Gln Gln Lys Pro Asp Gly Thr Val Lys Leu Leu Ile
           35                  40                  45
   Tyr Tyr Thr Ser Arg Leu His Ser Gly Val Pro Ser Arg Phe Ser Gly
       50                  55                  60
   Ser Gly Ser Gly Thr Asp Tyr Ser Leu Thr Ile Ser Asn Leu Glu Gln
   65                  70                  75                  80
   Glu Asp Ile Ala Thr Tyr Phe Cys Gln Gln Gly Asp Thr Leu Pro Pro
                   85                  90                  95
   Ala Phe Gly Gly Gly Thr Lys Leu Glu Ile Lys
               100                 105
<210> 100
   <211> 117
   <212> PRT
   <213> Artificial Sequence
<220>
   <221> source
   <223> /note="Description of Artificial Sequence: Synthetic polypeptide"
<400> 100
   Gln Val Gln Leu Lys Glu Ser Gly Pro Gly Leu Val Gln Pro Ser Gln
   1               5                   10                  15
   Ser Leu Ser Ile Thr Cys Thr Val Ser Gly Phe Ser Leu Thr Ser Tyr
               20                  25                  30
   Gly Val His Trp Val Arg Gln Ser Pro Gly Lys Gly Leu Glu Trp Leu
   35 40 45
   Gly Val Ile Trp Ser Gly Gly Ser Thr Asp Tyr Asn Ala Ala Phe Ile
   50 55 60
   Ser Arg Leu Ser Ile Ser Lys Asp Asn Ser Lys Ser Gln Val Phe Phe
   65 70 75 80
   Lys Met Asn Ser Leu Gln Ala Asn Asp Thr Ala Ile Tyr Tyr Cys Ala
   85 90 95
   Arg Asn Asp Gly Tyr Tyr Tyr Phe Asp Tyr Trp Gly Gln Gly Thr Thr
   100 105 110
   Leu Thr Val Ser Ser
   115
<210> 101
   <211> 111
   <212> PRT
   <213> Artificial Sequence
<220>
   <221> source
   <223> /note="Description of Artificial Sequence: Synthetic polypeptide"
<400> 101
   Asp Ile Val Leu Thr Gln Ser Pro Ala Ser Leu Thr Val Ser Leu Gly
   1 5 10 15
   Gln Arg Ala Thr Ile Ser Cys Arg Ala Ser Glu Ser Val Asp Ser Tyr
   20 25 30
   Gly Asn Thr Phe Met His Trp Tyr Gln Gln Lys Pro Gly Gln Pro Pro
   35 40 45
   Lys Leu Leu Ile Tyr Leu Ala Ser Asn Leu Glu Ser Gly Val Pro Ala
   50 55 60
   Arg Phe Ser Gly Ser Gly Ser Arg Thr Asp Phe Thr Leu Thr Ile Asp
   65 70 75 80
   Pro Val Glu Ala Asp Asp Ala Ala Thr Tyr Tyr Cys Gln Gln Asn Asn
   85 90 95
   Glu Asp Pro Tyr Thr Phe Gly Gly Gly Thr Lys Leu Glu Ile Lys
   100 105 110
<210> 102
   <211> 123
   <212> PRT
   <213> Artificial Sequence
<220>
   <221> source
   <223> /note="Description of Artificial Sequence: Synthetic polypeptide"
<400> 102
   Gln Val Gln Leu Gln Gln Ser Gly Ala Glu Leu Val Arg Pro Gly Val
   1               5                   10                  15
   Ser Val Lys Ile Ser Cys Lys Gly Ser Gly Tyr Thr Phe Thr Asp Tyr
               20                  25                  30
   Val Ile His Trp Val Lys Gln Ser His Ala Lys Ser Leu Glu Trp Ile
           35                  40                  45
   Gly Val Ile Thr Thr Tyr Tyr Gly Asp Ala Ser Tyr Asn Gln Lys Phe
       50                  55                  60
   Lys Gly Lys Ala Thr Met Thr Val Asp Lys Ser Ser Ser Thr Ala Tyr
   65                  70                  75                  80
   Met Glu Leu Ala Arg Leu Thr Ser Glu Asp Ser Ala Ile Tyr Tyr Cys
                   85                  90                  95
   Ala Arg Gly Ala Tyr Gly Asn Ser Pro Ser Tyr Trp Tyr Phe Asp Val
               100                 105                 110
   Trp Gly Ala Gly Thr Ser Val Thr Val Ser Ser
           115                 120
<210> 103
   <211> 107
   <212> PRT
   <213> Artificial Sequence
<220>
   <221> source
   <223> /note="Description of Artificial Sequence: Synthetic polypeptide"
<400> 103
   Asp Ile Lys Met Thr Gln Ser Pro Ser Ser Met Tyr Ala Ser Leu Gly
   1               5                   10                  15
   Glu Arg Val Thr Ile Thr Cys Lys Ala Ser Gln Asp Ile Asp Ser Tyr
               20                  25                  30
   Leu Ser Trp Phe Gln Gln Lys Pro Gly Lys Ser Pro Lys Thr Leu Ile
   35 40 45
   Tyr Leu Thr Asn Arg Leu Ile Asp Gly Val Pro Ser Arg Phe Ser Gly
   50 55 60
   Ser Gly Ser Gly Gln Asp Tyr Ser Leu Thr Ile Asn Ser Leu Glu Tyr
   65 70 75 80
   Glu Asp Met Gly Ile Tyr Tyr Cys Leu Gln Tyr Asp Glu Phe Pro Val
   85 90 95
   Thr Phe Gly Ala Gly Thr Arg Leu Glu Ile Lys
   100 105
<210> 104
   <211> 119
   <212> PRT
   <213> Artificial Sequence
<220>
   <221> source
   <223> /note="Description of Artificial Sequence: Synthetic polypeptide"
<400> 104
   Glu Val Gln Leu Gln Glu Ser Gly Pro Ser Leu Val Lys Pro Ser Gln
   1 5 10 15
   Thr Leu Ser Leu Thr Cys Ser Val Thr Gly Asp Ser Ile Thr Ser Gly
   20 25 30
   Tyr Trp Ser Trp Ile Arg Lys Phe Pro Gly Asn Lys Leu Glu Phe Met
   35 40 45
   Gly Tyr Ile Ser Phe Ser Gly Lys Thr Tyr Tyr Ile Pro Ser Leu Lys
   50 55 60
   Ser Arg Val Ser Ile Thr Arg Asp Thr Ser Lys Asn Gln Tyr Tyr Leu
   65 70 75 80
   Gln Leu Asn Ser Val Thr Thr Glu Asp Thr Ala Thr Tyr Tyr Cys Ala
   85 90 95
   Thr Tyr His Gly Tyr Gly Gly Pro Trp Phe Ala Tyr Trp Gly Gln Gly
   100 105 110
   Thr Leu Val Thr Val Ser Ser
   115
<210> 105
   <211> 110
   <212> PRT
   <213> Artificial Sequence
<220>
   <221> source
   <223> /note="Description of Artificial Sequence: Synthetic polypeptide"
<400> 105
   Gln Pro Val Leu Thr Gln Ser Pro Ser Val Ser Ala Ser Leu Gly Ala
   1               5                   10                  15
   Ser Val Lys Leu Thr Cys Thr Leu Ser Ser Gln His Ser Thr Asn Tyr
               20                  25                  30
   Ile Glu Trp Tyr Gln Gln His Pro Asp Lys Ser Pro Lys Phe Leu Met
           35                  40                  45
   Gln Val Arg Asp Gly Ser His Ser Lys Gly Asp Gly Thr Pro Asp Arg
       50                  55                  60
   Phe Ser Gly Ser Ser Ser Gly Ala His Arg Tyr Leu Ser Ile Ser Asn
   65                  70                  75                  80
   Leu Gln Leu Glu Asp Glu Ala Ile Tyr Tyr Cys Gly Leu Ser Asp Val
                   85                  90                  95
   Ser Leu Tyr Leu Phe Gly Ser Gly Thr Gln Leu Thr Leu Leu
               100                 105                 110
<210> 106
   <211> 119
   <212> PRT
   <213> Artificial Sequence
<220>
   <221> source
   <223> /note="Description of Artificial Sequence: Synthetic polypeptide"
<400> 106
   Glu Val Gln Leu Val Glu Ser Gly Gly Gly Leu Val Lys Pro Glu Gly
   1               5                   10                  15
   Ser Leu Lys Leu Ser Cys Val Ala Ser Gly Phe Thr Phe Ser Asp Tyr
               20                  25                  30
   Phe Met Ser Trp Val Arg Gln Ala Pro Gly Gln Gly Leu Glu Trp Val
           35                  40                  45
   Ala His Ile Tyr Thr Lys Thr Tyr Asn Tyr Ala Thr Tyr Tyr Ser Gly
       50                  55                  60
   Ser Val Lys Gly Arg Phe Ser Ile Ser Arg Asp Asp Ser Arg Asn Met
   65                  70                  75                  80
   Val Tyr Leu Gln Met Asn Asn Leu Arg Thr Glu Asp Thr Ala Thr Tyr
                   85                  90                  95
   Tyr Cys Thr Thr Asp Glu Asp Trp Tyr Phe Asp Phe Trp Gly Gln Gly
               100                 105                 110
   Thr Gln Val Thr Val Ser Ser
           115
<210> 107
   <211> 111
   <212> PRT
   <213> Artificial Sequence
<220>
   <221> source
   <223> /note="Description of Artificial Sequence: Synthetic polypeptide"
<400> 107
   Gln Pro Val Leu Thr Gln Ser Pro Ser Ala Ser Ala Ser Leu Gly Ala
   1               5                   10                  15
   Ser Val Lys Leu Thr Cys Thr Leu Ser Ser Gln His Ser Ser Tyr Gly
               20                  25                  30
   Ile Thr Trp Leu Gln Gln His Pro Asp Lys Ala Pro Lys Cys Val Met
           35                  40                  45
   Tyr Leu Arg Ser Asp Gly Ser His Ser Lys Gly Asp Gly Ile Pro Asp
       50                  55                  60
   Arg Phe Ser Gly Ser Ser Ser Gly Ala His Arg Tyr Leu Ser Ile Ser
   65                  70                  75                  80
   Asn Val Gln Pro Glu Asp Glu Ala Ile Tyr Phe Cys Val Thr Tyr Asp
                   85                  90                  95
   Ser Thr Val Gly Val Phe Gly Ser Gly Thr Gln Leu Thr Val Pro
               100                 105                 110
<210> 108
   <211> 117
   <212> PRT
   <213> Artificial Sequence
<220>
   <221> source
   <223> /note="Description of Artificial Sequence: Synthetic polypeptide"
<400> 108
   Gln Val Gln Leu Gln Gln Ser Gly Pro Gln Leu Val Lys Pro Gly Phe
   1               5                   10                  15
   Ser Val Lys Phe Ser Cys Lys Ala Ser Gly Ile Thr Phe Thr Glu Tyr
               20                  25                  30
   Tyr Val Thr Trp Val Lys Gln Arg Ala Gly Gln Gly Leu Glu Trp Val
           35                  40                  45
   Gly Asp Ile Asp Pro Glu Asn Gly Asp Thr Asp Tyr Asn Gln Lys Phe
       50                  55                  60
   Gln Gly Lys Ala Thr Ile Thr Ala Asp Lys Ser Ser Ser Thr Ala Tyr
   65                  70                  75                  80
   Met Glu Leu Ser Ser Leu Thr Ser Glu Asp Ser Ala Val Tyr Tyr Cys
                   85                  90                  95
   Ala Thr Gly Tyr Asp Tyr Ala Phe Asp Ser Trp Gly Gln Gly Thr Leu
               100                 105                 110
   Val Thr Val Ser Ser
           115
<210> 109
   <211> 109
   <212> PRT
   <213> Artificial Sequence
<220>
   <221> source
   <223> /note="Description of Artificial Sequence: Synthetic polypeptide"
<400> 109
   Glu Leu Val Phe Thr Gln Pro Gln Ser Val Ser Gly Ser Leu Gly Gln
   1               5                   10                  15
   Glu Ile Ser Ile Ser Cys Thr Arg Ser Ser Gly Asn Ile Gly Ser Asn
               20                  25                  30
   Tyr Val Ser Trp Tyr Gln Gln Gln Ser Ser Asn Lys Pro Arg Leu Leu
           35                  40                  45
   Ile Tyr Lys Phe Asp Gln Arg Pro Ser Gly Val Pro Asp Arg Phe Ser
       50                  55                  60
   Gly Ser Thr Asp Ser Ser Ser Asn Ser Gly Ile Leu Thr Ile Ser Arg
   65                  70                  75                  80
   Leu Gln Pro Glu Asp Glu Gly Asp Tyr Tyr Cys Leu Ser Gly Tyr Asp
                   85                  90                  95
   Lys Tyr Val Phe Gly Ser Gly Thr Gln Leu Thr Leu Leu
               100                 105
<210> 110
   <211> 119
   <212> PRT
   <213> Artificial Sequence
<220>
   <221> source
   <223> /note="Description of Artificial Sequence: Synthetic polypeptide"
<400> 110
   Gln Ile Gln Leu Gln Glu Ser Gly Pro Gly Leu Val Lys Pro Ser Gln
   1               5                   10                  15
   Ser Leu Ser Leu Thr Cys Ser Val Thr Gly Asn Ser Ile Thr Ser Ser
               20                  25                  30
   Asp Trp Ser Trp Ile Arg Gln Phe Pro Gly Lys Lys Leu Glu Trp Met
           35                  40                  45
   Gly Tyr Met Asn Tyr Gly Gly Gly Thr Tyr Tyr Asn Pro Ser Leu Glu
       50                  55                  60
   Asn Arg Ile Ser Ile Thr Arg Asp Thr Ser Lys Asn Gln Phe Phe Leu
   65                  70                  75                  80
   His Leu Lys Ser Val Thr Thr Glu Asp Thr Ala Thr Tyr Tyr Cys Ala
                   85                  90                  95
   Arg Glu Arg Pro His Pro Tyr Ala Tyr Phe Asp Val Trp Gly Gln Gly
               100                 105                 110
   Ile Gln Val Thr Val Ser Ser
           115
<210> 111
   <211> 110
   <212> PRT
   <213> Artificial Sequence
<220>
   <221> source
   <223> /note="Description of Artificial Sequence: Synthetic polypeptide"
<400> 111
   Gln Pro Leu Leu Thr Gln Ser Pro Ser Val Ser Ala Ser Leu Gly Ala
   1               5                   10                  15
   Ser Val Lys Leu Thr Cys Thr Leu Ser Ser Gln Tyr Asn Thr Tyr Tyr
               20                  25                  30
   Ile Glu Trp Tyr Gln Gln His Pro Asp Lys Ser Pro Lys Phe Leu Met
           35                  40                  45
   Gln Leu Ser Asp Gly Ser His Ser Lys Gly Asp Gly Ile Pro Asp Arg
       50                  55                  60
   Phe Ser Gly Ser Ser Ser Gly Ala His Arg Tyr Leu Ser Ile Ser Asn
   65                  70                  75                  80
   Leu Gln Leu Glu Asp Glu Ala Ile Tyr Tyr Cys Gly Val Ser Asp Val
                   85                  90                  95
   Ser Leu Tyr Val Phe Gly Ser Gly Thr Gln Leu Thr Val Leu
               100                 105                 110
<210> 112
   <211> 118
   <212> PRT
   <213> Artificial Sequence
<220>
   <221> source
   <223> /note="Description of Artificial Sequence: Synthetic polypeptide"
<400> 112
   Gln Val Gln Leu Lys Glu Ser Gly Pro Gly Leu Val Gln Pro Ser Gln
   1               5                   10                  15
   Thr Leu Ser Leu Thr Cys Thr Val Ser Gly Phe Ser Leu Thr Ser Tyr
               20                  25                  30
   Asn Ile His Trp Val Arg Gln Pro Pro Gly Lys Gly Leu Glu Trp Met
           35                  40                  45
   Gly Ala Val Trp Arg Gly Gly Gly Thr Tyr Tyr Asn Ser Asn Leu Lys
       50                  55                  60
   Ser Arg Val Ile Ile Thr Arg Asp Thr Ser Lys Ser Gln Val Leu Leu
   65                  70                  75                  80
   Lys Leu Asn Asn Leu Gln His Glu Asp Thr Ala Met Tyr Tyr Cys Ala
   85 90 95
   Arg Glu Glu Leu Arg Tyr Val Tyr Phe Asp Val Trp Gly Gln Gly Ile
   100 105 110
   Gln Val Thr Val Ser Ser
   115
<210> 113
   <211> 19
   <212> DNA
   <213> Artificial Sequence
<220>
   <221> source
   <223> /note="Description of Artificial Sequence: Synthetic primer"
<400> 113
   cttgcggaaa ggatgttgg 19
<210> 114
   <211> 19
   <212> DNA
   <213> Artificial Sequence
<220>
   <221> source
   <223> /note="Description of Artificial Sequence: Synthetic primer"
<400> 114
   actactcgca tcgcgcact 19
<210> 115
   <211> 20
   <212> DNA
   <213> Artificial Sequence
<220>
   <221> source
   <223> /note="Description of Artificial Sequence: Synthetic primer"
<400> 115
   aaactcggca tggatacgac 20
<210> 116
   <211> 20
   <212> DNA
   <213> Artificial Sequence
<220>
   <221> source
   <223> /note="Description of Artificial Sequence: Synthetic primer"
<400> 116
   tgcagtcaat gctccaactt 20
<210> 117
   <211> 20
   <212> DNA
   <213> Artificial Sequence
<220>
   <221> source
   <223> /note="Description of Artificial Sequence: Synthetic primer"
<400> 117
   aagcccatca acctctctca 20
<210> 118
   <211> 20
   <212> DNA
   <213> Artificial Sequence
<220>
   <221> source
   <223> /note="Description of Artificial Sequence: Synthetic primer"
<400> 118
   ctctacaccc ccaagtgcat 20
<210> 119
   <211> 20
   <212> DNA
   <213> Artificial Sequence
<220>
   <221> source
   <223> /note="Description of Artificial Sequence: Synthetic primer"
<400> 119
   aacaggagac ccgtacatgc 20
<210> 120
   <211> 21
   <212> DNA
   <213> Artificial Sequence
<220>
   <221> source
   <223> /note="Description of Artificial Sequence: Synthetic primer"
<400> 120
   ccagctgcta gctactgtgg a 21
<210> 121
   <211> 21
   <212> DNA
   <213> Artificial Sequence
<220>
   <221> source
   <223> /note="Description of Artificial Sequence: Synthetic primer"
<400> 121
   tgaaccgaag tttagcaatg g 21
<210> 122
   <211> 20
   <212> DNA
   <213> Artificial Sequence
<220>
   <221> source
   <223> /note="Description of Artificial Sequence: Synthetic primer"
<400> 122
   tgatgaactt tgcagccact 20
<210> 123
   <211> 20
   <212> DNA
   <213> Artificial Sequence
<220>
   <221> source
   <223> /note="Description of Artificial Sequence: Synthetic primer"
<400> 123
   agggccagat ttgagtgtgt 20
<210> 124
   <211> 20
   <212> DNA
   <213> Artificial Sequence
<220>
   <221> source
   <223> /note="Description of Artificial Sequence: Synthetic primer"
<400> 124
   gtgtatggcg tcgtgatgtc 20
<210> 125
   <211> 20
   <212> DNA
   <213> Artificial Sequence
<220>
   <221> source
   <223> /note="Description of Artificial Sequence: Synthetic primer"
<400> 125
   catgtcggag aacatctgga 20
<210> 126
   <211> 20
   <212> DNA
   <213> Artificial Sequence
<220>
   <221> source
   <223> /note="Description of Artificial Sequence: Synthetic primer"
<400> 126
   ccttactgcc ttgtgggaga 20
<210> 127
   <211> 20
   <212> DNA
   <213> Artificial Sequence
<220>
   <221> source
   <223> /note="Description of Artificial Sequence: Synthetic primer"
<400> 127
   cagagacccg tgctgagttt 20
<210> 128
   <211> 20
   <212> DNA
   <213> Artificial Sequence
<220>
   <221> source
   <223> /note="Description of Artificial Sequence: Synthetic primer"
<400> 128
   gactttggtg ccctcaacat 20
<210> 129
   <211> 20
   <212> DNA
   <213> Artificial Sequence
<220>
   <221> source
   <223> /note="Description of Artificial Sequence: Synthetic primer"
<400> 129
   aacgggaact cttagcagca 20
<210> 130
   <211> 20
   <212> DNA
   <213> Artificial Sequence
<220>
   <221> source
   <223> /note="Description of Artificial Sequence: Synthetic primer"
<400> 130
   gagacttcat gcgggagttc 20
<210> 131
   <211> 21
   <212> DNA
   <213> Artificial Sequence
<220>
   <221> source
   <223> /note="Description of Artificial Sequence: Synthetic primer"
<400> 131
   tggccttcgc taactacaag a 21
<210> 132
   <211> 18
   <212> DNA
   <213> Artificial Sequence
<220>
   <221> source
   <223> /note="Description of Artificial Sequence: Synthetic primer"
<400> 132
   gctctttggc gcggatta 18
<210> 133
   <211> 20
   <212> DNA
   <213> Artificial Sequence
<220>
   <221> source
   <223> /note="Description of Artificial Sequence: Synthetic primer"
<400> 133
   gttgcaaaag gcttgctgat 20
<210> 134
   <211> 20
   <212> DNA
   <213> Artificial Sequence
<220>
   <221> source
   <223> /note="Description of Artificial Sequence: Synthetic primer"
<400> 134
   tgattgatgc tgccaaacat 20
<210> 135
   <211> 20
   <212> DNA
   <213> Artificial Sequence
<220>
   <221> source
   <223> /note="Description of Artificial Sequence: Synthetic primer"
<400> 135
   atgaacctta tctcggccct 20
<210> 136
   <211> 20
   <212> DNA
   <213> Artificial Sequence
<220>
   <221> source
   <223> /note="Description of Artificial Sequence: Synthetic primer"
<400> 136
   atgtgtacgc agaagagcca 20
<210> 137
   <211> 21
   <212> DNA
   <213> Artificial Sequence
<220>
   <221> source
   <223> /note="Description of Artificial Sequence: Synthetic primer"
<400> 137
   ggaaaatcct catatttgcc a 21
<210> 138
   <211> 20
   <212> DNA
   <213> Artificial Sequence
<220>
   <221> source
   <223> /note="Description of Artificial Sequence: Synthetic primer"
<400> 138
   agacccagga ggagtgaggt 20
<210> 139
   <211> 20
   <212> DNA
   <213> Artificial Sequence
<220>
   <221> source
   <223> /note="Description of Artificial Sequence: Synthetic primer"
<400> 139
   agatgcccag atgcaaaagt 20
<210> 140
   <211> 20
   <212> DNA
   <213> Artificial Sequence
<220>
   <221> source
   <223> /note="Description of Artificial Sequence: Synthetic primer"
<400> 140
   ggctgagggt ggagtttgta 20
<210> 141
   <211> 20
   <212> DNA
   <213> Artificial Sequence
<220>
   <221> source
   <223> /note="Description of Artificial Sequence: Synthetic primer"
<400> 141
   ccccagttag aaggggaaga 20
<210> 142
   <211> 20
   <212> DNA
   <213> Artificial Sequence
<220>
   <221> source
   <223> /note="Description of Artificial Sequence: Synthetic primer"
<400> 142
   tggtgatcca gagaagaagc 20
<210> 143
   <211> 20
   <212> DNA
   <213> Artificial Sequence
<220>
   <221> source
   <223> /note="Description of Artificial Sequence: Synthetic primer"
<400> 143
   gggaggactc agagggagac 20
<210> 144
   <211> 20
   <212> DNA
   <213> Artificial Sequence
<220>
   <221> source
   <223> /note="Description of Artificial Sequence: Synthetic primer"
<400> 144
   tgcaggcact ctccatactg 20
<210> 145
   <211> 20
   <212> DNA
   <213> Artificial Sequence
<220>
   <221> source
   <223> /note="Description of Artificial Sequence: Synthetic primer"
<400> 145
   gcttcatgcc aattctttcc 20
<210> 146
   <211> 20
   <212> DNA
   <213> Artificial Sequence
<220>
   <221> source
   <223> /note="Description of Artificial Sequence: Synthetic primer"
<400> 146
   gccaattctt tccagagcaa 20
<210> 147
   <211> 20
   <212> DNA
   <213> Artificial Sequence
<220>
   <221> source
   <223> /note="Description of Artificial Sequence: Synthetic primer"
<400> 147
   gggctgaggt tgtagcactc 20
<210> 148
   <211> 21
   <212> DNA
   <213> Artificial Sequence
<220>
   <221> source
   <223> /note="Description of Artificial Sequence: Synthetic primer"
<400> 148
   tgacaccata atggattcct g 21
<210> 149
   <211> 20
   <212> DNA
   <213> Artificial Sequence
<220>
   <221> source
   <223> /note="Description of Artificial Sequence: Synthetic primer"
<400> 149
   aaagggcaca gattgccata 20
<210> 150
   <211> 20
   <212> DNA
   <213> Artificial Sequence
<220>
   <221> source
   <223> /note="Description of Artificial Sequence: Synthetic primer"
<400> 150
   actaggtggt ccagggtgtg 20
<210> 151
   <211> 20
   <212> DNA
   <213> Artificial Sequence
<220>
   <221> source
   <223> /note="Description of Artificial Sequence: Synthetic primer"
<400> 151
   tgctcaagca ggtaagatgc 20
<210> 152
   <211> 20
   <212> DNA
   <213> Artificial Sequence
<220>
   <221> source
   <223> /note="Description of Artificial Sequence: Synthetic primer"
<400> 152
   atggtctcca tcagctctcg 20
<210> 153
   <211> 20
   <212> DNA
   <213> Artificial Sequence
<220>
   <221> source
   <223> /note="Description of Artificial Sequence: Synthetic primer"
<400> 153
   aaactgaaaa tccccgctgt 20
<210> 154
   <211> 20
   <212> DNA
   <213> Artificial Sequence
<220>
   <221> source
   <223> /note="Description of Artificial Sequence: Synthetic primer"
<400> 154
   gctccagtca ccaaaaggag 20
<210> 155
   <211> 20
   <212> DNA
   <213> Artificial Sequence
<220>
   <221> source
   <223> /note="Description of Artificial Sequence: Synthetic primer"
<400> 155
   tgtggagtct cttgcgtgtc 20
<210> 156
   <211> 20
   <212> DNA
   <213> Artificial Sequence
<220>
   <221> source
   <223> /note="Description of Artificial Sequence: Synthetic primer"
<400> 156
   tggggatgag gtcgatgtat 20
<210> 157
   <211> 20
   <212> DNA
   <213> Artificial Sequence
<220>
   <221> source
   <223> /note="Description of Artificial Sequence: Synthetic primer"
<400> 157
   ccaaaaggtg tttcgtcctt 20
<210> 158
   <211> 21
   <212> DNA
   <213> Artificial Sequence
<220>
   <221> source
   <223> /note="Description of Artificial Sequence: Synthetic primer"
<400> 158
   caatttttcc actccaacac c 21
<210> 159
   <211> 20
   <212> DNA
   <213> Artificial Sequence
<220>
   <221> source
   <223> /note="Description of Artificial Sequence: Synthetic primer"
<400> 159
   catgtcaaac caccatccac 20
<210> 160
   <211> 20
   <212> DNA
   <213> Artificial Sequence
<220>
   <221> source
   <223> /note="Description of Artificial Sequence: Synthetic primer"
<400> 160
   atctggaagc aggggtcttt 20
<210> 161
   <211> 20
   <212> DNA
   <213> Artificial Sequence
<220>
   <221> source
   <223> /note="Description of Artificial Sequence: Synthetic primer"
<400> 161
   tccccatatt tctgcactcc 20
<210> 162
   <211> 18
   <212> DNA
   <213> Artificial Sequence
<220>
   <221> source
   <223> /note="Description of Artificial Sequence: Synthetic primer"
<400> 162
   ggagctacct gtggccct 18
<210> 163
   <211> 20
   <212> DNA
   <213> Artificial Sequence
<220>
   <221> source
   <223> /note="Description of Artificial Sequence: Synthetic primer"
<400> 163
   acgaaggctt cctcacagaa 20
<210> 164
   <211> 20
   <212> DNA
   <213> Artificial Sequence
<220>
   <221> source
   <223> /note="Description of Artificial Sequence: Synthetic primer"
<400> 164
   cacgcttttc atattcccgt 20
<210> 165
   <211> 20
   <212> DNA
   <213> Artificial Sequence
<220>
   <221> source
   <223> /note="Description of Artificial Sequence: Synthetic primer"
<400> 165
   tcccaaaggc ttcttcttga 20
<210> 166
   <211> 19
   <212> DNA
   <213> Artificial Sequence
<220>
   <221> source
   <223> /note="Description of Artificial Sequence: Synthetic primer"
<400> 166
   gtcgtgtacc ccagaggct 19
<210> 167
   <211> 20
   <212> DNA
   <213> Artificial Sequence
<220>
   <221> source
   <223> /note="Description of Artificial Sequence: Synthetic primer"
<400> 167
   gtgcaggaat tgggctatgt 20
<210> 168
   <211> 20
   <212> DNA
   <213> Artificial Sequence
<220>
   <221> source
   <223> /note="Description of Artificial Sequence: Synthetic primer"
<400> 168
   agcagggaag cctcctagtc 20
<210> 169
   <211> 20
   <212> DNA
   <213> Artificial Sequence
<220>
   <221> source
   <223> /note="Description of Artificial Sequence: Synthetic primer"
<400> 169
   ggtcagccag tgaggtcttc 20
<210> 170
   <211> 20
   <212> DNA
   <213> Artificial Sequence
<220>
   <221> source
   <223> /note="Description of Artificial Sequence: Synthetic primer"
<400> 170
   caaagcagac tttccaacgc 20
<210> 171
   <211> 20
   <212> DNA
   <213> Artificial Sequence
<220>
   <221> source
   <223> /note="Description of Artificial Sequence: Synthetic primer"
<400> 171
   cttctgatcg aagctttccg 20
<210> 172
   <211> 20
   <212> DNA
   <213> Artificial Sequence
<220>
   <221> source
   <223> /note="Description of Artificial Sequence: Synthetic primer"
<400> 172
   cactctcatc tctgggctcc 20
<210> 173
   <211> 1019
   <212> DNA
   <213> Artificial Sequence
<220>
   <221> source
   <223> /note="Description of Artificial Sequence: Synthetic polynucleotide"
<400> 173
   gagcacagac tcccttttct ttggcaagat ggcggagtac gacttgacta ctcgcatcgc      60
   gcactttttg gatcggcatc tagtctttcc gcttcttgaa tttctctctg taaaggaggt    120
   tcgtggcgga gagatgctga tcgcgctgaa ctgaccggtg cggcccgggg gtgagtggcg    180
   agtctccctc tgagtcctcc ccagcagcgc ggccggcgcc ggctctttgg gcgaaccctc    240
   cagttcctag actttgagag gcgtctctcc cccgcccgac cgcccagatg cagtttcgcc    300
   ttttctcctt tgccctcatc attctgaact gcatggatta cagccactgc caaggcaacc    360
   gatggagacg cagtaagcga gctagttatg tatcaaatcc catttgcaag ggttgtttgt    420
   cttgttcaaa ggacaatggg tgtagccgat gtcaacagaa gttgttcttc ttccttcgaa    480
   gagaagggat gcgccagtat ggagagtgcc tgcattcctg cccatccggg tactatggac    540
   accgagcccc agatatgaac agatgtgcaa gatgcagaat agaaaactgt gattcttgct    600
   ttagcaaaga cttttgtacc aagtgcaaag taggctttta tttgcataga ggccgttgct    660
   ttgatgaatg tccagatggt tttgcaccat tagaagaaac catggaatgt gtggaaggat    720
   gtgaagttgg tcattggagc gaatggggaa cttgtagcag aaataatcgc acatgtggat    780
   ttaaatgggg tctggaaacc agaacacggc aaattgttaa aaagccagtg aaagacacaa    840
   tactgtgtcc aaccattgct gaatccagga gatgcaagat gacaatgagg cattgtccag    900
   gagggaagag aacaccaaag gcgaaggaga agaggaacaa gaaaaagaaa aggaagctga    960
   tagaaagggc ccaggagcaa cacagcgtct tcctagctac agacagagct aaccaataa    1019
<210> 174
   <211> 284
   <212> PRT
   <213> Artificial Sequence
<220>
   <221> source
   <223> /note="Description of Artificial Sequence: Synthetic polypeptide"
<400> 174
   Met Ala Glu Tyr Asp Leu Thr Thr Arg Ile Ala His Phe Leu Asp Arg
   1               5                   10                  15
   His Leu Val Phe Pro Leu Leu Glu Phe Leu Ser Val Lys Glu Val Arg
               20                  25                  30
   Gly Gly Glu Met Leu Ile Ala Leu Asn Met Gln Phe Arg Leu Phe Ser
           35                  40                  45
   Phe Ala Leu Ile Ile Leu Asn Cys Met Asp Tyr Ser His Cys Gln Gly
       50                  55                  60
   Asn Arg Trp Arg Arg Ser Lys Arg Ala Ser Tyr Val Ser Asn Pro Ile
   65                  70                  75                  80
   Cys Lys Gly Cys Leu Ser Cys Ser Lys Asp Asn Gly Cys Ser Arg Cys
                   85                  90                  95
   Gln Gln Lys Leu Phe Phe Phe Leu Arg Arg Glu Gly Met Arg Gln Tyr
               100                 105                 110
   Gly Glu Cys Leu His Ser Cys Pro Ser Gly Tyr Tyr Gly His Arg Ala
           115                 120                 125
   Pro Asp Met Asn Arg Cys Ala Arg Cys Arg Ile Glu Asn Cys Asp Ser
       130                 135                 140
   Cys Phe Ser Lys Asp Phe Cys Thr Lys Cys Lys Val Gly Phe Tyr Leu
   145 150 155 160
   His Arg Gly Arg Cys Phe Asp Glu Cys Pro Asp Gly Phe Ala Pro Leu
   165 170 175
   Glu Glu Thr Met Glu Cys Val Glu Gly Cys Glu Val Gly His Trp Ser
   180 185 190
   Glu Trp Gly Thr Cys Ser Arg Asn Asn Arg Thr Cys Gly Phe Lys Trp
   195 200 205
   Gly Leu Glu Thr Arg Thr Arg Gln Ile Val Lys Lys Pro Val Lys Asp
   210 215 220
   Thr Ile Leu Cys Pro Thr Ile Ala Glu Ser Arg Arg Cys Lys Met Thr
   225 230 235 240
   Met Arg His Cys Pro Gly Gly Lys Arg Thr Pro Lys Ala Lys Glu Lys
   245 250 255
   Arg Asn Lys Lys Lys Lys Arg Lys Leu Ile Glu Arg Ala Gln Glu Gln
   260 265 270
   His Ser Val Phe Leu Ala Thr Asp Arg Ala Asn Gln
   275 280
<210> 175
   <211> 822
   <212> DNA
   <213> Artificial Sequence
<220>
   <221> source
   <223> /note="Description of Artificial Sequence: Synthetic polynucleotide"
<400> 175
   atggatacga ctgcggcggc ggcgctgcct gcttttgtgg cgctcttgct cctctctcct 60
   tggcctctcc tgggatcggc ccaaggccag ttctccgcag tgcatcctaa cgttagtcaa 120
   ggctgccaag gaggctgtgc aacatgctca gattacaatg gatgtttgtc atgtaagccc 180
   agactatttt ttgctctgga aagaattggc atgaagcaga ttggagtatg tctctcttca 240
   tgtccaagtg gatattatgg aactcgatat ccagatataa ataagtgtac aaaatgcaaa 300
   gctgactgtg atacctgttt caacaaaaat ttctgcacaa aatgtaaaag tggattttac 360
   ttacaccttg gaaagtgcct tgacaattgc ccagaagggt tggaagccaa caaccatact 420
   atggagtgtg tcagtattgt gcactgtgag gtcagtgaat ggaatccttg gagtccatgc      480
   acgaagaagg gaaaaacatg tggcttcaaa agagggactg aaacacgggt ccgagaaata      540
   atacagcatc cttcagcaaa gggtaacctg tgtcccccaa caaatgagac aagaaagtgt      600
   acagtgcaaa ggaagaagtg tcagaaggga gaacgaggaa aaaaaggaag ggagaggaaa      660
   agaaaaaaac ctaataaagg agaaagtaaa gaagcaatac ctgacagcaa aagtctggaa      720
   tccagcaaag aaatcccaga gcaacgagaa aacaaacagc agcagaagaa gcgaaaagtc      780
   caagataaac agaaatcggt atcagtcagc actgtacact ag                         822
<210> 176
   <211> 217
   <212> PRT
   <213> Artificial Sequence
<220>
   <221> source
   <223> /note="Description of Artificial Sequence: Synthetic polypeptide"
<400> 176
   Met Asp Thr Thr Ala Ala Ala Ala Leu Pro Ala Phe Val Ala Leu Leu
   1               5                   10                  15
   Leu Leu Ser Pro Trp Pro Leu Leu Gly Ser Ala Gln Gly Gln Phe Ser
               20                  25                  30
   Ala Val His Pro Asn Val Ser Gln Gly Cys Gln Gly Gly Cys Ala Thr
           35                  40                  45
   Cys Ser Asp Tyr Asn Gly Cys Leu Ser Cys Lys Pro Arg Leu Phe Phe
       50                  55                  60
   Ala Leu Glu Arg Ile Gly Met Lys Gln Ile Gly Val Cys Leu Ser Ser
   65                  70                  75                  80
   Cys Pro Ser Gly Tyr Tyr Gly Thr Arg Tyr Pro Asp Ile Asn Lys Cys
                   85                  90                  95
   Thr Lys Cys Lys Ala Asp Cys Asp Thr Cys Phe Asn Lys Asn Phe Cys
               100                 105                 110
   Thr Lys Cys Lys Ser Gly Phe Tyr Leu His Leu Gly Lys Cys Leu Asp
           115                 120                 125
   Asn Cys Pro Glu Gly Leu Glu Ala Asn Asn His Thr Met Glu Cys Val
       130                 135                 140
   Ser Ile Val His Cys Glu Val Ser Glu Trp Asn Pro Trp Ser Pro Cys
   145                 150                 155                 160
   Thr Lys Lys Gly Lys Thr Cys Gly Phe Lys Arg Gly Thr Glu Thr Arg
                   165                 170                 175
   Val Arg Glu Ile Ile Gln His Pro Ser Ala Lys Gly Asn Leu Cys Pro
               180                 185                 190
   Pro Thr Asn Glu Thr Arg Lys Cys Thr Val Gln Arg Lys Lys Cys Gln
           195                 200                 205
   Lys Gly Glu Arg Gly Lys Lys Gly Arg
       210                 215
<210> 177
   <211> 1884
   <212> DNA
   <213> Artificial Sequence
<220>
   <221> source
   <223> /note="Description of Artificial Sequence: Synthetic polynucleotide"
<400> 177
   atggatacga ctgcggcggc ggcgctgcct gcttttgtgg cgctcttgct cctctctcct      60
   tggcctctcc tgggatcggc ccaaggccag ttctccgcag gtggctgtac ttttgatgat     120
   ggtccagggg cctgtgatta ccaccaggat ctgtatgatg actttgaatg ggtgcatgtt     180
   agtgctcaag agcctcatta tctaccaccc gagatgcccc aaggttccta tatgatagtg     240
   gactcttcag atcacgaccc tggagaaaaa gccagacttc agctgcctac aatgaaggag     300
   aacgacactc actgcattga tttcagttac ctattatata gccagaaagg actgaatcct     360
   ggcactttga acatattagt tagggtgaat aaaggacctc ttgccaatcc aatttggaat     420
   gtgactggat tcacgggtag agattggctt cgggctgagc tagcagtgag caccttttgg     480
   cccaatgaat atcaggtaat atttgaagct gaagtctcag gagggagaag tggttatatt     540
   gccattgatg acatccaagt actgagttat ccttgtgata aatctcctca tttcctccgt     600
   ctaggggatg tagaggtgaa tgcagggcaa aacgctacat ttcagtgcat tgccacaggg     660
   agagatgctg tgcataacaa gttatggctc cagagacgaa atggagaaga tataccagta     720
   gcccagacta agaacatcaa tcatagaagg tttgccgctt ccttcagatt gcaagaagtg     780
   acaaaaactg accaggattt gtatcgctgt gtaactcagt cagaacgagg ttccggtgtg     840
   tccaattttg ctcaacttat tgtgagagaa ccgccaagac ccattgctcc tcctcagctt     900
   cttggtgttg ggcctacata tttgctgatc caactaaatg ccaactcgat cattggcgat     960
   ggtcctatca tcctgaaaga agtagagtac cgaatgacat caggatcctg gacagaaacc    1020
   catgcagtca atgctccaac ttacaaatta tggcatttag atccagatac cgaatatgag     1080
   atccgagttc tacttacaag acctggtgaa ggtggaacgg ggctcccagg acctccacta     1140
   atcaccagaa caaaatgtgc agtgcatcct aacgttagtc aaggctgcca aggaggctgt     1200
   gcaacatgct cagattacaa tggatgtttg tcatgtaagc ccagactatt ttttgctctg     1260
   gaaagaattg gcatgaagca gattggagta tgtctctctt catgtccaag tggatattat     1320
   ggaactcgat atccagatat aaataagtgt acaaaatgca aagctgactg tgatacctgt     1380
   ttcaacaaaa atttctgcac aaaatgtaaa agtggatttt acttacacct tggaaagtgc     1440
   cttgacaatt gcccagaagg gttggaagcc aacaaccata ctatggagtg tgtcagtatt     1500
   gtgcactgtg aggtcagtga atggaatcct tggagtccat gcacgaagaa gggaaaaaca     1560
   tgtggcttca aaagagggac tgaaacacgg gtccgagaaa taatacagca tccttcagca     1620
   aagggtaacc tgtgtccccc aacaaatgag acaagaaagt gtacagtgca aaggaagaag     1680
   tgtcagaagg gagaacgagg aaaaaaagga agggagagga aaagaaaaaa acctaataaa     1740
   ggagaaagta aagaagcaat acctgacagc aaaagtctgg aatccagcaa agaaatccca     1800
   gagcaacgag aaaacaaaca gcagcagaag aagcgaaaag tccaagataa acagaaatcg     1860
   gtatcagtca gcactgtaca ctag                                            1884
<210> 178
   <211> 627
   <212> PRT
   <213> Artificial Sequence
<220>
   <221> source
   <223> /note="Description of Artificial Sequence: Synthetic polypeptide"
<400> 178
   Met Asp Thr Thr Ala Ala Ala Ala Leu Pro Ala Phe Val Ala Leu Leu
   1               5                   10                  15
   Leu Leu Ser Pro Trp Pro Leu Leu Gly Ser Ala Gln Gly Gln Phe Ser
               20                  25                  30
   Ala Gly Gly Cys Thr Phe Asp Asp Gly Pro Gly Ala Cys Asp Tyr His
           35                  40                  45
   Gln Asp Leu Tyr Asp Asp Phe Glu Trp Val His Val Ser Ala Gln Glu
       50                  55                  60
   Pro His Tyr Leu Pro Pro Glu Met Pro Gln Gly Ser Tyr Met Ile Val
   65                  70                  75                  80
   Asp Ser Ser Asp His Asp Pro Gly Glu Lys Ala Arg Leu Gln Leu Pro
   85 90 95
   Thr Met Lys Glu Asn Asp Thr His Cys Ile Asp Phe Ser Tyr Leu Leu
   100 105 110
   Tyr Ser Gln Lys Gly Leu Asn Pro Gly Thr Leu Asn Ile Leu Val Arg
   115 120 125
   Val Asn Lys Gly Pro Leu Ala Asn Pro Ile Trp Asn Val Thr Gly Phe
   130 135 140
   Thr Gly Arg Asp Trp Leu Arg Ala Glu Leu Ala Val Ser Thr Phe Trp
   145 150 155 160
   Pro Asn Glu Tyr Gln Val Ile Phe Glu Ala Glu Val Ser Gly Gly Arg
   165 170 175
   Ser Gly Tyr Ile Ala Ile Asp Asp Ile Gln Val Leu Ser Tyr Pro Cys
   180 185 190
   Asp Lys Ser Pro His Phe Leu Arg Leu Gly Asp Val Glu Val Asn Ala
   195 200 205
   Gly Gln Asn Ala Thr Phe Gln Cys Ile Ala Thr Gly Arg Asp Ala Val
   210 215 220
   His Asn Lys Leu Trp Leu Gln Arg Arg Asn Gly Glu Asp Ile Pro Val
   225 230 235 240
   Ala Gln Thr Lys Asn Ile Asn His Arg Arg Phe Ala Ala Ser Phe Arg
   245 250 255
   Leu Gln Glu Val Thr Lys Thr Asp Gln Asp Leu Tyr Arg Cys Val Thr
   260 265 270
   Gln Ser Glu Arg Gly Ser Gly Val Ser Asn Phe Ala Gln Leu Ile Val
   275 280 285
   Arg Glu Pro Pro Arg Pro Ile Ala Pro Pro Gln Leu Leu Gly Val Gly
   290 295 300
   Pro Thr Tyr Leu Leu Ile Gln Leu Asn Ala Asn Ser Ile Ile Gly Asp
   305 310 315 320
   Gly Pro Ile Ile Leu Lys Glu Val Glu Tyr Arg Met Thr Ser Gly Ser
   325 330 335
   Trp Thr Glu Thr His Ala Val Asn Ala Pro Thr Tyr Lys Leu Trp His
               340                 345                 350
   Leu Asp Pro Asp Thr Glu Tyr Glu Ile Arg Val Leu Leu Thr Arg Pro
           355                 360                 365
   Gly Glu Gly Gly Thr Gly Leu Pro Gly Pro Pro Leu Ile Thr Arg Thr
       370                 375                 380
   Lys Cys Ala Val His Pro Asn Val Ser Gln Gly Cys Gln Gly Gly Cys
   385                 390                 395                 400
   Ala Thr Cys Ser Asp Tyr Asn Gly Cys Leu Ser Cys Lys Pro Arg Leu
                   405                 410                 415
   Phe Phe Ala Leu Glu Arg Ile Gly Met Lys Gln Ile Gly Val Cys Leu
               420                 425                 430
   Ser Ser Cys Pro Ser Gly Tyr Tyr Gly Thr Arg Tyr Pro Asp Ile Asn
           435                 440                 445
   Lys Cys Thr Lys Cys Lys Ala Asp Cys Asp Thr Cys Phe Asn Lys Asn
       450                 455                 460
   Phe Cys Thr Lys Cys Lys Ser Gly Phe Tyr Leu His Leu Gly Lys Cys
   465                 470                 475                 480
   Leu Asp Asn Cys Pro Glu Gly Leu Glu Ala Asn Asn His Thr Met Glu
                   485                 490                 495
   Cys Val Ser Ile Val His Cys Glu Val Ser Glu Trp Asn Pro Trp Ser
               500                 505                 510
   Pro Cys Thr Lys Lys Gly Lys Thr Cys Gly Phe Lys Arg Gly Thr Glu
           515                 520                 525
   Thr Arg Val Arg Glu Ile Ile Gln His Pro Ser Ala Lys Gly Asn Leu
       530                 535                 540
   Cys Pro Pro Thr Asn Glu Thr Arg Lys Cys Thr Val Gln Arg Lys Lys
   545                 550                 555                 560
   Cys Gln Lys Gly Glu Arg Gly Lys Lys Gly Arg Glu Arg Lys Arg Lys
                   565                 570                 575
   Lys Pro Asn Lys Gly Glu Ser Lys Glu Ala Ile Pro Asp Ser Lys Ser
               580                 585                 590
   Leu Glu Ser Ser Lys Glu Ile Pro Glu Gln Arg Glu Asn Lys Gln Gln
           595                 600                 605
   Gln Lys Lys Arg Lys Val Gln Asp Lys Gln Lys Ser Val Ser Val Ser
       610                 615                 620
   Thr Val His
   625
<210> 179
   <211> 68
   <212> DNA
   <213> Artificial Sequence
<220>
   <221> source
   <223> /note="Description of Artificial Sequence: Synthetic primer"
<400> 179
   caccccgctg cctctaggtt ctgggaagat ggcgaaggtc tcagagcttt acgatgtcac      60
   ttgggaag                                                                68
<210> 180
   <211> 70
   <212> DNA
   <213> Artificial Sequence
<220>
   <221> source
   <223> /note="Description of Artificial Sequence: Synthetic primer"
<400> 180
   gttcgtggcg gagagatgct gatcgcgctg aactgaccgg tgcggcccgg gggtgagtgg      60
   cgagtctccc                                                              70
<210> 181
   <211> 9
   <212> PRT
   <213> Artificial Sequence
<220>
   <221> source
   <223> /note="Description of Artificial Sequence: Synthetic peptide"
<400> 181
   Tyr Pro Tyr Asp Val Pro Asp Tyr Ala
   1               5
<210> 182
   <211> 11
   <212> PRT
   <213> Artificial Sequence
<220>
   <221> source
   <223> /note="Description of Artificial Sequence: Synthetic peptide"
<400> 182
   Lys Ala Ser Gln Asp Val Gly Thr Ala Val Ala
   1 5 10
<210> 183
   <211> 7
   <212> PRT
   <213> Artificial Sequence
<220>
   <221> source
   <223> /note="Description of Artificial Sequence: Synthetic peptide"
<400> 183
   Trp Ala Ser Thr Arg His Thr
   1 5
<210> 184
   <211> 8
   <212> PRT
   <213> Artificial Sequence
<220>
   <221> source
   <223> /note="Description of Artificial Sequence: Synthetic peptide"
<400> 184
   Gln Gln Tyr Ser Ser Ser Ile Thr
   1 5
<210> 185
   <211> 5
   <212> PRT
   <213> Artificial Sequence
<220>
   <221> source
   <223> /note="Description of Artificial Sequence: Synthetic peptide"
<400> 185
   Asp Tyr Ala Met His
   1 5
<210> 186
   <211> 17
   <212> PRT
   <213> Artificial Sequence
<220>
   <221> source
   <223> /note="Description of Artificial Sequence: Synthetic peptide"
<400> 186
   Ile Ile Ser Ile Tyr Tyr Asp Asn Thr Asn Tyr Asn Gln Lys Phe Lys
   1 5 10 15
   Gly
<210> 187
   <211> 11
   <212> PRT
   <213> Artificial Sequence
<220>
   <221> source
   <223> /note="Description of Artificial Sequence: Synthetic peptide"
<400> 187
   Gly Gly Asn Gly Tyr Tyr Tyr Val Met Asp Tyr
   1 5 10
<210> 188
   <211> 11
   <212> PRT
   <213> Artificial Sequence
<220>
   <221> source
   <223> /note="Description of Artificial Sequence: Synthetic peptide"
<400> 188
   Tyr Tyr Gly Tyr Gly Gly Pro Phe Phe Ala Tyr
   1 5 10
<210> 189
   <211> 11
   <212> PRT
   <213> Artificial Sequence
<220>
   <221> source
   <223> /note="Description of Artificial Sequence: Synthetic peptide"
<400> 189
   Tyr Tyr Gly Tyr Gly Gly Pro His Phe Ala Tyr
   1 5 10
<210> 190
   <211> 107
   <212> PRT
   <213> Artificial Sequence
<220>
   <221> source
   <223> /note="Description of Artificial Sequence: Synthetic polypeptide"
<400> 190
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Gln Asp Ile Asp Ser Tyr
               20                  25                  30
   Leu Ser Trp Phe Gln Gln Lys Pro Gly Lys Ala Pro Lys Thr Leu Ile
           35                  40                  45
   Tyr Leu Thr Asn Arg Leu Val Asp Gly Val Pro Ser Arg Phe Ser Gly
       50                  55                  60
   Ser Gly Ser Gly Gln Asp Tyr Thr Leu Thr Ile Ser Ser Leu Gln Pro
   65                  70                  75                  80
   Glu Asp Phe Ala Thr Tyr Tyr Cys Leu His Tyr Asp Glu Phe Pro Leu
                   85                  90                  95
   Thr Phe Gly Gln Gly Thr Lys Val Glu Ile Lys
               100                 105
<210> 191
   <211> 119
   <212> PRT
   <213> Artificial Sequence
<220>
   <221> source
   <223> /note="Description of Artificial Sequence: Synthetic polypeptide"
<400> 191
   Glu Val Gln Leu Val Glu Ser Gly Pro Gly Leu Val Lys Pro Ser Glu
   1               5                   10                  15
   Thr Leu Ser Leu Thr Cys Thr Val Ser Gly Asp Ser Ile Thr Ser Gly
               20                  25                  30
   Tyr Trp Asn Trp Ile Arg Gln Pro Pro Gly Lys Gly Leu Glu Tyr Met
           35                  40                  45
   Gly Tyr Ile Ser Tyr Ser Gly Lys Thr Tyr Gln Asn Pro Ser Leu Lys
       50                  55                  60
   Ser Arg Ile Thr Ile Ser Arg Asp Thr Ser Lys Asn Gln Tyr Ser Leu
   65                  70                  75                  80
   Lys Leu Ser Ser Val Thr Ala Ala Asp Thr Ala Val Tyr Tyr Cys Ala
                   85                  90                  95
   Thr Tyr Tyr Gly Tyr Gly Gly Pro Trp Phe Ala Tyr Trp Gly Gln Gly
               100                 105                 110
   Thr Leu Val Thr Val Ser Ser
           115
<210> 192
   <211> 107
   <212> PRT
   <213> Artificial Sequence
<220>
   <221> source
   <223> /note="Description of Artificial Sequence: Synthetic polypeptide"
<400> 192
   Asp Ile Gln Met Thr Gln Ser Pro Ser Ser Leu Ser Ala Ser Val Gly
   1 5 10 15
   Asp Arg Val Thr Ile Thr Cys Lys Ala Ser Gln Asp Ile Asp Ser Tyr
   20 25 30
   Leu Ser Trp Tyr Gln Gln Lys Pro Gly Lys Ala Pro Lys Thr Leu Ile
   35 40 45
   Tyr Leu Thr Asn Arg Leu Val Asp Gly Val Pro Ser Arg Phe Ser Gly
   50 55 60
   Ser Gly Ser Gly Gln Asp Tyr Thr Leu Thr Ile Ser Ser Leu Gln Pro
   65 70 75 80
   Glu Asp Phe Ala Thr Tyr Tyr Cys Leu His Tyr Asp Glu Phe Pro Leu
   85 90 95
   Thr Phe Gly Gln Gly Thr Lys Val Glu Ile Lys
   100 105
<210> 193
   <211> 119
   <212> PRT
   <213> Artificial Sequence
<220>
   <221> source
   <223> /note="Description of Artificial Sequence: Synthetic polypeptide"
<400> 193
   Glu Val Gln Leu Val Glu Ser Gly Pro Gly Leu Val Lys Pro Ser Glu
   1               5                   10                  15
   Thr Leu Ser Leu Thr Cys Thr Val Ser Gly Asp Ser Ile Thr Ser Gly
               20                  25                  30
   Tyr Trp Asn Trp Ile Arg Gln Pro Pro Gly Lys Gly Leu Glu Tyr Met
           35                  40                  45
   Gly Tyr Ile Ser Tyr Ser Gly Lys Thr Tyr Gln Asn Pro Ser Leu Lys
       50                  55                  60
   Ser Arg Ile Thr Ile Ser Arg Asp Thr Ser Lys Asn Gln Tyr Ser Leu
   65                  70                  75                  80
   Lys Leu Ser Ser Val Thr Ala Ala Asp Thr Ala Val Tyr Tyr Cys Ala
                   85                  90                  95
   Thr Tyr Tyr Gly Tyr Gly Gly Pro Trp Phe Ala Tyr Trp Gly Gln Gly
               100                 105                 110
   Thr Leu Val Thr Val Ser Ser
           115
<210> 194
   <211> 107
   <212> PRT
   <213> Artificial Sequence
<220>
   <221> source
   <223> /note="Description of Artificial Sequence: Synthetic polypeptide"
<400> 194
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Gln Asp Ile Asp Ser Tyr
               20                  25                  30
   Leu Ser Trp Phe Gln Gln Lys Pro Gly Lys Ala Pro Lys Leu Leu Ile
           35                  40                  45
   Tyr Leu Thr Asn Arg Leu Val Asp Gly Val Pro Ser Arg Phe Ser Gly
       50                  55                  60
   Ser Gly Ser Gly Gln Asp Tyr Thr Leu Thr Ile Ser Ser Leu Gln Pro
   65                  70                  75                  80
   Glu Asp Phe Ala Thr Tyr Tyr Cys Leu His Tyr Asp Glu Phe Pro Leu
   85 90 95
   Thr Phe Gly Gln Gly Thr Lys Val Glu Ile Lys
   100 105
<210> 195
   <211> 119
   <212> PRT
   <213> Artificial Sequence
<220>
   <221> source
   <223> /note="Description of Artificial Sequence: Synthetic polypeptide"
<400> 195
   Glu Val Gln Leu Val Glu Ser Gly Pro Gly Leu Val Lys Pro Ser Glu
   1 5 10 15
   Thr Leu Ser Leu Thr Cys Thr Val Ser Gly Asp Ser Ile Thr Ser Gly
   20 25 30
   Tyr Trp Asn Trp Ile Arg Gln Pro Pro Gly Lys Gly Leu Glu Tyr Met
   35 40 45
   Gly Tyr Ile Ser Tyr Ser Gly Lys Thr Tyr Gln Asn Pro Ser Leu Lys
   50 55 60
   Ser Arg Ile Thr Ile Ser Arg Asp Thr Ser Lys Asn Gln Tyr Ser Leu
   65 70 75 80
   Lys Leu Ser Ser Val Thr Ala Ala Asp Thr Ala Val Tyr Tyr Cys Ala
   85 90 95
   Thr Tyr Tyr Gly Tyr Gly Gly Pro Trp Phe Ala Tyr Trp Gly Gln Gly
   100 105 110
   Thr Leu Val Thr Val Ser Ser
   115
<210> 196
   <211> 107
   <212> PRT
   <213> Artificial Sequence
<220>
   <221> source
   <223> /note="Description of Artificial Sequence: Synthetic polypeptide"
<210> 197
   <211> 119
   <212> PRT
   <213> Artificial Sequence
<220>
   <221> source
   <223> /note="Description of Artificial Sequence: Synthetic polypeptide"
<400> 197
   Glu Val Gln Leu Val Glu Ser Gly Pro Gly Leu Val Lys Pro Ser Glu
   1               5                   10                  15
   Thr Leu Ser Leu Thr Cys Thr Val Ser Gly Asp Ser Ile Thr Ser Gly
               20                  25                  30
   Tyr Trp Asn Trp Ile Arg Gln Pro Pro Gly Lys Gly Leu Glu Tyr Met
           35                  40                  45
   Gly Tyr Ile Ser Tyr Ser Gly Lys Thr Tyr Gln Asn Pro Ser Leu Lys
       50                  55                  60
   Ser Arg Ile Thr Ile Ser Arg Asp Thr Ser Lys Asn Gln Tyr Ser Leu
   65                  70                  75                  80
   Lys Leu Ser Ser Val Thr Ala Ala Asp Thr Ala Val Tyr Tyr Cys Ala
                   85                  90                  95
   Thr Tyr Tyr Gly Tyr Gly Gly Pro Trp Phe Ala Tyr Trp Gly Gln Gly
               100                 105                 110
   Thr Leu Val Thr Val Ser Ser
           115
<210> 198
   <211> 107
   <212> PRT
   <213> Artificial Sequence
<220>
   <221> source
   <223> /note="Description of Artificial Sequence: Synthetic polypeptide"
<400> 198
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Gln Asp Ile Asp Ser Tyr
               20                  25                  30
   Leu Ser Trp Phe Gln Gln Lys Pro Gly Lys Ala Pro Lys Thr Leu Ile
           35                  40                  45
   Tyr Leu Thr Asn Arg Leu Val Asp Gly Val Pro Ser Arg Phe Ser Gly
       50                  55                  60
   Ser Gly Ser Gly Gln Asp Phe Thr Leu Thr Ile Ser Ser Leu Gln Pro
   65                  70                  75                  80
   Glu Asp Phe Ala Thr Tyr Tyr Cys Leu His Tyr Asp Glu Phe Pro Leu
                   85                  90                  95
   Thr Phe Gly Gln Gly Thr Lys Val Glu Ile Lys
               100                 105
<210> 199
   <211> 119
   <212> PRT
   <213> Artificial Sequence
<220>
   <221> source
   <223> /note="Description of Artificial Sequence: Synthetic polypeptide"
<400> 199
   Glu Val Gln Leu Val Glu Ser Gly Pro Gly Leu Val Lys Pro Ser Glu
   1               5                   10                  15
   Thr Leu Ser Leu Thr Cys Thr Val Ser Gly Asp Ser Ile Thr Ser Gly
   20 25 30
   Tyr Trp Asn Trp Ile Arg Gln Pro Pro Gly Lys Gly Leu Glu Tyr Met
   35 40 45
   Gly Tyr Ile Ser Tyr Ser Gly Lys Thr Tyr Gln Asn Pro Ser Leu Lys
   50 55 60
   Ser Arg Ile Thr Ile Ser Arg Asp Thr Ser Lys Asn Gln Tyr Ser Leu
   65 70 75 80
   Lys Leu Ser Ser Val Thr Ala Ala Asp Thr Ala Val Tyr Tyr Cys Ala
   85 90 95
   Thr Tyr Tyr Gly Tyr Gly Gly Pro Trp Phe Ala Tyr Trp Gly Gln Gly
   100 105 110
   Thr Leu Val Thr Val Ser Ser
   115
<210> 200
   <211> 107
   <212> PRT
   <213> Artificial Sequence
<220>
   <221> source
   <223> /note="Description of Artificial Sequence: Synthetic polypeptide"
<400> 200
   Asp Ile Gln Met Thr Gln Ser Pro Ser Ser Leu Ser Ala Ser Val Gly
   1 5 10 15
   Asp Arg Val Thr Ile Thr Cys Lys Ala Ser Gln Asp Ile Asp Ser Tyr
   20 25 30
   Leu Ser Trp Phe Gln Gln Lys Pro Gly Lys Ala Pro Lys Thr Leu Ile
   35 40 45
   Tyr Leu Thr Asn Arg Leu Val Asp Gly Val Pro Ser Arg Phe Ser Gly
   50 55 60
   Ser Gly Ser Gly Gln Asp Tyr Thr Leu Thr Ile Ser Ser Leu Gln Pro
   65 70 75 80
   Glu Asp Phe Ala Thr Tyr Tyr Cys Leu His Tyr Asp Glu Phe Pro Leu
   85 90 95
   Thr Phe Gly Gln Gly Thr Lys Val Glu Ile Lys
   100 105
<210> 201
   <211> 119
   <212> PRT
   <213> Artificial Sequence
<220>
   <221> source
   <223> /note="Description of Artificial Sequence: Synthetic polypeptide"
<400> 201
   Glu Val Gln Leu Val Glu Ser Gly Pro Gly Leu Val Lys Pro Ser Glu
   1 5 10 15
   Thr Leu Ser Leu Thr Cys Thr Val Ser Gly Asp Ser Ile Thr Ser Gly
   20 25 30
   Tyr Trp Asn Trp Ile Arg Gln Pro Pro Gly Lys Gly Leu Glu Tyr Met
   35 40 45
   Gly Tyr Ile Ser Tyr Ser Gly Lys Thr Tyr Gln Asn Pro Ser Leu Lys
   50 55 60
   Ser Arg Ile Thr Ile Ser Val Asp Thr Ser Lys Asn Gln Tyr Ser Leu
   65 70 75 80
   Lys Leu Ser Ser Val Thr Ala Ala Asp Thr Ala Val Tyr Tyr Cys Ala
   85 90 95
   Thr Tyr Tyr Gly Tyr Gly Gly Pro Trp Phe Ala Tyr Trp Gly Gln Gly
   100 105 110
   Thr Leu Val Thr Val Ser Ser
   115
<210> 202
   <211> 107
   <212> PRT
   <213> Artificial Sequence
<220>
   <221> source
   <223> /note="Description of Artificial Sequence: Synthetic polypeptide"
<400> 202
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Gln Asp Ile Asp Ser Tyr
               20                  25                  30
   Leu Ser Trp Phe Gln Gln Lys Pro Gly Lys Ala Pro Lys Thr Leu Ile
           35                  40                  45
   Tyr Leu Thr Asn Arg Leu Val Asp Gly Val Pro Ser Arg Phe Ser Gly
       50                  55                  60
   Ser Gly Ser Gly Gln Asp Tyr Thr Leu Thr Ile Ser Ser Leu Gln Pro
   65                  70                  75                  80
   Glu Asp Phe Ala Thr Tyr Tyr Cys Leu His Tyr Asp Glu Phe Pro Leu
                   85                  90                  95
   Thr Phe Gly Gln Gly Thr Lys Val Glu Ile Lys
               100                 105
<210> 203
   <211> 119
   <212> PRT
   <213> Artificial Sequence
<220>
   <221> source
   <223> /note="Description of Artificial Sequence: Synthetic polypeptide"
<400> 203
   Glu Val Gln Leu Val Glu Ser Gly Pro Gly Leu Val Lys Pro Ser Glu
   1               5                   10                  15
   Thr Leu Ser Leu Thr Cys Thr Val Ser Gly Asp Ser Ile Thr Ser Gly
               20                  25                  30
   Tyr Trp Asn Trp Ile Arg Gln Pro Pro Gly Lys Gly Leu Glu Tyr Met
           35                  40                  45
   Gly Tyr Ile Ser Tyr Ser Gly Lys Thr Tyr Gln Asn Pro Ser Leu Lys
       50                  55                  60
   Ser Arg Ile Thr Ile Ser Arg Asp Thr Ser Lys Asn Gln Tyr Ser Leu
   65                  70                  75                  80
   Lys Leu Ser Ser Val Thr Ala Ala Asp Thr Ala Val Tyr Tyr Cys Ala
                   85                  90                  95
   Arg Tyr Tyr Gly Tyr Gly Gly Pro Trp Phe Ala Tyr Trp Gly Gln Gly
               100                 105                 110
   Thr Leu Val Thr Val Ser Ser
   115
<210> 204
   <211> 107
   <212> PRT
   <213> Artificial Sequence
<220>
   <221> source
   <223> /note="Description of Artificial Sequence: Synthetic polypeptide"
<400> 204
   Asp Ile Gln Met Thr Gln Ser Pro Ser Ser Leu Ser Ala Ser Val Gly
   1 5 10 15
   Asp Arg Val Thr Ile Thr Cys Lys Ala Ser Gln Asp Ile Asp Ser Tyr
   20 25 30
   Leu Ser Trp Phe Gln Gln Lys Pro Gly Lys Ala Pro Lys Thr Leu Ile
   35 40 45
   Tyr Leu Thr Asn Arg Leu Val Asp Gly Val Pro Ser Arg Phe Ser Gly
   50 55 60
   Ser Gly Ser Gly Gln Asp Tyr Thr Leu Thr Ile Ser Ser Leu Gln Pro
   65 70 75 80
   Glu Asp Phe Ala Thr Tyr Tyr Cys Leu His Tyr Asp Glu Phe Pro Leu
   85 90 95
   Thr Phe Gly Gln Gly Thr Lys Val Glu Ile Lys
   100 105
<210> 205
   <211> 119
   <212> PRT
   <213> Artificial Sequence
<220>
   <221> source
   <223> /note="Description of Artificial Sequence: Synthetic polypeptide"
<400> 205
   Glu Val Gln Leu Val Glu Ser Gly Pro Gly Leu Val Lys Pro Ser Glu
   1 5 10 15
   Thr Leu Ser Leu Thr Cys Thr Val Ser Gly Asp Ser Ile Thr Ser Gly
   20 25 30
   Tyr Trp Asn Trp Ile Arg Gln Pro Pro Gly Lys Gly Leu Glu Trp Ile
   35 40 45
   Gly Tyr Ile Ser Tyr Ser Gly Lys Thr Tyr Gln Asn Pro Ser Leu Lys
   50 55 60
   Ser Arg Ile Thr Ile Ser Arg Asp Thr Ser Lys Asn Gln Phe Ser Leu
   65 70 75 80
   Lys Leu Ser Ser Val Thr Ala Ala Asp Thr Ala Val Tyr Tyr Cys Ala
   85 90 95
   Thr Tyr Tyr Gly Tyr Gly Gly Pro Trp Phe Ala Tyr Trp Gly Gln Gly
   100 105 110
   Thr Leu Val Thr Val Ser Ser
   115
<210> 206
   <211> 107
   <212> PRT
   <213> Artificial Sequence
<220>
   <221> source
   <223> /note="Description of Artificial Sequence: Synthetic polypeptide"
<400> 206
   Asp Ile Gln Met Thr Gln Ser Pro Ser Ser Leu Ser Ala Ser Val Gly
   1 5 10 15
   Asp Arg Val Thr Ile Thr Cys Lys Ala Ser Gln Asp Ile Asp Ser Tyr
   20 25 30
   Leu Ser Trp Phe Gln Gln Lys Pro Gly Lys Ala Pro Lys Thr Leu Ile
   35 40 45
   Tyr Leu Thr Asn Arg Leu Val Asp Gly Val Pro Ser Arg Phe Ser Gly
   50 55 60
   Ser Gly Ser Gly Gln Asp Tyr Thr Leu Thr Ile Ser Ser Leu Gln Pro
   65 70 75 80
   Glu Asp Phe Ala Thr Tyr Tyr Cys Leu His Tyr Asp Glu Phe Pro Leu
   85 90 95
   Thr Phe Gly Gln Gly Thr Lys Val Glu Ile Lys
   100 105
<210> 207
   <211> 119
   <212> PRT
   <213> Artificial Sequence
<220>
   <221> source
   <223> /note="Description of Artificial Sequence: Synthetic polypeptide"
<400> 207
   Glu Val Gln Leu Val Glu Ser Gly Pro Gly Leu Val Lys Pro Ser Glu
   1               5                   10                  15
   Thr Leu Ser Leu Thr Cys Thr Val Ser Gly Asp Ser Ile Thr Ser Gly
               20                  25                  30
   Tyr Trp Asn Trp Ile Arg Gln Pro Pro Gly Lys Gly Leu Glu Trp Ile
           35                  40                  45
   Gly Tyr Ile Ser Tyr Ser Gly Lys Thr Tyr Gln Asn Pro Ser Leu Lys
       50                  55                  60
   Ser Arg Val Thr Ile Ser Arg Asp Thr Ser Lys Asn Gln Phe Ser Leu
   65                  70                  75                  80
   Lys Leu Ser Ser Val Thr Ala Ala Asp Thr Ala Val Tyr Tyr Cys Ala
                   85                  90                  95
   Thr Tyr Tyr Gly Tyr Gly Gly Pro Trp Phe Ala Tyr Trp Gly Gln Gly
               100                 105                 110
   Thr Leu Val Thr Val Ser Ser
           115
<210> 208
   <211> 107
   <212> PRT
   <213> Artificial Sequence
<220>
   <221> source
   <223> /note="Description of Artificial Sequence: Synthetic polypeptide"
<400> 208
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Gln Asp Ile Asp Ser Tyr
               20                  25                  30
   Leu Ser Trp Phe Gln Gln Lys Pro Gly Lys Ala Pro Lys Thr Leu Ile
           35                  40                  45
   Tyr Leu Thr Asn Arg Leu Val Asp Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Tyr Cys Leu His Tyr Asp Glu Phe Pro Leu
                   85                  90                  95
   Thr Phe Gly Gln Gly Thr Lys Val Glu Ile Lys
               100                 105
<210> 209
   <211> 119
   <212> PRT
   <213> Artificial Sequence
<220>
   <221> source
   <223> /note="Description of Artificial Sequence: Synthetic polypeptide"
<400> 209
   Glu Val Gln Leu Val Glu Ser Gly Pro Gly Leu Val Lys Pro Ser Glu
   1 5 10 15
   Thr Leu Ser Leu Thr Cys Thr Val Ser Gly Asp Ser Ile Thr Ser Gly
   20 25 30
   Tyr Trp Asn Trp Ile Arg Gln Pro Pro Gly Lys Gly Leu Glu Trp Ile
   35 40 45
   Gly Tyr Ile Ser Tyr Ser Gly Lys Thr Tyr Gln Asn Pro Ser Leu Lys
   50 55 60
   Ser Arg Val Thr Ile Ser Arg Asp Thr Ser Lys Asn Gln Phe Ser Leu
   65 70 75 80
   Lys Leu Ser Ser Val Thr Ala Ala Asp Thr Ala Val Tyr Tyr Cys Ala
   85 90 95
   Thr Tyr Tyr Gly Tyr Gly Gly Pro Trp Phe Ala Tyr Trp Gly Gln Gly
   100 105 110
   Thr Leu Val Thr Val Ser Ser
   115
<210> 210
   <211> 107
   <212> PRT
   <213> Artificial Sequence
<220>
   <221> source
   <223> /note="Description of Artificial Sequence: Synthetic polypeptide"
<400> 210
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Gln Asp Ile Asp Ser Tyr
               20                  25                  30
   Leu Ser Trp Phe Gln Gln Lys Pro Gly Lys Ala Pro Lys Thr Leu Ile
           35                  40                  45
   Tyr Leu Thr Asn Arg Leu Val Asp Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Tyr Cys Leu His Tyr Asp Glu Phe Pro Leu
                   85                  90                  95
   Thr Phe Gly Gln Gly Thr Lys Val Glu Ile Lys
               100                 105
<210> 211
   <211> 119
   <212> PRT
   <213> Artificial Sequence
<220>
   <221> source
   <223> /note="Description of Artificial Sequence: Synthetic polypeptide"
<400> 211
   Glu Val Gln Leu Val Glu Ser Gly Pro Gly Leu Val Lys Pro Ser Glu
   1               5                   10                  15
   Thr Leu Ser Leu Thr Cys Thr Val Ser Gly Asp Ser Ile Thr Ser Gly
               20                  25                  30
   Tyr Trp Asn Trp Ile Arg Gln Pro Pro Gly Lys Gly Leu Glu Trp Ile
           35                  40                  45
   Gly Tyr Ile Ser Tyr Ser Gly Lys Thr Tyr Gln Asn Pro Ser Leu Lys
       50                  55                  60
   Ser Arg Ile Thr Ile Ser Arg Asp Thr Ser Lys Asn Gln Phe Ser Leu
   65 70 75 80
   Lys Leu Ser Ser Val Thr Ala Ala Asp Thr Ala Val Tyr Tyr Cys Ala
   85 90 95
   Thr Tyr Tyr Gly Tyr Gly Gly Pro Trp Phe Ala Tyr Trp Gly Gln Gly
   100 105 110
   Thr Leu Val Thr Val Ser Ser
   115
<210> 212
   <211> 107
   <212> PRT
   <213> Artificial Sequence
<220>
   <221> source
   <223> /note="Description of Artificial Sequence: Synthetic polypeptide"
<400> 212
   Asp Ile Gln Met Thr Gln Ser Pro Ser Ser Leu Ser Ala Ser Val Gly
   1 5 10 15
   Asp Arg Val Thr Ile Thr Cys Lys Ala Ser Gln Asp Ile Asp Ser Tyr
   20 25 30
   Leu Ser Trp Phe Gln Gln Lys Pro Gly Lys Ala Pro Lys Thr Leu Ile
   35 40 45
   Tyr Leu Thr Asn Arg Leu Val Asp Gly Val Pro Ser Arg Phe Ser Gly
   50 55 60
   Ser Gly Ser Gly Thr Asp Tyr Thr Leu Thr Ile Ser Ser Leu Gln Pro
   65 70 75 80
   Glu Asp Phe Ala Thr Tyr Tyr Cys Leu His Tyr Asp Glu Phe Pro Leu
   85 90 95
   Thr Phe Gly Gln Gly Thr Lys Val Glu Ile Lys
   100 105
<210> 213
   <211> 119
   <212> PRT
   <213> Artificial Sequence
<220>
   <221> source
   <223> /note="Description of Artificial Sequence: Synthetic polypeptide"
<400> 213
   Glu Val Gln Leu Val Glu Ser Gly Pro Gly Leu Val Lys Pro Ser Glu
   1               5                   10                  15
   Thr Leu Ser Leu Thr Cys Thr Val Ser Gly Asp Ser Ile Thr Ser Gly
               20                  25                  30
   Tyr Trp Asn Trp Ile Arg Gln Pro Pro Gly Lys Gly Leu Glu Trp Ile
           35                  40                  45
   Gly Tyr Ile Ser Tyr Ser Gly Lys Thr Tyr Gln Asn Pro Ser Leu Lys
       50                  55                  60
   Ser Arg Val Thr Ile Ser Arg Asp Thr Ser Lys Asn Gln Phe Ser Leu
   65                  70                  75                  80
   Lys Leu Ser Ser Val Thr Ala Ala Asp Thr Ala Val Tyr Tyr Cys Ala
                   85                  90                  95
   Thr Tyr Tyr Gly Tyr Gly Gly Pro Phe Phe Ala Tyr Trp Gly Gln Gly
               100                 105                 110
   Thr Leu Val Thr Val Ser Ser
           115
<210> 214
   <211> 107
   <212> PRT
   <213> Artificial Sequence
<220>
   <221> source
   <223> /note="Description of Artificial Sequence: Synthetic polypeptide"
<400> 214
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Gln Asp Ile Asp Ser Tyr
               20                  25                  30
   Leu Ser Trp Phe Gln Gln Lys Pro Gly Lys Ala Pro Lys Thr Leu Ile
           35                  40                  45
   Tyr Leu Thr Asn Arg Leu Val Asp Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Tyr Cys Leu His Tyr Asp Glu Phe Pro Leu
                   85                  90                  95
   Thr Phe Gly Gln Gly Thr Lys Val Glu Ile Lys
               100                 105
<210> 215
   <211> 119
   <212> PRT
   <213> Artificial Sequence
<220>
   <221> source
   <223> /note="Description of Artificial Sequence: Synthetic polypeptide"
<400> 215
   Glu Val Gln Leu Val Glu Ser Gly Pro Gly Leu Val Lys Pro Ser Glu
   1               5                   10                  15
   Thr Leu Ser Leu Thr Cys Thr Val Ser Gly Asp Ser Ile Thr Ser Gly
               20                  25                  30
   Tyr Trp Asn Trp Ile Arg Gln Pro Pro Gly Lys Gly Leu Glu Trp Ile
           35                  40                  45
   Gly Tyr Ile Ser Tyr Ser Gly Lys Thr Tyr Gln Asn Pro Ser Leu Lys
       50                  55                  60
   Ser Arg Val Thr Ile Ser Arg Asp Thr Ser Lys Asn Gln Phe Ser Leu
   65                  70                  75                  80
   Lys Leu Ser Ser Val Thr Ala Ala Asp Thr Ala Val Tyr Tyr Cys Ala
                   85                  90                  95
   Thr Tyr Tyr Gly Tyr Gly Gly Pro His Phe Ala Tyr Trp Gly Gln Gly
               100                 105                 110
   Thr Leu Val Thr Val Ser Ser
           115
<210> 216
   <211> 11
   <212> PRT
   <213> Artificial Sequence
<220>
   <221> source
   <223> /note="Description of Artificial Sequence: Synthetic peptide"
<220>
   <221> MOD_RES
   <222> (2) .. (2)
   <223> /replace="His"
<220>
   <221> MOD_RES
   <222> (8) .. (8)
   <223> /replace="Phe" or "His"
<220>
   <221> misc_feature
   <222> (1)..(11)
   <223> /note="Variant residues given in the sequence have no preference with respect to those in the annotations for variant positions"
<400> 216
   Tyr Tyr Gly Tyr Gly Gly Pro Trp Phe Ala Tyr
   1               5                   10

## Description

### FIELD

Provided herein are anti-RSPO antibodies, in particular anti-RSPO2 antibodies and/or anti-RSPO3 antibodies, and methods of using the same. The detailed technical disclosure set out below may in some respects go beyond the disclosure of the invention per se, and may also provide technical background for related technical developments. It will be appreciated that the additional technical background provided is not intended to define the invention as such (which is defined exclusively by the appended claims), but rather to place it in a broader technical context. Accordingly, it will be appreciated that the terms "embodiments", "aspects", "subject-matter" reflect specific details of the disclosure, but insofar as they refer to a part of the additional technical background, are not intended to define as part of the invention subject-matter that does not fall within the scope of the appended claims.

### BACKGROUND

The R-spondin (RSPO) family is a small group of four secreted proteins (RSPO1-RSPO4) that are widely expressed in vertebrate embryos and the adult. RSPOs have pleiotropic functions in development and stem cell growth by strongly enhancing Wnt pathway activation (Kazanskaya et al. Dev. Cell 7:525-534 (2004); Kim et al., Cell Cycle 5:23-26 (2006); WO 2005/040418). Mammalian RSPO1-RSPO4 share 40%-60% amino acid sequence identities and consist of a signal peptide, two adjacent furin-like cysteine-rich domains (FU-CRDs) followed by a thrombospondin type I repeat (TSR) domain, and a positively charged C-terminal region. The two FU-CRDs are essential and sufficient to promote Wnt/β-catenin signaling (Kazanskaya et al., Dev. Cell 7:525-534 (2004); WO 2005/040418).

LGR4 (leucine-rich repeat [LRR]-containing G-protein-coupled receptor [GPCR] 4), LGR5, and LGR6 (Hsu et al., Mol. Endocrinol. 12:1830-1845 (1998) and Hsu et al., Mol. Endocinol. 14:1257-1271 (2000)) are receptors for RSPOs. A common feature of the LGR4/5/6 receptors is their expression in distinct types of adult stem cells. LGR5 has already been described as a marker for resident stem cells in Wnt-dependent compartments, including the small intestine, colon, stomach, and hair follicle (Barker and Clevers Gastroenterology 138:1681-1696 (2010); Seshagiri et al., Nature 488:660-664 (2012)). LGR6 also serves as a marker of multipotent stem cells in the epidermis (Snippert et al., Science 327:1385-1389 (2010)). LGR4 is widely expressed in proliferating cells (Van Schoore et al., Histochem Cell Biol. 124:35-50 (2005)), and its knockout mice show developmental defects in many organs, including bone, kidney, testis, skin, and gall bladder (Mustata et al., EMBO Rep 12:558-564 (2011)). LGR4/5/6 receptors have a central array of 17 LRRs flanked by cysteine-rich sequences at both the N- and C-termini in the extracellular domain before seven transmembrane helices, and the extracellular domain is essential and sufficient for high-affinity binding with RSPOs (de Lau et al., Genome Biol. 13:242 (2011) and Wang et al, Genes & Dev. 27:1339-1344 (2013)).

LGR4/5/6 receptors may physically interact with low-density lipoprotein receptor-related protein 5/6 (LRP5/6) after RSPO recognition, and thereby RSPOs and Wnt ligands work together to activate Wnt/β-catenin signaling (de Lau et al., Genome Biol. 13:242 (2011); Carmon et al., Proc Natl Acad Sci 108:11452-11457 (2012)). RSPOs are also able to promote Wnt/β-catenin signaling by stabilizing the Frizzled and LRP5/6 receptors (Hao et al., Nature 485:195-200 (2012)). Zinc and RING finger 3 (ZNRF3) and its homolog, RING finger 43 (RNF43), are transmembrane E3 ubiquitin ligases that promote turnover of the Frizzled and LRP6 receptors on the cell surface (Hao et al., Nature 485:195-200 (2012); Koo et al., Nature 488:665-669 (2012)). RSPOs may induce clearance of ZNRF3 from the membrane by interacting with the extracellular domains of LGR4/5/6 and ZNRF3/RNF43, which stabilizes the Frizzled and LRP6 receptors to enhance Wnt/β-catenin signaling (Hao et al., Nature 485:195-200 (2012)).

### SUMMARY

The invention provides anti-RSPO antibodies, in particular antibodies that bind RSPO2, RSPO3, and/or both RSPO2 and RSPO3, and methods of using the same.

Provided herein are isolated antibodies that bind to RSPO2, wherein the antibody inhibits the interaction of RSPO2 with a transmembrane E3 ubiquitinase. In some embodiments, the transmembrane E3 ubiquitinase is ZNRF3 and/or RNF43.

In some embodiments, the antibody does not inhibit the interaction of RSPO2 with one or more of LGR4, LGR5, and/or LGR6 (*e.g*., enhances the interaction of RSPO2 with one or more of LGR4, LGR5, and/or LGR6). For example, provided herein are isolated antibodies that bind to RSPO2, wherein the antibody comprises (a) a light chain variable domain (VL) comprising (i) hyper variable region-Ll (HVR-L1) comprising the amino acid sequence of SEQ ID NO:53, (ii) HVR-L2 comprising the amino acid sequence of SEQ ID NO:54, and (iii) HVR-L3 comprising the amino acid sequence of SEQ ID NO:55, and (b) a heavy chain variable domain (VH) comprising (i) HVR-H1 comprising the amino acid sequence of SEQ ID NO:56, (ii) HVR-H2 comprising the amino acid sequence of SEQ ID NO:57, and (iii) HVR-H3 comprising the amino acid sequence of SEQ ID NO:58. In some embodiments, the antibody comprises (a) a VL sequence of SEQ ID NO:105 and a VH sequence of SEQ ID NO:106.

In some embodiments, the antibody inhibits the interaction of RSPO2 with one or more of LGR4, LGR5, and/or LGR6. For example, provided herein are isolated antibodies that bind to RSPO2, wherein the antibody comprises: (a) a VL comprising (i) HVR-L1 comprising the amino acid sequence of SEQ ID NO:59, (ii) HVR-L2 comprising the amino acid sequence of SEQ ID NO:60, and (iii) HVR-L3 comprising the amino acid sequence of SEQ ID NO:61; and a VH comprising (i) HVR-H1 comprising the amino acid sequence of SEQ ID NO:62, (ii) HVR-H2 comprising the amino acid sequence of SEQ ID NO:63, and (iii) HVR-H3 comprising the amino acid sequence of SEQ ID NO:64; (b) a VL comprising (i) HVR-L1 comprising the amino acid sequence of SEQ ID NO:65, (ii) HVR-L2 comprising the amino acid sequence of SEQ ID NO:66, and (iii) HVR-L3 comprising the amino acid sequence of SEQ ID NO:67; and a VH comprising (i) HVR-H1 comprising the amino acid sequence of SEQ ID NO:68, (ii) HVR-H2 comprising the amino acid sequence of SEQ ID NO:69, and (iii) HVR-H3 comprising the amino acid sequence of SEQ ID NO:70; or (c) a VL comprising (i) HVR-L1 comprising the amino acid sequence of SEQ ID NO:71, (ii) HVR-L2 comprising the amino acid sequence of SEQ ID NO:72, and (iii) HVR-L3 comprising the amino acid sequence of SEQ ID NO:73; and a VH comprising (i) HVR-H1 comprising the amino acid sequence of SEQ ID NO:74, (ii) HVR-H2 comprising the amino acid sequence of SEQ ID NO:75, and (iii) HVR-H3 comprising the amino acid sequence of SEQ ID NO:76. In some embodiments, the isolated antibody comprises (a) a VL sequence of SEQ ID NO:107 and a VH sequence of SEQ ID NO:108; (b) a VL sequence of SEQ ID NO:109 and a VH sequence of SEQ ID NO:110; or (c) a VL sequence of SEQ ID NO:111 and a VH sequence of SEQ ID NO:112.

Provided herein are also isolated antibodies that bind to RSPO3, wherein the antibody inhibits the interaction of RSPO3 with a transmembrane E3 ubiquitinase. In some embodiments, the transmembrane E3 ubiquitinase is ZNRF3 and/or RNF43.

In some embodiments, the antibody does not inhibit the interaction of RSPO3 with one or more of LGR4, LGR5, and/or LGR6 (*e.g*., enhances binding of RSPO3 to one or more of LGR4, LGR5, and/or LGR6).

In some embodiments, the antibody inhibits the interaction of RSPO3 with one or more of LGR4, LGR5, and/or LGR6. For example, provided herein are isolated antibodies that bind to RSPO3, wherein the antibody comprises: (a) VL comprising (i) HVR-L1 comprising the amino acid sequence of SEQ ID NO:5, (ii) HVR-L2 comprising the amino acid sequence of SEQ ID NO:6, and (iii) HVR-L3 comprising the amino acid sequence of SEQ ID NO:7; and a VH comprising (i) HVR-H1 comprising the amino acid sequence of SEQ ID NO:8, (ii) HVR-H2 comprising the amino acid sequence of SEQ ID NO:9, and (iii) HVR-H3 comprising the amino acid sequence of SEQ ID NO:10; (b) a VL comprising (i) HVR-L1 comprising the amino acid sequence of SEQ ID NO:11, (ii) HVR-L2 comprising the amino acid sequence of SEQ ID NO:12, and (iii) HVR-L3 comprising the amino acid sequence of SEQ ID NO:13; and a VH comprising (i) HVR-H1 comprising the amino acid sequence of SEQ ID NO:14, (ii) HVR-H2 comprising the amino acid sequence of SEQ ID NO:15, and (iii) HVR-H3 comprising the amino acid sequence of SEQ ID NO:16; (c) a VL comprising (i) HVR-L1 comprising the amino acid sequence of SEQ ID NO:17, (ii) HVR-L2 comprising the amino acid sequence of SEQ ID NO:18, and (iii) HVR-L3 comprising the amino acid sequence of SEQ ID NO:19; and a VH comprising (i) HVR-H1 comprising the amino acid sequence of SEQ ID NO:20, (ii) HVR-H2 comprising the amino acid sequence of SEQ ID NO:21, and (iii) HVR-H3 comprising the amino acid sequence of SEQ ID NO:22; (d) a VL comprising (i) HVR-L1 comprising the amino acid sequence of SEQ ID NO:23, (ii) HVR-L2 comprising the amino acid sequence of SEQ ID NO:24, and (iii) HVR-L3 comprising the amino acid sequence of SEQ ID NO:25; and a VH comprising (i) HVR-H1 comprising the amino acid sequence of SEQ ID NO:26, (ii) HVR-H2 comprising the amino acid sequence of SEQ ID NO:27, and (iii) HVR-H3 comprising the amino acid sequence of SEQ ID NO:28; (e) a VL comprising (i) HVR-L1 comprising the amino acid sequence of SEQ ID NO:29, (ii) HVR-L2 comprising the amino acid sequence of SEQ ID NO:30, and (iii) HVR-L3 comprising the amino acid sequence of SEQ ID NO:31; and a VH comprising (i) HVR-H1 comprising the amino acid sequence of SEQ ID NO:32, (ii) HVR-H2 comprising the amino acid sequence of SEQ ID NO:33, and (iii) HVR-H3 comprising the amino acid sequence of SEQ ID NO:34; (f) a VL comprising (i) HVR-L1 comprising the amino acid sequence of SEQ ID NO:35, (ii) HVR-L2 comprising the amino acid sequence of SEQ ID NO:36, and (iii) HVR-L3 comprising the amino acid sequence of SEQ ID NO:37; and a VH comprising (i) HVR-H1 comprising the amino acid sequence of SEQ ID NO:38, (ii) HVR-H2 comprising the amino acid sequence of SEQ ID NO:39, and (iii) HVR-H3 comprising the amino acid sequence of SEQ ID NO:40; or (g) a VL comprising (i) HVR-L1 comprising the amino acid sequence of SEQ ID NO:41, (ii) HVR-L2 comprising the amino acid sequence of SEQ ID NO:42, and (iii) HVR-L3 comprising the amino acid sequence of SEQ ID NO:43; and a VH comprising (i) HVR-H1 comprising the amino acid sequence of SEQ ID NO:44, (ii) HVR-H2 comprising the amino acid sequence of SEQ ID NO:45, and (iii) HVR-H3 comprising the amino acid sequence of SEQ ID NO:46. In some embodiments, the antibody comprises a VL comprising (i) HVR-L1 comprising the amino acid sequence of SEQ ID NO:23, (ii) HVR-L2 comprising the amino acid sequence of SEQ ID NO:24, and (iii) HVR-L3 comprising the amino acid sequence of SEQ ID NO:25; and a VH comprising (i) HVR-H1 comprising the amino acid sequence of SEQ ID NO:26, (ii) HVR-H2 comprising the amino acid sequence of SEQ ID NO:27, and (iii) HVR-H3 comprising the amino acid sequence of SEQ ID NO:28. In some embodiments, the antibody comprises a VL comprising (i) HVR-L1 comprising the amino acid sequence of SEQ ID NO:29, (ii) HVR-L2 comprising the amino acid sequence of SEQ ID NO:30, and (iii) HVR-L3 comprising the amino acid sequence of SEQ ID NO:31; and a VH comprising (i) HVR-H1 comprising the amino acid sequence of SEQ ID NO:32, (ii) HVR-H2 comprising the amino acid sequence of SEQ ID NO:33, and (iii) HVR-H3 comprising the amino acid sequence of SEQ ID NO:34. Further, provided herein are isolated antibodies that bind to RSPO3, wherein the antibody comprises (a) a VL comprising (i) HVR-L1 comprising the amino acid sequence of SEQ ID NO:23, (ii) HVR-L2 comprising the amino acid sequence of SEQ ID NO:24, and (iii) HVR-L3 comprising the amino acid sequence of SEQ ID NO:25; and a VH comprising (i) HVR-H1 comprising the amino acid sequence of SEQ ID NO:26, (ii) HVR-H2 comprising the amino acid sequence of SEQ ID NO:27, and (iii) HVR-H3 comprising the amino acid sequence of SEQ ID NO:188; or (b) a VL comprising (i) HVR-L1 comprising the amino acid sequence of SEQ ID NO:23, (ii) HVR-L2 comprising the amino acid sequence of SEQ ID NO:24, and (iii) HVR-L3 comprising the amino acid sequence of SEQ ID NO:25; and a VH comprising (i) HVR-H1 comprising the amino acid sequence of SEQ ID NO:26, (ii) HVR-H2 comprising the amino acid sequence of SEQ ID NO:27, and (iii) HVR-H3 comprising the amino acid sequence of SEQ ID NO: 189. In some embodiments, the isolated antibody that binds to RSPO comprises a VL comprising (i) HVR-L1 comprising the amino acid sequence of SEQ ID NO:23, (ii) HVR-L2 comprising the amino acid sequence of SEQ ID NO:24, and (iii) HVR-L3 comprising the amino acid sequence of SEQ ID NO:25; and a VH comprising (i) HVR-H1 comprising the amino acid sequence of SEQ ID NO:26, (ii) HVR-H2 comprising the amino acid sequence of SEQ ID NO:27, and (iii) HVR-H3 comprising the amino acid sequence of SEQ ID NO:28. In some embodiments, the isolated antibody that binds to RSPO comprises a VL comprising (i) HVR-L1 comprising the amino acid sequence of SEQ ID NO:23, (ii) HVR-L2 comprising the amino acid sequence of SEQ ID NO:24, and (iii) HVR-L3 comprising the amino acid sequence of SEQ ID NO:25; and a VH comprising (i) HVR-H1 comprising the amino acid sequence of SEQ ID NO:26, (ii) HVR-H2 comprising the amino acid sequence of SEQ ID NO:27, and (iii) HVR-H3 comprising the amino acid sequence of SEQ ID NO: 188. In some embodiments, the isolated antibody that binds to RSPO comprises a VL comprising (i) HVR-L1 comprising the amino acid sequence of SEQ ID NO:23, (ii) HVR-L2 comprising the amino acid sequence of SEQ ID NO:24, and (iii) HVR-L3 comprising the amino acid sequence of SEQ ID NO:25; and a VH comprising (i) HVR-H1 comprising the amino acid sequence of SEQ ID NO:26, (ii) HVR-H2 comprising the amino acid sequence of SEQ ID NO:27, and (iii) HVR-H3 comprising the amino acid sequence of SEQ ID NO:189.

In some embodiments, the antibody comprises (a) a VL sequence of SEQ ID NO:89 and a VH sequence of SEQ ID NO:90; (b) a VL sequence of SEQ ID NO:91 and a VH sequence of SEQ ID NO:92; (c) a VL sequence of SEQ ID NO:93 and a VH sequence of SEQ ID NO:94; (d) a VL sequence of SEQ ID NO:95 and a VH sequence of SEQ ID NO:96; (e) a VL sequence of SEQ ID NO:97 and a VH sequence of SEQ ID NO:98; (f) a VL sequence of SEQ ID NO:99 and a VH sequence of SEQ ID NO: 100; or (g) a VL sequence of SEQ ID NO:101 and a VH sequence of SEQ ID NO:102. In some embodiments, the isolated antibody that binds to RSPO3 comprises (a) a VL sequence of SEQ ID NO:208 and a VH sequence of SEQ ID NO:209, (b) a VL sequence of SEQ ID NO:212 and a VH sequence of SEQ ID NO:213, or (c) a VL sequence of SEQ ID NO:214 and a VH sequence of SEQ ID NO:205. In some embodiments, the isolated antibody that binds to RSPO3 comprises (a) a VL sequence of SEQ ID NO:208 and a VH sequence of SEQ ID NO:209. In some embodiments, the isolated antibody that binds to RSPO3 comprises (a) a VL sequence of SEQ ID NO:212 and a VH sequence of SEQ ID NO:213. In some embodiments, the isolated antibody that binds to RSPO3 comprises (a) a VL sequence of SEQ ID NO:214 and a VH sequence of SEQ ID NO:215.

Provided herein are isolated antibodies that bind to RSPO2 and RSPO3 (anti-RSP02/3 antibody). In some embodiments, the antibody inhibits the interaction of RSPO2 and RSPO3 with a transmembrane E3 ubiquitinase. In some embodiments, the transmembrane E3 ubiquitinase is ZNRF3 and/or RNF43. In some embodiments, the antibody inhibits the interaction of RSP03 with one or more of LGR4, LGR5, and/or LGR6. In some embodiments, the antibody does not inhibit the interaction of RSPO3 with one or more of LGR4, LGR5, and/or LGR6 (*e.g*., enhances binding of RSPO3 to one or more of LGR4, LGR5, and/or LGR6). In some embodiments, the antibody inhibits the interaction of RSP02 with one or more of LGR4, LGR5, and/or LGR6. In some embodiments, the antibody does not inhibit the interaction of RSPO2 with one or more of LGR4, LGR5, and/or LGR6 (e.g., enhances binding of RSPO2 to one or more of LGR4, LGR5, and/or LGR6).

For example, provided herein are antibodies that bind to RSPO2 and RSP03, wherein the antibody comprises: (a) a VL comprising (i) HVR-L1 comprising the amino acid sequence of SEQ ID NO:47, (ii) HVR-L2 comprising the amino acid sequence of SEQ ID NO:48, and (iii) HVR-L3 comprising the amino acid sequence of SEQ ID NO:49; and (b) a VH comprising (i) HVR-H1 comprising the amino acid sequence of SEQ ID NO:50, (ii) HVR-H2 comprising the amino acid sequence of SEQ ID NO:51, and (iii) HVR-H3 comprising the amino acid sequence of SEQ ID NO:52. In some embodiments, the antibody comprises (a) a VL sequence of SEQ ID NO:103 and a VH sequence of SEQ ID NO:104.

In some embodiments of any of the anti-RSPO2/3 antibodies, the antibody comprises a first variable domain and a second variable domain, wherein the first variable domain comprises a first set of six HVRs and the second variable domain comprises a second set of six HVRs, and wherein the first and second set of six HVRs are identical. In some embodiments, the first set of six HVRs and the second set of six HVRs are the six HVRs of 26E11.

In some embodiments of any of the anti-RSPO2/3 antibodies, the antibody comprises the antibody comprises a first variable domain and a second variable domain, wherein the first variable domain comprises a first set of six HVRs and the second variable domain comprises a second set of six HVRs, and wherein the first and second set of six HVRs are different. In some embodiments, the first set of six HVRs are the six HVRs of any one of 4H1, 4D4, 5C2, 5D6, 5E11, 6E9, and 21C2 and the second set of six HVRs are the six HVRs of any one of 1A1, 11F11, 36D2, and 49G5. In some embodiments, the first set of six HVRs are the six HVRs of any one of 4H1, 4D4, 5C2, 5D6, 5E11, 6E9, and 21C2 and the second set of six HVRs are the six HVRs of 1A1.

Provided herein are also isolated antibodies that bind to RSPO3, wherein the antibody comprises: (a) a VL comprising (i) HVR-L1 comprising the amino acid sequence of SEQ ID NO:77, (ii) HVR-L2 comprising the amino acid sequence of SEQ ID NO:78, and (iii) HVR-L3 comprising the amino acid sequence of SEQ ID NO:79; and a VH comprising (i) HVR-H1 comprising the amino acid sequence of SEQ ID NO:80, (ii) HVR-H2 comprising the amino acid sequence of SEQ ID NO:81, and (iii) HVR-H3 comprising the amino acid sequence of SEQ ID NO:82; or (b) a VL comprising (i) HVR-L1 comprising the amino acid sequence of SEQ ID NO:83, (ii) HVR-L2 comprising the amino acid sequence of SEQ ID NO:84, and (iii) HVR-L3 comprising the amino acid sequence of SEQ ID NO:85; and a VH comprising (i) HVR-H1 comprising the amino acid sequence of SEQ ID NO:86, (ii) HVR-H2 comprising the amino acid sequence of SEQ ID NO:87, and (iii) HVR-H3 comprising the amino acid sequence of SEQ ID NO:88. Provided herein are also isolated antibodies that bind to RSPO3, wherein the antibody comprises: (a) a VL comprising (i) HVR-L1 comprising the amino acid sequence of SEQ ID NO:77, (ii) HVR-L2 comprising the amino acid sequence of SEQ ID NO:78, and (iii) HVR-L3 comprising the amino acid sequence of SEQ ID NO:79; and a VH comprising (i) HVR-H1 comprising the amino acid sequence of SEQ ID NO:80, (ii) HVR-H2 comprising the amino acid sequence of SEQ ID NO:81, and (iii) HVR-H3 comprising the amino acid sequence of SEQ ID NO:216.

Provided herein are also isolated antibodies that bind to RSPO3, wherein the antibody binds to a region within amino acids 47-108 (*e.g.,* 49-108) of RSPO3.

Provided herein are also isolated antibodies that bind to an RSPO3 epitope and in some embodiments of any of the antibodies, wherein the RSPO3 epitope comprises amino acid residues of RSPO3: Gln72, Pro90, Asp91, and Lys94. In some embodiments, the RSPO3 epitope comprises amino acids of RSPO3: Asn 52, Leu55, Phe63, Gln72, Tyr89, Pro90, Asp91, Lys94, and Lys97. In some embodiments, the RSPO3 epitope comprises amino acid residues of RSPO3: Ser49, Asn52, Cys54, Leu55, Ser56, Phe63, Leu65, Gln72, Ile73, Gly74, Tyr84, Tyr89, Pro90, Asp91, Ile92, Lys94, Lys97, and Lys108.

Provided herein are also isolated antibodies that bind to an RSPO3 epitope and in some embodiments of any of the antibodies, wherein the RSPO3 epitope comprises amino acids of RSPO3: Thr47, Leu55, Gln72, Pro90, Asp91, and Lys94. In some embodiments, the RSPO3 epitope comprises amino acids of RSPO3: Thr47, Asn52, Leu55, Phe63, Gln72, Tyr89, Pro90, Asp91, Ile92, Lys94, and Lys97. In some embodiments, the RSPO3 epitope comprises amino acid residues of RSPO3: Thr47, Asn52, Cys54, Leu55, Ser56, Phe63, Leu65, Gln72, Tyr84, Tyr89, Pro90, Asp91, Ile92, Asn93, Lys94, Lys97, and Lys108.

Provided herein are also isolated antibodies that bind to an RSPO3 epitope and in some embodiments of any of the antibodies, wherein the RSPO3 epitope comprises one or more amino acids selected from Ser 49, Asn52, Cys54, Leu55, Ser56, Phe63, Leu65, Gln72, Ile73, Gly74, Tyr84, Tyr89, Pro90, Asp91, Ile92, Lys94, and Lys108 of RSPO3. In some embodiments, the RSPO3 epitope comprises amino acid residues of RSP03: Ser 49, Asn52, Cys54, Leu55, Ser56, Phe63, Leu65, Gln72, Ile73, Gly74, Tyr84, Tyr89, Pro90, Asp91, Ile92, Lys94, and Lys108.

Provided herein are also isolated antibodies that bind to an RSPO3 epitope and in some embodiments of any of the antibodies, wherein the RSPO3 epitope comprises one or more amino acids selected from Ser 49, Asn52, Cys54, Leu55, Ser56, Phe63, Leu65, Gln72, Ile73, Gly74, Tyr84, Tyr89, Pro90, Asp91, Ile92, Lys94, Lys97, and Lys108 of RSPO3. In some embodiments, the RSPO3 epitope comprises amino acid residues of RSPO3: Ser 49, Asn52, Cys54, Leu55, Ser56, Phe63, Leu65, Gln72, Ile73, Gly74, Tyr84, Tyr89, Pro90, Asp91, Ile92, Lys94, Lys97, and Lys108.

Provided herein are also isolated antibodies that bind to an RSPO3 epitope, wherein the RSPO3 epitope comprises one or more amino acids selected from Thr47, Asn52, Cys54, Leu55, Ser56, Phe63, Leu65, Gln72, Tyr84, Tyr89, Pro90, Asp91, Ile92, Asn93, Lys94, Lys97, and Lys108 of RSPO3. In some embodiments, the RSPO3 epitope comprises amino acid residues of RSPO3: Thr47, Asn52, Cys54, Leu55, Ser56, Phe63, Leu65, Gln72, Tyr84, Tyr89, Pro90, Asp91, Ile92, Asn93, Lys94, Lys97, and Lys108.

In some embodiments of any of the antibodies, the antibody inhibits the interaction of RSPO2 and RSPO3 with a transmembrane E3 ubiquitinase. In some embodiments, the transmembrane E3 ubiquitinase is ZNRF3 and/or RNF43. In some embodiments, the antibody inhibits the interaction of RSPO3 with one or more of LGR4, LGR5, and/or LGR6.

In some embodiments of any of the anti-RSPO antibodies, the antibody inhibits RSPO2 and/or RSPO3 mediated wnt signaling. In some embodiments of any of the anti-RSPO antibodies, the antibody is an antibody fragment that binds RSPO2 and/or RSPO3. In some embodiments of any of the anti-RSPO antibodies, the antibody fragment inhibits RSPO2 and/or RSPO3 mediated wnt signaling. In some embodiments of any of the anti-RSPO antibodies, the antibody inhibits cancer stem cell growth. In some embodiments of any of the anti-RSPO antibodies, the antibody induces and/or promotes cancer cell (*e.g.,* cancer stem cell) differentiation (*e.g.,* terminal differentiation and/or differentiation into progenitor cell).

In some embodiments of any of the anti-RSPO antibodies, the antibody is a monoclonal antibody. In some embodiments of any of the anti-RSPO antibodies, the antibody is a human, humanized, or chimeric antibody. In some embodiments of any of the anti-RSPO antibodies, the antibody is a full length IgG1 antibody. In some embodiments of any of the anti-RSPO antibodies, the antibody has reduced or depleted effector function. In some embodiments of any of the anti-RSPO antibodies, the anti-RSPO antibody comprises an engineered alanine at amino acid position 297 according to EU numbering convention. In some embodiments of any of the anti-RSPO antibodies, the anti-RSPO antibody comprises an engineered alanine at amino acid position 265 according to EU numbering convention.

In some embodiments of any of the anti-RSPO antibodies, the antibody is for use as a medicament. In some embodiments of any of the anti-RSPO antibodies, the antibody is for use in treating cancer. In some embodiments, the cancer is gastrointestinal cancer, stomach cancer, colon cancer, colorectal cancer, or rectal cancer. In some embodiments, the cancer is characterized by increased expression of one or more RSPO (*e.g*., RSPO2 and/or RSPO3) compared to a reference. In some embodiments, the cancer is characterized by a RSPO translocation (*e.g*., RSPO2 translocation and/or RSPO3 translocation. In some embodiments of any of the anti-RSPO antibodies, the antibody is for use in inhibiting wnt signaling, inhibiting angiogenesis and/or vasculogenesis, and/or inhibiting cell proliferation.

Provided here are also isolated nucleic acids encoding an antibody described herein. Further provided herein are host cells comprising the nucleic acid of an antibody described herein. Provided here in are methods of producing an antibody described herein comprising culturing the host cell comprising the nucleic acid of an antibody described herein so that the antibody is produced. In some embodiments, the method of producing further comprising recovering the antibody from the host cell.

Provided here are immunoconjugates comprising an antibody described herein and a cytotoxic agent.

Further provided herein are pharmaceutical formulations comprising an antibody described herein and a pharmaceutically acceptable carrier. In some embodiments, the pharmaceutical formulation further comprises an additional therapeutic agent. In some embodiments, the additional therapeutic agent is a taxane. In some embodiments, the taxane is paclitaxel or docetaxel. In some embodiments, the additional therapeutic agent is a platinum agent. In some embodiments, the platinum agent is carboplatin, oxaliplatin, and/or cisplatin. In some embodiments, the additional therapeutic agent is a topoisomerase inhibitor. In some embodiments, the topoisomerase inhibitor is irinotecan, topotecan, etoposide, and/or mitoxantrone. In some embodiments, the additional therapeutic agent is folinic acid (*e.g*., Leucovorin). In some embodiments, the additional therapeutic agent is a nucleoside metabolic inhibitor. In some embodiments, the nucleoside metabolic inhibitor is fluorouracil, capecitabine, and/or gemcitabine. In some embodiments, the additional therapeutic agent is folinic acid, 5-fluorouracil, and/or oxaliplatin. In some embodiments, the additional therapeutic agent is 5-fluorouracil and irinotecan. In some embodiments, the additional therapeutic agent is a taxane and platinum agent. In some embodiments, the additional therapeutic agent is paclitaxel and carboplatin. In some embodiments, the additional therapeutic agent is pemetrexate. In some embodiments, the additional therapeutic agent is a hedgehog inhibitor (*e.g*., vismodegib).

Provided herein are uses of an antibody described herein in the manufacture of a medicament for treatment of cancer. In some embodiments, the cancer is gastrointestinal cancer, stomach cancer, colon cancer, colorectal cancer, or rectal cancer. In some embodiments, the cancer is lung cancer. In some embodiments, the cancer is characterized by increased expression of one or more RSPO (*e.g.,* RSPO2 and/or RSPO3) compared to a reference. In some embodiments, the cancer is characterized by a RSPO translocation (e.g., RSPO2 translocation and/or RSPO3 translocation). Further, provided herein are uses of an antibody described herein in the manufacture of a medicament for inhibiting wnt signaling, inhibiting angiogenesis and/or vasculogenesis, and/or inhibiting cell proliferation. In some embodiments, the anti-RSPO antibody is used in combination with an additional therapeutic agent (e.g., administered sequentially or concurrently). In some embodiments, the additional therapeutic agent is a taxane. In some embodiments, the taxane is paclitaxel or docetaxel. In some embodiments, the additional therapeutic agent is a platinum agent. In some embodiments, the platinum agent is carboplatin, oxaliplatin, and/or cisplatin. In some embodiments, the additional therapeutic agent is a topoisomerase inhibitor. In some embodiments, the topoisomerase inhibitor is irinotecan, topotecan, etoposide, and/or mitoxantrone. In some embodiments, the additional therapeutic agent is folinic acid (*e.g*., Leucovorin). In some embodiments, the additional therapeutic agent is a nucleoside metabolic inhibitor. In some embodiments, the nucleoside metabolic inhibitor is fluorouracil, capecitabine, and/or gemcitabine. In some embodiments, the additional therapeutic agent is folinic acid, 5-fluorouracil, and/or oxaliplatin. In some embodiments, the additional therapeutic agent is 5-fluorouracil and irinotecan. In some embodiments, the additional therapeutic agent is a taxane and platinum agent. In some embodiments, the additional therapeutic agent is paclitaxel and carboplatin. In some embodiments, the additional therapeutic agent is pemetrexate. In some embodiments, the additional therapeutic agent is a hedgehog inhibitor (*e.g*., vismodegib).

Provided herein are methods of treating an individual having cancer comprising administering to the individual an effective amount of an antibody described herein. In some embodiments, the cancer is gastrointestinal cancer, stomach cancer, colon cancer, colorectal cancer, or rectal cancer. In some embodiments, the cancer is lung cancer. In some embodiments, the method further comprises administering an additional therapeutic agent to the individual. In some embodiments, the cancer is characterized by increased expression of one or more RSPO (*e.g*., RSPO2 and/or RSPO3) compared to a reference. In some embodiments, the cancer is characterized by a RSPO translocation (*e.g*., RSPO2 translocation and/or RSPO3 translocation). Also provided herein are methods of inhibiting wnt signaling, inhibiting angiogenesis and/or vasculogenesis, and/or inhibiting cell proliferation in an individual comprising administering to the individual an effective amount of an antibody described herein to inhibit wnt signaling, inhibit angiogenesis and/or vasculogenesis, and/or inhibit cell proliferation. In some embodiments, the method comprises administering an additional therapeutic agent. In some embodiments, the additional therapeutic agent is a taxane. In some embodiments, the taxane is paclitaxel or docetaxel. In some embodiments, the additional therapeutic agent is a platinum agent. In some embodiments, the platinum agent is carboplatin, oxaliplatin, and/or cisplatin. In some embodiments, the additional therapeutic agent is a topoisomerase inhibitor. In some embodiments, the topoisomerase inhibitor is irinotecan, topotecan, etoposide, and/or mitoxantrone. In some embodiments, the additional therapeutic agent is folinic acid (*e.g*., Leucovorin). In some embodiments, the additional therapeutic agent is a nucleoside metabolic inhibitor. In some embodiments, the nucleoside metabolic inhibitor is fluorouracil, capecitabine, and/or gemcitabine. In some embodiments, the additional therapeutic agent is folinic acid, 5-fluorouracil, and/or oxaliplatin. In some embodiments, the additional therapeutic agent is 5-fluorouracil and irinotecan. In some embodiments, the additional therapeutic agent is a taxane and platinum agent. In some embodiments, the additional therapeutic agent is paclitaxel and carboplatin. In some embodiments, the additional therapeutic agent is pemetrexate. In some embodiments, the additional therapeutic agent is a hedgehog inhibitor (*e.g*., vismodegib).

### BRIEF DESCRIPTION OF THE FIGURES

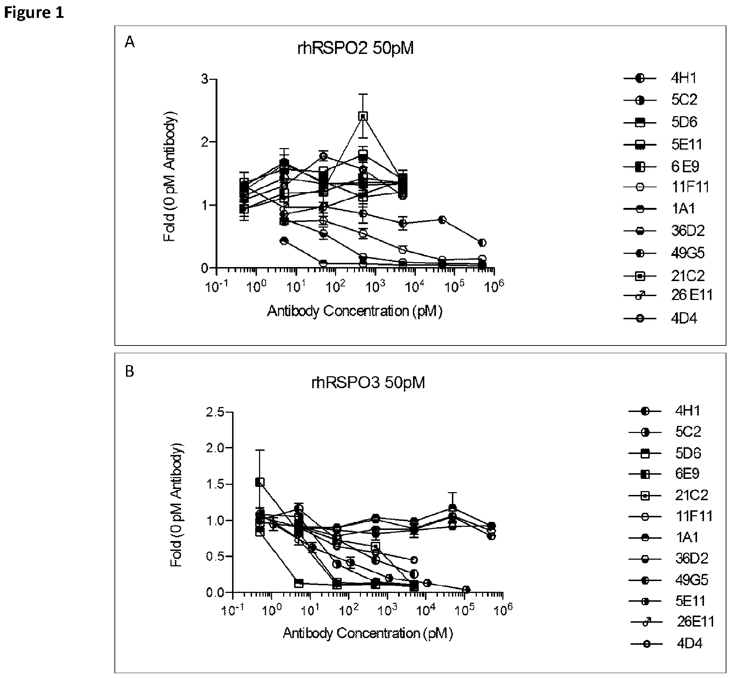

**Figure 1A-B**. A panel of anti-RSPO2 and anti-RSPO3 antibodies were tested for ability to block recombinant human (rh) RSPO2-stimulated (A) and/or rhRSPO3-stimulated (B) WNT reporter activity. A subset of the antibodies block rhRSPO2- and/or rhRSPO3-stimulated WNT reporter activity. WNT reporter cells were stimulated with 10 ng/ml recombinant mouse (rm)Wnt3a, 50 pM rhRSPO2 (A) or rhRSPO3 (B), and increasing concentrations of the indicated antibody clones. Data were normalized to the amount of stimulation present in the absence of antibody.

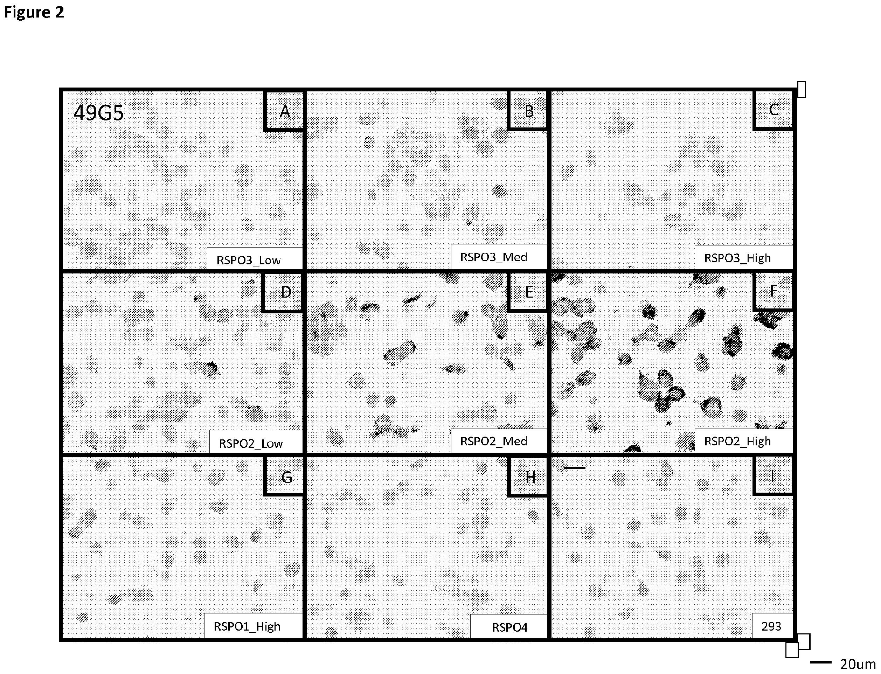

**Figure 2A-I**. A panel of anti-RSPO2 and anti-RSPO3 antibodies were tested for IHC reactive to RSPO3-expressing cell pellets (A-C), RSPO2-expressing cell pellets (D-F), RSPO1-expressing cell pellet (G), RSPO4-expressing cell pellet (H), and non-RSPO1-4 expressing cells (293 cells) (I). As shown in Figure 2, the antibody 49G5 recognized as determined by IHC reactivity to RSPO2-expressing cell pellets, while not recognizing RSPO3, RSPO1, RSPO4, and non-RSPO1-4 expressing cell pellets. A complete table of antibodies tested for IHC reactivity is shown in Table 4. All tested antibodies in Table 4 did not recognize as determined by IHC reactivity RSPO1, RSPO4, and non-RSPO1-4 expressing cell pellets.

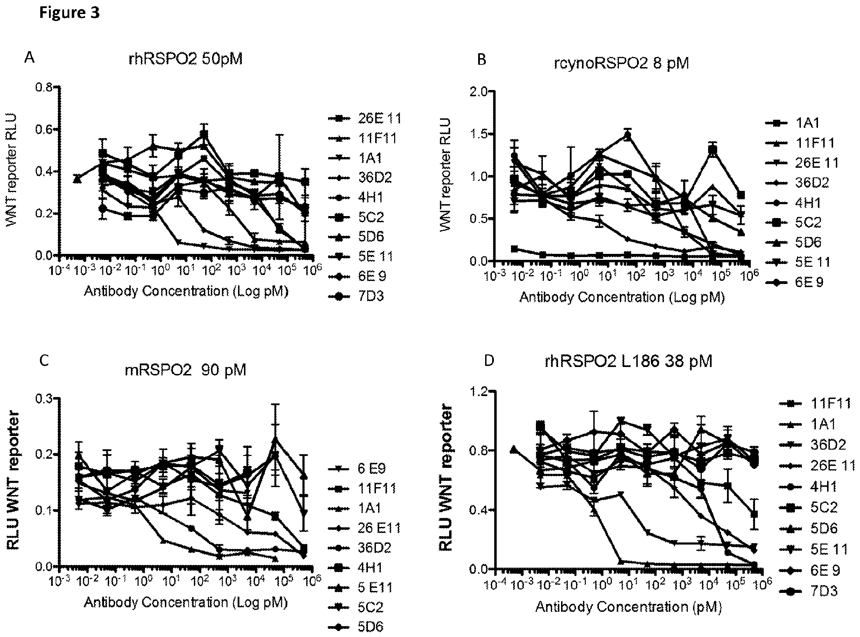

**Figure 3A-D**. A panel of anti-RSPO2 and anti-RSP03 antibodies were tested for ability to inhibit rhRSPO2 (A), recombinant cynomolgus (rcyno) RSPO2 (B), mouse (m) RSPO2 (C), and rhRSPO2 L186P variant (D) stimulation of wnt reporter activity. WNT reporter cells were stimulated with 10 ng/ml rmWnt3a, either 50 pM rhRSPO2 (A), 8 pM rcynoRSPO2 (Genentech) (B), 90 pM mRSPO2 (R&D Systems) (C), or 38 pM rhRSPO2 L186P (Genentech) (D) and increasing concentrations of the indicated antibody clones.

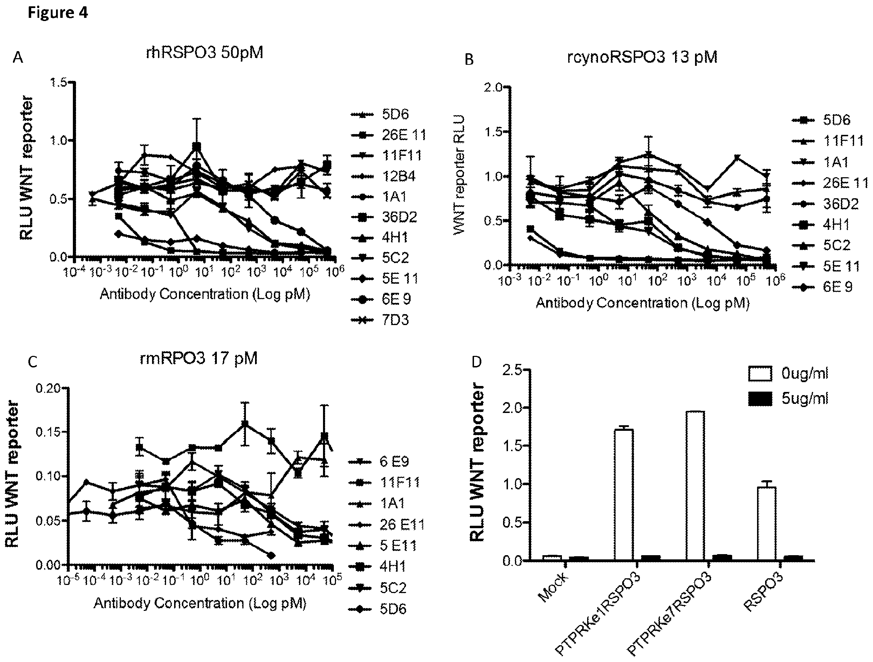

**Figure 4A-D**. A panel of anti-RSPO2 and anti-RSP03 antibodies were tested for ability to inhibit WNT reporter activity stimulated by rhRSPO3 (A), rcynoRSPO3 (B), mRSPO3 (C), and PTPRK fusion-RSPO3 (D). WNT reporter cells were stimulated with 10 ng/ml rmWnt3a, either 50 pM rhRSPO3 (A), 13 pM cynoRSPO3 (Genentech) (B), or 17 pM mRSPO3 (R&D Systems) (C) and increasing concentrations of the indicated antibody clones. In Figure 4D, WNT reporter cells were stimulated with 10 ng/ml rmWNT3a, conditioned media prepared from 293T cells transfected with the indicated DNA, in the absence or presence of anti-RSPO3 at 5 ug/ml.

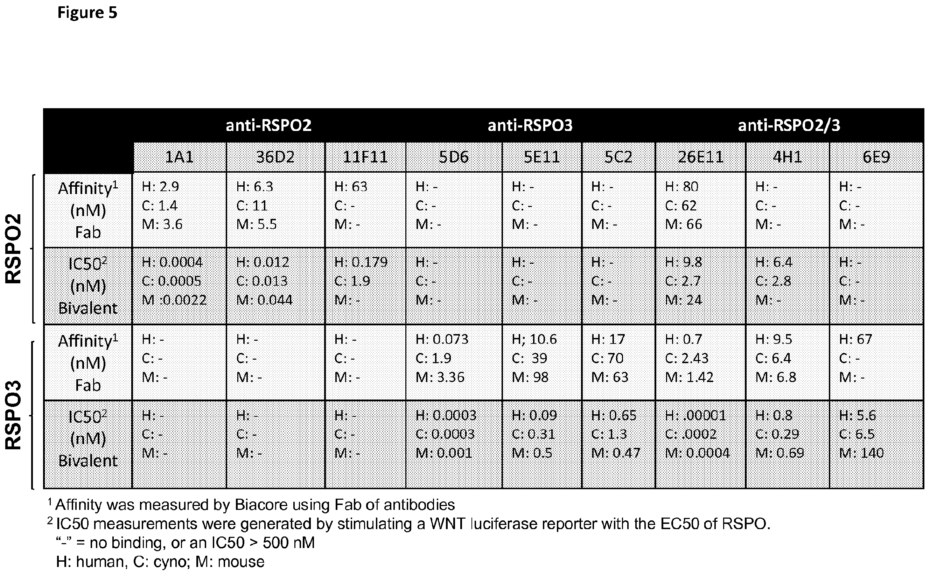

**Figure 5****.** Affinities and IC50 measurements of nine anti-RSPO2 and anti-RSPO3 clones. The affinity of the Fab of the indicated clones for the indicated recombinant (r) RSPO2 and rRSPO3 was determined by Surface Plasmon Resonance. The IC50 measurements of the indicated clone was determined by stimulating a WNT reporter assay with the EC50 of the indicated rRSPO and increasing concentrations of each antibody. H, human; C, cynomolgus; M, mouse; -, no binding or IC50 > 500 nM.

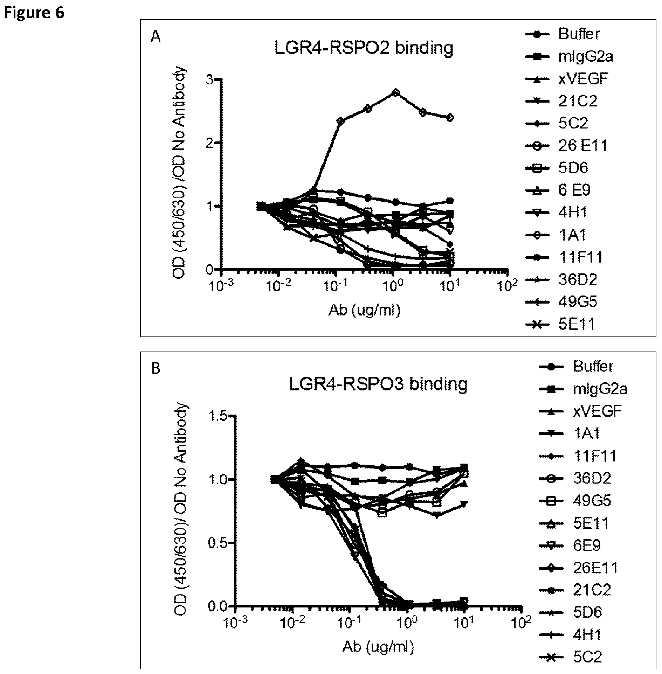

**Figure 6A-B**. A panel of anti-RSPO2 and anti-RSP03 antibodies were tested for their ability to inhibit LGR4 binding to rhRSPO2 (A) and rhRSPO3 (B). Individual antibody clones were tested for the ability to inhibit the binding of either LGR4-ECD to rhRSPO2 (A) or rhRSPO3 (B) by competitive binding ELISA. Similar results were seen with LGR5 (data not shown). *See* Table 5 for a summary of the results.

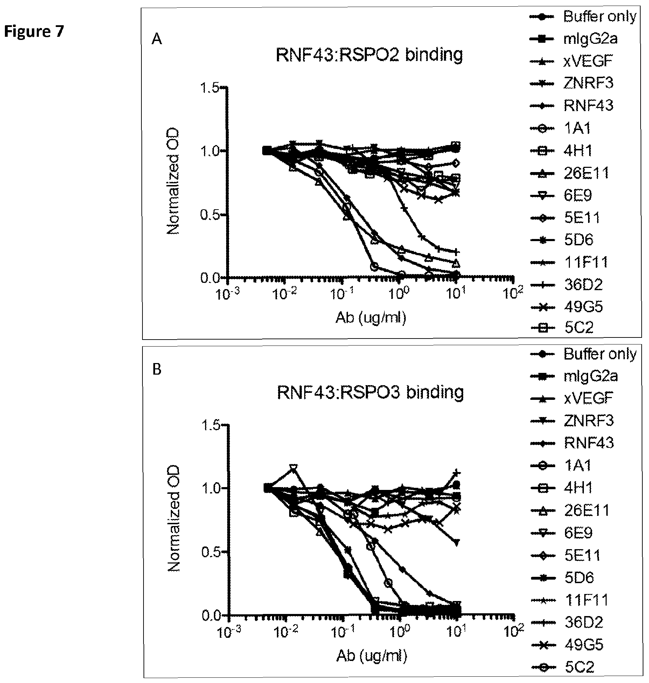

**Figure 7A-B**. A panel of anti-RSPO2 and anti-RSP03 antibodies were tested for their ability to inhibit RNF43 binding to rhRSPO2 (A) and rhRSPO3 (B). Individual antibody clones were tested for the ability to inhibit the binding of RNF43-ECD to rhRSPO2 (A) or rhRSPO3 (B) by competitive binding ELISA. Similar results were seen with LGR5 (data not shown). *See* Table 5 for a summary of the results.

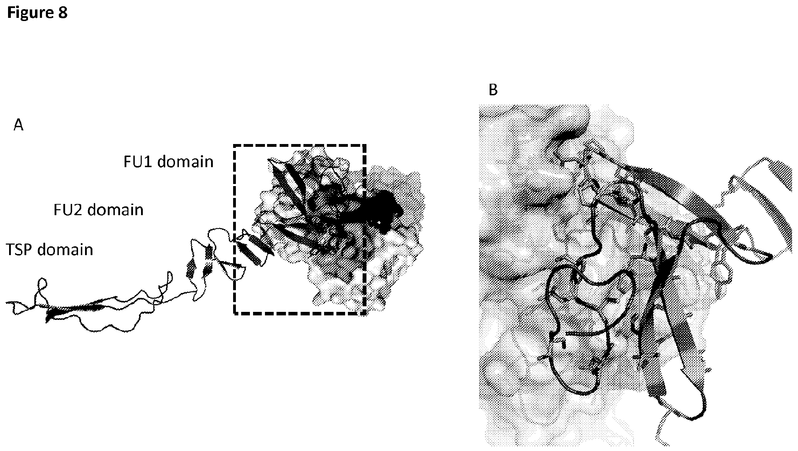

**Figure 8A-B**. Model of crystallized RSPO3 (33-210) in complex with Fab 26E11 (A). An enlargement of the Fab26E11/RSPO3 interaction is shown in (B).

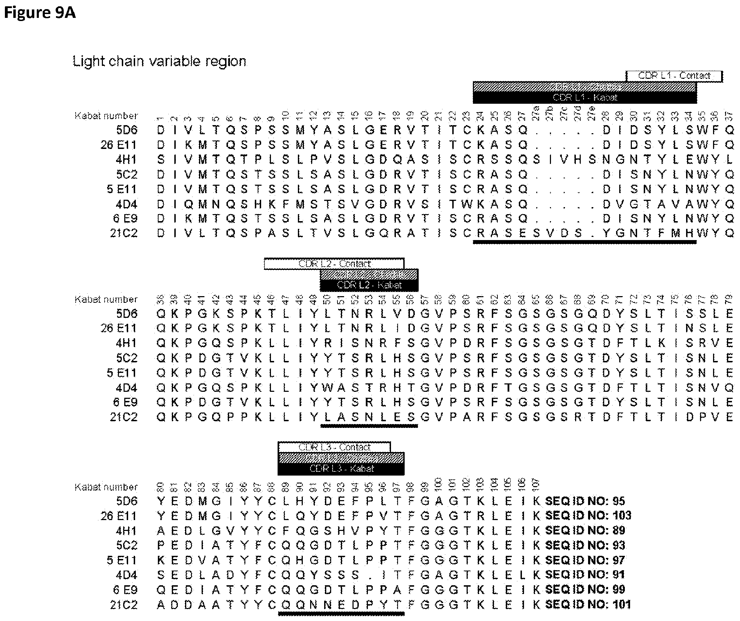

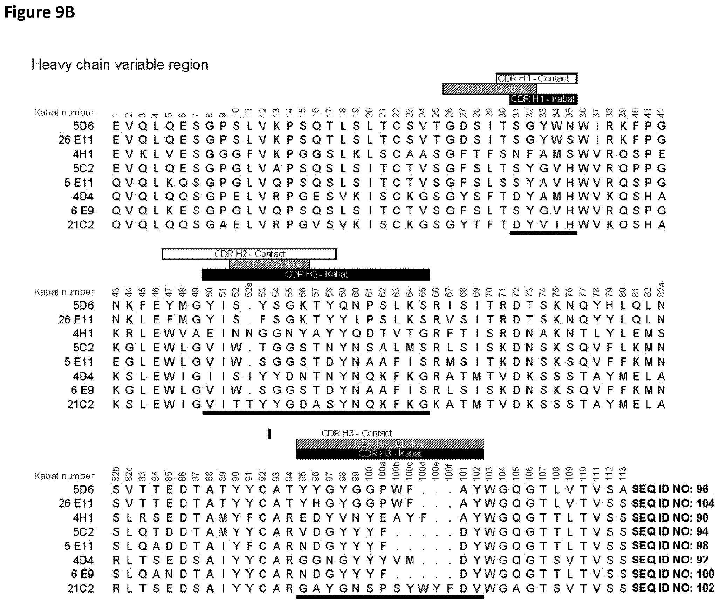

**Figure 9A-B**. Alignment of variable light chain region sequences (A) and variable heavy chain region sequences (B) of 5D6, 26E11, 4H1, 5C2, 5E11, 4D4, 6E9 and 21C2. CDR sequences according to Kabat definition are underlined.

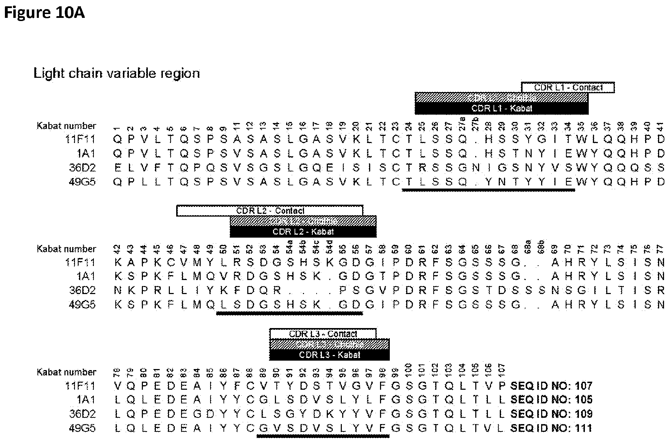

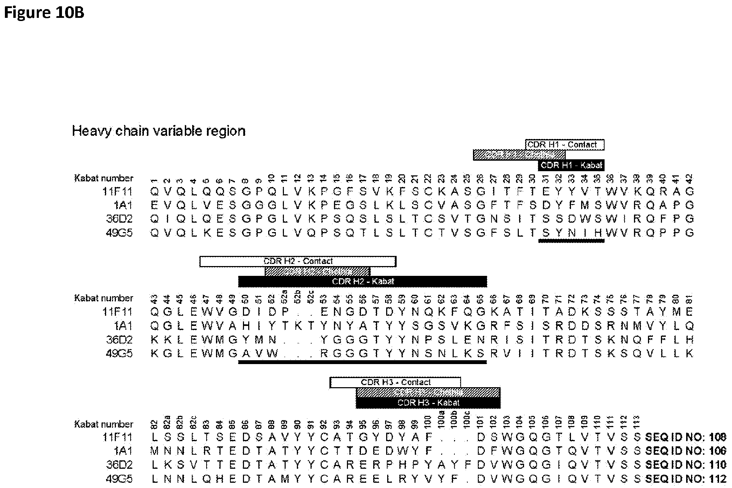

**Figure 10A-B**. Alignment of variable light chain region sequences (A) and variable heavy chain region sequences (B) of 11F11, 1A1, 36D2, and 49G5. CDR sequences according to Kabat definition are underlined.

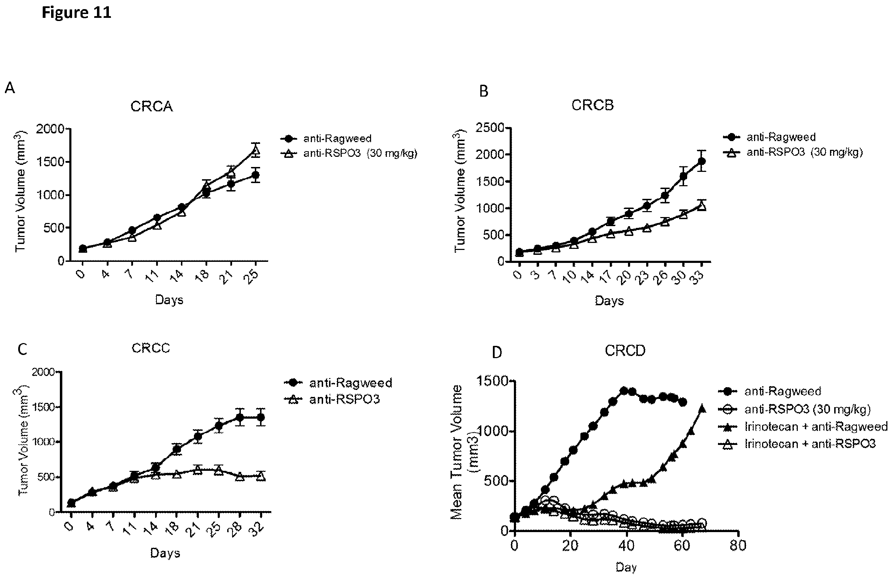

**Figure 11A-D**. Change in mean tumor volume (mm³) of four colorectal cancer patient derived models (A-D) upon treatment with anti-RSPO3 antibody (5D6) at 30 mg/kg or anti-Ragweed antibody (control). Figure 11D also shows change in mean tumor volume (mm³) of CRCD colorectal cancer patient derived model upon treatment with anti-RSPO3 antibody (5D6) in combination with Irinotecan (100 mg/kg, day 0 and day 3) or anti-Ragweed antibody (control) and Irinotecan (100 mg/kg, day 0 and day 3).

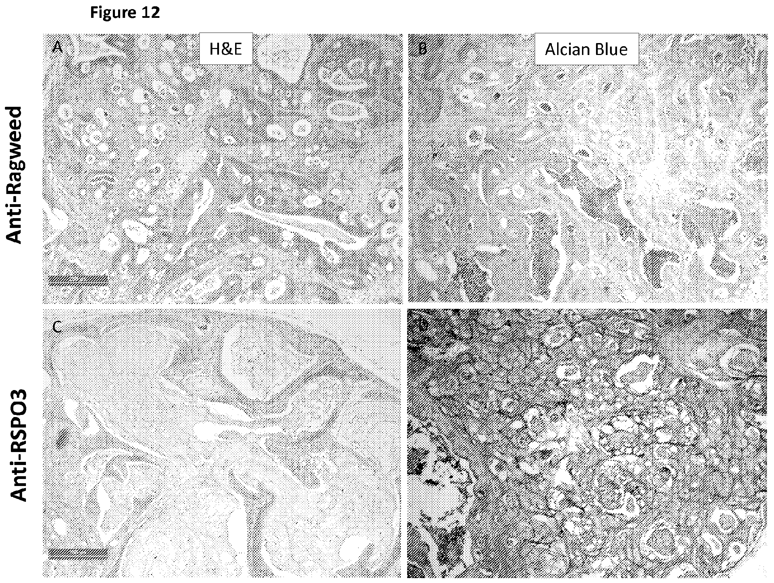

**Figure 12A-D**. Staining of anti-Ragweed antibody (control) or anti-RSPO3 antibody (5D6) at 30 mg/kg treated colorectal cancer patient derived model tumors 2-3 weeks after last dose with hematoxylin and eosin stain (H&E stain) (A and C) and Alcian Blue stain (B and D). anti-RSPO3 treated tumors have a distinct histopathology, in particular a significant increase in mucous as indicated by Alcian Blue staining compared to the anti-Ragweed antibody control.

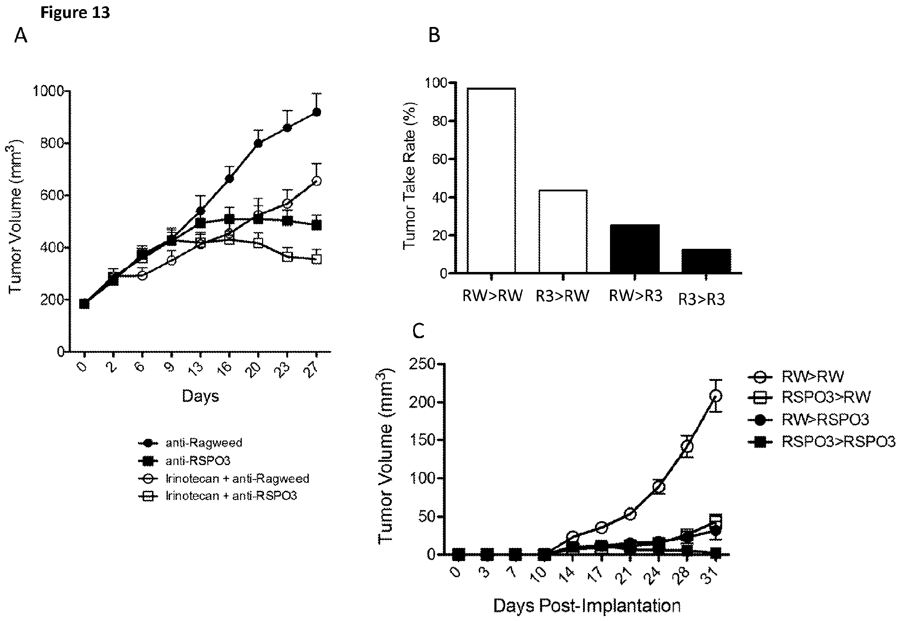

**Figure 13A-C****.** (A) shows mean tumor volume (mm³) of CRCC colorectal cancer patient derived models upon treatment with (i) anti-RSPO3 antibody (5D6), (ii) anti-Ragweed antibody (control), (iii) anti-RSPO3 antibody (5D6) in combination with Irinotecan (100 mg/kg, Day 0), or anti-Ragweed antibody (control) and Irinotecan (100 mg/kg, Day 0). (B-C) shows serial transplant experiments in which colorectal cancer patient derived models were (a) treated with either anti-RSPO3 antibody (5D6) or anti-Ragweed antibody (control; 30 mg/kg) and (b) transplanted and treated with anti-RSPO3 antibody (5D6) or anti-Ragweed antibody (control). (B) shows a substantial reduction in percentage tumor (transplant) engraftment rate of serial transplanted tumors upon treatment with anti-RSPO3 antibody (5D6) either initially or at the time of serial transplant. (C) shows a significant decrease in change mean tumor volume of serial transplanted tumors upon treatment with anti-RSPO3 antibody (5D6) either initially or at the time of serial transplant.

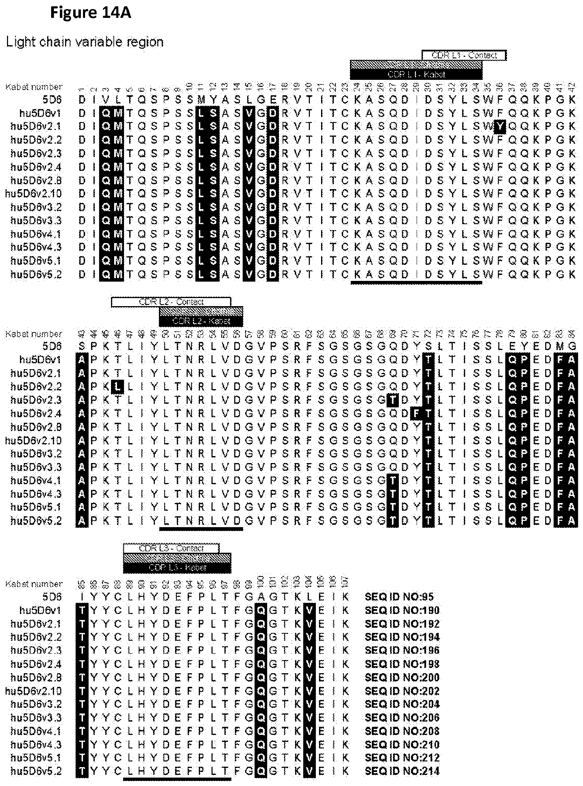

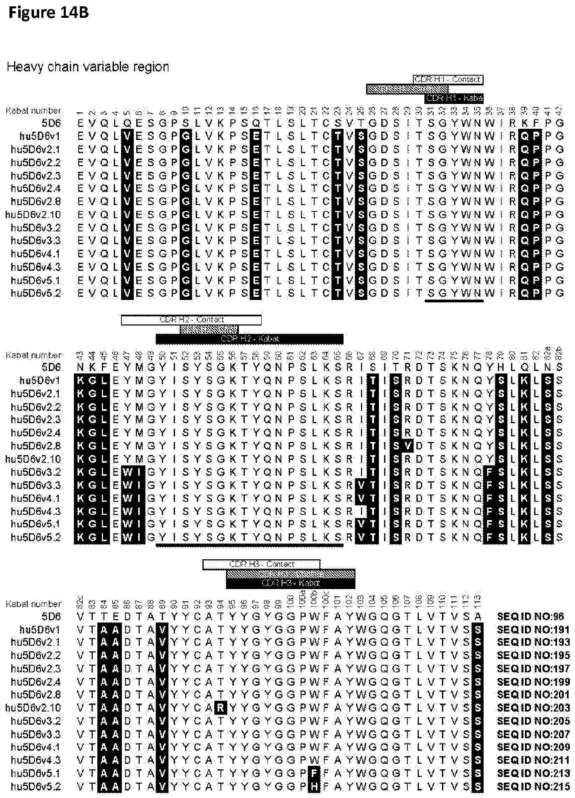

**Figure 14A-B**. Alignment of variable light chain region sequences (A) and variable heavy chain region sequences (B) of 5D6, 5D6v1, 5D6v2.1, 5D6v2.2, 5D6v2.3, 5D6v2.4, 5D6v2.8, 5D6v2.10, 5D6v3.2, 5D6v3.3, 5D6v4.1, 5D6v4.3, 5D6v5.1, and 5D6v5.2. CDR sequences according to Kabat definition are underlined.

### DETAILED DESCRIPTION

### I. DEFINITIONS

The terms "R-spondin" and "RSPO," as used herein, refer to any native RSPO (*e.g.,* RSPO1, RSPO2, RSPO3, and/or RSPO4) from any vertebrate source, including mammals such as primates (*e.g.,* humans) and rodents (*e.g.,* mice and rats), unless otherwise indicated. The term encompasses "full-length," unprocessed RSPO as well as any form of RSPO that results from processing in the cell. The term also encompasses naturally occurring variants of RSPO, *e.g.,* splice variants or allelic variants. In some embodiments, the amino acid sequence of an exemplary human RSPO is RSPO1, for example, as shown in SEQ ID NO:3. In some embodiments, the amino acid sequence of an exemplary human RSPO is RSPO2, for example, as shown in SEQ ID NO:1. In some embodiments, the amino acid sequence of an exemplary human RSPO is RSPO3, for example, as shown in SEQ ID NO:2. In some embodiments, the amino acid sequence of an exemplary human RSPO is RSPO4, for example, as shown in SEQ ID NO:4.

The terms "R-spondin 2" and "RSPO2," as used herein, refers to any native RSPO2 from any vertebrate source, including mammals such as primates (*e.g.,* humans) and rodents (*e.g.,* mice and rats), unless otherwise indicated. The term encompasses "full-length," unprocessed RSPO2 as well as any form of RSPO2 that results from processing in the cell. The term also encompasses naturally occurring variants of RSPO2, *e.g*., splice variants or allelic variants. In some embodiments, the amino acid sequence of an exemplary human RSPO2 is UNIPROT Q6UXX9-1 as of October 18, 2013. In some embodiments, the amino acid sequence of an exemplary human RSPO2 is UNIPROT Q6UXX9-2 as of October 18, 2013. In some embodiments, the amino acid sequence of an exemplary human RSPO2 is UNIPROT Q6UXX9-3 as of October 18, 2013. In some embodiments, the amino acid sequence of an exemplary human RSPO2 is shown in SEQ ID NO:1.

The terms "R-spondin 3" and "RSPO3," as used herein, refers to any native RSPO3 from any vertebrate source, including mammals such as primates (*e.g.,* humans) and rodents (*e.g.,* mice and rats), unless otherwise indicated. The term encompasses "full-length," unprocessed RSPO3 as well as any form of RSPO3 that results from processing in the cell. The term also encompasses naturally occurring variants of RSPO3, *e.g*., splice variants or allelic variants. In some embodiments, the amino acid sequence of an exemplary human RSPO2 is UNIPROT Q9BXY4-1 as of October 18, 2013. In some embodiments, the amino acid sequence of an exemplary human RSPO2 is UNIPROT Q9BXY4-2 as of October 18, 2013. In some embodiments, the amino acid sequence of an exemplary human RSPO3 is shown in SEQ ID NO:2.

An "acceptor human framework" for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In some embodiments, the number of amino acid changes are 10 or less, 9 or less, 8 or less, 7 or less, 6 or less, 5 or less, 4 or less, 3 or less, or 2 or less. In some embodiments, the VL acceptor human framework is identical in sequence to the VL human immunoglobulin framework sequence or human consensus framework sequence.

"Affinity" refers to the strength of the sum total of noncovalent interactions between a single binding site of a molecule (*e.g.,* an antibody) and its binding partner (*e.g.,* an antigen). Unless indicated otherwise, as used herein, "binding affinity" refers to intrinsic binding affinity which reflects a 1:1 interaction between members of a binding pair (*e.g*., antibody and antigen). The affinity of a molecule X for its partner Y can generally be represented by the dissociation constant (Kd). Affinity can be measured by common methods known in the art, including those described herein. Specific illustrative and exemplary embodiments for measuring binding affinity are described in the following.

An "affinity matured" antibody refers to an antibody with one or more alterations in one or more hypervariable regions (HVRs), compared to a parent antibody which does not possess such alterations, such alterations resulting in an improvement in the affinity of the antibody for antigen.

The terms "anti-RSPO2 antibody" and "an antibody that binds to RSPO2" refer to an antibody that is capable of binding RSPO2 with sufficient affinity such that the antibody is useful as a diagnostic and/or therapeutic agent in targeting RSPO2. In one embodiment, the extent of binding of an anti-RSPO2 antibody to a non-RSP02 protein is less than about 10% of the binding of the antibody to RSPO2 as measured, *e.g*., by a radioimmunoassay (RIA). In certain embodiments, an antibody that binds to RSPO2 has a dissociation constant (Kd) of ≤ 1µM, ≤ 100 nM, ≤ 10 nM, ≤ 1 nM, ≤ 0.1 nM, ≤ 0.01 nM, or ≤ 0.001 nM (*e.g.,* 10⁻⁸ M or less, *e.g.* from 10⁻⁸ M to 10⁻¹³ M, *e.g.,* from 10⁻⁹ M to 10⁻¹³ M). In certain embodiments, an anti-RSPO2 antibody binds to an epitope of RSPO2 that is conserved among RSPO2 from different species.

The terms "anti-RSPO3 antibody" and "an antibody that binds to RSPO3" refer to an antibody that is capable of binding RSPO3 with sufficient affinity such that the antibody is useful as a diagnostic and/or therapeutic agent in targeting RSPO3.In one embodiment, the extent of binding of an anti-RSPO3 antibody to a non-RSP03 protein is less than about 10% of the binding of the antibody to RSPO3 as measured, *e.g*., by a radioimmunoassay (RIA). In certain embodiments, an antibody that binds to RSPO3 has a dissociation constant (Kd) of ≤ 1µM, ≤ 100 nM, ≤ 10 nM, ≤ 1 nM, ≤ 0.1 nM, ≤ 0.01 nM, or ≤ 0.001 nM (*e.g.,* 10⁻⁸ M or less, *e.g.* from 10⁻⁸ M to 10⁻¹³ M, *e.g.,* from 10⁻⁹ M to 10⁻¹³ M). In certain embodiments, an anti-RSPO3 antibody binds to an epitope of RSPO3 that is conserved among RSPO3 from different species.

The terms "anti-RSPO2/3 antibody" and "an antibody that binds to RSPO2 and RSPO3" refer to an antibody that is capable of binding RSPO2 and RSPO3 with sufficient affinity such that the antibody is useful as a diagnostic and/or therapeutic agent in targeting RSPO2 and RSPO3. In one embodiment, the extent of binding of an anti-RSPO2/3 antibody to a non-RSPO2 or non-RSPO3 protein is less than about 10% of the binding of the antibody to RSPO2 and RSPO3 as measured, e.g., by a radioimmunoassay (RIA). In certain embodiments, an antibody that binds to RSPO2 and RSPO3 has a dissociation constant (Kd) of ≤ 1µM, ≤ 100 nM, ≤ 10 nM, ≤ 1 nM, ≤ 0.1 nM, ≤ 0.01 nM, or ≤ 0.001 nM (*e.g.,* 10⁻⁸ M or less, *e.g.* from 10⁻⁸ M to 10⁻¹³ M, *e.g*., from 10⁻⁹ M to 10⁻¹³ M). In certain embodiments, an anti-RSPO2/3 antibody binds to an epitope of RSPO2 and/or RSPO3 that is conserved among RSPO2 and/or RSPO3 from different species.

The term "antibody" herein is used in the broadest sense and encompasses various antibody structures, including but not limited to monoclonal antibodies, polyclonal antibodies, multispecific antibodies (*e.g*., bispecific antibodies), and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; single-chain antibody molecules (*e*.*g*,. scFv); and multispecific antibodies formed from antibody fragments.

An "antibody that competes for binding with" a reference antibody refers to an antibody that blocks binding of the reference antibody to its antigen in a competition assay by 50% or more, and conversely, the reference antibody blocks binding of the antibody to its antigen in a competition assay by 50% or more. An exemplary competition assay is provided herein.

The term "chimeric" antibody refers to an antibody in which a portion of the heavy and/or light chain is derived from a particular source or species, while the remainder of the heavy and/or light chain is derived from a different source or species.

The "class" of an antibody refers to the type of constant domain or constant region possessed by its heavy chain. There are five major classes of antibodies: IgA, IgD, IgE, IgG, and IgM, and several of these may be further divided into subclasses (isotypes), *e.g*., IgG₁, IgG₂, IgG₃, IgG₄, IgA₁, and IgA₂. The heavy chain constant domains that correspond to the different classes of immunoglobulins are called α, δ, ε, γ, and µ, respectively.

"Effector functions" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (*e.g.* B cell receptor); and B cell activation.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The terms "full length antibody," "intact antibody," and "whole antibody" are used herein interchangeably to refer to an antibody having a structure substantially similar to a native antibody structure or having heavy chains that contain an Fc region as defined herein.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., Sequences of Proteins of Immunological Interest, Fifth Edition, NIH Publication 91-3242, Bethesda MD (1991), vols. 1-3. In one embodiment, for the VL, the subgroup is subgroup kappa I as in Kabat et al., *supra.* In one embodiment, for the VH, the subgroup is subgroup III as in Kabat et al., *supra.*

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, *e.g*., a non-human antibody, refers to an antibody that has undergone humanization.

The term "hypervariable region" or "HVR" as used herein refers to each of the regions of an antibody variable domain which are hypervariable in sequence ("complementarity determining regions" or "CDRs") and/or form structurally defined loops ("hypervariable loops") and/or contain the antigen-contacting residues ("antigen contacts"). Generally, antibodies comprise six HVRs: three in the VH (H1, H2, H3), and three in the VL (L1, L2, L3). Exemplary HVRs herein include:
(a) hypervariable loops occurring at amino acid residues 26-32 (L1), 50-52 (L2), 91-96 (L3), 26-32 (HI), 53-55 (H2), and 96-101 (H3) (Chothia and Lesk, J. Mol. Biol. 196:901-917 (1987));
(b) CDRs occurring at amino acid residues 24-34 (L1), 50-56 (L2), 89-97 (L3), 31-35b (HI), 50-65 (H2), and 95-102 (H3) (Kabat et al., Sequences of Proteins of Immunological Interest, 5th Ed. Public Health Service, National Institutes of Health, Bethesda, MD (1991));
(c) antigen contacts occurring at amino acid residues 27c-36 (L1), 46-55 (L2), 89-96 (L3), 30-35b (HI), 47-58 (H2), and 93-101 (H3) (MacCallum et al. J. Mol. Biol. 262: 732-745 (1996)); and
(d) combinations of (a), (b), and/or (c), including HVR amino acid residues 46-56 (L2), 47-56 (L2), 48-56 (L2), 49-56 (L2), 26-35 (H1), 26-35b (H1), 49-65 (H2), 93-102 (H3), and 94-102 (H3).

In one embodiment, HVR residues comprise those identified in Figures 9A-B and/or Figures 10A-B or elsewhere in the specification.

Unless otherwise indicated, HVR residues and other residues in the variable domain (e.g., FR residues) are numbered herein according to Kabat et al., *supra.*

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (*See, e.g.,* Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. *See, e.g.,* Portolano et al., J. Immunol. 150:880-887 (1993); Clarkson et al., Nature 352:624-628 (1991).

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

An "immunoconjugate" is an antibody conjugated to one or more heterologous molecule(s), including but not limited to a cytotoxic agent.

An "isolated" antibody is one which has been separated from a component of its natural environment. In some embodiments, an antibody is purified to greater than 95% or 99% purity as determined by, for example, electrophoretic (*e.g*., SDS-PAGE, isoelectric focusing (IEF), capillary electrophoresis) or chromatographic (*e.g*., ion exchange or reverse phase HPLC). For review of methods for assessment of antibody purity, *see, e.g.,* Flatman et al., J. Chromatogr. B 848:79-87 (2007).

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Isolated nucleic acid encoding an anti-RSPO2 antibody" refers to one or more nucleic acid molecules encoding antibody heavy and light chains (or fragments thereof), including such nucleic acid molecule(s) in a single vector or separate vectors, and such nucleic acid molecule(s) present at one or more locations in a host cell.

"Isolated nucleic acid encoding an anti-RSPO3 antibody" refers to one or more nucleic acid molecules encoding antibody heavy and light chains (or fragments thereof), including such nucleic acid molecule(s) in a single vector or separate vectors, and such nucleic acid molecule(s) present at one or more locations in a host cell.

The term "monoclonal antibody" as used herein refers to an antibody obtained from a population of substantially homogeneous antibodies, *i.e.,* the individual antibodies comprising the population are identical and/or bind the same epitope, except for possible variant antibodies, *e.g*., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

A "naked antibody" refers to an antibody that is not conjugated to a heterologous moiety (*e.g*., a cytotoxic moiety) or radiolabel. The naked antibody may be present in a pharmaceutical formulation.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CH1, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa (κ) and lambda (λ), based on the amino acid sequence of its constant domain.

The term "package insert" is used to refer to instructions customarily included in commercial packages of therapeutic products, that contain information about the indications, usage, dosage, administration, combination therapy, contraindications and/or warnings concerning the use of such therapeutic products.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The terms "R-spondin translocation" and "RSPO translocation" refer herein to an R-spondin wherein a portion of a broken chromosome including, for example, R-spondin, variant, or fragment thereof or a second gene, variant, or fragment thereof, reattaches in a different chromosome location, for example, a chromosome location different from R-spondin native location or a chromosome location in and/or around the R-spondin native location which is different from the second gene's native location. The R-spondin translocation may be a RSPO1 translocation, RSPO2 translocation, RSPO3 translocation, and/or RSPO4 translocation.

The terms "R-spondin-translocation fusion polynucleotide" and "RSPO-translocation fusion polynucleotide" refer herein to the nucleic acid sequence of an R-spondin translocation gene product or fusion polynucleotide. The R-spondin-translocation fusion polynucleotide may be a RSPO1-translocation fusion polynucleotide, RSPO2-translocation fusion polynucleotide, RSPO3-translocation fusion polynucleotide, and/or RSPO4-translocation fusion polynucleotide. The terms "R-spondin-translocation fusion polypeptide" and "RSPO-translocation fusion polypeptide" refer herein to the amino acid sequence of an R-spondin translocation gene product or fusion polynucleotide. The R-spondin-translocation fusion polypeptide may be a RSPO1-translocation fusion polypeptide, RSPO2-translocation fusion polypeptide, RSP03-translocation fusion polypeptide, and/or RSPO4-translocation fusion polypeptide.

The term "detection" includes any means of detecting, including direct and indirect detection.

The term "biomarker" as used herein refers to an indicator, *e.g*., a predictive, diagnostic, and/or prognostic indicator, which can be detected in a sample. The biomarker may serve as an indicator of a particular subtype of a disease or disorder (*e.g*., cancer) characterized by certain, molecular, pathological, histological, and/or clinical features. In some embodiments, the biomarker is a gene. In some embodiments, the biomarker is a variation (*e.g*., mutation and/or polymorphism) of a gene. In some embodiments, the biomarker is a translocation. Biomarkers include, but are not limited to, polynucleotides (*e.g*., DNA, and/or RNA), polypeptides, polypeptide and polynucleotide modifications (*e.g*., posttranslational modifications), carbohydrates, and/or glycolipid-based molecular markers.

The "presence," "amount," or "level" of a biomarker associated with an increased clinical benefit to an individual is a detectable level in a sample. These can be measured by methods known to one skilled in the art and also disclosed herein. The expression level or amount of biomarker assessed can be used to determine the response to the treatment.

The terms "level of expression" or "expression level" in general are used interchangeably and generally refer to the amount of a biomarker in a sample. "Expression" generally refers to the process by which information (*e.g*., gene-encoded and/or epigenetic) is converted into the structures present and operating in the cell. Therefore, as used herein, "expression" may refer to transcription into a polynucleotide, translation into a polypeptide, or even polynucleotide and/or polypeptide modifications (*e.g*., posttranslational modification of a polypeptide). Fragments of the transcribed polynucleotide, the translated polypeptide, or polynucleotide and/or polypeptide modifications (*e.g*., posttranslational modification of a polypeptide) shall also be regarded as expressed whether they originate from a transcript generated by alternative splicing or a degraded transcript, or from a posttranslational processing of the polypeptide, *e.g*., by proteolysis. "Expressed genes" include those that are transcribed into a polynucleotide as mRNA and then translated into a polypeptide, and also those that are transcribed into RNA but not translated into a polypeptide (for example, transfer and ribosomal RNAs).

"Elevated expression," "elevated expression levels," or "elevated levels" refers to increased expression or increased levels of a biomarker in an individual relative to a control, such as an individual or individuals who are not suffering from the disease or disorder (*e.g*., cancer) or an internal control (*e.g*., housekeeping biomarker).

"Reduced expression," "reduced expression levels," or "reduced levels" refers to decrease expression or decreased levels of a biomarker in an individual relative to a control, such as an individual or individuals who are not suffering from the disease or disorder (*e.g*., cancer) or an internal control (*e.g*., housekeeping biomarker).

The term "housekeeping biomarker" refers to a biomarker or group of biomarkers (*e.g*., polynucleotides and/or polypeptides) which are typically similarly present in all cell types. In some embodiments, the housekeeping biomarker is a "housekeeping gene." A "housekeeping gene" refers herein to a gene or group of genes which encode proteins whose activities are essential for the maintenance of cell function and which are typically similarly present in all cell types.

"Amplification," as used herein generally refers to the process of producing multiple copies of a desired sequence. "Multiple copies" mean at least two copies. A "copy" does not necessarily mean perfect sequence complementarity or identity to the template sequence. For example, copies can include nucleotide analogs such as deoxyinosine, intentional sequence alterations (such as sequence alterations introduced through a primer comprising a sequence that is hybridizable, but not complementary, to the template), and/or sequence errors that occur during amplification.

The term "diagnosis" is used herein to refer to the identification or classification of a molecular or pathological state, disease or condition (*e.g*., cancer). For example, "diagnosis" may refer to identification of a particular type of cancer. "Diagnosis" may also refer to the classification of a particular subtype of cancer, *e.g.,* by histopathological criteria, or by molecular features (*e.g.,* a subtype characterized by expression of one or a combination of biomarkers (*e.g*., particular genes or proteins encoded by said genes)).

Samples include, but are not limited to, primary or cultured cells or cell lines, cell supernatants, cell lysates, platelets, serum, plasma, vitreous fluid, lymph fluid, synovial fluid, follicular fluid, seminal fluid, amniotic fluid, milk, whole blood, blood-derived cells, urine, cerebrospinal fluid, saliva, sputum, tears, perspiration, mucus, tumor lysates, and tissue culture medium, tissue extracts such as homogenized tissue, tumor tissue, cellular extracts, and combinations thereof.

A "reference sample", "reference cell", "reference tissue", "control sample", "control cell", or "control tissue", as used herein, refers to a sample, cell, tissue, standard, or level that is used for comparison purposes. In one embodiment, a reference sample, reference cell, reference tissue, control sample, control cell, or control tissue is obtained from a healthy and/or non-diseased part of the body (e.g., tissue or cells) of the same subject or individual. For example, healthy and/or non-diseased cells or tissue adjacent to the diseased cells or tissue (*e.g*., cells or tissue adjacent to a tumor). In another embodiment, a reference sample is obtained from an untreated tissue and/or cell of the body of the same subject or individual. In yet another embodiment, a reference sample, reference cell, reference tissue, control sample, control cell, or control tissue is obtained from a healthy and/or non-diseased part of the body (*e.g.,* tissues or cells) of an individual who is not the subject or individual. In even another embodiment, a reference sample, reference cell, reference tissue, control sample, control cell, or control tissue is obtained from an untreated tissue and/or cell of the body of an individual who is not the subject or individual.

The phrase "substantially similar," as used herein, refers to a sufficiently high degree of similarity between two numeric values (generally one associated with a molecule and the other associated with a reference/comparator molecule) such that one of skill in the art would consider the difference between the two values to not be of statistical significance within the context of the biological characteristic measured by said values (*e.g.,* Kd values). The difference between said two values may be, for example, less than about 20%, less than about 10%, and/or less than about 5% as a function of the reference/comparator value.

The phrase "substantially different," refers to a sufficiently high degree of difference between two numeric values (generally one associated with a molecule and the other associated with a reference/comparator molecule) such that one of skill in the art would consider the difference between the two values to be of statistical significance within the context of the biological characteristic measured by said values (*e.g.,* Kd values). The difference between said two values may be, for example, greater than about 10%, greater than about 20%, greater than about 30%, greater than about 40%, and/or greater than about 50% as a function of the value for the reference/comparator molecule.

The term "cytotoxic agent" as used herein refers to a substance that inhibits or prevents a cellular function and/or causes cell death or destruction. Cytotoxic agents include, but are not limited to, radioactive isotopes (*e.g*., At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu); chemotherapeutic agents or drugs (*e.g.*, methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

A "chemotherapeutic agent" refers to a chemical compound useful in the treatment of cancer. Examples of chemotherapeutic agents include alkylating agents such as thiotepa and cyclosphosphamide (CYTOXAN®); alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); delta-9-tetrahydrocannabinol (dronabinol, MARINOL®); beta-lapachone; lapachol; colchicines; betulinic acid; a camptothecin (including the synthetic analogue topotecan (HYCAMTIN®), CPT-11 (irinotecan, CAMPTOSAR®), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e. g., calicheamicin, especially calicheamicin gamma1I and calicheamicin omegaI1 (see, *e.g.,* Nicolaou et al., Angew. Chem Intl. Ed. Engl., 33: 183-186 (1994)); CDP323, an oral alpha-4 integrin inhibitor; dynemicin, including dynemicin A; an esperamicin; as well as neocarzinostatin chromophore and related chromoprotein enediyne antibiotic chromophores), aclacinomysins, actinomycin, authramycin, azaserine, bleomycins, cactinomycin, carabicin, carminomycin, carzinophilin, chromomycins, dactinomycin, daunorubicin, detorubicin, 6-diazo-5-oxo-L-norleucine, doxorubicin (including ADRIAMYCIN®, morpholino-doxorubicin, cyanomorpholino-doxorubicin, 2-pyrrolino-doxorubicin, doxorubicin HCl liposome injection (DOXIL®), liposomal doxorubicin TLC D-99 (MYOCET®), peglylated liposomal doxorubicin (CAELYX®), and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate, gemcitabine (GEMZAR®), tegafur (UFTORAL®), capecitabine (XELODA®), an epothilone, and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2'-trichlorotriethylamine; trichothecenes (especially T-2 toxin, verracurin A, roridin A and anguidine); urethan; vindesine (ELDISINE®, FILDESIN®); dacarbazine; mannomustine; mitobronitol; mitolactol; pipobroman; gacytosine; arabinoside ("Ara-C"); thiotepa; taxoid, e.g., paclitaxel (TAXOL®), albumin-engineered nanoparticle formulation of paclitaxel (ABRAXANE™), and docetaxel (TAXOTERE®); chloranbucil; 6-thioguanine; mercaptopurine; methotrexate; platinum agents such as cisplatin, oxaliplatin (*e.g*., ELOXATIN®), and carboplatin; vincas, which prevent tubulin polymerization from forming microtubules, including vinblastine (VELBAN®), vincristine (ONCOVIN®), vindesine (ELDISINE®, FILDESIN®), and vinorelbine (NAVELBINE®); etoposide (VP-16); ifosfamide; mitoxantrone; leucovorin; novantrone; edatrexate; daunomycin; aminopterin; ibandronate; topoisomerase inhibitor RFS 2000; difluoromethylornithine (DMFO); retinoids such as retinoic acid, including bexarotene (TARGRETIN®); bisphosphonates such as clodronate (for example, BONEFOS® or OSTAC®), etidronate (DIDROCAL®), NE-58095, zoledronic acid/zoledronate (ZOMETA®), alendronate (FOSAMAX®), pamidronate (AREDIA®), tiludronate (SKELID®), or risedronate (ACTONEL®); troxacitabine (a 1,3-dioxolane nucleoside cytosine analog); antisense oligonucleotides, particularly those that inhibit expression of genes in signaling pathways implicated in aberrant cell proliferation, such as, for example, PKC-alpha, Raf, H-Ras, and epidermal growth factor receptor (EGF-R); vaccines such as THERATOPE® vaccine and gene therapy vaccines, for example, ALLOVECTIN® vaccine, LEUVECTIN® vaccine, and VAXID® vaccine; topoisomerase 1 inhibitor (*e.g.,* LURTOTECAN®); rmRH (*e.g.,* ABARELIX®); BAY439006 (sorafenib; Bayer); SU-11248 (sunitinib, SUTENT®, Pfizer); perifosine, COX-2 inhibitor (*e.g.* celecoxib or etoricoxib), proteosome inhibitor (*e.g.* PS341); bortezomib (VELCADE®); CCI-779; tipifarnib (R11577); orafenib, ABT510; Bcl-2 inhibitor such as oblimersen sodium (GENASENSE®); pixantrone; EGFR inhibitors (see definition below); tyrosine kinase inhibitors (see definition below); serine-threonine kinase inhibitors such as rapamycin (sirolimus, RAPAMUNE®); farnesyltransferase inhibitors such as lonafarnib (SCH 6636, SARASAR™); and pharmaceutically acceptable salts, acids or derivatives of any of the above; as well as combinations of two or more of the above such as CHOP, an abbreviation for a combined therapy of cyclophosphamide, doxorubicin, vincristine, and prednisolone; and FOLFOX, an abbreviation for a treatment regimen with oxaliplatin (ELOXATIN™) combined with 5-FU and leucovorin.

Chemotherapeutic agents as defined herein include "anti-hormonal agents" or "endocrine therapeutics" which act to regulate, reduce, block, or inhibit the effects of hormones that can promote the growth of cancer. They may be hormones themselves, including, but not limited to: anti-estrogens with mixed agonist/antagonist profile, including, tamoxifen (NOLVADEX®), 4-hydroxytamoxifen, toremifene (FARESTON®), idoxifene, droloxifene, raloxifene (EVISTA®), trioxifene, keoxifene, and selective estrogen receptor modulators (SERMs) such as SERM3; pure anti-estrogens without agonist properties, such as fulvestrant (FASLODEX®), and EM800 (such agents may block estrogen receptor (ER) dimerization, inhibit DNA binding, increase ER turnover, and/or suppress ER levels); aromatase inhibitors, including steroidal aromatase inhibitors such as formestane and exemestane (AROMASIN®), and nonsteroidal aromatase inhibitors such as anastrazole (ARIMIDEX®), letrozole (FEMARA®) and aminoglutethimide, and other aromatase inhibitors include vorozole (RIVISOR®), megestrol acetate (MEGASE®), fadrozole, and 4(5)-imidazoles; lutenizing hormone-releaseing hormone agonists, including leuprolide (LUPRON® and ELIGARD®), goserelin, buserelin, and tripterelin; sex steroids, including progestines such as megestrol acetate and medroxyprogesterone acetate, estrogens such as diethylstilbestrol and premarin, and androgens/retinoids such as fluoxymesterone, all transretionic acid and fenretinide; onapristone; anti-progesterones; estrogen receptor down-regulators (ERDs); anti-androgens such as flutamide, nilutamide and bicalutamide; and pharmaceutically acceptable salts, acids or derivatives of any of the above; as well as combinations of two or more of the above.

The term "cytostatic agent" refers to a compound or composition which arrests growth of a cell either in vitro or in vivo. Thus, a cytostatic agent may be one which significantly reduces the percentage of cells in S phase. Further examples of cytostatic agents include agents that block cell cycle progression by inducing G0/G1 arrest or M-phase arrest. The humanized anti-Her2 antibody trastuzumab (HERCEPTIN®) is an example of a cytostatic agent that induces G0/G1 arrest. Classical M-phase blockers include the vincas (vincristine and vinblastine), taxanes, and topoisomerase II inhibitors such as doxorubicin, epirubicin, daunorubicin, etoposide, and bleomycin. Certain agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in Mendelsohn and Israel, eds., The Molecular Basis of Cancer, Chapter 1, entitled "Cell cycle regulation, oncogenes, and antineoplastic drugs" by Murakami et al. (W.B. Saunders, Philadelphia, 1995), *e.g.,* p. 13. The taxanes (paclitaxel and docetaxel) are anticancer drugs both derived from the yew tree. Docetaxel (TAXOTERE®, Rhone-Poulenc Rorer), derived from the European yew, is a semisynthetic analogue of paclitaxel (TAXOL®, Bristol-Myers Squibb). Paclitaxel and docetaxel promote the assembly of microtubules from tubulin dimers and stabilize microtubules by preventing depolymerization, which results in the inhibition of mitosis in cells.

As used herein, the term "EGFR inhibitor" refers to compounds that bind to or otherwise interact directly with EGFR and prevent or reduce its signaling activity, and is alternatively referred to as an "EGFR antagonist." Examples of such agents include antibodies and small molecules that bind to EGFR. Examples of antibodies which bind to EGFR include MAb 579 (ATCC CRL HB 8506), MAb 455 (ATCC CRL HB8507), MAb 225 (ATCC CRL 8508), MAb 528 (ATCC CRL 8509) (see, US Patent No. 4,943, 533, Mendelsohn et al.) and variants thereof, such as chimerized 225 (C225 or Cetuximab; ERBUTIX®) and reshaped human 225 (H225) (see, WO 96/40210, Imclone Systems Inc.); IMC-11F8, a fully human, EGFR-targeted antibody (Imclone); antibodies that bind type II mutant EGFR (US Patent No. 5,212,290); humanized and chimeric antibodies that bind EGFR as described in US Patent No. 5,891,996; and human antibodies that bind EGFR, such as ABX-EGF or Panitumumab (*see* WO98/50433, Abgenix/Amgen); EMD 55900 (Stragliotto et al. Eur. J. Cancer 32A:636-640 (1996)); EMD7200 (matuzumab) a humanized EGFR antibody directed against EGFR that competes with both EGF and TGF-alpha for EGFR binding (EMD/Merck); human EGFR antibody, HuMax-EGFR (GenMab); fully human antibodies known as E1.1, E2.4, E2.5, E6.2, E6.4, E2.11, E6. 3 and E7.6.3 and described in US 6,235,883; MDX-447 (Medarex Inc); and mAb 806 or humanized mAb 806 (Johns et al., J. Biol. Chem. 279(29):30375-30384 (2004)). The anti-EGFR antibody may be conjugated with a cytotoxic agent, thus generating an immunoconjugate (*see, e.g.,* EP659,439A2, Merck Patent GmbH). EGFR antagonists include small molecules such as compounds described in US Patent Nos: 5,616,582, 5,457,105, 5,475,001, 5,654,307, 5,679,683, 6,084,095, 6,265,410, 6,455,534, 6,521,620, 6,596,726, 6,713,484, 5,770,599, 6,140,332, 5,866,572, 6,399,602, 6,344,459, 6,602,863, 6,391,874, 6,344,455, 5,760,041, 6,002,008, and 5,747,498, as well as the following PCT publications: WO98/14451, WO98/50038, WO99/09016, and WO99/24037. Particular small molecule EGFR antagonists include OSI-774 (CP-358774, erlotinib, TARCEVA® Genentech/OSI Pharmaceuticals); PD 183805 (CI 1033, 2-propenamide, N-[4-[(3-chloro-4-fluorophenyl)amino]-7-[3-(4-morpholinyl)propoxy]-6-quinazolinyl]-, dihydrochloride, Pfizer Inc.); ZD1839, gefitinib (IRESSAJ) 4-(3'-Chloro-4'-fluoroanilino)-7-methoxy-6-(3-morpholinopropoxy)quinazoline, AstraZeneca); ZM 105180 ((6-amino-4-(3-methylphenyl-amino)-quinazoline, Zeneca); BIBX-1382 (N8-(3-chloro-4-fluoro-phenyl)-N2-(1-methyl-piperidin-4-yl)-pyrimido[5,4-d]pyrimidine-2,8-diamine, Boehringer Ingelheim); PKI-166 ((R)-4-[4-[(1-phenylethyl)amino]-1H-pyrrolo[2,3-d]pyrimidin-6-yl]-phenol); (R)-6-(4-hydroxyphenyl)-4-[(1-phenylethyl)amino]-7H-pyrrolo[2,3-d]pyrimidine); CL-387785 (N-[4-[(3-bromophenyl)amino]-6-quinazolinyl]-2-butynamide); EKB-569 (N-[4-[(3-chloro-4-fluorophenyl)amino]-3-cyano-7-ethoxy-6-quinolinyl]-4-(dimethylamino)-2-butenamide) (Wyeth); AG1478 (Pfizer); AG1571 (SU 5271; Pfizer); dual EGFR/HER2 tyrosine kinase inhibitors such as lapatinib (TYKERB®, GSK572016 or N-[3-chloro-4-[(3 fluorophenyl)methoxy]phenyl]6[5[[[2methylsulfonyl)ethyl]amino]methyl]-2-furanyl]-4-quinazolinamine; Glaxo-SmithKline).

The term "tumor" refers to all neoplastic cell growth and proliferation, whether malignant or benign, and all pre-cancerous and cancerous cells and tissues. The terms "cancer," "cancerous," "cell proliferative disorder," "proliferative disorder" and "tumor" are not mutually exclusive as referred to herein.

The terms "cell proliferative disorder" and "proliferative disorder" refer to disorders that are associated with some degree of abnormal cell proliferation. In one embodiment, the cell proliferative disorder is cancer.

The terms "cancer" and "cancerous" refer to or describe the physiological condition in mammals that is typically characterized by unregulated cell growth/proliferation. Examples of cancer include, but are not limited to, carcinoma, lymphoma (*e.g*., Hodgkin's and non-Hodgkin's lymphoma), blastoma, sarcoma, and leukemia. More particular examples of such cancers include squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, glioma, cervical cancer, ovarian cancer, liver cancer, bladder cancer, hepatoma, breast cancer, colon cancer, colorectal cancer, endometrial or uterine carcinoma, salivary gland carcinoma, kidney cancer, liver cancer, prostate cancer, vulval cancer, thyroid cancer, hepatic carcinoma, leukemia and other lymphoproliferative disorders, and various types of head and neck cancer.

The term "colon tumor" or "colon cancer" refers to any tumor or cancer of the colon (the large intestine from the cecum to the rectum).

The term "colorectal tumor" or "colorectal cancer" refers to any tumor or cancer of the large bowel, which includes the colon (the large intestine from the cecum to the rectum) and the rectum, including, *e.g*., adenocarcinomas and less prevalent forms, such as lymphomas and squamous cell carcinomas.

An "effective amount" of an agent, *e.g.,* a pharmaceutical formulation, refers to an amount effective, at dosages and for periods of time necessary, to achieve the desired therapeutic or prophylactic result.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject., A pharmaceutically acceptable carrier includes, but is not limited to, a buffer, excipient, stabilizer, or preservative.

An "individual" or "subject" is a mammal. Mammals include, but are not limited to, domesticated animals (*e.g.,* cows, sheep, cats, dogs, and horses), primates (*e.g.,* humans and non-human primates such as monkeys), rabbits, and rodents (*e.g*., mice and rats). In certain embodiments, the individual or subject is a human.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the invention are used to delay development of a disease or to slow the progression of a disease.

By "reduce" or "inhibit" is meant the ability to cause an overall decrease of 20%, 30%, 40%, 50%, 60%, 70%, 75%, 80%, 85%, 90%, 95%, or greater. In some embodiments, reduce or inhibit can refer to a relative reduction compared to a reference (*e.g.,* reference level of biological activity (*e.g.,* wnt signaling) or binding). In some embodiments, reduce or inhibit can refer to the symptoms of the disorder being treated, the presence or size of metastases, or the size of the primary tumor.

As is understood by one skilled in the art, reference to "about" a value or parameter herein includes (and describes) embodiments that are directed to that value or parameter per se. For example, description referring to "about X" includes description of "X".

It is understood that aspect and embodiments of the invention described herein include "consisting" and/or "consisting essentially of" aspects and embodiments. As used herein, the singular form "a", "an", and "the" includes plural references unless indicated otherwise.

### II. COMPOSITIONS AND METHODS

Provided herein are anti-RSPO antibodies and uses thereof. In certain embodiments, antibodies that bind to RSPO2 and/or RSPO3 are provided. Antibodies provided are useful, *e.g*., for the diagnosis or treatment of cancer, such as colorectal cancer.

In some aspects, provided herein are a panel of anti-RSPO antibodies. The panel of antibodies where characterized for multiple properties, including but not limited to, based upon the ability to bind to RSPO2 and/or RSPO3, the ability to detect RSPO2 and/or RSPO3 by IHC, the ability to inhibit the interaction of RSPO2 and/or RSPO3 and an LGR polypeptide, for example LGR4 and/or LGR5, the ability to inhibit the interaction of RSPO2 and/or RSPO3 and an E3 ubiquitinase polypeptide, for example RNF43 and/or ZNRF3, and the ability to inhibit wnt signaling stimulated by RSPO2, RSPO3, RSPO2 polymorphisms, and/or RSPO2 translocation products, and subsets were identified.

In one aspect, provided herein are isolated antibodies that bind to RSPO2 and/or RSPO3.In some embodiments, the antibody binds to RSPO2. In some embodiments, the antibody binds to RSPO2 and do not significantly bind to RSPO3. In some embodiments, the antibody binds to RSPO3. In some embodiments, the antibody binds to RSPO3 and does not significantly bind to RSPO2. In some embodiments, the antibody binds to both RSPO2 and RSPO3. In some embodiments, the antibody is a multispecific antibody. In some embodiments, the multispecific antibody is a bispecific antibody. In some embodiments, the bispecific antibody comprises a first variable domain which binds to RSPO2 and a second variable domain which binds to RSPO3.

In certain embodiments, the antibody that binds to RSPO2 and/or RSPO3 is an antibody that binds RSPO2. In some embodiments, the anti-RSPO2 antibody binds RSPO2, wherein the RSPO2 has the sequence set forth in SEQ ID NO:1. In some embodiments, the anti-RSPO2 antibody binds RSPO2, wherein the RSPO2 lacks the signaling peptide sequence (*e.g*., binds to amino acids within amino acids 22-243 of SEQ ID NO:1). In some embodiments, the anti-RSPO2 antibody binds to one or more furin-like cysteine-rich domains of RSPO2. In some embodiments, the anti-RSPO2 antibody binds a region within amino acids 34 to 134 of SEQ ID NO:1. In some embodiments, the anti-RSPO2 antibody binds a region within amino acids 39 to 134 of SEQ ID NO:1. In some embodiments, the anti-RSPO2 antibody binds a region within amino acids 34 to 84 of SEQ ID NO:1. In some embodiments, the anti-RSPO2 antibody binds a region within amino acids 90 to 134 of SEQ ID NO:1. In some embodiments, the anti-RSPO2 antibody does not bind to the thrombospondin type 1 domain of RSPO2 (*e.g*., does not bind a region within amino acids 144-204 of SEQ ID NO:1). In some embodiments, the anti-RSPO2 antibody binds to the thrombospondin type 1 domain of RSPO2. In some embodiments, the anti-RSPO2 antibody binds a region within amino acids 144-204 of SEQ ID NO:1. In some embodiments, the anti-RSPO2 antibody inhibits wnt signaling. In some embodiments, the anti-RSPO2 antibody inhibits wnt signaling in an individual and/or cancer with an RSPO2 polymorphism (*e.g*., RSPO2 L186P polymorphism). In some embodiments, the anti-RSPO2 antibody inhibits the interaction of RSPO2 and one or more of LGR4, LGR5, and/or LGR6. In some embodiments, the anti-RSPO2 antibody does not inhibit the interaction of RSPO2 and one or more of LGR4, LGR5, and/or LGR6 (*e.g*., enhances binding of RSP02 to one or more of LGR4, LGR5, and/or LGR6). In some embodiments, the anti-RSPO2 antibody inhibits the interaction of RSPO2 and a transmembrane E3 ubiquitinase (*e.g*., one or more of ZNRF3 and/or RNF43). In some embodiments, the anti-RSPO2 antibody inhibits the interaction of RSPO2 with a syndecan (*e.g*., Sdc4). In some embodiments, the anti-RSPO2 antibody inhibits the interaction of RSPO2 and one or more of LGR4, LGR5, and/or LGR6 and inhibits the interaction of RSPO2 and a transmembrane E3 ubiquitinase (*e.g.,* one or more of ZNRF3 and/or RNF43) (*e.g.,* 11F11, 36D2, 49G5, and/or 26E11). In some embodiments, the anti-RSPO2 antibody inhibits the interaction of RSPO2 and a transmembrane E3 ubiquitinase (*e.g*., one or more of ZNRF3 and/or RNF43) and does not inhibit the interaction of RSPO2 and one or more of LGR4, LGR5, and/or LGR6 (*e.g*., enhances binding of RSPO2 to one or more of LGR4, LGR5, and/or LGR6) (*e.g.,* 1A1). In some embodiments, the anti-RSPO2 antibody inhibits cancer stem cell growth. In some embodiments, the anti-RSPO2 antibody induces and/or promotes cancer cell (*e.g.,* cancer stem cell) differentiation (*e.g.,* terminal differentiation and/or differentiation into progenitor cell). In some embodiments, the anti-RSPO3 antibody induces and/or promotes cancer cell (*e.g.,* cancer stem cell) differentiation into enterocyte, goblet cell, and/or enteroendocrine cell.

In certain embodiments, the antibody that binds to RSPO2 and/or RSPO3 is an antibody that binds RSPO3. In some embodiments, the anti-RSPO3 antibody binds RSPO3, wherein the RSPO3 has the sequence set forth in SEQ ID NO:2. In some embodiments, the anti-RSPO3 antibody binds RSPO3, wherein the RSPO3 lacks the signaling peptide sequence (*e.g*., binds to amino acids within amino acids 22-272 of SEQ ID NO:2). In some embodiments, the anti-RSPO3 antibody binds to one or more furin-like cysteine-rich domains of RSPO3. In some embodiments, the anti-RSPO3 antibody binds a region within amino acids 35 to 135 of SEQ ID NO:2. In some embodiments, the anti-RSPO3 antibody binds a region within amino acids 35 to 86 of SEQ ID NO:2. In some embodiments, the anti-RSPO3 antibody binds to a region within amino acids 92 to 135 of SEQ ID NO:2. In some embodiments, the anti-RSPO3 antibody does not bind to the thrombospondin type 1 domain of RSPO3 (*e.g.,* does not bind amino acids within amino acids 147-207 of SEQ ID NO:2). In some embodiments, the anti-RSPO3 antibody binds to the thrombospondin type 1 domain of RSPO3. In some embodiments, the anti-RSPO3 antibody binds a region within amino acids 147-207 of SEQ ID NO:2. In some embodiments, the anti-RSPO3 antibody inhibits wnt signaling. In some embodiments, the anti-RSPO3 antibody inhibits the interaction of RSPO3 and one or more of LGR4, LGR5, and/or LGR6. In some embodiments, the anti-RSPO3 antibody does not inhibit the interaction of RSPO3 and one or more of LGR4, LGR5, and/or LGR6 (*e.g*., enhances binding of RSPO3 to one or more of LGR4, LGR5, and/or LGR6). In some embodiments, the anti-RSPO3 antibody inhibits the interaction of RSPO3 and a transmembrane E3 ubiquitinase (*e.g*., one or more of ZNRF3 and/or RNF43). In some embodiments, the anti-RSPO3 antibody inhibits the interaction of RSPO3 with a syndecan (*e.g*., Sdc4). In some embodiments, the anti-RSPO3 antibody inhibits the interaction of RSPO3 and one or more of LGR4, LGR5, and/or LGR6 and inhibits the interaction of RSPO3 and a transmembrane E3 ubiquitinase (*e.g*., one or more of ZNRF3 and/or RNF43). In some embodiments, the anti-RSPO3 antibody inhibits cancer stem cell growth. In some embodiments, the anti-RSPO3 antibody induces and/or promotes cancer cell (*e.g.,* cancer stem cell) differentiation (*e.g.,* terminal differentiation and/or differentiation into progenitor cell). In some embodiments, the anti-RSPO3 antibody induces and/or promotes cancer cell (*e.g.,* cancer stem cell) differentiation into a transit-amplifying cell. In some embodiments, the anti-RSPO3 antibody induces and/or promotes cancer cell (*e.g.,* cancer stem cell) differentiation into enterocyte, goblet cell, and/or enteroendocrine cell.

In some embodiments, the anti-RSPO3 antibody binds to a region within amino acids 49 to 108 of SEQ ID NO:2. In some embodiments, the anti-RSPO3 antibody binds to an epitope comprising one or more amino acids selected from Ser49, Asn52, Cys54, Leu55, Ser56, Phe63, Leu65, Gln72, Ile73, Gly74, Tyr84, Tyr89, Pro90, Asp91, Ile92, Lys94, and Lys108 of RSPO3 (*e.g.,* SEQ ID NO:2). In some embodiments, the anti-RSPO3 antibody binds to an epitope comprising amino acids Ser 49, Asn52, Cys54, Leu55, Ser56, Phe63, Leu65, Gln72, Ile73, Gly74, Tyr84, Tyr89, Pro90, Asp91, Ile92, Lys94, and Lys108 of RSPO3 (*e.g.,* SEQ ID NO:2). In some embodiments, the anti-RSPO3 antibody binds to an epitope comprising amino acids residues of RSP03 (*e.g.,* SEQ ID NO:2): Ser 49, Asn52, Cys54, Leu55, Ser56, Phe63, Leu65, Gln72, Ile73, Gly74, Tyr84, Tyr89, Pro90, Asp91, Ile92, Lys94, and Lys108. In some embodiments, the anti-RSPO3 antibody binds to an epitope comprising one or more amino acids selected from Ser49, Asn52, Cys54, Leu55, Ser56, Phe63, Leu65, Gln72, Ile73, Gly74, Tyr84, Tyr89, Pro90, Asp91, Ile92, Lys94, Lys97, and Lys108 of RSPO3 (*e.g.,* SEQ ID NO:2). In some embodiments, the anti-RSPO3 antibody binds to an epitope comprising amino acids Ser 49, Asn52, Cys54, Leu55, Ser56, Phe63, Leu65, Gln72, Ile73, Gly74, Tyr84, Tyr89, Pro90, Asp91, Ile92, Lys94, Lys97, and Lys108 of RSPO3 (*e.g.,* SEQ ID NO:2). In some embodiments, the anti-RSPO3 antibody when bound to RSPO3 is positioned 4 angstroms or less from one or more amino acids Ser 49, Asn52, Cys54, Leu55, Ser56, Phe63, Leu65, Gln72, Ile73, Gly74, Tyr84, Tyr89, Pro90, Asp91, Ile92, Lys94, and Lys108 of RSPO3 (*e.g.,* SEQ ID NO:2). In some embodiments, the anti-RSPO3 antibody when bound to RSPO3 is positioned 4 angstroms or less from amino acids residues of RSPO3 (*e.g.,* SEQ ID NO:2): Ser 49, Asn52, Cys54, Leu55, Ser56, Phe63, Leu65, Gln72, Ile73, Gly74, Tyr84, Tyr89, Pro90, Asp91, Ile92, Lys94, and Lys108. In some embodiments, the anti-RSPO3 antibody when bound to RSPO3 is positioned 4 angstroms or less from one or more amino acids Ser 49, Asn52, Cys54, Leu55, Ser56, Phe63, Leu65, Gln72, Ile73, Gly74, Tyr84, Tyr89, Pro90, Asp91, Ile92, Lys94, Lys97, and Lys108 of RSPO3 (*e.g.,* SEQ ID NO:2). In some embodiments, the anti-RSPO3 antibody when bound to RSPO3 is positioned 4 angstroms or less from amino acids residues of RSPO3 (*e.g.,* SEQ ID NO:2): Ser 49, Asn52, Cys54, Leu55, Ser56, Phe63, Leu65, Gln72, Ile73, Gly74, Tyr84, Tyr89, Pro90, Asp91, Ile92, Lys94, Lys97,and Lys108. In some embodiments, the anti-RSPO3 antibody when bound to RSPO3 is positioned 3.5 angstroms or less from one or more amino acids Asn 52, Leu55, Phe63, Gln72, Tyr89, Pro90, Asp91, Lys94, and Lys97 of RSPO3 (*e.g.,* SEQ ID NO:2). In some embodiments, the anti-RSPO3 antibody when bound to RSPO3 is positioned 3.5 angstroms or less from Asn 52, Leu55, Phe63, Gln72, Tyr89, Pro90, Asp91, Lys94, and Lys97 of RSPO3 (*e.g.,* SEQ ID NO:2). In some embodiments, the anti-RSPO3 antibody when bound to RSPO3 is positioned 3 angstroms or less from one or more amino acids Gln72, Pro90, Asp91, and Lys94 of RSPO3 (*e.g.,* SEQ ID NO:2). In some embodiments, the anti-RSPO3 antibody when bound to RSPO3 is positioned 3 angstroms or less from Gln72, Pro90, Asp91, and Lys94 of RSPO3 (*e.g.,* SEQ ID NO:2). In some embodiments, the anti-RSPO3 antibody when bound to RSPO3 is positioned about any of 4, 3.75, 3.5, 3.25, or 3 angstroms from one or more amino acids provided above. In some embodiments, the one or more amino acids and/or the one or more amino acid residues is about any of 1, 2, 3, 4, 5, 6, 7, 8, 9, 10, 11, and/or 12 amino acids and/or amino acid residues. In some embodiments, the epitope is determined by crystallography (*e.g*., crystallography methods described in the Examples).

In some embodiments, the anti-RSPO3 antibody binds to amino acids within amino acids 47 to 108 of SEQ ID NO:2. In some embodiments, the anti-RSPO3 antibody binds to an epitope comprising one or more amino acids selected from Thr47, Asn52, Cys54, Leu55, Ser56, Phe63, Leu65, Gln72, Tyr84, Tyr89, Pro90, Asp91, Ile92, Asn93, Lys94, Lys97, and Lys108 of RSPO3 (*e.g.,* SEQ ID NO:2). In some embodiments, the anti-RSPO3 antibody binds to an epitope comprising amino acids Thr47, Asn52, Cys54, Leu55, Ser56, Phe63, Leu65, Gln72, Tyr84, Tyr89, Pro90, Asp91, Ile92, Asn93, Lys94, Lys97, and Lys108 of RSPO3 (*e.g.,* SEQ ID NO:2). In some embodiments, the anti-RSPO3 antibody binds to an epitope comprising amino acids residues of RSP03 (*e.g.,* SEQ ID NO:2): Thr47, Asn52, Cys54, Leu55, Ser56, Phe63, Leu65, Gln72, Tyr84, Tyr89, Pro90, Asp91, Ile92, Asn93, Lys94, Lys97, and Lys108. In some embodiments, the anti-RSPO3 antibody when bound to RSPO3 is positioned 4 angstroms or less from one or more amino Thr47, Asn52, Cys54, Leu55, Ser56, Phe63, Leu65, Gln72, Tyr84, Tyr89, Pro90, Asp91, Ile92, Asn93, Lys94, Lys97, and Lys108 of RSPO3 (*e.g.,* SEQ ID NO:2). In some embodiments, the anti-RSPO3 antibody when bound to RSPO3 is positioned 4 angstroms or less from amino acids residues of RSPO3 (*e.g.,* SEQ ID NO:2): Thr47, Asn52, Cys54, Leu55, Ser56, Phe63, Leu65, Gln72, Tyr84, Tyr89, Pro90, Asp91, Ile92, Asn93, Lys94, Lys97, and Lys108. In some embodiments, the anti-RSPO3 antibody when bound to RSPO3 is positioned 3.5 angstroms or less from one or more amino acids Thr47, Asn52, Leu55, Phe63, Gln72, Tyr89, Pro90, Asp91, Ile92, Lys94, and Lys97 of RSPO3 (*e.g*., SEQ ID NO:2). In some embodiments, the anti-RSPO3 antibody when bound to RSPO3 is positioned 3.5 angstroms or less from amino acids Thr47, Asn52, Leu55, Phe63, Gln72, Tyr89, Pro90, Asp91, Ile92, Lys94, and Lys97 of RSPO3 (*e.g.,* SEQ ID NO:2). In some embodiments, the anti-RSPO3 antibody when bound to RSPO3 is positioned 3 angstroms or less from one or more amino acids Thr47, Leu55, Gln72, Pro90, Asp91, and Lys94 of RSPO3 (*e.g.,* SEQ ID NO:2). In some embodiments, the anti-RSPO3 antibody when bound to RSPO3 is positioned 3 angstroms or less from amino acids Thr47, Leu55, Gln72, Pro90, Asp91, and Lys94 of RSPO3 (*e.g.,* SEQ ID NO:2). In some embodiments, the anti-RSPO3 antibody when bound to RSPO3 is positioned about any of 4, 3.75, 3.5, 3.25, or 3 angstroms from one or more amino acids provided above. In some embodiments, the one or more amino acids and/or the one or more amino acid residues is about any of 1, 2, 3, 4, 5, 6, 7, 8, 9, 10, 11, and/or 12 amino acids and/or amino acid residues. In some embodiments, the anti-RSPO3 antibody also binds RSPO2. In some embodiments, the epitope is determined by crystallography (e.g., crystallography methods described in the Examples).

In some embodiment, the epitope as determined by crystallography is determined using amino acids M33-E210 of RSPO3. In some embodiments, the epitope as determined by crystallography is permformed by using an Labcyte Echo liquid handler to set several sparse matrix crystal screens using 100 nL sitting drops. Screens were stored at 18°C. In some embodiments, crystals may be obtained in a drop containing 100 mM MIB pH 9 and 25% PEG 1500 as the mother liquor. In some embodiments, crystals may be obtained in a drop containing 200 mM Sodium formate and 20% (w/v) PEG 3,350 as the mother liquor. In some embodiments, the crystal may be harvested and soaked in cryoprotectant solution for 10 seconds and flash-frozen in liquid nitrogen. In some embodiments, the cryoprotectant solution may be made by mixing 1 µL 70% glycerol with 1.8µL reservoir solution. In some embodiments, the crystals may be grown in PEG-based conditions, for example, about 20-25% PEG 3,350. In some embodiments, the crystals may be grown in about 20% PEG 6,000, about 20-25% PEG 4,000, and about 25% PEG 1,500. In some embodiments, the pH may range from about 3.5 - 9, for example, between about 7 and about 8. In some embodiments, the salt concentration is about 200 mM.

In certain embodiments, the antibody that binds to RSPO2 and/or RSPO3 is an antibody that binds RSPO2 and RSPO3 (e.g., anti-RSPO2/3 antibody). In some embodiments, the anti-RSPO2/3 antibody binds RSPO2, wherein the RSPO2 has the sequence set forth in SEQ ID NO:1 and binds RSPO3, wherein the RSPO3 has the sequence set forth in SEQ ID NO:2. In some embodiments, the anti-RSPO2/3 antibody inhibits wnt signaling.

In some aspects, antibodies were identified that were able to cross react with both RSPO2 and RSPO3. A nonlimiting example of activities of these anti-RSPO2/3 antibodies may include the ability to bind to RSPO2 and RSPO3, detect RSPO2 and RSPO3 by IHC, inhibit the interaction of RSPO2 and RSPO3 and an LGR polypeptide, for example LGR4 and/or LGR5, inhibit the interaction of RSPO2 and RSPO3 and an E3 ubiquitinase polypeptide, for example RNF43 and/or ZNRF3, and/or inhibit wnt signaling stimulated by RSPO2, RSPO3, RSPO2 polymorphisms, and RSPO2 translocation products.

One skilled in the art would further appreciate that in some embodiments, any anti-RSPO2 antibody and/or anti-RSPO3 antibody could be engineered into an antibody format, in particular bispecific format, which would allow reactivity with both RSPO2 and RSPO3. These anti-RSPO2/3 bispecific antibodies be able to may include the ability to bind to RSPO2 and RSP03, detect RSPO2 and RSPO3 by IHC, inhibit the interaction of RSPO2 and RSPO3 and an LGR polypeptide, for example LGR4 and/or LGR5, inhibit the interaction of RSP02 and RSPO3 and an E3 ubiquitinase polypeptide, for example, RNF43 and/or ZNRF3, and/or inhibit wnt signaling stimulated by RSPO2, RSPO3, RSPO2 polymorphisms, and RSPO2 translocation products.

In some embodiments of any of the anti-RSPO2/3 antibodies, the anti-RSPO2/3 antibody inhibits the interaction of RSPO2 and RSPO3 with one or more of LGR4, LGR5, and/or LGR6. In some embodiments, the antibody is a dual arm antibody. In some embodiments, the anti-RSPO2/3 antibody comprises a first and second variable domain comprising on each variable domain the six HVRs of 26E11. In some embodiments, the antibody is a bispecific antibody. In some embodiments, the anti-RSPO2/3 antibody comprises a first variable domain comprising the six HVRs of 5D6 or 5E11 and a second variable domain comprising the six HVRs of 36D2.

In some embodiments of any of the anti-RSPO2/3 antibodies, the anti-RSPO2/3 antibody inhibits the interaction of RSPO3 and one or more of LGR4, LGR5, and/or LGR6 and does not inhibit the interaction of RSPO2 and one or more of LGR4, LGR5, and/or LGR6 (e.g., enhances binding of RSPO2 to one or more of LGR4, LGR5, and/or LGR6). In some embodiments, the antibody is a bispecific antibody. In some embodiments, the anti-RSPO2/3 antibody comprises a first variable domain comprising the six HVRs of 5D6 or 5E11 and a second variable domain comprising the six HVRs of 1A1.

In some embodiments of any of the anti-RSPO2/3 antibodies, the anti-RSPO2/3 antibody inhibits the interaction of RSPO2 and RSPO3 with a transmembrane E3 ubiquitinase (e.g., one or more of ZNRF3 and/or RNF43). In some embodiments, the antibody is a dual arm antibody. In some embodiments, the anti-RSPO2/3 antibody comprises a first and second variable domain comprising on each variable domain the six HVRs of 26E11. In some embodiments, the antibody is a bispecific antibody. In some embodiments, the anti-RSPO2/3 antibody comprises a first variable domain comprising the six HVRs of 5D6 or 5E11 and a second variable domain comprising the six HVRs of 36D2 or 1A1.

In some embodiments of any of the anti-RSPO2/3 antibodies, the anti-RSPO2/3 antibody inhibits the interaction of RSPO2 and RSPO3 with one or more of LGR4, LGR5, and/or LGR6 and RSPO2 and inhibits the interaction of RSPO2 and RSPO3 with a transmembrane E3 ubiquitinase (*e.g.,* one or more of ZNRF3 and/or RNF43). In some embodiments, the antibody is a dual arm antibody. In some embodiments, the anti-RSPO2/3 antibody comprises a first and second variable domain comprising on each variable domain the six HVRs of 26E11. In some embodiments, the antibody is a bispecific antibody. In some embodiments, the anti-RSPO2/3 antibody comprises a first variable domain comprising the six HVRs of 5D6 or 5E11 and a second variable domain comprising the six HVRs of 36D2.

In some embodiments of any of the anti-RSPO2/3 antibodies, the anti-RSPO2/3 antibody inhibits the interaction of RSPO3 and one or more of LGR4, LGR5, and/or LGR6 and RSPO2 and inhibits the interaction of RSPO2 and RSPO3 with a transmembrane E3 ubiquitinase (e.g., one or more of ZNRF3 and/or RNF43) and does not inhibit the interaction of RSPO2 and one or more of LGR4, LGR5, and/or LGR6 (e.g., enhances binding of RSP02 to one or more of LGR4, LGR5, and/or LGR6). In some embodiments, the antibody is a bispecific antibody. In some embodiments, the anti-RSPO2/3 antibody comprises a first variable domain comprising the six HVRs of 5D6 or 5E11 and a second variable domain comprising the six HVRs of 1A1.

In one aspect provided herein are anti-RSPO3 antibodies that bind the same or overlapping epitope as one or more of the antibodies 4H1, 4D4, 5C2, 5D6, 5E11, 6E9, 21C2, and/or 26E11. Further, in one aspect provided herein are anti-RSPO3 antibodies that compete for binding to RSPO3 with one or more of the antibodies 4H1, 4D4, 5C2, 5D6, 5E11, 6E9, 21C2, and/or 26E11. In one aspect provided herein are anti-RSPO2 antibodies that bind the same or overlapping epitope as one or more of antibodies 1A1, 11F11, 26E11, 36D2, and/or 49G5. Further, in one aspect provided herein are anti-RSPO2 antibodies that compete for binding to RSPO2 with one or more of antibodies 1A1, 11F11, 26E11, 36D2, and/or 49G5. In one aspect provided herein are anti-RSPO2 antibodies that bind the same or overlapping epitope as 1A1. Further, in one aspect provided herein are anti-RSPO2 antibodies that compete for binding to RSPO2 with 1A1. In some embodiments, the antibody competes for binding with another antibody by BIACORE, competitive ELISA, and/or any other methods described herein and known in the art. Methods of determining epitopes are known in the art and described herein. In some embodiments, the epitope is a linear epitope. In some embodiments, the epitope is a conformational epitope. In some embodiments, the epitope is determined by antibody binding to peptide fragments. In some embodiments, the epitope is determined by mass spectrometry. In some embodiments, the epitope is determined by crystallography (e.g., analysis of crystal structure).

### Monoclonal Antibody 4H1 and Certain Other Antibody Embodiments

In one aspect, the invention provides an anti-RSPO3 antibody comprising at least one, two, three, four, five, or six HVRs selected from (a) HVR-H1 comprising the amino acid sequence of SEQ ID NO:8; (b) HVR-H2 comprising the amino acid sequence of SEQ ID NO:9; (c) HVR-H3 comprising the amino acid sequence of SEQ ID NO:10; (d) HVR-L1 comprising the amino acid sequence of SEQ ID NO:5; (e) HVR-L2 comprising the amino acid sequence of SEQ ID NO:6; and (f) HVR-L3 comprising the amino acid sequence of SEQ ID NO:7.

In one aspect, the invention provides an antibody comprising at least one, at least two, or all three VH HVR sequences selected from (a) HVR-H1 comprising the amino acid sequence of SEQ ID NO:8; (b) HVR-H2 comprising the amino acid sequence of SEQ ID NO:9; and (c) HVR-H3 comprising the amino acid sequence of SEQ ID NO:10. In one embodiment, the antibody comprises HVR-H3 comprising the amino acid sequence of SEQ ID NO:10. In another embodiment, the antibody comprises HVR-H3 comprising the amino acid sequence of SEQ ID NO:10 and HVR-L3 comprising the amino acid sequence of SEQ ID NO:7. In a further embodiment, the antibody comprises HVR-H3 comprising the amino acid sequence of SEQ ID NO:10, HVR-L3 comprising the amino acid sequence of SEQ ID NO:7, and HVR-H2 comprising the amino acid sequence of SEQ ID NO:9. In a further embodiment, the antibody comprises (a) HVR-H1 comprising the amino acid sequence of SEQ ID NO:8; (b) HVR-H2 comprising the amino acid sequence of SEQ ID NO:9; and (c) HVR-H3 comprising the amino acid sequence of SEQ ID NO:10.

In another aspect, the invention provides an antibody comprising at least one, at least two, or all three VL HVR sequences selected from (a) HVR-L1 comprising the amino acid sequence of SEQ ID NO:5; (b) HVR-L2 comprising the amino acid sequence of SEQ ID NO:6; and (c) HVR-L3 comprising the amino acid sequence of SEQ ID NO:7. In one embodiment, the antibody comprises (a) HVR-L1 comprising the amino acid sequence of SEQ ID NO:5; (b) HVR-L2 comprising the amino acid sequence of SEQ ID NO:6; and (c) HVR-L3 comprising the amino acid sequence of SEQ ID NO:7.

In another aspect, an antibody of the invention comprises (a) a VH domain comprising at least one, at least two, or all three VH HVR sequences selected from (i) HVR-H1 comprising the amino acid sequence of SEQ ID NO:8, (ii) HVR-H2 comprising the amino acid sequence of SEQ ID NO:9, and (iii) HVR-H3 comprising an amino acid sequence selected from SEQ ID NO:10; and (b) a VL domain comprising at least one, at least two, or all three VL HVR sequences selected from (i) HVR-L1 comprising the amino acid sequence of SEQ ID NO:5, (ii) HVR-L2 comprising the amino acid sequence of SEQ ID NO:6, and (c) HVR-L3 comprising the amino acid sequence of SEQ ID NO:7.

In another aspect, the invention provides an antibody comprising (a) HVR-H1 comprising the amino acid sequence of SEQ ID NO:8; (b) HVR-H2 comprising the amino acid sequence of SEQ ID NO:9; (c) HVR-H3 comprising the amino acid sequence of SEQ ID NO:10; (d) HVR-L1 comprising the amino acid sequence of SEQ ID NO:5; (e) HVR-L2 comprising the amino acid sequence of SEQ ID NO:6; and (f) HVR-L3 comprising an amino acid sequence selected from SEQ ID NO:7.

In another aspect, an anti-RSP03 antibody is provided, wherein the antibody comprises a VH as in any of the embodiments provided above, and a VL as in any of the embodiments provided above. In one embodiment, the antibody comprises the VH and VL sequences in SEQ ID NO:89 and SEQ ID NO:90, respectively, including post-translational modifications of those sequences.

### Monoclonal Antibody 4D4 and Certain Other Antibody Embodiments

In one aspect, the invention provides an anti-RSPO3 antibody comprising at least one, two, three, four, five, or six HVRs selected from (a) HVR-H1 comprising the amino acid sequence of SEQ ID NO:14; (b) HVR-H2 comprising the amino acid sequence of SEQ ID NO:15; (c) HVR-H3 comprising the amino acid sequence of SEQ ID NO:16; (d) HVR-L1 comprising the amino acid sequence of SEQ ID NO:11; (e) HVR-L2 comprising the amino acid sequence of SEQ ID NO:12; and (f) HVR-L3 comprising the amino acid sequence of SEQ ID NO:13.

In one aspect, the invention provides an antibody comprising at least one, at least two, or all three VH HVR sequences selected from (a) HVR-H1 comprising the amino acid sequence of SEQ ID NO:14; (b) HVR-H2 comprising the amino acid sequence of SEQ ID NO:15; and (c) HVR-H3 comprising the amino acid sequence of SEQ ID NO:16. In one embodiment, the antibody comprises HVR-H3 comprising the amino acid sequence of SEQ ID NO:16. In another embodiment, the antibody comprises HVR-H3 comprising the amino acid sequence of SEQ ID NO:16 and HVR-L3 comprising the amino acid sequence of SEQ ID NO:13. In a further embodiment, the antibody comprises HVR-H3 comprising the amino acid sequence of SEQ ID NO:16, HVR-L3 comprising the amino acid sequence of SEQ ID NO:13, and HVR-H2 comprising the amino acid sequence of SEQ ID NO:15. In a further embodiment, the antibody comprises (a) HVR-H1 comprising the amino acid sequence of SEQ ID NO:14; (b) HVR-H2 comprising the amino acid sequence of SEQ ID NO:15; and (c) HVR-H3 comprising the amino acid sequence of SEQ ID NO:16.

In another aspect, the invention provides an antibody comprising at least one, at least two, or all three VL HVR sequences selected from (a) HVR-L1 comprising the amino acid sequence of SEQ ID NO:11; (b) HVR-L2 comprising the amino acid sequence of SEQ ID NO:12; and (c) HVR-L3 comprising the amino acid sequence of SEQ ID NO:13. In one embodiment, the antibody comprises (a) HVR-L1 comprising the amino acid sequence of SEQ ID NO:11; (b) HVR-L2 comprising the amino acid sequence of SEQ ID NO:12; and (c) HVR-L3 comprising the amino acid sequence of SEQ ID NO:13.

In another aspect, an antibody of the invention comprises (a) a VH domain comprising at least one, at least two, or all three VH HVR sequences selected from (i) HVR-H1 comprising the amino acid sequence of SEQ ID NO:14, (ii) HVR-H2 comprising the amino acid sequence of SEQ ID NO:15, and (iii) HVR-H3 comprising an amino acid sequence selected from SEQ ID NO:16; and (b) a VL domain comprising at least one, at least two, or all three VL HVR sequences selected from (i) HVR-L1 comprising the amino acid sequence of SEQ ID NO:11, (ii) HVR-L2 comprising the amino acid sequence of SEQ ID NO:12, and (c) HVR-L3 comprising the amino acid sequence of SEQ ID NO:13.

In another aspect, the invention provides an antibody comprising (a) HVR-H1 comprising the amino acid sequence of SEQ ID NO:14; (b) HVR-H2 comprising the amino acid sequence of SEQ ID NO:15; (c) HVR-H3 comprising the amino acid sequence of SEQ ID NO:16; (d) HVR-L1 comprising the amino acid sequence of SEQ ID NO:11; (e) HVR-L2 comprising the amino acid sequence of SEQ ID NO:12; and (f) HVR-L3 comprising an amino acid sequence selected from SEQ ID NO:13.

In another aspect, an anti-RSP03 antibody is provided, wherein the antibody comprises a VH as in any of the embodiments provided above, and a VL as in any of the embodiments provided above. In one embodiment, the antibody comprises the VH and VL sequences in SEQ ID NO:91 and SEQ ID NO:92, respectively, including post-translational modifications of those sequences.

### Monoclonal Antibody 5C2 and Certain Other Antibody Embodiments

In one aspect, the invention provides an anti-RSPO3 antibody comprising at least one, two, three, four, five, or six HVRs selected from (a) HVR-H1 comprising the amino acid sequence of SEQ ID NO:20; (b) HVR-H2 comprising the amino acid sequence of SEQ ID NO:21; (c) HVR-H3 comprising the amino acid sequence of SEQ ID NO:22; (d) HVR-L1 comprising the amino acid sequence of SEQ ID NO:17; (e) HVR-L2 comprising the amino acid sequence of SEQ ID NO:18; and (f) HVR-L3 comprising the amino acid sequence of SEQ ID NO:19.

In one aspect, the invention provides an antibody comprising at least one, at least two, or all three VH HVR sequences selected from (a) HVR-H1 comprising the amino acid sequence of SEQ ID NO:20; (b) HVR-H2 comprising the amino acid sequence of SEQ ID NO:21; and (c) HVR-H3 comprising the amino acid sequence of SEQ ID NO:22. In one embodiment, the antibody comprises HVR-H3 comprising the amino acid sequence of SEQ ID NO:22. In another embodiment, the antibody comprises HVR-H3 comprising the amino acid sequence of SEQ ID NO:22 and HVR-L3 comprising the amino acid sequence of SEQ ID NO:19. In a further embodiment, the antibody comprises HVR-H3 comprising the amino acid sequence of SEQ ID NO:22, HVR-L3 comprising the amino acid sequence of SEQ ID NO:19, and HVR-H2 comprising the amino acid sequence of SEQ ID NO:21. In a further embodiment, the antibody comprises (a) HVR-H1 comprising the amino acid sequence of SEQ ID NO:20; (b) HVR-H2 comprising the amino acid sequence of SEQ ID NO:21; and (c) HVR-H3 comprising the amino acid sequence of SEQ ID NO:22.

In another aspect, the invention provides an antibody comprising at least one, at least two, or all three VL HVR sequences selected from (a) HVR-L1 comprising the amino acid sequence of SEQ ID NO:17; (b) HVR-L2 comprising the amino acid sequence of SEQ ID NO:18; and (c) HVR-L3 comprising the amino acid sequence of SEQ ID NO:19. In one embodiment, the antibody comprises (a) HVR-L1 comprising the amino acid sequence of SEQ ID NO:17; (b) HVR-L2 comprising the amino acid sequence of SEQ ID NO:18; and (c) HVR-L3 comprising the amino acid sequence of SEQ ID NO:19.

In another aspect, an antibody of the invention comprises (a) a VH domain comprising at least one, at least two, or all three VH HVR sequences selected from (i) HVR-H1 comprising the amino acid sequence of SEQ ID NO:20, (ii) HVR-H2 comprising the amino acid sequence of SEQ ID NO:21, and (iii) HVR-H3 comprising an amino acid sequence selected from SEQ ID NO:22; and (b) a VL domain comprising at least one, at least two, or all three VL HVR sequences selected from (i) HVR-L1 comprising the amino acid sequence of SEQ ID NO:17, (ii) HVR-L2 comprising the amino acid sequence of SEQ ID NO:18, and (c) HVR-L3 comprising the amino acid sequence of SEQ ID NO:19.

In another aspect, the invention provides an antibody comprising (a) HVR-H1 comprising the amino acid sequence of SEQ ID NO:20; (b) HVR-H2 comprising the amino acid sequence of SEQ ID NO:21; (c) HVR-H3 comprising the amino acid sequence of SEQ ID NO:22; (d) HVR-L1 comprising the amino acid sequence of SEQ ID NO:17; (e) HVR-L2 comprising the amino acid sequence of SEQ ID NO:18; and (f) HVR-L3 comprising an amino acid sequence selected from SEQ ID NO:19.

In another aspect, an anti-RSP03 antibody is provided, wherein the antibody comprises a VH as in any of the embodiments provided above, and a VL as in any of the embodiments provided above. In one embodiment, the antibody comprises the VH and VL sequences in SEQ ID NO:93 and SEQ ID NO:94, respectively, including post-translational modifications of those sequences.

### Monoclonal Antibody 5D6 and Certain Other Antibody Embodiments

In one aspect, the invention provides an anti-RSPO3 antibody comprising at least one, two, three, four, five, or six HVRs selected from (a) HVR-H1 comprising the amino acid sequence of SEQ ID NO:26; (b) HVR-H2 comprising the amino acid sequence of SEQ ID NO:27; (c) HVR-H3 comprising the amino acid sequence of SEQ ID NO:28; (d) HVR-L1 comprising the amino acid sequence of SEQ ID NO:23; (e) HVR-L2 comprising the amino acid sequence of SEQ ID NO:24; and (f) HVR-L3 comprising the amino acid sequence of SEQ ID NO:25.

In one aspect, the invention provides an antibody comprising at least one, at least two, or all three VH HVR sequences selected from (a) HVR-H1 comprising the amino acid sequence of SEQ ID NO:26; (b) HVR-H2 comprising the amino acid sequence of SEQ ID NO:27; and (c) HVR-H3 comprising the amino acid sequence of SEQ ID NO:28. In one embodiment, the antibody comprises HVR-H3 comprising the amino acid sequence of SEQ ID NO:28. In another embodiment, the antibody comprises HVR-H3 comprising the amino acid sequence of SEQ ID NO:28 and HVR-L3 comprising the amino acid sequence of SEQ ID NO:25. In a further embodiment, the antibody comprises HVR-H3 comprising the amino acid sequence of SEQ ID NO:28, HVR-L3 comprising the amino acid sequence of SEQ ID NO:25, and HVR-H2 comprising the amino acid sequence of SEQ ID NO:27. In a further embodiment, the antibody comprises (a) HVR-H1 comprising the amino acid sequence of SEQ ID NO:26; (b) HVR-H2 comprising the amino acid sequence of SEQ ID NO:27; and (c) HVR-H3 comprising the amino acid sequence of SEQ ID NO:28.

In another aspect, the invention provides an antibody comprising at least one, at least two, or all three VL HVR sequences selected from (a) HVR-L1 comprising the amino acid sequence of SEQ ID NO:23; (b) HVR-L2 comprising the amino acid sequence of SEQ ID NO:24; and (c) HVR-L3 comprising the amino acid sequence of SEQ ID NO:25. In one embodiment, the antibody comprises (a) HVR-L1 comprising the amino acid sequence of SEQ ID NO:23; (b) HVR-L2 comprising the amino acid sequence of SEQ ID NO:24; and (c) HVR-L3 comprising the amino acid sequence of SEQ ID NO:25.

In another aspect, an antibody of the invention comprises (a) a VH domain comprising at least one, at least two, or all three VH HVR sequences selected from (i) HVR-H1 comprising the amino acid sequence of SEQ ID NO:26, (ii) HVR-H2 comprising the amino acid sequence of SEQ ID NO:27, and (iii) HVR-H3 comprising an amino acid sequence selected from SEQ ID NO:28; and (b) a VL domain comprising at least one, at least two, or all three VL HVR sequences selected from (i) HVR-L1 comprising the amino acid sequence of SEQ ID NO:23, (ii) HVR-L2 comprising the amino acid sequence of SEQ ID NO:24, and (c) HVR-L3 comprising the amino acid sequence of SEQ ID NO:25.

In another aspect, the invention provides an antibody comprising (a) HVR-H1 comprising the amino acid sequence of SEQ ID NO:26; (b) HVR-H2 comprising the amino acid sequence of SEQ ID NO:27; (c) HVR-H3 comprising the amino acid sequence of SEQ ID NO:28; (d) HVR-L1 comprising the amino acid sequence of SEQ ID NO:23; (e) HVR-L2 comprising the amino acid sequence of SEQ ID NO:24; and (f) HVR-L3 comprising an amino acid sequence selected from SEQ ID NO:25.

In one aspect, the invention provides an anti-RSPO3 antibody comprising at least one, two, three, four, five, or six HVRs selected from (a) HVR-H1 comprising the amino acid sequence of SEQ ID NO:26; (b) HVR-H2 comprising the amino acid sequence of SEQ ID NO:27; (c) HVR-H3 comprising the amino acid sequence of SEQ ID NO:188 or SEQ ID NO:189; (d) HVR-L1 comprising the amino acid sequence of SEQ ID NO:23; (e) HVR-L2 comprising the amino acid sequence of SEQ ID NO:24; and (f) HVR-L3 comprising the amino acid sequence of SEQ ID NO:25. In some embodiments, HVR-H3 comprises the amino acid sequence of SEQ ID NO:188. In some embodiments, HVR-H3 comprises the amino acid sequence of SEQ ID NO:189.

In one aspect, the invention provides an antibody comprising at least one, at least two, or all three VH HVR sequences selected from (a) HVR-H1 comprising the amino acid sequence of SEQ ID NO:26; (b) HVR-H2 comprising the amino acid sequence of SEQ ID NO:27; and (c) HVR-H3 comprising the amino acid sequence of SEQ ID NO:188 or SEQ ID NO:189. In one embodiment, the antibody comprises HVR-H3 comprising the amino acid sequence of SEQ ID NO:188 or SEQ ID NO: 189. In another embodiment, the antibody comprises HVR-H3 comprising the amino acid sequence of SEQ ID NO:188 or SEQ ID NO:189 and HVR-L3 comprising the amino acid sequence of SEQ ID NO:25. In a further embodiment, the antibody comprises HVR-H3 comprising the amino acid sequence of SEQ ID NO:188 or SEQ ID NO:189, HVR-L3 comprising the amino acid sequence of SEQ ID NO:25, and HVR-H2 comprising the amino acid sequence of SEQ ID NO:27. In a further embodiment, the antibody comprises (a) HVR-H1 comprising the amino acid sequence of SEQ ID NO:26; (b) HVR-H2 comprising the amino acid sequence of SEQ ID NO:27; and (c) HVR-H3 comprising the amino acid sequence of SEQ ID NO: 188 or SEQ ID NO:189. In some embodiments, HVR-H3 comprises the amino acid sequence of SEQ ID NO:188. In some embodiments, HVR-H3 comprises the amino acid sequence of SEQ ID NO:189.

In another aspect, the invention provides an antibody comprising at least one, at least two, or all three VL HVR sequences selected from (a) HVR-L1 comprising the amino acid sequence of SEQ ID NO:23; (b) HVR-L2 comprising the amino acid sequence of SEQ ID NO:24; and (c) HVR-L3 comprising the amino acid sequence of SEQ ID NO:25. In one embodiment, the antibody comprises (a) HVR-L1 comprising the amino acid sequence of SEQ ID NO:23; (b) HVR-L2 comprising the amino acid sequence of SEQ ID NO:24; and (c) HVR-L3 comprising the amino acid sequence of SEQ ID NO:25.

In another aspect, an antibody of the invention comprises (a) a VH domain comprising at least one, at least two, or all three VH HVR sequences selected from (i) HVR-H1 comprising the amino acid sequence of SEQ ID NO:26, (ii) HVR-H2 comprising the amino acid sequence of SEQ ID NO:27, and (iii) HVR-H3 comprising an amino acid sequence selected from SEQ ID NO:188 or SEQ ID NO:189; and (b) a VL domain comprising at least one, at least two, or all three VL HVR sequences selected from (i) HVR-L1 comprising the amino acid sequence of SEQ ID NO:23, (ii) HVR-L2 comprising the amino acid sequence of SEQ ID NO:24, and (c) HVR-L3 comprising the amino acid sequence of SEQ ID NO:25. In some embodiments, HVR-H3 comprises the amino acid sequence of SEQ ID NO:188. In some embodiments, HVR-H3 comprises the amino acid sequence of SEQ ID NO:189.

In another aspect, the invention provides an antibody comprising (a) HVR-H1 comprising the amino acid sequence of SEQ ID NO:26; (b) HVR-H2 comprising the amino acid sequence of SEQ ID NO:27; (c) HVR-H3 comprising the amino acid sequence of SEQ ID NO:188 or SEQ ID NO:189; (d) HVR-L1 comprising the amino acid sequence of SEQ ID NO:23; (e) HVR-L2 comprising the amino acid sequence of SEQ ID NO:24; and (f) HVR-L3 comprising an amino acid sequence selected from SEQ ID NO:25. In some embodiments, HVR-H3 comprises the amino acid sequence of SEQ ID NO:188. In some embodiments, HVR-H3 comprises the amino acid sequence of SEQ ID NO:189.

In another aspect, an anti-RSP03 antibody is provided, wherein the antibody comprises a VH as in any of the embodiments provided above, and a VL as in any of the embodiments provided above. In one embodiment, the antibody comprises the VH and VL sequences in SEQ ID NO:95 and SEQ ID NO:96, respectively, including post-translational modifications of those sequences.

In any of the above embodiments, an anti-RSPO3 antibody is humanized. In one embodiment, an anti-RSPO3 antibody comprises HVRs as in any of the above embodiments, and further comprises a human acceptor framework, e.g. a human immunoglobulin framework or a human consensus framework. In certain embodiments, the human acceptor framework is the human VL kappa I consensus (VL_{KI}) framework and/or the VH framework VH₁. In certain embodiments, the human acceptor framework is the human VL kappa I consensus (VL_{KI}) framework and/or the VH framework VH₁ comprising any one of the following mutations.

In another aspect, an anti-RSP03 antibody comprises a heavy chain variable domain (VH) sequence having at least 90%, 91%, 92%, 93%, 94%, 95%, 96%, 97%, 98%, 99%, or 100% sequence identity to the amino acid sequence of SEQ ID NO:191, 193, 195, 197, 199, 201, 203, 205, 207, 209, 211, 213, or 215. In certain embodiments, a VH sequence having at least 90%, 91%, 92%, 93%, 94%, 95%, 96%, 97%, 98%, or 99% identity to the amino acid sequence of SEQ ID NO:191, 193, 195, 197, 199, 201, 203, 205, 207, 209, 211, 213, or 215 contains substitutions (e.g., conservative substitutions), insertions, or deletions relative to the reference sequence, but an anti-RSPO3 antibody comprising that sequence retains the ability to bind to RSPO3. In certain embodiments, a total of 1 to 10 amino acids have been substituted, inserted and/or deleted in SEQ ID NO:191, 193, 195, 197, 199, 201, 203, 205, 207, 209, 211, 213, or 215. In certain embodiments, a total of 1 to 5 amino acids have been substituted, inserted and/or deleted in SEQ ID NO:191, 193, 195, 197, 199, 201, 203, 205, 207, 209, 211, 213, or 215. In certain embodiments, substitutions, insertions, or deletions occur in regions outside the HVRs (*i.e.,* in the FRs). Optionally, the anti-RSP03 antibody comprises the VH sequence of SEQ ID NO:191, 193, 195, 197, 199, 201, 203, 205, 207, 209, 211, 213, or 215, including post-translational modifications of that sequence. In a particular embodiment, the VH comprises one, two or three HVRs selected from: (a) HVR-H1 comprising the amino acid sequence of SEQ ID NO:26, (b) HVR-H2 comprising the amino acid sequence of SEQ ID NO:27, and (c) HVR-H3 comprising the amino acid sequence of SEQ ID NO:28, SEQ ID NO:188, or SEQ ID NO:189.

In another aspect, an anti-RSPO3 antibody is provided, wherein the antibody comprises a light chain variable domain (VL) having at least 90%, 91%, 92%, 93%, 94%, 95%, 96%, 97%, 98%, 99%, or 100% sequence identity to the amino acid sequence of SEQ ID NO:190, 192, 194, 196, 198, 200, 202, 204, 206, 208, 210, 212, or 214. In certain embodiments, a VL sequence having at least 90%, 91%, 92%, 93%, 94%, 95%, 96%, 97%, 98%, or 99% identity to the amino acid sequence of SEQ ID NO:190, 192, 194, 196, 198, 200, 202, 204, 206, 208, 210, 212, or 214contains substitutions (e.g., conservative substitutions), insertions, or deletions relative to the reference sequence, but an anti-RSPO3 antibody comprising that sequence retains the ability to bind to RSPO3. In certain embodiments, a total of 1 to 10 amino acids have been substituted, inserted and/or deleted in SEQ ID NO:190, 192, 194, 196, 198, 200, 202, 204, 206, 208, 210, 212, or 214. In certain embodiments, a total of 1 to 5 amino acids have been substituted, inserted and/or deleted in SEQ ID NO:190, 192, 194, 196, 198, 200, 202, 204, 206, 208, 210, 212, or 214. In certain embodiments, the substitutions, insertions, or deletions occur in regions outside the HVRs (*i.e.,* in the FRs). Optionally, the anti-RSPO3 antibody comprises the VL sequence of SEQ ID NO:190, 192, 194, 196, 198, 200, 202, 204, 206, 208, 210, 212, or 214, including post-translational modifications of that sequence. In a particular embodiment, the VL comprises one, two or three HVRs selected from (a) HVR-L1 comprising the amino acid sequence of SEQ ID NO:23; (b) HVR-L2 comprising the amino acid sequence of SEQ ID NO:24; and (c) HVR-L3 comprising the amino acid sequence of SEQ ID NO:25.

In another aspect, an anti-RSP03 antibody is provided, wherein the antibody comprises a VH as in any of the embodiments provided above, and a VL as in any of the embodiments provided above.

In one embodiment, the antibody comprises the VH and VL sequences in SEQ ID NO:190 and SEQ ID NO:191, respectively, including post-translational modifications of those sequences. In one embodiment, the antibody comprises the VH and VL sequences in SEQ ID NO:192 and SEQ ID NO:193, respectively, including post-translational modifications of those sequences. In one embodiment, the antibody comprises the VH and VL sequences in SEQ ID NO:194 and SEQ ID NO:195, respectively, including post-translational modifications of those sequences. In one embodiment, the antibody comprises the VH and VL sequences in SEQ ID NO:196 and SEQ ID NO:197, respectively, including post-translational modifications of those sequences. In one embodiment, the antibody comprises the VH and VL sequences in SEQ ID NO:198 and SEQ ID NO:199, respectively, including post-translational modifications of those sequences. In one embodiment, the antibody comprises the VH and VL sequences in SEQ ID NO:200 and SEQ ID NO:201, respectively, including post-translational modifications of those sequences. In one embodiment, the antibody comprises the VH and VL sequences in SEQ ID NO:202 and SEQ ID NO:203, respectively, including post-translational modifications of those sequences. In one embodiment, the antibody comprises the VH and VL sequences in SEQ ID NO:204 and SEQ ID NO:205, respectively, including post-translational modifications of those sequences. In one embodiment, the antibody comprises the VH and VL sequences in SEQ ID NO:206 and SEQ ID NO:207, respectively, including post-translational modifications of those sequences. In one embodiment, the antibody comprises the VH and VL sequences in SEQ ID NO:208 and SEQ ID NO:209, respectively, including post-translational modifications of those sequences. In one embodiment, the antibody comprises the VH and VL sequences in SEQ ID NO:210 and SEQ ID NO:211, respectively, including post-translational modifications of those sequences. In one embodiment, the antibody comprises the VH and VL sequences in SEQ ID NO:212 and SEQ ID NO:213, respectively, including post-translational modifications of those sequences. In one embodiment, the antibody comprises the VH and VL sequences in SEQ ID NO:214 and SEQ ID NO:215, respectively, including post-translational modifications of those sequences.

In a further aspect, provided are herein are antibodies that bind to the same epitope as an anti-RSPO3 antibody provided herein. For example, in certain embodiments, an antibody is provided that binds to the same epitope as an anti-RSPO3 antibody comprising a VH sequence of SEQ ID NO: 191, 193, 195, 197, 199, 201, 203, 205, 207, 209, 211, 213, or 215 and a VL sequence of SEQ ID NO:190, 192, 194, 196, 198, 200, 202, 204, 206, 208, 210, 212, or 214, respectively. In some embodiments, the epitope is determined by crystallography.

In a further aspect of the invention, an anti-RSPO3 antibody according to any of the above embodiments is a monoclonal antibody, including a human antibody. In one embodiment, an anti-RSPO3 antibody is an antibody fragment, e.g., a Fv, Fab, Fab', scFv, diabody, or F(ab')₂ fragment. In another embodiment, the antibody is a substantially full length antibody, e.g., an IgG1 antibody, IgG2a antibody or other antibody class or isotype as defined herein.

### Monoclonal Antibody 5E11 and Certain Other Antibody Embodiments

In one aspect, the invention provides an anti-RSPO3 antibody comprising at least one, two, three, four, five, or six HVRs selected from (a) HVR-H1 comprising the amino acid sequence of SEQ ID NO:32; (b) HVR-H2 comprising the amino acid sequence of SEQ ID NO:33; (c) HVR-H3 comprising the amino acid sequence of SEQ ID NO:34; (d) HVR-L1 comprising the amino acid sequence of SEQ ID NO:29; (e) HVR-L2 comprising the amino acid sequence of SEQ ID NO:30; and (f) HVR-L3 comprising the amino acid sequence of SEQ ID NO:31.

In one aspect, the invention provides an antibody comprising at least one, at least two, or all three VH HVR sequences selected from (a) HVR-H1 comprising the amino acid sequence of SEQ ID NO:32; (b) HVR-H2 comprising the amino acid sequence of SEQ ID NO:33; and (c) HVR-H3 comprising the amino acid sequence of SEQ ID NO:34. In one embodiment, the antibody comprises HVR-H3 comprising the amino acid sequence of SEQ ID NO:34. In another embodiment, the antibody comprises HVR-H3 comprising the amino acid sequence of SEQ ID NO:34 and HVR-L3 comprising the amino acid sequence of SEQ ID NO:31. In a further embodiment, the antibody comprises HVR-H3 comprising the amino acid sequence of SEQ ID NO:34, HVR-L3 comprising the amino acid sequence of SEQ ID NO:31, and HVR-H2 comprising the amino acid sequence of SEQ ID NO:33. In a further embodiment, the antibody comprises (a) HVR-H1 comprising the amino acid sequence of SEQ ID NO:32; (b) HVR-H2 comprising the amino acid sequence of SEQ ID NO:33; and (c) HVR-H3 comprising the amino acid sequence of SEQ ID NO:34.

In another aspect, the invention provides an antibody comprising at least one, at least two, or all three VL HVR sequences selected from (a) HVR-L1 comprising the amino acid sequence of SEQ ID NO:29; (b) HVR-L2 comprising the amino acid sequence of SEQ ID NO:30; and (c) HVR-L3 comprising the amino acid sequence of SEQ ID NO:31. In one embodiment, the antibody comprises (a) HVR-L1 comprising the amino acid sequence of SEQ ID NO:29; (b) HVR-L2 comprising the amino acid sequence of SEQ ID NO:30; and (c) HVR-L3 comprising the amino acid sequence of SEQ ID NO:31.

In another aspect, an antibody of the invention comprises (a) a VH domain comprising at least one, at least two, or all three VH HVR sequences selected from (i) HVR-H1 comprising the amino acid sequence of SEQ ID NO:32, (ii) HVR-H2 comprising the amino acid sequence of SEQ ID NO:33, and (iii) HVR-H3 comprising an amino acid sequence selected from SEQ ID NO:34; and (b) a VL domain comprising at least one, at least two, or all three VL HVR sequences selected from (i) HVR-L1 comprising the amino acid sequence of SEQ ID NO:29, (ii) HVR-L2 comprising the amino acid sequence of SEQ ID NO:30, and (c) HVR-L3 comprising the amino acid sequence of SEQ ID NO:31.

In another aspect, the invention provides an antibody comprising (a) HVR-H1 comprising the amino acid sequence of SEQ ID NO:32; (b) HVR-H2 comprising the amino acid sequence of SEQ ID NO:33; (c) HVR-H3 comprising the amino acid sequence of SEQ ID NO:34; (d) HVR-L1 comprising the amino acid sequence of SEQ ID NO:29; (e) HVR-L2 comprising the amino acid sequence of SEQ ID NO:30; and (f) HVR-L3 comprising an amino acid sequence selected from SEQ ID NO:31.

In another aspect, an anti-RSP03 antibody is provided, wherein the antibody comprises a VH as in any of the embodiments provided above, and a VL as in any of the embodiments provided above. In one embodiment, the antibody comprises the VH and VL sequences in SEQ ID NO:97 and SEQ ID NO:98, respectively, including post-translational modifications of those sequences.

### Monoclonal Antibody 6E9 and Certain Other Antibody Embodiments

In one aspect, the invention provides an anti-RSPO3 antibody comprising at least one, two, three, four, five, or six HVRs selected from (a) HVR-H1 comprising the amino acid sequence of SEQ ID NO:38; (b) HVR-H2 comprising the amino acid sequence of SEQ ID NO:39; (c) HVR-H3 comprising the amino acid sequence of SEQ ID NO:40; (d) HVR-L1 comprising the amino acid sequence of SEQ ID NO:35; (e) HVR-L2 comprising the amino acid sequence of SEQ ID NO:36; and (f) HVR-L3 comprising the amino acid sequence of SEQ ID NO:37.

In one aspect, the invention provides an antibody comprising at least one, at least two, or all three VH HVR sequences selected from (a) HVR-H1 comprising the amino acid sequence of SEQ ID NO:38; (b) HVR-H2 comprising the amino acid sequence of SEQ ID NO:39; and (c) HVR-H3 comprising the amino acid sequence of SEQ ID NO:40. In one embodiment, the antibody comprises HVR-H3 comprising the amino acid sequence of SEQ ID NO:40. In another embodiment, the antibody comprises HVR-H3 comprising the amino acid sequence of SEQ ID NO:40 and HVR-L3 comprising the amino acid sequence of SEQ ID NO:37. In a further embodiment, the antibody comprises HVR-H3 comprising the amino acid sequence of SEQ ID NO:40, HVR-L3 comprising the amino acid sequence of SEQ ID NO:37, and HVR-H2 comprising the amino acid sequence of SEQ ID NO:39. In a further embodiment, the antibody comprises (a) HVR-H1 comprising the amino acid sequence of SEQ ID NO:38; (b) HVR-H2 comprising the amino acid sequence of SEQ ID NO:39; and (c) HVR-H3 comprising the amino acid sequence of SEQ ID NO:40.

In another aspect, the invention provides an antibody comprising at least one, at least two, or all three VL HVR sequences selected from (a) HVR-L1 comprising the amino acid sequence of SEQ ID NO:35; (b) HVR-L2 comprising the amino acid sequence of SEQ ID NO:36; and (c) HVR-L3 comprising the amino acid sequence of SEQ ID NO:37. In one embodiment, the antibody comprises (a) HVR-L1 comprising the amino acid sequence of SEQ ID NO:35; (b) HVR-L2 comprising the amino acid sequence of SEQ ID NO:36; and (c) HVR-L3 comprising the amino acid sequence of SEQ ID NO:37.

In another aspect, an antibody of the invention comprises (a) a VH domain comprising at least one, at least two, or all three VH HVR sequences selected from (i) HVR-H1 comprising the amino acid sequence of SEQ ID NO:38, (ii) HVR-H2 comprising the amino acid sequence of SEQ ID NO:39, and (iii) HVR-H3 comprising an amino acid sequence selected from SEQ ID NO:40; and (b) a VL domain comprising at least one, at least two, or all three VL HVR sequences selected from (i) HVR-L1 comprising the amino acid sequence of SEQ ID NO:35, (ii) HVR-L2 comprising the amino acid sequence of SEQ ID NO:36, and (c) HVR-L3 comprising the amino acid sequence of SEQ ID NO:37.

In another aspect, the invention provides an antibody comprising (a) HVR-H1 comprising the amino acid sequence of SEQ ID NO:38; (b) HVR-H2 comprising the amino acid sequence of SEQ ID NO:39; (c) HVR-H3 comprising the amino acid sequence of SEQ ID NO:40; (d) HVR-L1 comprising the amino acid sequence of SEQ ID NO:35; (e) HVR-L2 comprising the amino acid sequence of SEQ ID NO:36; and (f) HVR-L3 comprising an amino acid sequence selected from SEQ ID NO:37.

In another aspect, an anti-RSP03 antibody is provided, wherein the antibody comprises a VH as in any of the embodiments provided above, and a VL as in any of the embodiments provided above. In one embodiment, the antibody comprises the VH and VL sequences in SEQ ID NO:99 and SEQ ID NO: 100, respectively, including post-translational modifications of those sequences.

### Monoclonal Antibody 21C2 and Certain Other Antibody Embodiments

In one aspect, the invention provides an anti-RSPO3 antibody comprising at least one, two, three, four, five, or six HVRs selected from (a) HVR-H1 comprising the amino acid sequence of SEQ ID NO:44; (b) HVR-H2 comprising the amino acid sequence of SEQ ID NO:45; (c) HVR-H3 comprising the amino acid sequence of SEQ ID NO:46; (d) HVR-L1 comprising the amino acid sequence of SEQ ID NO:41; (e) HVR-L2 comprising the amino acid sequence of SEQ ID NO:42; and (f) HVR-L3 comprising the amino acid sequence of SEQ ID NO:43.

In one aspect, the invention provides an antibody comprising at least one, at least two, or all three VH HVR sequences selected from (a) HVR-H1 comprising the amino acid sequence of SEQ ID NO:44; (b) HVR-H2 comprising the amino acid sequence of SEQ ID NO:45; and (c) HVR-H3 comprising the amino acid sequence of SEQ ID NO:46. In one embodiment, the antibody comprises HVR-H3 comprising the amino acid sequence of SEQ ID NO:46. In another embodiment, the antibody comprises HVR-H3 comprising the amino acid sequence of SEQ ID NO:46 and HVR-L3 comprising the amino acid sequence of SEQ ID NO:43. In a further embodiment, the antibody comprises HVR-H3 comprising the amino acid sequence of SEQ ID NO:46, HVR-L3 comprising the amino acid sequence of SEQ ID NO:43, and HVR-H2 comprising the amino acid sequence of SEQ ID NO:45. In a further embodiment, the antibody comprises (a) HVR-H1 comprising the amino acid sequence of SEQ ID NO:44; (b) HVR-H2 comprising the amino acid sequence of SEQ ID NO:45; and (c) HVR-H3 comprising the amino acid sequence of SEQ ID NO:46.

In another aspect, the invention provides an antibody comprising at least one, at least two, or all three VL HVR sequences selected from (a) HVR-L1 comprising the amino acid sequence of SEQ ID NO:41; (b) HVR-L2 comprising the amino acid sequence of SEQ ID NO:42; and (c) HVR-L3 comprising the amino acid sequence of SEQ ID NO:43. In one embodiment, the antibody comprises (a) HVR-L1 comprising the amino acid sequence of SEQ ID NO:41; (b) HVR-L2 comprising the amino acid sequence of SEQ ID NO:42; and (c) HVR-L3 comprising the amino acid sequence of SEQ ID NO:43.

In another aspect, an antibody of the invention comprises (a) a VH domain comprising at least one, at least two, or all three VH HVR sequences selected from (i) HVR-H1 comprising the amino acid sequence of SEQ ID NO:44, (ii) HVR-H2 comprising the amino acid sequence of SEQ ID NO:45, and (iii) HVR-H3 comprising an amino acid sequence selected from SEQ ID NO:46; and (b) a VL domain comprising at least one, at least two, or all three VL HVR sequences selected from (i) HVR-L1 comprising the amino acid sequence of SEQ ID NO:41, (ii) HVR-L2 comprising the amino acid sequence of SEQ ID NO:42, and (c) HVR-L3 comprising the amino acid sequence of SEQ ID NO:43.

In another aspect, the invention provides an antibody comprising (a) HVR-H1 comprising the amino acid sequence of SEQ ID NO:44; (b) HVR-H2 comprising the amino acid sequence of SEQ ID NO:45; (c) HVR-H3 comprising the amino acid sequence of SEQ ID NO:46; (d) HVR-L1 comprising the amino acid sequence of SEQ ID NO:41; (e) HVR-L2 comprising the amino acid sequence of SEQ ID NO:42; and (f) HVR-L3 comprising an amino acid sequence selected from SEQ ID NO:43.

In another aspect, an anti-RSP03 antibody is provided, wherein the antibody comprises a VH as in any of the embodiments provided above, and a VL as in any of the embodiments provided above. In one embodiment, the antibody comprises the VH and VL sequences in SEQ ID NO:101 and SEQ ID NO:102, respectively, including post-translational modifications of those sequences.

### Monoclonal Antibody 26E11 and Certain Other Antibody Embodiments

In one aspect, the invention provides an anti-RSPO2/3 antibody comprising at least one, two, three, four, five, or six HVRs selected from (a) HVR-H1 comprising the amino acid sequence of SEQ ID NO:50; (b) HVR-H2 comprising the amino acid sequence of SEQ ID NO:51; (c) HVR-H3 comprising the amino acid sequence of SEQ ID NO:52; (d) HVR-L1 comprising the amino acid sequence of SEQ ID NO:47; (e) HVR-L2 comprising the amino acid sequence of SEQ ID NO:48; and (f) HVR-L3 comprising the amino acid sequence of SEQ ID NO:49.

In one aspect, the invention provides an antibody comprising at least one, at least two, or all three VH HVR sequences selected from (a) HVR-H1 comprising the amino acid sequence of SEQ ID NO:50; (b) HVR-H2 comprising the amino acid sequence of SEQ ID NO:51; and (c) HVR-H3 comprising the amino acid sequence of SEQ ID NO:52. In one embodiment, the antibody comprises HVR-H3 comprising the amino acid sequence of SEQ ID NO:52. In another embodiment, the antibody comprises HVR-H3 comprising the amino acid sequence of SEQ ID NO:52 and HVR-L3 comprising the amino acid sequence of SEQ ID NO:49. In a further embodiment, the antibody comprises HVR-H3 comprising the amino acid sequence of SEQ ID NO:52, HVR-L3 comprising the amino acid sequence of SEQ ID NO:49, and HVR-H2 comprising the amino acid sequence of SEQ ID NO:51. In a further embodiment, the antibody comprises (a) HVR-H1 comprising the amino acid sequence of SEQ ID NO:50; (b) HVR-H2 comprising the amino acid sequence of SEQ ID NO:51; and (c) HVR-H3 comprising the amino acid sequence of SEQ ID NO:52.

In another aspect, the invention provides an antibody comprising at least one, at least two, or all three VL HVR sequences selected from (a) HVR-L1 comprising the amino acid sequence of SEQ ID NO:47; (b) HVR-L2 comprising the amino acid sequence of SEQ ID NO:48; and (c) HVR-L3 comprising the amino acid sequence of SEQ ID NO:49. In one embodiment, the antibody comprises (a) HVR-L1 comprising the amino acid sequence of SEQ ID NO:47; (b) HVR-L2 comprising the amino acid sequence of SEQ ID NO:48; and (c) HVR-L3 comprising the amino acid sequence of SEQ ID NO:49.

In another aspect, an antibody of the invention comprises (a) a VH domain comprising at least one, at least two, or all three VH HVR sequences selected from (i) HVR-H1 comprising the amino acid sequence of SEQ ID NO:50, (ii) HVR-H2 comprising the amino acid sequence of SEQ ID NO:51, and (iii) HVR-H3 comprising an amino acid sequence selected from SEQ ID NO:52; and (b) a VL domain comprising at least one, at least two, or all three VL HVR sequences selected from (i) HVR-L1 comprising the amino acid sequence of SEQ ID NO:47, (ii) HVR-L2 comprising the amino acid sequence of SEQ ID NO:48, and (c) HVR-L3 comprising the amino acid sequence of SEQ ID NO:49.

In another aspect, the invention provides an antibody comprising (a) HVR-H1 comprising the amino acid sequence of SEQ ID NO:50; (b) HVR-H2 comprising the amino acid sequence of SEQ ID NO:51; (c) HVR-H3 comprising the amino acid sequence of SEQ ID NO:52; (d) HVR-L1 comprising the amino acid sequence of SEQ ID NO:47; (e) HVR-L2 comprising the amino acid sequence of SEQ ID NO:48; and (f) HVR-L3 comprising an amino acid sequence selected from SEQ ID NO:49.

In another aspect, an anti-RSP02/3 antibody is provided, wherein the antibody comprises a VH as in any of the embodiments provided above, and a VL as in any of the embodiments provided above. In one embodiment, the antibody comprises the VH and VL sequences in SEQ ID NO:103 and SEQ ID NO: 104, respectively, including post-translational modifications of those sequences.

### Anti-RSPO3 Monoclonal Antibodies and Certain Other Antibody Embodiments

In one aspect, the invention provides an anti-RSPO3 antibody comprising at least one, two, three, four, five, or six HVRs selected from (a) HVR-H1 comprising the amino acid sequence of SEQ ID NO:80; (b) HVR-H2 comprising the amino acid sequence of SEQ ID NO:81; (c) HVR-H3 comprising the amino acid sequence of SEQ ID NO:82; (d) HVR-L1 comprising the amino acid sequence of SEQ ID NO:77; (e) HVR-L2 comprising the amino acid sequence of SEQ ID NO:78; and (f) HVR-L3 comprising the amino acid sequence of SEQ ID NO:79.

In one aspect, the invention provides an antibody comprising at least one, at least two, or all three VH HVR sequences selected from (a) HVR-H1 comprising the amino acid sequence of SEQ ID NO:80; (b) HVR-H2 comprising the amino acid sequence of SEQ ID NO:81; and (c) HVR-H3 comprising the amino acid sequence of SEQ ID NO:82. In one embodiment, the antibody comprises HVR-H3 comprising the amino acid sequence of SEQ ID NO:82. In another embodiment, the antibody comprises HVR-H3 comprising the amino acid sequence of SEQ ID NO:82 and HVR-L3 comprising the amino acid sequence of SEQ ID NO:79. In a further embodiment, the antibody comprises HVR-H3 comprising the amino acid sequence of SEQ ID NO:82, HVR-L3 comprising the amino acid sequence of SEQ ID NO:79, and HVR-H2 comprising the amino acid sequence of SEQ ID NO:81. In a further embodiment, the antibody comprises (a) HVR-H1 comprising the amino acid sequence of SEQ ID NO:80; (b) HVR-H2 comprising the amino acid sequence of SEQ ID NO:81; and (c) HVR-H3 comprising the amino acid sequence of SEQ ID NO:82.

In another aspect, the invention provides an antibody comprising at least one, at least two, or all three VL HVR sequences selected from (a) HVR-L1 comprising the amino acid sequence of SEQ ID NO:77; (b) HVR-L2 comprising the amino acid sequence of SEQ ID NO:78; and (c) HVR-L3 comprising the amino acid sequence of SEQ ID NO:79. In one embodiment, the antibody comprises (a) HVR-L1 comprising the amino acid sequence of SEQ ID NO:77; (b) HVR-L2 comprising the amino acid sequence of SEQ ID NO:78; and (c) HVR-L3 comprising the amino acid sequence of SEQ ID NO:79.

In another aspect, an antibody of the invention comprises (a) a VH domain comprising at least one, at least two, or all three VH HVR sequences selected from (i) HVR-H1 comprising the amino acid sequence of SEQ ID NO:80, (ii) HVR-H2 comprising the amino acid sequence of SEQ ID NO:81, and (iii) HVR-H3 comprising an amino acid sequence selected from SEQ ID NO:82; and (b) a VL domain comprising at least one, at least two, or all three VL HVR sequences selected from (i) HVR-L1 comprising the amino acid sequence of SEQ ID NO:77, (ii) HVR-L2 comprising the amino acid sequence of SEQ ID NO:78, and (c) HVR-L3 comprising the amino acid sequence of SEQ ID NO:79.

In another aspect, the invention provides an antibody comprising (a) HVR-H1 comprising the amino acid sequence of SEQ ID NO:80; (b) HVR-H2 comprising the amino acid sequence of SEQ ID NO:81; (c) HVR-H3 comprising the amino acid sequence of SEQ ID NO:82; (d) HVR-L1 comprising the amino acid sequence of SEQ ID NO:77; (e) HVR-L2 comprising the amino acid sequence of SEQ ID NO:78; and (f) HVR-L3 comprising an amino acid sequence selected from SEQ ID NO:79.

In one aspect, the invention provides an anti-RSPO3 antibody comprising at least one, two, three, four, five, or six HVRs selected from (a) HVR-H1 comprising the amino acid sequence of SEQ ID NO:86; (b) HVR-H2 comprising the amino acid sequence of SEQ ID NO:87; (c) HVR-H3 comprising the amino acid sequence of SEQ ID NO:88; (d) HVR-L1 comprising the amino acid sequence of SEQ ID NO:83; (e) HVR-L2 comprising the amino acid sequence of SEQ ID NO:84; and (f) HVR-L3 comprising the amino acid sequence of SEQ ID NO:85.

In one aspect, the invention provides an antibody comprising at least one, at least two, or all three VH HVR sequences selected from (a) HVR-H1 comprising the amino acid sequence of SEQ ID NO:86; (b) HVR-H2 comprising the amino acid sequence of SEQ ID NO:87; and (c) HVR-H3 comprising the amino acid sequence of SEQ ID NO:88. In one embodiment, the antibody comprises HVR-H3 comprising the amino acid sequence of SEQ ID NO:88. In another embodiment, the antibody comprises HVR-H3 comprising the amino acid sequence of SEQ ID NO:88 and HVR-L3 comprising the amino acid sequence of SEQ ID NO:85. In a further embodiment, the antibody comprises HVR-H3 comprising the amino acid sequence of SEQ ID NO:88, HVR-L3 comprising the amino acid sequence of SEQ ID NO:85, and HVR-H2 comprising the amino acid sequence of SEQ ID NO:87. In a further embodiment, the antibody comprises (a) HVR-H1 comprising the amino acid sequence of SEQ ID NO:86; (b) HVR-H2 comprising the amino acid sequence of SEQ ID NO:87; and (c) HVR-H3 comprising the amino acid sequence of SEQ ID NO:88.

In another aspect, the invention provides an antibody comprising at least one, at least two, or all three VL HVR sequences selected from (a) HVR-L1 comprising the amino acid sequence of SEQ ID NO:83; (b) HVR-L2 comprising the amino acid sequence of SEQ ID NO:84; and (c) HVR-L3 comprising the amino acid sequence of SEQ ID NO:85. In one embodiment, the antibody comprises (a) HVR-L1 comprising the amino acid sequence of SEQ ID NO:83; (b) HVR-L2 comprising the amino acid sequence of SEQ ID NO:84; and (c) HVR-L3 comprising the amino acid sequence of SEQ ID NO:85.

In another aspect, an antibody of the invention comprises (a) a VH domain comprising at least one, at least two, or all three VH HVR sequences selected from (i) HVR-H1 comprising the amino acid sequence of SEQ ID NO:86, (ii) HVR-H2 comprising the amino acid sequence of SEQ ID NO:87, and (iii) HVR-H3 comprising an amino acid sequence selected from SEQ ID NO:88; and (b) a VL domain comprising at least one, at least two, or all three VL HVR sequences selected from (i) HVR-L1 comprising the amino acid sequence of SEQ ID NO:83, (ii) HVR-L2 comprising the amino acid sequence of SEQ ID NO:84, and (c) HVR-L3 comprising the amino acid sequence of SEQ ID NO:85.

In another aspect, the invention provides an antibody comprising (a) HVR-H1 comprising the amino acid sequence of SEQ ID NO:86; (b) HVR-H2 comprising the amino acid sequence of SEQ ID NO:87; (c) HVR-H3 comprising the amino acid sequence of SEQ ID NO:88; (d) HVR-L1 comprising the amino acid sequence of SEQ ID NO:83; (e) HVR-L2 comprising the amino acid sequence of SEQ ID NO:84; and (f) HVR-L3 comprising an amino acid sequence selected from SEQ ID NO:85.

### Monoclonal Antibody 1A1 and Certain Other Antibody Embodiments

In one aspect, the invention provides an anti-RSPO2 antibody comprising at least one, two, three, four, five, or six HVRs selected from (a) HVR-H1 comprising the amino acid sequence of SEQ ID NO:56; (b) HVR-H2 comprising the amino acid sequence of SEQ ID NO:57; (c) HVR-H3 comprising the amino acid sequence of SEQ ID NO:58; (d) HVR-L1 comprising the amino acid sequence of SEQ ID NO:53; (e) HVR-L2 comprising the amino acid sequence of SEQ ID NO:54; and (f) HVR-L3 comprising the amino acid sequence of SEQ ID NO:55.

In one aspect, the invention provides an antibody comprising at least one, at least two, or all three VH HVR sequences selected from (a) HVR-H1 comprising the amino acid sequence of SEQ ID NO:56; (b) HVR-H2 comprising the amino acid sequence of SEQ ID NO:57; and (c) HVR-H3 comprising the amino acid sequence of SEQ ID NO:58. In one embodiment, the antibody comprises HVR-H3 comprising the amino acid sequence of SEQ ID NO:58. In another embodiment, the antibody comprises HVR-H3 comprising the amino acid sequence of SEQ ID NO:58 and HVR-L3 comprising the amino acid sequence of SEQ ID NO:55. In a further embodiment, the antibody comprises HVR-H3 comprising the amino acid sequence of SEQ ID NO:58, HVR-L3 comprising the amino acid sequence of SEQ ID NO:55, and HVR-H2 comprising the amino acid sequence of SEQ ID NO:57. In a further embodiment, the antibody comprises (a) HVR-H1 comprising the amino acid sequence of SEQ ID NO:56; (b) HVR-H2 comprising the amino acid sequence of SEQ ID NO:57; and (c) HVR-H3 comprising the amino acid sequence of SEQ ID NO:58.

In another aspect, the invention provides an antibody comprising at least one, at least two, or all three VL HVR sequences selected from (a) HVR-L1 comprising the amino acid sequence of SEQ ID NO:53; (b) HVR-L2 comprising the amino acid sequence of SEQ ID NO:54; and (c) HVR-L3 comprising the amino acid sequence of SEQ ID NO:55. In one embodiment, the antibody comprises (a) HVR-L1 comprising the amino acid sequence of SEQ ID NO:53; (b) HVR-L2 comprising the amino acid sequence of SEQ ID NO:54; and (c) HVR-L3 comprising the amino acid sequence of SEQ ID NO:55.

In another aspect, an antibody of the invention comprises (a) a VH domain comprising at least one, at least two, or all three VH HVR sequences selected from (i) HVR-H1 comprising the amino acid sequence of SEQ ID NO:56, (ii) HVR-H2 comprising the amino acid sequence of SEQ ID NO:57, and (iii) HVR-H3 comprising an amino acid sequence selected from SEQ ID NO:58; and (b) a VL domain comprising at least one, at least two, or all three VL HVR sequences selected from (i) HVR-L1 comprising the amino acid sequence of SEQ ID NO:53, (ii) HVR-L2 comprising the amino acid sequence of SEQ ID NO:54, and (c) HVR-L3 comprising the amino acid sequence of SEQ ID NO:55.

In another aspect, the invention provides an antibody comprising (a) HVR-H1 comprising the amino acid sequence of SEQ ID NO:56; (b) HVR-H2 comprising the amino acid sequence of SEQ ID NO:57; (c) HVR-H3 comprising the amino acid sequence of SEQ ID NO:58; (d) HVR-L1 comprising the amino acid sequence of SEQ ID NO:53; (e) HVR-L2 comprising the amino acid sequence of SEQ ID NO:54; and (f) HVR-L3 comprising an amino acid sequence selected from SEQ ID NO:55.

In another aspect, an anti-RSP02 antibody is provided, wherein the antibody comprises a VH as in any of the embodiments provided above, and a VL as in any of the embodiments provided above. In one embodiment, the antibody comprises the VH and VL sequences in SEQ ID NO: 105 and SEQ ID NO: 106, respectively, including post-translational modifications of those sequences.

### Monoclonal Antibody 11F11 and Certain Other Antibody Embodiments

In one aspect, the invention provides an anti-RSPO2 antibody comprising at least one, two, three, four, five, or six HVRs selected from (a) HVR-H1 comprising the amino acid sequence of SEQ ID NO:62; (b) HVR-H2 comprising the amino acid sequence of SEQ ID NO:63; (c) HVR-H3 comprising the amino acid sequence of SEQ ID NO:64; (d) HVR-L1 comprising the amino acid sequence of SEQ ID NO:59; (e) HVR-L2 comprising the amino acid sequence of SEQ ID NO:60; and (f) HVR-L3 comprising the amino acid sequence of SEQ ID NO:61.

In one aspect, the invention provides an antibody comprising at least one, at least two, or all three VH HVR sequences selected from (a) HVR-H1 comprising the amino acid sequence of SEQ ID NO:62; (b) HVR-H2 comprising the amino acid sequence of SEQ ID NO:63; and (c) HVR-H3 comprising the amino acid sequence of SEQ ID NO:64. In one embodiment, the antibody comprises HVR-H3 comprising the amino acid sequence of SEQ ID NO:64. In another embodiment, the antibody comprises HVR-H3 comprising the amino acid sequence of SEQ ID NO:64 and HVR-L3 comprising the amino acid sequence of SEQ ID NO:61. In a further embodiment, the antibody comprises HVR-H3 comprising the amino acid sequence of SEQ ID NO:64, HVR-L3 comprising the amino acid sequence of SEQ ID NO:61, and HVR-H2 comprising the amino acid sequence of SEQ ID NO:63. In a further embodiment, the antibody comprises (a) HVR-H1 comprising the amino acid sequence of SEQ ID NO:62; (b) HVR-H2 comprising the amino acid sequence of SEQ ID NO:63; and (c) HVR-H3 comprising the amino acid sequence of SEQ ID NO:64.

In another aspect, the invention provides an antibody comprising at least one, at least two, or all three VL HVR sequences selected from (a) HVR-L1 comprising the amino acid sequence of SEQ ID NO:59; (b) HVR-L2 comprising the amino acid sequence of SEQ ID NO:60; and (c) HVR-L3 comprising the amino acid sequence of SEQ ID NO:61. In one embodiment, the antibody comprises (a) HVR-L1 comprising the amino acid sequence of SEQ ID NO:59; (b) HVR-L2 comprising the amino acid sequence of SEQ ID NO:60; and (c) HVR-L3 comprising the amino acid sequence of SEQ ID NO:61.

In another aspect, an antibody of the invention comprises (a) a VH domain comprising at least one, at least two, or all three VH HVR sequences selected from (i) HVR-H1 comprising the amino acid sequence of SEQ ID NO:62, (ii) HVR-H2 comprising the amino acid sequence of SEQ ID NO:63, and (iii) HVR-H3 comprising an amino acid sequence selected from SEQ ID NO:64; and (b) a VL domain comprising at least one, at least two, or all three VL HVR sequences selected from (i) HVR-L1 comprising the amino acid sequence of SEQ ID NO:59, (ii) HVR-L2 comprising the amino acid sequence of SEQ ID NO:60, and (c) HVR-L3 comprising the amino acid sequence of SEQ ID NO:61.

In another aspect, the invention provides an antibody comprising (a) HVR-H1 comprising the amino acid sequence of SEQ ID NO:62; (b) HVR-H2 comprising the amino acid sequence of SEQ ID NO:63; (c) HVR-H3 comprising the amino acid sequence of SEQ ID NO:64; (d) HVR-L1 comprising the amino acid sequence of SEQ ID NO:59; (e) HVR-L2 comprising the amino acid sequence of SEQ ID NO:60; and (f) HVR-L3 comprising an amino acid sequence selected from SEQ ID NO:61.

In another aspect, an anti-RSP02 antibody is provided, wherein the antibody comprises a VH as in any of the embodiments provided above, and a VL as in any of the embodiments provided above. In one embodiment, the antibody comprises the VH and VL sequences in SEQ ID NO:107 and SEQ ID NO:108, respectively, including post-translational modifications of those sequences.

### Monoclonal Antibody 36D2 and Certain Other Antibody Embodiments

In one aspect, the invention provides an anti-RSPO2 antibody comprising at least one, two, three, four, five, or six HVRs selected from (a) HVR-H1 comprising the amino acid sequence of SEQ ID NO:68; (b) HVR-H2 comprising the amino acid sequence of SEQ ID NO:69; (c) HVR-H3 comprising the amino acid sequence of SEQ ID NO:70; (d) HVR-L1 comprising the amino acid sequence of SEQ ID NO:65; (e) HVR-L2 comprising the amino acid sequence of SEQ ID NO:66; and (f) HVR-L3 comprising the amino acid sequence of SEQ ID NO:67.

In one aspect, the invention provides an antibody comprising at least one, at least two, or all three VH HVR sequences selected from (a) HVR-H1 comprising the amino acid sequence of SEQ ID NO:68; (b) HVR-H2 comprising the amino acid sequence of SEQ ID NO:69; and (c) HVR-H3 comprising the amino acid sequence of SEQ ID NO:70. In one embodiment, the antibody comprises HVR-H3 comprising the amino acid sequence of SEQ ID NO:70. In another embodiment, the antibody comprises HVR-H3 comprising the amino acid sequence of SEQ ID NO:70 and HVR-L3 comprising the amino acid sequence of SEQ ID NO:67. In a further embodiment, the antibody comprises HVR-H3 comprising the amino acid sequence of SEQ ID NO:70, HVR-L3 comprising the amino acid sequence of SEQ ID NO:67, and HVR-H2 comprising the amino acid sequence of SEQ ID NO:69. In a further embodiment, the antibody comprises (a) HVR-H1 comprising the amino acid sequence of SEQ ID NO:68; (b) HVR-H2 comprising the amino acid sequence of SEQ ID NO:69; and (c) HVR-H3 comprising the amino acid sequence of SEQ ID NO:70.

In another aspect, the invention provides an antibody comprising at least one, at least two, or all three VL HVR sequences selected from (a) HVR-L1 comprising the amino acid sequence of SEQ ID NO:65; (b) HVR-L2 comprising the amino acid sequence of SEQ ID NO:66; and (c) HVR-L3 comprising the amino acid sequence of SEQ ID NO:67. In one embodiment, the antibody comprises (a) HVR-L1 comprising the amino acid sequence of SEQ ID NO:65; (b) HVR-L2 comprising the amino acid sequence of SEQ ID NO:66; and (c) HVR-L3 comprising the amino acid sequence of SEQ ID NO:67.

In another aspect, an antibody of the invention comprises (a) a VH domain comprising at least one, at least two, or all three VH HVR sequences selected from (i) HVR-H1 comprising the amino acid sequence of SEQ ID NO:68, (ii) HVR-H2 comprising the amino acid sequence of SEQ ID NO:69, and (iii) HVR-H3 comprising an amino acid sequence selected from SEQ ID NO:70; and (b) a VL domain comprising at least one, at least two, or all three VL HVR sequences selected from (i) HVR-L1 comprising the amino acid sequence of SEQ ID NO:65, (ii) HVR-L2 comprising the amino acid sequence of SEQ ID NO:66, and (c) HVR-L3 comprising the amino acid sequence of SEQ ID NO:67.

In another aspect, the invention provides an antibody comprising (a) HVR-H1 comprising the amino acid sequence of SEQ ID NO:68; (b) HVR-H2 comprising the amino acid sequence of SEQ ID NO:69; (c) HVR-H3 comprising the amino acid sequence of SEQ ID NO:70; (d) HVR-L1 comprising the amino acid sequence of SEQ ID NO:65; (e) HVR-L2 comprising the amino acid sequence of SEQ ID NO:66; and (f) HVR-L3 comprising an amino acid sequence selected from SEQ ID NO:67.

In another aspect, an anti-RSPO2 antibody is provided, wherein the antibody comprises a VH as in any of the embodiments provided above, and a VL as in any of the embodiments provided above. In one embodiment, the antibody comprises the VH and VL sequences in SEQ ID NO: 109 and SEQ ID NO:110, respectively, including post-translational modifications of those sequences.

### Monoclonal Antibody 49G5 and Certain Other Antibody Embodiments

In one aspect, the invention provides an anti-RSPO2 antibody comprising at least one, two, three, four, five, or six HVRs selected from (a) HVR-H1 comprising the amino acid sequence of SEQ ID NO:74; (b) HVR-H2 comprising the amino acid sequence of SEQ ID NO:75; (c) HVR-H3 comprising the amino acid sequence of SEQ ID NO:76; (d) HVR-L1 comprising the amino acid sequence of SEQ ID NO:71; (e) HVR-L2 comprising the amino acid sequence of SEQ ID NO:72; and (f) HVR-L3 comprising the amino acid sequence of SEQ ID NO:73.

In one aspect, the invention provides an antibody comprising at least one, at least two, or all three VH HVR sequences selected from (a) HVR-H1 comprising the amino acid sequence of SEQ ID NO:74; (b) HVR-H2 comprising the amino acid sequence of SEQ ID NO:75; and (c) HVR-H3 comprising the amino acid sequence of SEQ ID NO:76. In one embodiment, the antibody comprises HVR-H3 comprising the amino acid sequence of SEQ ID NO:76. In another embodiment, the antibody comprises HVR-H3 comprising the amino acid sequence of SEQ ID NO:76 and HVR-L3 comprising the amino acid sequence of SEQ ID NO:73. In a further embodiment, the antibody comprises HVR-H3 comprising the amino acid sequence of SEQ ID NO:76, HVR-L3 comprising the amino acid sequence of SEQ ID NO:73, and HVR-H2 comprising the amino acid sequence of SEQ ID NO:75. In a further embodiment, the antibody comprises (a) HVR-H1 comprising the amino acid sequence of SEQ ID NO:74; (b) HVR-H2 comprising the amino acid sequence of SEQ ID NO:75; and (c) HVR-H3 comprising the amino acid sequence of SEQ ID NO:76.

In another aspect, the invention provides an antibody comprising at least one, at least two, or all three VL HVR sequences selected from (a) HVR-L1 comprising the amino acid sequence of SEQ ID NO:71; (b) HVR-L2 comprising the amino acid sequence of SEQ ID NO:72; and (c) HVR-L3 comprising the amino acid sequence of SEQ ID NO:73. In one embodiment, the antibody comprises (a) HVR-L1 comprising the amino acid sequence of SEQ ID NO:71; (b) HVR-L2 comprising the amino acid sequence of SEQ ID NO:72; and (c) HVR-L3 comprising the amino acid sequence of SEQ ID NO:73.

In another aspect, an antibody of the invention comprises (a) a VH domain comprising at least one, at least two, or all three VH HVR sequences selected from (i) HVR-H1 comprising the amino acid sequence of SEQ ID NO:74, (ii) HVR-H2 comprising the amino acid sequence of SEQ ID NO:75, and (iii) HVR-H3 comprising an amino acid sequence selected from SEQ ID NO:76; and (b) a VL domain comprising at least one, at least two, or all three VL HVR sequences selected from (i) HVR-L1 comprising the amino acid sequence of SEQ ID NO:71, (ii) HVR-L2 comprising the amino acid sequence of SEQ ID NO:72, and (c) HVR-L3 comprising the amino acid sequence of SEQ ID NO:73.

In another aspect, the invention provides an antibody comprising (a) HVR-H1 comprising the amino acid sequence of SEQ ID NO:74; (b) HVR-H2 comprising the amino acid sequence of SEQ ID NO:75; (c) HVR-H3 comprising the amino acid sequence of SEQ ID NO:76; (d) HVR-L1 comprising the amino acid sequence of SEQ ID NO:71; (e) HVR-L2 comprising the amino acid sequence of SEQ ID NO:72; and (f) HVR-L3 comprising an amino acid sequence selected from SEQ ID NO:73.

In another aspect, an anti-RSP02 antibody is provided, wherein the antibody comprises a VH as in any of the embodiments provided above, and a VL as in any of the embodiments provided above. In one embodiment, the antibody comprises the VH and VL sequences in SEQ ID NO:111 and SEQ ID NO:112, respectively, including post-translational modifications of those sequences.

In any of the above embodiments, an anti-RSPO antibody is humanized. For example, humanized forms of any of the above anti-RSPO antibodies. In one embodiment, an anti-RSPO antibody comprises HVRs as in any of the above embodiments, and further comprises an acceptor human framework, e.g. a human immunoglobulin framework or a human consensus framework.

In a further aspect of the invention, an anti-RSPO antibody according to any of the above embodiments is a monoclonal antibody, including a chimeric, humanized or human antibody. In one embodiment, an anti-RSPO antibody is an antibody fragment, *e.g*., a Fv, Fab, Fab', scFv, diabody, or F(ab')₂ fragment. In another embodiment, the antibody is a full length antibody, *e.g*., an intact IgG1 or IgG2a antibody or other antibody class or isotype as defined herein.

In a further aspect, an anti-RSPO antibody according to any of the above embodiments may incorporate any of the features, singly or in combination, as described in Sections 1-7 below:

### 1. Antibody Affinity

In certain embodiments, an antibody provided herein has a dissociation constant (Kd) of ≤ 1µM, ≤ 100 nM, ≤ 10 nM, ≤ 1 nM, ≤ 0.1 nM, ≤ 0.01 nM, or ≤ 0.001 nM (*e.g.* 10⁻⁸ M or less, *e.g.* from 10⁻⁸ M to 10⁻¹³ M, *e.g*., from 10⁻⁹ M to 10⁻¹³ M).

In one embodiment, Kd is measured by a radiolabeled antigen binding assay (RIA). In one embodiment, an RIA is performed with the Fab version of an antibody of interest and its antigen. For example, solution binding affinity of Fabs for antigen is measured by equilibrating Fab with a minimal concentration of (¹²⁵I)-labeled antigen in the presence of a titration series of unlabeled antigen, then capturing bound antigen with an anti-Fab antibody-coated plate (*see, e.g.,* Chen et al., J. Mol. Biol. 293:865-881(1999)). To establish conditions for the assay, MICROTITER® multi-well plates (Thermo Scientific) are coated overnight with 5 µg/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23°C). In a non-adsorbent plate (Nunc #269620), 100 pM or 26 pM [¹²⁵I]-antigen are mixed with serial dilutions of a Fab of interest (e.g., consistent with assessment of the anti-VEGF antibody, Fab-12, in Presta et al., Cancer Res. 57:4593-4599 (1997)). The Fab of interest is then incubated overnight; however, the incubation may continue for a longer period (e.g., about 65 hours) to ensure that equilibrium is reached. Thereafter, the mixtures are transferred to the capture plate for incubation at room temperature (e.g., for one hour). The solution is then removed and the plate washed eight times with 0.1% polysorbate 20 (TWEEN-20®) in PBS. When the plates have dried, 150 µl/well of scintillant (MICROSCINT-20™; Packard) is added, and the plates are counted on a TOPCOUNT™ gamma counter (Packard) for ten minutes. Concentrations of each Fab that give less than or equal to 20% of maximal binding are chosen for use in competitive binding assays.

According to another embodiment, Kd is measured using a BIACORE® surface plasmon resonance assay. For example, an assay using a BIACORE®-2000 or a BIACORE®-3000 (BIAcore, Inc., Piscataway, NJ) is performed at 25°C with immobilized antigen CM5 chips at ∼10 response units (RU). In one embodiment, carboxymethylated dextran biosensor chips (CM5, BIACORE, Inc.) are activated with *N-ethyl-N'-*(3-dimethylaminopropyl)-carbodiimide hydrochloride (EDC) and *N-*hydroxysuccinimide (NHS) according to the supplier's instructions. Antigen is diluted with 10 mM sodium acetate, pH 4.8, to 5 µg/ml (∼0.2 µM) before injection at a flow rate of 5 µl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20™) surfactant (PBST) at 25°C at a flow rate of approximately 25 µl/min. Association rates (kₒₙ) and dissociation rates (k_{off}) are calculated using a simple one-to-one Langmuir binding model (BIACORE ® Evaluation Software version 3.2) by simultaneously fitting the association and dissociation sensorgrams. The equilibrium dissociation constant (Kd) is calculated as the ratio k_{off}/kₒₙ. *See, e.g.,* Chen et al., J. Mol. Biol. 293:865-881 (1999). If the on-rate exceeds 10⁶ M⁻¹ s⁻¹ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-AMINCO™ spectrophotometer (ThermoSpectronic) with a stirred cuvette.

### 2. Antibody Fragments

In certain embodiments, an antibody provided herein is an antibody fragment. Antibody fragments include, but are not limited to, Fab, Fab', Fab'-SH, F(ab')₂, Fv, and scFv fragments, and other fragments described below. For a review of certain antibody fragments, *see* Hudson et al. Nat. Med. 9:129-134 (2003). For a review of scFv fragments, *see, e.g.,* Pluckthün, in The Pharmacology of Monoclonal Antibodies, vol. 113, Rosenburg and Moore eds., (Springer-Verlag, New York), pp. 269-315 (1994); *see also* WO 93/16185; and U.S. Patent Nos. 5,571,894 and 5,587,458. For discussion of Fab and F(ab')₂ fragments comprising salvage receptor binding epitope residues and having increased in vivo half-life, *see* U.S. Patent No. 5,869,046.

Diabodies are antibody fragments with two antigen-binding sites that may be bivalent or bispecific. *See,* for example, EP 404,097; WO 1993/01161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9:129-134 (2003).

Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; *see, e.g.,* U.S. Patent No. 6,248,516).

Antibody fragments can be made by various techniques, including but not limited to proteolytic digestion of an intact antibody as well as production by recombinant host cells (*e.g. E. coli* or phage), as described herein.

### 3. Chimeric and Humanized Antibodies

In certain embodiments, an antibody provided herein is a chimeric antibody. Certain chimeric antibodies are described, *e.g.,* in U.S. Patent No. 4,816,567; and Morrison et al., Proc. Natl. Acad. Sci. USA, 81:6851-6855 (1984)). In one example, a chimeric antibody comprises a non-human variable region *(e.g.,* a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, *e.g*., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody *(e.g.,* the antibody from which the HVR residues are derived), *e.g.,* to restore or improve antibody specificity or affinity.

Humanized antibodies and methods of making them are reviewed, *e.g*., in Almagro and Fransson, Front. Biosci. 13:1619-1633 (2008), and are further described, *e.g.,* in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing specificity determining region (SDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (*see, e.g.,* Sims et al. J. Immunol. 151:2296 (1993)); framework regions derived from the consensus sequence of human antibodies of a particular subgroup of light or heavy chain variable regions (*see, e.g.,* Carter et al. Proc. Natl. Acad. Sci. USA, 89:4285 (1992); and Presta et al. J. Immunol., 151:2623 (1993)); human mature (somatically mutated) framework regions or human germline framework regions (*see, e.g.,* Almagro and Fransson, Front. Biosci. 13:1619-1633 (2008)); and framework regions derived from screening FR libraries (*see, e.g.,* Baca et al., J. Biol. Chem. 272:10678-10684 (1997) and Rosok et al., J. Biol. Chem. 271:22611-22618 (1996)).

### 4. Human Antibodies

In certain embodiments, an antibody provided herein is a human antibody. Human antibodies can be produced using various techniques known in the art. Human antibodies are described generally in van Dijk and van de Winkel, Curr. Opin. Pharmacol. 5: 368-74 (2001) and Lonberg, Curr. Opin. Immunol. 20:450-459 (2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, *see* Lonberg, Nat. Biotech. 23:1117-1125 (2005). *See also, e.g.,* U.S. Patent Nos. 6,075,181 and 6,150,584 describing XENOMOUSE™ technology; U.S. Patent No. 5,770,429 describing HUMAB® technology; U.S. Patent No. 7,041,870 describing K-M MOUSE® technology, and U.S. Patent Application Publication No. US 2007/0061900, describing VELOCIMOUSE® technology). Human variable regions from intact antibodies generated by such animals may be further modified, *e.g*., by combining with a different human constant region.

Human antibodies can also be made by hybridoma-based methods. Human myeloma and mouse-human heteromyeloma cell lines for the production of human monoclonal antibodies have been described. (*See, e.g.,* Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).) Human antibodies generated via human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clinical Pharmacology, 27(3):185-91 (2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

### 5. Library-Derived Antibodies

Antibodies of the invention may be isolated by screening combinatorial libraries for antibodies with the desired activity or activities. For example, a variety of methods are known in the art for generating phage display libraries and screening such libraries for antibodies possessing the desired binding characteristics. Such methods are reviewed, *e.g.,* in Hoogenboom et al. in Methods in Molecular Biology 178:1-37 (O'Brien et al., ed., Human Press, Totowa, NJ, 2001) and further described, *e.g.,* in the McCafferty et al., Nature 348:552-554; Clackson et al., Nature 352: 624-628 (1991); Marks et al., J. Mol. Biol. 222: 581-597 (1992); Marks and Bradbury, in Methods in Molecular Biology 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of VH and VL genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (*e.g.,* from human) to provide a single source of antibodies to a wide range of non-self and also self antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement *in vitro,* as described by Hoogenboom and Winter, J. Mol. Biol., 227: 381-388 (1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

### 6. Multispecific Antibodies

In certain embodiments, an antibody provided herein is a multispecific antibody, e.g. a bispecific antibody. Multispecific antibodies are monoclonal antibodies that have binding specificities for at least two different sites. In certain embodiments, one of the binding specificities is RSPO (*e.g.,* RSPO2 and/or RSPO3). and the other is for any other antigen. In certain embodiments, bispecific antibodies may bind to two different epitopes of RSPO. Bispecific antibodies may also be used to localize cytotoxic agents to cells which express RSPO (e.g., RSPO2 and/or RSPO3). In some embodiments, the multispecific antibody (*e.g*., bispecific antibody) binds to RSPO2 and RSPO3. In some embodiments, the multispecific antibody (*e.g*., bispecific antibody) comprises a first variable domain comprising the HVRs of 5E11 and a second variable domain comprising the HVRs of 36D2. In some embodiments, the multispecific antibody (*e.g*., bispecific antibody) comprises a first variable domain comprising the HVRs of 5D6 and a second variable domain comprising the HVRs of 36D2. In some embodiments, the multispecific antibody (*e.g*., bispecific antibody) comprises a first variable domain comprising the HVRs of 5E11 and a second variable domain comprising the HVRs of 1A1. In some embodiments, the multispecific antibody (*e.g*., bispecific antibody) comprises a first variable domain comprising the HVRs of 5D6 and a second variable domain comprising the HVRs of 1A1. Bispecific antibodies can be prepared as full length antibodies or antibody fragments.

Techniques for making multispecific antibodies include, but are not limited to, recombinant co-expression of two immunoglobulin heavy chain-light chain pairs having different specificities *(see* Milstein and Cuello, Nature 305: 537 (1983)), WO 93/08829, and Traunecker et al., EMBO J. 10: 3655 (1991)), and "knob-in-hole" engineering (*see, e.g.,* U.S. Patent No. 5,731,168). Multi-specific antibodies may also be made by engineering electrostatic steering effects for making antibody Fc-heterodimeric molecules (WO 2009/089004A1); cross-linking two or more antibodies or fragments *(see, e.g.,* US Patent No. 4,676,980, and Brennan et al., Science, 229: 81 (1985)); using leucine zippers to produce bi-specific antibodies *(see, e.g.,* Kostelny et al., J. Immunol., 148(5): 1547-1553 (1992)); using "diabody" technology for making bispecific antibody fragments (*see, e.g.,* Hollinger et al., Proc. Natl. Acad. Sci. USA, 90:6444-6448 (1993)); and using single-chain Fv (sFv) dimers *(see, e.g.* Gruber et al., J. Immunol., 152:5368 (1994)); and preparing trispecific antibodies as described, *e.g.,* in Tutt et al. J. Immunol. 147: 60 (1991).

Engineered antibodies with three or more functional antigen binding sites, including "Octopus antibodies," are also included herein (*see*, *e.g.* US 2006/0025576).

The antibody or fragment herein also includes a "Dual Acting FAb" or "DAF" comprising an antigen binding site that binds to multiple RSPOs (*e.g.,* RSPO2 and/or RSPO3) (*see*, US 2008/0069820, for example).

### 7. Antibody Variants

In certain embodiments, amino acid sequence variants of the antibodies provided herein are contemplated. For example, it may be desirable to improve the binding affinity and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, *e.g*., antigen-binding.

### a) Substitution, Insertion, and Deletion Variants

In certain embodiments, antibody variants having one or more amino acid substitutions are provided. Sites of interest for substitutional mutagenesis include the HVRs and FRs. Conservative substitutions are shown in Table 1 under the heading of "preferred substitutions." More substantial changes are provided in Table 1 under the heading of "exemplary substitutions," and as further described below in reference to amino acid side chain classes. Amino acid substitutions may be introduced into an antibody of interest and the products screened for a desired activity, *e.g*., retained/improved antigen binding, decreased immunogenicity, or improved ADCC or CDC.

**TABLE 1**

| **Original Residue** | **Exemplary Substitutions** | **Preferred Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

One type of substitutional variant involves substituting one or more hypervariable region residues of a parent antibody (*e.g*. a humanized or human antibody). Generally, the resulting variant(s) selected for further study will have modifications (*e.g*., improvements) in certain biological properties (*e.g*., increased affinity, reduced immunogenicity) relative to the parent antibody and/or will have substantially retained certain biological properties of the parent antibody. An exemplary substitutional variant is an affinity matured antibody, which may be conveniently generated, *e.g*., using phage display-based affinity maturation techniques such as those described herein. Briefly, one or more HVR residues are mutated and the variant antibodies displayed on phage and screened for a particular biological activity (*e.g*. binding affinity).

Alterations (*e.g.,* substitutions) may be made in HVRs, *e.g.,* to improve antibody affinity. Such alterations may be made in HVR "hotspots," *i.e*., residues encoded by codons that undergo mutation at high frequency during the somatic maturation process (*see, e.g.,* Chowdhury, Methods Mol. Biol. 207:179-196 (2008)), and/or residues that contact antigen, with the resulting variant VH or VL being tested for binding affinity. Affinity maturation by constructing and reselecting from secondary libraries has been described, *e.g.,* in Hoogenboom et al. in Methods in Molecular Biology 178:1-37 (O'Brien et al., ed., Human Press, Totowa, NJ, (2001).) In some embodiments of affinity maturation, diversity is introduced into the variable genes chosen for maturation by any of a variety of methods (*e.g*., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (*e.g*., 4-6 residues at a time) are randomized. HVR residues involved in antigen binding may be specifically identified, *e.g*., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antibody to bind antigen. For example, conservative alterations (*e.g.*, conservative substitutions as provided herein) that do not substantially reduce binding affinity may be made in HVRs. Such alterations may, for example, be outside of antigen contacting residues in the HVRs. In certain embodiments of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (*e.g.,* charged residues such as Arg, Asp, His, Lys, and Glu) are identified and replaced by a neutral or negatively charged amino acid (*e.g*., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (*e.g*., for ADEPT) or a polypeptide which increases the serum half-life of the antibody.

### b) Glycosylation variants

In certain embodiments, an antibody provided herein is altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. *See, e.g.,* Wright et al. TIBTECH 15:26-32 (1997). The oligosaccharide may include various carbohydrates, *e.g*., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody of the invention may be made in order to create antibody variants with certain improved properties.

In one embodiment, antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (Eu numbering of Fc region residues); however, Asn297 may also be located about ± 3 amino acids upstream or downstream of position 297, *i.e*., between positions 294 and 300, due to minor sequence variations in antibodies. Such fucosylation variants may have improved ADCC function. *See, e.g.,* US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/0157108, Presta, L; and WO 2004/056312 A1, Adams *et al*., especially at Example 11), and knockout cell lines, such as alpha-1,6-fucosyltransferase gene, *FUT8*, knockout CHO cells *(see, e.g.,* Yamane-Ohnuki et al. Biotech. Bioeng. 87: 614 (2004); Kanda, Y. et al., Biotechnol. Bioeng., 94(4):680-688 (2006); and WO2003/085107).

Antibodies variants are further provided with bisected oligosaccharides, *e.g*., in which a biantennary oligosaccharide attached to the Fc region of the antibody is bisected by GlcNAc. Such antibody variants may have reduced fucosylation and/or improved ADCC function. Examples of such antibody variants are described, *e.g*., in WO 2003/011878 (Jean-Mairet et al.); US Patent No. 6,602,684 (Umana et al.); and US 2005/0123546 (Umana et al.). Antibody variants with at least one galactose residue in the oligosaccharide attached to the Fc region are also provided. Such antibody variants may have improved CDC function. Such antibody variants are described, *e.g*., in WO 1997/30087 (Patel et al.); WO 1998/58964 (Raju, S.); and WO 1999/22764 (Raju, S.).

### c) Fc region variants

In certain embodiments, one or more amino acid modifications may be introduced into the Fc region of an antibody provided herein, thereby generating an Fc region variant. The Fc region variant may comprise a human Fc region sequence (*e.g*., a human IgG1, IgG2, IgG3 or IgG4 Fc region) comprising an amino acid modification (*e.g*., a substitution) at one or more amino acid positions.

In certain embodiments, the invention contemplates an antibody variant that possesses some but not all effector functions, which make it a desirable candidate for applications in which the half-life of the antibody *in vivo* is important yet certain effector functions (such as complement and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcγR binding (hence likely lacking ADCC activity), but retains FcRn binding ability. The primary cells for mediating ADCC, NK cells, express Fc(RIII only, whereas monocytes express Fc(RI, Fc(RII and Fc(RIII. FcR expression on hematopoietic cells is summarized in Table 3 on page 464 of Ravetch and Kinet, Annu. Rev. Immunol. 9:457-492 (1991). Non-limiting examples of *in vitro* assays to assess ADCC activity of a molecule of interest is described in U.S. Patent No. 5,500,362 (*see, e.g*,. Hellstrom, I. et al. Proc. Nat'l Acad. Sci. USA 83:7059-7063 (1986)) and Hellstrom, I et al., Proc. Nat'l Acad. Sci. USA 82:1499-1502 (1985); 5,821,337 (*see* Bruggemann, M. et al., J. Exp. Med. 166:1351-1361 (1987)). Alternatively, non-radioactive assays methods may be employed (*see,* for example, ACTI™ non-radioactive cytotoxicity assay for flow cytometry (CellTechnology, Inc. Mountain View, CA; and CytoTox 96® non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo, e.g.,* in an animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. *See, e.g.,* C1q and C3c binding ELISA in WO 2006/029879 and WO 2005/100402. To assess complement activation, a CDC assay may be performed (*see,* for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)). FcRn binding and *in vivo* clearance/half-life determinations can also be performed using methods known in the art (*see, e.g.,* Petkova, S.B. et al., Int'l. Immunol. 18(12):1759-1769 (2006)).

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581). In some embodiments, the antibody comprises an engineered alanine at amino acid position 265 according to EU numbering convention. In some embodiments, the antibody comprises an engineered alanine at amino acid position 297 according to EU numbering convention.

Certain antibody variants with improved or diminished binding to FcRs are described. (*See, e.g.,* U.S. Patent No. 6,737,056; WO 2004/056312, and Shields et al., J. Biol. Chem. 9(2): 6591-6604 (2001).)

In certain embodiments, an antibody variant comprises an Fc region with one or more amino acid substitutions which improve ADCC, *e.g*., substitutions at positions 298, 333, and/or 334 of the Fc region (EU numbering of residues).

In some embodiments, alterations are made in the Fc region that result in altered (*i.e.,* either improved or diminished) C1q binding and/or Complement Dependent Cytotoxicity (CDC), *e.g.,* as described in US Patent No. 6,194,551, WO 99/51642, and Idusogie et al. J. Immunol. 164: 4178-4184 (2000).

Antibodies with increased half-lives and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934 (Hinton et al.). Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, *e.g.,* substitution of Fc region residue 434 (US Patent No. 7,371,826). *See* also Duncan & Winter, Nature 322:738-40 (1988); U.S. Patent No. 5,648,260; U.S. Patent No. 5,624,821; and WO 94/29351 concerning other examples of Fc region variants.

### d) Cysteine engineered antibody variants

In certain embodiments, it may be desirable to create cysteine engineered antibodies, *e.g*., "thioMAbs," in which one or more residues of an antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, *e.g*., in U.S. Patent No. 7,521,541.

### e) Antibody Derivatives

In certain embodiments, an antibody provided herein may be further modified to contain additional nonproteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer is attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another embodiment, conjugates of an antibody and nonproteinaceous moiety that may be selectively heated by exposure to radiation are provided. In one embodiment, the nonproteinaceous moiety is a carbon nanotube (Kam et al., Proc. Natl. Acad. Sci. USA 102: 11600-11605 (2005)). The radiation may be of any wavelength, and includes, but is not limited to, wavelengths that do not harm ordinary cells, but which heat the nonproteinaceous moiety to a temperature at which cells proximal to the antibody-nonproteinaceous moiety are killed.

### B. Recombinant Methods and Compositions

Antibodies may be produced using recombinant methods and compositions, *e.g*., as described in U.S. Patent No. 4,816,567. In one embodiment, isolated nucleic acid encoding an anti-RSPO antibody described herein is provided. Such nucleic acid may encode an amino acid sequence comprising the VL and/or an amino acid sequence comprising the VH of the antibody (e.g., the light and/or heavy chains of the antibody). In a further embodiment, one or more vectors (e.g., expression vectors) comprising such nucleic acid are provided. In a further embodiment, a host cell comprising such nucleic acid is provided. In one such embodiment, a host cell comprises (e.g., has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In one embodiment, the host cell is eukaryotic, e.g. a Chinese Hamster Ovary (CHO) cell or lymphoid cell (*e.g.,* Y0, NS0, Sp20 cell). In one embodiment, a method of making an anti-RSPO antibody is provided, wherein the method comprises culturing a host cell comprising a nucleic acid encoding the antibody, as provided above, under conditions suitable for expression of the antibody, and optionally recovering the antibody from the host cell (or host cell culture medium).

For recombinant production of an anti-RSPO antibody, nucleic acid encoding an antibody, *e.g.,* as described above, is isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, *see, e.g.,* U.S. Patent Nos. 5,648,237, 5,789,199, and 5,840,523. (*See* also Charlton, Methods in Molecular Biology, Vol. 248 (B.K.C. Lo, ed., Humana Press, Totowa, NJ, 2003), pp. 245-254, describing expression of antibody fragments *in E. coli*.) After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. *See* Gerngross, Nat. Biotech. 22:1409-1414 (2004), and Li et al., Nat. Biotech. 24:210-215 (2006).

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. *See, e.g.,* US Patent Nos. 5,959,177, 6,040,498, 6,420,548, 7,125,978, and 6,417,429 (describing PLANTIBODIES™ technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, *e.g.,* in Graham et al., J. Gen Virol. 36:59 (1977)); baby hamster kidney cells (BHK); mouse sertoli cells (TM4 cells as described, *e.g.,* in Mather, Biol. Reprod. 23:243-251 (1980)); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, *e.g.,* in Mather et al., Annals N.Y. Acad. Sci. 383:44-68 (1982); MRC 5 cells; and FS4 cells. Other useful mammalian host cell lines include Chinese hamster ovary (CHO) cells, including DHFR⁻CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, *see, e.g.,* Yazaki and Wu, Methods in Molecular Biology, Vol. 248 (B.K.C. Lo, ed., Humana Press, Totowa, NJ), pp. 255-268 (2003).

### C. Assays

Anti-RSPO antibodies provided herein may be identified, screened for, or characterized for their physical/chemical properties and/or biological activities by various assays known in the art.

### 1. Binding assays and other assays

In one aspect, an antibody of the invention is tested for its antigen binding activity, *e.g*., by known methods such as ELISA, Western blot, etc.

Methods of determining binding affinity are known in the art. In some embodiments, the binding affinity may be determined according to a BIAcore® assay as described herein in Example 1. Specifically, in some embodiments, Kd may be measured using surface plasmon resonance assays using a BIACORE®-3000 (BIAcore, Inc., Piscataway, NJ).

Methods of determining the ability of an anti-RSPO antibody to disrupt and/or inhibit the binding of an RSPO to LGR (*e.g.,* LGR4, 5, and/or 6), syndecan (*e.g.,* SDC4), and/or an E3 ubiquitinase (*e.g.,* ZNRF3 and/or RNF43) are known in the art. *See e.g.,* WO2011/076932, WO2013012747, Lau et al. Nature 476:293-297 (2011), Hao et al. Nature 485:195-200 (2012). In some embodiments, the ability of an anti-RSPO antibody to significantly disrupt the binding of an R-spondon (RSPO) to an LGR, syndecan and/or E3 ubiquitinase may be determined by flow cytometry, BIAcore assay, and/or ELISA (*e.g*., Competitive Binding ELISA). In some embodiments, the ability of an anti-RSPO antibody to disrupt and/or inhibit the binding of an RSPO to LGR (*e.g.,* LGR4, 5, and/or 6), syndecan (SDC4), and/or an E3 ubiquitinase (*e.g*., ZNRF3 and/or RNF43) may be determined according to a Competitive Binding ELISA as described herein in Example 1.

In another aspect, competition assays may be used to identify an antibody that competes with 4H1, 4D4, 5C2, 5D6, 5E11, 6E9, 21C2, 26E11, 1A1, 11F11, 36D2, and/or 49G5 for binding to RSPO *(e.g.,* RSPO2 and/or RSPO3).

Methods of determining antibody competition are known in the art. In an exemplary competition assay, immobilized RSPO (*e.g*., RSPO2 and/or RSPO3) is incubated in a solution comprising a first labeled antibody that binds to RSPO (*e.g.,* RSPO2 and/or RSPO3) (*e.g.,* 4H1, 4D4, 5C2, 5D6, 5E11, 6E9, 21C2, 26E11, 1A1, 11F11, 36D2, and/or 49G5) and a second unlabeled antibody that is being tested for its ability to compete with the first antibody for binding to RSPO (*e.g.,* RSPO2 and/or RSPO3). The second antibody may be present in a hybridoma supernatant. As a control, immobilized RSPO (*e.g*., RSPO2 and/or RSPO3) is incubated in a solution comprising the first labeled antibody but not the second unlabeled antibody. After incubation under conditions permissive for binding of the first antibody to RSPO (*e.g*., RSPO2 and/or RSPO3), excess unbound antibody is removed, and the amount of label associated with immobilized RSPO (*e.g*., RSPO2 and/or RSPO3) is measured. If the amount of label associated with immobilized RSPO (*e.g.*, RSPO2 and/or RSPO3) is substantially reduced in the test sample relative to the control sample, then that indicates that the second antibody is competing with the first antibody for binding to RSPO (*e.g*., RSPO2 and/or RSPO3). *See* Harlow and Lane (1988) Antibodies: A Laboratory Manual ch.14 (Cold Spring Harbor Laboratory, Cold Spring Harbor, NY).

Another exemplar competition assay is described in the Example 1 useful for epitope binning and/or determining whether two antibodies compete for binding. In some embodiments, epitope binning and/or determining whether two antibodies compete for binding may be determined according to a Octet® assay as described herein in Example 1.

In certain embodiments, an antibody binds to the same epitope (*e.g.,* a linear or a conformational epitope) that is bound 4H1, 4D4, 5C2, 5D6, 5E11, 6E9, 21C2, 26E11, 1A1, 11F11, 36D2, and/or 49G5. Detailed exemplary methods for mapping an epitope to which an antibody binds are provided in Morris (1996) "Epitope Mapping Protocols," in Methods in Molecular Biology vol. 66 (Humana Press, Totowa, NJ). In some embodiments, the epitope is determined by peptide competition. In some embodiments, the epitope is determined by mass spectrometry. In some embodiments, the epitope is determined by crystallography. An exemplary method of crystallography is described in Example 1.

### 2. Activity assays

In one aspect, assays are provided for identifying anti-RSPO antibodies thereof having biological activity. Biological activity may include, *e.g*., inhibit wnt signaling, inhibit angiogenesis, inhibit cell proliferation, inhibit cancer stem cell proliferation, and/or deplete cancer stem cells. Antibodies having such biological activity in vivo and/or in vitro are also provided.

Methods of determining ability of an anti-RSPO antibody to disrupt wnt/beta-catenin signaling are known in the art. *See e.g.,* WO2005/040418 and WO2013/012747. In some embodiments, the ability of an anti-RSPO antibody to significantly disrupt wnt/beta-catenin signaling may be determined using a reporter gene assay. In some embodiments, for example, a reporter construct comprising a reporter gene (such as, for example, a luciferase gene) under the control of a wnt/beta-catenin responsive promoter (such as, for example, a promoter comprising multimerized TCF/LEF DNA-binding sites) may be transfected into cells. The cells are then contacted with a Wnt ligand, such as Wnt3a, and an RSPO, such as RSPO1, RSPO2, RSPO3, and/or RSPO4, in the presence and absence of an RSPO antibody, and luciferase expression is measured.

Methods of determining ability of an anti-RSPO antibody inhibiting angiogenesis and/or vasculogenesis are known in the art. *See e.g.,* WO2008/04664. Examples of assays include the in vivo Matrigel plug and corneal neovascularization assays, the in vivo/in vitro chick chorioallantoic membrane (CAM) assay, the in vitro cellular (proliferation, migration, tube formation) and organotypic (aortic ring) assays, the chick aortic arch assays, and the Matrigel sponge assays.

Methods of determining the ability of an anti-RSPO antibody to induce stem cell differentiation and/or cancer stem cell depletion are known in the art. *See e.g.,* WO2013/036867. In some embodiments, stem cell differentiation may be assayed by determining ability to differentiation of crypt base columnar cells (CBCs), which are fast-cycling stem cells in the small intestine, into, for example, enterocytes, goblet cells, and/or enteroendocrine cells, in the presence and absence of an anti-RSPO antibody.

In certain embodiments, an antibody of the invention is tested for such biological activity and/or binding interactions by the assays described herein and in WO2005/040418, WO2008/046649, WO2011/076932, WO2013/012747, WO2013/054307, Lau et al. Nature 476:293-297 (2011), Hao et al. Nature 485:195-200 (2012).

In some embodiment, the epitope is determined by crystallography. In some embodiments, the epitope as determined by crystallography is determined using amino acids M33-E210 of RSPO3. In some embodiments, the epitope as determined by crystallography is performed by using an Labcyte Echo liquid handler to set several sparse matrix crystal screens using 100 nL sitting drops. Screens were stored at 18°C. In some embodiments, crystals may be obtained in a drop containing 100 mM MIB pH 9 and 25% PEG 1500 as the mother liquor. In some embodiments, crystals may be obtained in a drop containing 200 mM Sodium formate and 20% (w/v) PEG 3,350 as the mother liquor. In some embodiments, the crystal may be harvested and soaked in cryoprotectant solution for 10 seconds and flash-frozen in liquid nitrogen. In some embodiments, the cryoprotectant solution may be made by mixing 1 µL 70% glycerol with 1.8µL reservoir solution. In some embodiments, the crystals may be grown in PEG-based conditions, for example, about 20-25% PEG 3,350. In some embodiments, the crystals may be grown in about 20% PEG 6,000, about 20-25% PEG 4,000, and about 25% PEG 1,500. In some embodiments, the pH may range from about 3.5 - 9, for example, between about 7 and about 8. In some embodiments, the salt concentration is about 200 mM.

### D. Immunoconjugates

The invention also provides immunoconjugates comprising an anti-RSPO antibody herein conjugated to one or more cytotoxic agents, such as chemotherapeutic agents or drugs, growth inhibitory agents, toxins (e.g., protein toxins, enzymatically active toxins of bacterial, fungal, plant, or animal origin, or fragments thereof), or radioactive isotopes.

In one embodiment, an immunoconjugate is an antibody-drug conjugate (ADC) in which an antibody is conjugated to one or more drugs, including but not limited to a maytansinoid (*see* U.S. Patent Nos. 5,208,020, 5,416,064 and European Patent EP 0 425 235 B1); an auristatin such as monomethylauristatin drug moieties DE and DF (MMAE and MMAF) (*see* U.S. Patent Nos. 5,635,483 and 5,780,588, and 7,498,298); a dolastatin; a calicheamicin or derivative thereof (*see* U.S. Patent Nos. 5,712,374, 5,714,586, 5,739,116, 5,767,285, 5,770,701, 5,770,710, 5,773,001, and 5,877,296; Hinman et al., Cancer Res. 53:3336-3342 (1993); and Lode et al., Cancer Res. 58:2925-2928 (1998)); an anthracycline such as daunomycin or doxorubicin *(see* Kratz et al., Current Med. Chem. 13:477-523 (2006); Jeffrey et al., Bioorganic & Med. Chem. Letters 16:358-362 (2006); Torgov et al., Bioconj. Chem. 16:717-721 (2005); Nagy et al., Proc. Natl. Acad. Sci. USA 97:829-834 (2000); Dubowchik et al., Bioorg. & Med. Chem. Letters 12:1529-1532 (2002); King et al., J. Med. Chem. 45:4336-4343 (2002); and U.S. Patent No. 6,630,579); methotrexate; vindesine; a taxane such as docetaxel, paclitaxel, larotaxel, tesetaxel, and ortataxel; a trichothecene; and CC1065.

In another embodiment, an immunoconjugate comprises an antibody as described herein conjugated to an enzymatically active toxin or fragment thereof, including but not limited to diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes.

In another embodiment, an immunoconjugate comprises an antibody as described herein conjugated to a radioactive atom to form a radioconjugate. A variety of radioactive isotopes are available for the production of radioconjugates. Examples include At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu. When the radioconjugate is used for detection, it may comprise a radioactive atom for scintigraphic studies, for example Tc99m or I123, or a spin label for nuclear magnetic resonance (NMR) imaging (also known as magnetic resonance imaging, MRI), such as iodine-123 again, iodine-131, indium-111, fluorine-19, carbon-13, nitrogen-15, oxygen-17, gadolinium, manganese or iron.

Conjugates of an antibody and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., Science 238:1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. *See* WO94/11026. The linker may be a "cleavable linker" facilitating release of a cytotoxic drug in the cell. For example, an acid-labile linker, peptidase-sensitive linker, photolabile linker, dimethyl linker or disulfide-containing linker (Chari et al., Cancer Res. 52:127-131 (1992); U.S. Patent No. 5,208,020) may be used.

The immunuoconjugates or ADCs herein expressly contemplate, but are not limited to such conjugates prepared with cross-linker reagents including, but not limited to, BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate) which are commercially available (e.g., from Pierce Biotechnology, Inc., Rockford, IL., U.S.A).

### E. Methods and Compositions for Diagnostics and Detection

In certain embodiments, any of the anti-RSPO antibodies provided herein is useful for detecting the presence of RSPO in a sample. The term "detecting" as used herein encompasses quantitative or qualitative detection. In certain embodiments, a sample comprises a cell or tissue, such as gastrointestinal, stomach, esophageal, colon, rectal, and/or colorectal tissue. In some embodiments, a sample comprises a cell or tissue, such as adrenal, bladder, brain, breast, cervix, colon, head and neck, kidney, leukemia, liver, lung, lymphoid, ovarian, pancreas, prostate, rectum, skin, stomach, thyroid, and/or uterus tissue. In some embodiments, a sample comprises a cell or tissue, such as lung, ovarian, breast, liver, or multiple myeloma tissue.

In one embodiment, an anti-RSPO antibody for use in a method of diagnosis or detection is provided. In a further aspect, a method of detecting the presence of RSPO in a sample is provided. In certain embodiments, the method comprises contacting the sample with an anti-RSPO antibody as described herein under conditions permissive for binding of the anti-RSPO antibody to RSPO and detecting whether a complex is formed between the anti-RSPO antibody and RSPO. Such method may be an *in vitro* or *in vivo* method. In one embodiment, an anti-RSPO antibody is used to select subjects eligible for therapy with an anti-RSPO antibody, *e.g*. where RSPO is a biomarker for selection of patients. In some embodiments, the RSPO is RSPO2. In some embodiments, the RSPO is RSPO3. In some embodiments, the RSPO is RSPO2 and RSPO3. In some embodiments, the individual and/or cancer has increased expression of one or more stem cell biomarkers. In some embodiments, the stem cell biomarker comprises Myc, Axin2, LGR5, TERT, BIRC5, and/or Ascl2. In some embodiments, the individual and/or cancer has decreased expression of one or more biomarker of differentiation. In some embodiments, the biomarker of differentiation comprises CEACAM7, SLC26A3, CA1, SYTl5, CA4, TFF1, and/or KRT20.

For example, provided herein are methods of treating cancer in an individual, wherein the cancer comprises one or more biomarkers, comprising administering to the individual an effective amount of an anti-RSPO antibody. Also provided herein are methods of treating cancer in an individual comprising administering to the individual an effective amount of an anti-RSPO antibody, wherein treatment is based upon the individual having cancer comprising one or more biomarkers.

Translocation are exceptionally powerful cancer mutations, as they often have multiple effects on a target gene: in a single 'mutation' they can dramatically change expression, remove regulatory domains, force oligomerization, change the subcellular location of a protein or join it to novel binding domains. This is reflected clinically in the fact that some neoplasms are classified or managed according to the presence of a particular fusion gene. In some embodiments of any of the methods, the one or more biomarkers comprise a translocation (*e.g*., intrachromosomal translocation, interchromosomal translocation, rearrangement and/or fusion) of one or more genes listed in Table 2.

In some embodiments of any of the methods, the translocation is a PVT1. In some embodiments, the PVT1 translocation comprises *PVT1* and *MYC.* In some embodiments, the RSPO2 translocation comprises *PVT1* and *IncDNA.* In some embodiments of any of the methods, the translocation is an R-spondin translocation. In some embodiments, the R-spondin translocation is a RSPO1 translocation. In some embodiments, the R-spondin translocation is a RSPO2. In some embodiments, the RSPO2 translocation comprises *EMC2* and *RSPO2.* In some embodiments, the RSPO2 translocation comprises *EIF3E* and *RSPO2.* In some embodiments, the RSPO2 translocation comprises *EIF3E* exon 1 and *RSPO2* exon 2. In some embodiments, the RSPO2 translocation comprises *EIF3E* exon 1 and *RSPO2* exon 3. In some embodiments, the RSPO2 translocation comprises SEQ ID NO:71. In some embodiments, the RSPO2 translocation is detectable by primers which include SEQ ID NO:114, 143, and/or 145. In some embodiments, the RSP02 translocation is driven by the *EIF3E* promoter. In some embodiments, the RSPO2 translocation is driven by the *RSPO2* promoter. In some embodiments, the R-spondin translocation is a RSPO3 translocation. In some embodiments, the RSPO3 translocation comprises *PTPRK* and *RSPO3*. In some embodiments, the RSPO3 translocation comprises *PTPRK* exon 1 and *RSPO3* exon 2. In some embodiments, the RSPO3 translocation comprises *PTPRK* exon 7 and *RSPO3* exon 2. In some embodiments, the RSPO3 translocation comprises SEQ ID NO:171 and/or SEQ ID NO:172. In some embodiments, the RSPO3 translocation is detectable by primers which include SEQ ID NO:115, 116, 145, and/or 146. In some embodiments, the RSPO3 translocation is driven by the *PTPRK* promoter. In some embodiments, the RSPO3 translocation is driven by the *RSPO3* promoter. In some embodiments, the RSPO3 translocation) comprises the PTPRK secretion signal sequence (and/or does not comprise the RSPO3 secretion signal sequence).

**Table 2 - Gene Fusions**

| **5' GeneName** | **3' GeneName** | **Type** | **Genomic position** | **5' PCR primer** | **3' PCR primer** | **bp** |
|---|---|---|---|---|---|---|
| PVT1 | ENST00000502082 | intrachrom. | 8:128806980-8:128433074 | CTTGCGGAAAGGATGTTGG (SEQ ID NO:113) | TGGTGATCCAGAGAAGAAGC (SEQ ID NO:142) | 150 |
| EMC2 | RSPO2 | | 8:109455927-8:109095035 | | | |
| EIF3E(e1) | RSPO2(e2) | Deletion | 8:109260842-8:109095035 | ACTACTCGCATCGCGCACT (SEQ ID NO:114) | GGGAGGACTCAGAGGGAGAC (SEQ ID NO:143) | 155 |
| EIF3E(e1) | RSPO2(e2) | Deletion | 8:109260842-8:109095035 | ACTACTCGCATCGCGCACT (SEQ ID NO:114) | GGGAGGACTCAGAGGGAGAC (SEQ ID NO:143) | 155 |
| EIF3E(e1) | RSPO2(e3) | Deletion | 8:109260842-8:109001472 | ACTACTCGCATCGCGCACT (SEQ ID NO:114) | TGCAGGCACTCTCCATACTG (SEQ ID NO:144) | 205 |
| EIF3E(e1) | RSPO2(e3) | Deletion | 8:109260842-8:109001472 | ACTACTCGCATCGCGCACT (SEQ ID NO:114) | TGCAGGCACTCTCCATACTG (SEQ ID NO:144) | 205 |
| PTPRK(e1) | RSPO3(e2) | Inversion | 6:128841404-6:127469793 | AAACTCGGCATGGATACGAC (SEQ ID NO:115) | GCTTCATGCCAATTCTTTCC (SEQ ID NO:145) | 226 |
| PTPRK(e1) | RSPO3(e2) | Inversion | 6:128841404-6:127469793 | AAACTCGGCATGGATACGAC (SEQ ID NO:115) | GCTTCATGCCAATTCTTTCC (SEQ ID NO:145) | 226 |
| PTPRK(e1) | RSPO3(e2) | Inversion | 6:128841404-6:127469793 | AAACTCGGCATGGATACGAC (SEQ ID NO:115) | GCTTCATGCCAATTCTTTCC (SEQ ID NO:145) | 226 |
| PTPRK(e1) | RSPO3(e2) | Inversion | 6:128841404-6:127469793 | AAACTCGGCATGGATACGAC (SEQ ID NO:115) | GCTTCATGCCAATTCTTTCC (SEQ ID NO:145) | 226 |
| PTPRK(e7) | RSPO3(e2) | Inversion | 6:128505577-6:127469793 | TGCAGTCAATGCTCCAACTT (SEQ ID NO:116) | GCCAATTCTTTCCAGAGCAA (SEQ ID NO:146) | 250 |
| ETV6 | NTRK3 | Interchrom | 12:12022903-15:88483984 | AAGCCCATCAACCTCTCTCA (SEQ ID NO:117) | GGGCTGAGGTTGTAGCACTC (SEQ ID NO:147) | 206 |
| ANXA2 | RORA | intrachrom. | 15:60674541-15:60824050 | CTCTACACCCCCAAGTGCAT (SEQ ID NO:118) | TGACACCATAATGGATTCCTG (SEQ ID NO:148) | 164 |
| TUBGCP3 | PDS5B | Inversion | 13:113200013-13:33327470 | AACAGGAGACCCGTACATGC (SEQ ID NO:119) | AAAGGGCACAGATTGCCATA (SEQ ID NO:149) | 221 |
| ARHGEF18 | NCRNA00157 | Interchrom | 19:7460133-21:19212970 | CCAGCTGCTAGCTACTGTGGA (SEQ ID NO:120) | ACTAGGTGGTCCAGGGTGTG (SEQ ID NO:150) | 186 |
| NT5C2 | ASAH2 | Deletion | 10:104899163-10:51978390 | TGAACCGAAGTTTAGCAATGG (SEQ ID NO:121) | TGCTCAAGCAGGTAAGATGC (SEQ ID NO:151) | 156 |
| NRBP2 | VPS28 | intrachrom. | 8:144919211-8:145649651 | TGATGAACTTTGCAGCCACT (SEQ ID NO:122) | ATGGTCTCCATCAGCTCTCG (SEQ ID NO:152) | 208 |
| CDC42SE2 | KIAA0146 | Interchrom | 5:130651837-8:48612965 | AGGGCCAGATTTGAGTGTGT (SEQ ID NO:123) | AAACTGAAAATCCCCGCTGT (SEQ ID NO:153) | 188 |
| MED13L | LAG3 | Inversion | 12:116675273-12:6886957 | GTGTATGGCGTCGTGATGTC (SEQ ID NO:124) | GCTCCAGTCACCAAAAGGAG (SEQ ID NO:154) | 205 |
| PEX5 | LOC389634 | Inversion | 12:7362838-12:8509737 | CATGTCGGAGAACATCTGGA (SEQ ID NO:125) | TGTGGAGTCTCTTGCGTGTC (SEQ ID NO:155) | 230 |
| PLCE1 | CYP2C19 | Deletion | 10:95792009-10:96602594 | CCTTACTGCCTTGTGGGAGA (SEQ ID NO:126) | TGGGGATGAGGTCGATGTAT (SEQ ID NO:156) | 224 |
| TPM3 | NTRK1 | Inversion | 1:154142876-1:156844363 | CAGAGACCCGTGCTGAGTTT (SEQ ID NO:127) | CCAAAAGGTGTTTCGTCCTT (SEQ ID NO:157) | 124 |
| PAN3 | RFC3 | Deletion | 13:28752072-13:34395269 | GACTTTGGTGCCCTCAACAT (SEQ ID NO:128) | CAATTTTTCCACTCCAACACC (SEQ ID NO:158) | 150 |
| CWC27 | RNF180 | intrachrom. | 5:64181373-5:63665442 | AACGGGAACTCTTAGCAGCA (SEQ ID NO:129) | CATGTCAAACCACCATCCAC (SEQ ID NO:159) | 182 |
| CAPN1 | SPDYC | intrachrom. | 11:64956217-11:64939414 | GAGACTTCATGCGGGAGTTC (SEQ ID NO:130) | ATCTGGAAGCAGGGGTCTTT (SEQ ID NO:160) | 199 |
| COG8 | TERF2 | intrachrom. | 16:69373079-16:69391464 | TGGCCTTCGCTAACTACAAGA (SEQ ID NO:131) | TCCCCATATTTCTGCACTCC (SEQ ID NO:161) | 233 |
| TADA2A | MEF2B | Interchrom | 17:35767040-19:19293492 | GCTCTTTGGCGCGGATTA (SEQ ID NO:132) | GGAGCTACCTGTGGCCCT (SEQ ID NO:162) | 152 |
| STRBP | DENND1A | intrachrom. | 9:125935956-9:126220176 | GTTGCAAAAGGCTTGCTGAT (SEQ ID NO:133) | ACGAAGGCTTCCTCACAGAA (SEQ ID NO:163) | 155 |
| CXorf56 | UBE2A | Inversion | X:118694231-X:118717090 | TGATTGATGCTGCCAAACAT (SEQ ID NO:134) | CACGCTTTTCATATTCCCGT (SEQ ID NO:164) | 161 |
| MED13L | CD4 | Inversion | 12:116675273-12:6923308 | GTGTATGGCGTCGTGATGTC (SEQ ID NO:124) | TCCCAAAGGCTTCTTCTTGA (SEQ ID NO:165) | 151 |
| PRR12 | PRRG2 | intrachrom. | 19:50097872-19:50093157 | ATGAACCTTATCTCGGCCCT (SEQ ID NO:135) | GTCGTGTACCCCAGAGGCT (SEQ ID NO:166) | 227 |
| ATP9A | ARFGEF2 | Inversion | 20:50307278-20:47601266 | ATGTGTACGCAGAAGAGCCA (SEQ ID NO:136) | GTGCAGGAATTGGGCTATGT (SEQ ID NO:167) | 150 |
| ANKRD17 | HS3ST1 | Deletion | 4:73956384-4:11401737 | GGAAAATCCTCATATTTGCCA (SEQ ID NO:137) | AGCAGGGAAGCCTCCTAGTC (SEQ ID NO:168) | 158 |
| RBM47 | ATP8A1 | intrachrom. | 4:40517884-4:42629126 | AGACCCAGGAGGAGTGAGGT (SEQ ID NO:138) | GGTCAGCCAGTGAGGTCTTC (SEQ ID NO:169) | 151 |
| FRS2 | RAP1B | intrachrom. | 12:69924740-12:69042479 | AGATGCCCAGATGCAAAAGT (SEQ ID NO:139) | CAAAGCAGACTTTCCAACGC (SEQ ID NO:170) | 161 |
| CHEK2 | PARVB | Inversion | 22:29137757-22:44553862 | GGCTGAGGGTGGAGTTTGTA (SEQ ID NO:140) | CTTCTGATCGAAGCTTTCCG (SEQ ID NO:171) | 191 |
| SFI1 | TPST2 | Inversion | 22:31904362-22:26940641 | CCCCAGTTAGAAGGGGAAGA (SEQ ID NO:141) | CACTCTCATCTCTGGGCTCC (SEQ ID NO:172) | 190 |

In some embodiments, the R-spondin translocation is a RSPO4 translocation. In some embodiments, the R-spondin translocation results in elevated expression levels of R-spondin (e.g., compared to a reference without the R-spondin translocation). In some embodiments, the R-spondin translocation results in elevated activity and/or activation of R-spondin (e.g., compared to a reference without the R-spondin translocation). In some embodiments, the presence of one or more biomarkers comprises an R-spondin translocation), such as a translocation in Table 2, and *KRAS* and/or *BRAF.* In some embodiments, the presence of one or more biomarkers is presence of an R-spondin translocation *(e.g.,* rearrangement and/or fusion), such as a translocation in Table 2, and a variation (*e.g.,* polymorphism or mutation) *KRAS* and/or *BRAF.* In some embodiments, the individual and/or cancer comprises a variation (polymorphism or mutation) in *KRAS* and/or *BRAF.* In some embodiments, the presence of one or more biomarkers is presence of an R-spondin translocation, such as a translocation in Table 2, and the absence of one or more biomarkers is absence of a variation (*e.g*., polymorphism or mutation) *CTNNB1* and/or *APC.*

In some embodiments of any of the translocation (*e.g*., intrachromosomal translocation, interchromosomal translocation, rearrangement and/or fusion), the translocation (e.g., intrachromosomal translocation, interchromosomal translocation, rearrangement and/or fusion) is a somatic translocation (e.g., intrachromosomal translocation, interchromosomal translocation, rearrangement and/or fusion). In some embodiments, the translocation is an intrachromosomal translocation. In some embodiments, the translocation is an interchromosomal. In some embodiments, the translocation is an inversion. In some embodiments, the translocation is a deletion. In some embodiments, the translocation is a functional translocation fusion polynucleotide (*e.g*., functional R-spondin-translocation fusion polynucleotide) and/or functional translocation fusion polypeptide (e.g., functional R-spondin-translocation fusion polypeptide). In some embodiments, the functional translocation fusion polypeptide (e.g., functional R-spondin-translocation fusion polypeptide) activates a pathway known to be modulated by one of the tranlocated genes (*e.g*., wnt signaling pathway). In some embodiments, the pathway is canonical wnt signaling pathway. In some embodiments, the pathway is noncanonical wnt signaling pathway. In some embodiments, the Methods of determining pathway activation are known in the art and include luciferase reporter assays as described herein. In some embodiments, the method is one or more methods described in Seshagiri et al., Nature 488:660-664 (2012) and/or WO 2013/120056, which are incorporated by reference in their entirety.

Exemplary disorders that may be diagnosed using an antibody of the invention include tumors, cell proliferative disorders, cancer, gastrointestinal cancer, stomach cancer, colorectal cancer, colon cancer, and/or rectal cancer. Exemplary disorders that may be diagnosed using an antibody of the invention further include adrenal cancer, bladder cancer, brain cancer, breast cancer, cervix cancer, colon cancer, head and neck cancer, kidney cancer, leukemia, liver cancer, lung cancer (*e.g*., NSCLC), lymphoid cancer, ovarian cancer, pancreatic cancer, prostate cancer, rectum cancer, skin cancer (*e.g.,* melanoma), stomach cancer, thyroid cancer, and/or uterine cancer. Exemplary disorders that may be diagnosed using an antibody of the invention also include lung cancer (*e.g*., NSCLC), ovarian cancer, breast cancer, liver cancer, or multiple myeloma.

Samples include, but are not limited to, primary or cultured cells or cell lines, cell supernatants, cell lysates, platelets, serum, plasma, vitreous fluid, lymph fluid, synovial fluid, follicular fluid, seminal fluid, amniotic fluid, milk, whole blood, blood-derived cells, urine, cerebro-spinal fluid, saliva, sputum, tears, perspiration, mucus, tumor lysates, and tissue culture medium, tissue extracts such as homogenized tissue, tumor tissue, cellular extracts, and combinations thereof. In some embodiments, the sample is a sample from gastrointestinal, stomach, esophageal, colon, rectal, and/or colorectal tissue. In some embodiments, the sample is a sample from adrenal, bladder, brain, breast, cervix, colon, head and neck, kidney, leukemia, liver, lung, lymphoid, ovarian, pancreas, prostate, rectum, skin, stomach, thyroid, and/or uterus tissue. In some embodiments, the sample is a sample from lung, ovarian, breast, liver, or multiple myeloma tissue.

In certain embodiments, labeled anti-RSPO antibodies are provided. Labels include, but are not limited to, labels or moieties that are detected directly (such as fluorescent, chromophoric, electron-dense, chemiluminescent, and radioactive labels), as well as moieties, such as enzymes or ligands, that are detected indirectly, *e.g*., through an enzymatic reaction or molecular interaction. Exemplary labels include, but are not limited to, the radioisotopes ³²P, ¹⁴C, ¹²⁵I, ³H, and ¹³¹I, fluorophores such as rare earth chelates or fluorescein and its derivatives, rhodamine and its derivatives, dansyl, umbelliferone, luceriferases, *e.g*., firefly luciferase and bacterial luciferase (U.S. Patent No. 4,737,456), luciferin, 2,3-dihydrophthalazinediones, horseradish peroxidase (HRP), alkaline phosphatase, β-galactosidase, glucoamylase, lysozyme, saccharide oxidases, *e.g*., glucose oxidase, galactose oxidase, and glucose-6-phosphate dehydrogenase, heterocyclic oxidases such as uricase and xanthine oxidase, coupled with an enzyme that employs hydrogen peroxide to oxidize a dye precursor such as HRP, lactoperoxidase, or microperoxidase, biotin/avidin, spin labels, bacteriophage labels, stable free radicals, and the like.

In some embodiments of any of the methods, elevated expression refers to an overall increase of about any of 10%, 20%, 30%, 40%, 50%, 60%, 70%, 80%, 90%, 95%, 96%, 97%, 98%, 99% or greater, in the level of biomarker (*e.g.,* protein or nucleic acid (*e.g.,* gene or mRNA)), detected by standard art known methods such as those described herein, as compared to a reference sample, reference cell, reference tissue, control sample, control cell, or control tissue. In certain embodiments, the elevated expression refers to the increase in expression level/amount of a biomarker in the sample wherein the increase is at least about any of 1.5X, 1.75X, 2X, 3X, 4X, 5X, 6X, 7X, 8X, 9X, 10X, 25X, 50X, 75X, or 100X the expression level/amount of the respective biomarker in a reference sample, reference cell, reference tissue, control sample, control cell, or control tissue. In some embodiments, elevated expression refers to an overall increase of greater than about 1.5 fold, about 1.75 fold, about 2 fold, about 2.25 fold, about 2.5 fold, about 2.75 fold, about 3.0 fold, or about 3.25 fold as compared to a reference sample, reference cell, reference tissue, control sample, control cell, control tissue, or internal control (*e.g*., housekeeping gene).

In some embodiments of any of the methods, reduced expression refers to an overall reduction of about any of 10%, 20%, 30%, 40%, 50%, 60%, 70%, 80%, 90%, 95%, 96%, 97%, 98%, 99% or greater, in the level of biomarker (*e.g.,* protein or nucleic acid (*e.g.,* gene or mRNA)), detected by standard art known methods such as those described herein, as compared to a reference sample, reference cell, reference tissue, control sample, control cell, or control tissue. In certain embodiments, reduced expression refers to the decrease in expression level/amount of a biomarker in the sample wherein the decrease is at least about any of 0.9X, 0.8X, 0.7X, 0.6X, 0.5X, 0.4X, 0.3X, 0.2X, 0.1X, 0.05X, or 0.01X the expression level/amount of the respective biomarker in a reference sample, reference cell, reference tissue, control sample, control cell, or control tissue.

Presence and/or expression level/amount of various biomarkers in a sample can be analyzed by a number of methodologies, many of which are known in the art and understood by the skilled artisan, including, but not limited to, immunohistochemical ("IHC"), Western blot analysis, immunoprecipitation, molecular binding assays, ELISA, ELIFA, fluorescence activated cell sorting ("FACS"), MassARRAY, proteomics, quantitative blood based assays (as for example Serum ELISA), biochemical enzymatic activity assays, in situ hybridization, Southern analysis, Northern analysis, whole genome sequencing, polymerase chain reaction ("PCR") including quantitative real time PCR ("qRT-PCR") and other amplification type detection methods, such as, for example, branched DNA, SISBA, TMA and the like), RNA-Seq, FISH, microarray analysis, gene expression profiling, and/or serial analysis of gene expression ("SAGE"), as well as any one of the wide variety of assays that can be performed by protein, gene, and/or tissue array analysis. Typical protocols for evaluating the status of genes and gene products are found, for example in Ausubel et al., eds., 1995, Current Protocols In Molecular Biology, Units 2 (Northern Blotting), 4 (Southern Blotting), 15 (Immunoblotting) and 18 (PCR Analysis). Multiplexed immunoassays such as those available from Rules Based Medicine or Meso Scale Discovery ("MSD") may also be used.

In some embodiments, presence and/or expression level/amount of a biomarker is determined using a method comprising: (a) performing gene expression profiling, PCR (such as rtPCR), RNA-seq, microarray analysis, SAGE, MassARRAY technique, or FISH on a sample (such as a subject cancer sample); and b) determining presence and/or expression level/amount of a biomarker in the sample. In some embodiments, the microarray method comprises the use of a microarray chip having one or more nucleic acid molecules that can hybridize under stringent conditions to a nucleic acid molecule encoding a gene mentioned above or having one or more polypeptides (such as peptides or antibodies) that can bind to one or more of the proteins encoded by the genes mentioned above. In one embodiment, the PCR method is qRT-PCR. In one embodiment, the PCR method is multiplex-PCR. In some embodiments, gene expression is measured by microarray. In some embodiments, gene expression is measured by qRT-PCR. In some embodiments, expression is measured by multiplex-PCR.

### F. Pharmaceutical Formulations

Pharmaceutical formulations of an anti-RSPO antibody as described herein are prepared by mixing such antibody having the desired degree of purity with one or more optional pharmaceutically acceptable carriers (Remington's Pharmaceutical Sciences 16th edition, Osol, A. Ed. (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g*. Zn-protein complexes); and/or non-ionic surfactants such as polyethylene glycol (PEG). Exemplary pharmaceutically acceptable carriers herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX®, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody formulations are described in US Patent No. 6,267,958. Aqueous antibody formulations include those described in US Patent No. 6,171,586 and WO2006/044908, the latter formulations including a histidine-acetate buffer.

The formulation herein may also contain more than one active ingredients as necessary for the particular indication being treated, preferably those with complementary activities that do not adversely affect each other. Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the antibody, which matrices are in the form of shaped articles, *e.g*. films, or microcapsules.

The formulations to be used for *in vivo* administration are generally sterile. Sterility may be readily accomplished, *e.g*., by filtration through sterile filtration membranes.

### G. Therapeutic Methods and Compositions

Any of the anti-RSPO antibodies provided herein may be used in therapeutic methods.

In one aspect, an anti-RSPO antibody for use as a medicament is provided. In further aspects, an anti-RSPO antibody for use in treating tumor, cell proliferative disorder, and/or cancer is provided. In some embodiments, an anti-RSPO antibody is provided for use in promoting differentiation of cells including terminal differentiation of cancer cells. In certain embodiments, an anti-RSPO antibody for use in a method of treatment is provided. In certain embodiments, the invention provides an anti-RSPO antibody for use in a method of treating an individual having tumor, cell proliferative disorder, and/or cancer comprising administering to the individual an effective amount of the anti-RSPO antibody. In some embodiments, the cancer is colorectal cancer. In one such embodiment, the method further comprises administering to the individual an effective amount of at least one additional therapeutic agent, *e.g.,* as described below. In some embodiments, the RSPO is RSPO2. In some embodiments, the RSPO is RSPO3. In some embodiments, the RSPO is RSPO2 and RSPO3. In further embodiments, the invention provides an anti-RSPO antibody for use in inhibiting wnt signaling, inhibiting angiogenesis, inhibiting cell proliferation, inhibiting cancer stem cell proliferation, and/or depleting cancer stem cells. In certain embodiments, the invention provides an anti-RSPO antibody for use in a method of inhibiting wnt signaling, inhibiting angiogenesis, inhibiting cell proliferation, inhibiting cancer stem cell proliferation, and/or depleting cancer stem cells in an individual comprising administering to the individual an effective of the anti-RSPO antibody to inhibit wnt signaling, inhibit angiogenesis, inhibit cell proliferation, inhibit cancer stem cell proliferation, and/or deplete cancer stem cells. An "individual" according to any of the above embodiments is preferably a human. In some embodiments, the individual and/or cancer has one or more biomarker. In some embodiments, the one or more biomarkers comprises an RSPO translocation. In some embodiments, the RSPO translocation comprises and RSPO2 and/or RSPO3 translocation. In some embodiments, the individual and/or cancer has increased expression of one or more biomarker. In some embodiments, the one or more biomarker comprises RSPO, *e.g*., RSPO2 and/or RSPO3. In some embodiments, the one or more biomarker comprises a stem cell biomarker. In some embodiments, the stem cell biomarker comprises Myc, Axin2, LGR5, TERT, BIRC5, and/or Ascl2. In some embodiments, the individual and/or cancer has decreased expression of one or more biomarker of differentiation. In some embodiments, the biomarker of differentiation comprises CEACAM7, SLC26A3, CA1, SYT15, CA4, TFF1, and/or KRT20. In some embodiments, treatment with the anti-RSPO antibody reduces expression of one or more stem cell biomarker, *e.g*., Myc, Axin2, LGR5, TERT, BIRC5, and/or Ascl2. In some embodiments, treatment with the anti-RSPO antibody increases expression of one or more biomarker of differentiation, *e.g*., CEACAM7, SLC26A3, CA1, SYTl5, CA4, TFF1, and/or KRT20. In some embodiments, the cancer is adrenal cancer, bladder cancer, brain cancer, breast cancer, cervix cancer, colon cancer, head and neck cancer, kidney cancer, leukemia, liver cancer, lung cancer (*e.g*., NSCLC), lymphoid cancer, ovarian cancer, pancreatic cancer, prostate cancer, rectum cancer, skin cancer (*e.g.,* melanoma), stomach cancer, thyroid cancer, and/or uterine cancer. In some embodiments, the cancer is lung cancer (*e.g.*, NSCLC), ovarian cancer, breast cancer, liver cancer, or multiple myeloma. In some embodiments, the cancer is colorectal cancer.

In a further aspect, the invention provides for the use of an anti-RSPO antibody in the manufacture or preparation of a medicament. In one embodiment, the medicament is for treatment of tumor, cell proliferative disorder, and/or cancer. In a further embodiment, the medicament is for use in a method of treating tumor, cell proliferative disorder, and/or cancer comprising administering to an individual having tumor, cell proliferative disorder, and/or cancer an effective amount of the medicament. In some embodiments, the cancer is colorectal cancer. In one such embodiment, the method further comprises administering to the individual an effective amount of at least one additional therapeutic agent, *e.g.,* as described below. In a further embodiment, the medicament is for inhibiting wnt signaling, inhibiting angiogenesis, inhibiting cell proliferation, inhibiting cancer stem cell proliferation, and/or depleting cancer stem cells. In a further embodiment, the medicament is for use in a method of inhibiting wnt signaling, inhibiting angiogenesis, inhibiting cell proliferation, inhibiting cancer stem cell proliferation, and/or depleting cancer stem cells in an individual comprising administering to the individual an amount effective of the medicament to inhibit wnt signaling, inhibit angiogenesis, inhibit cell proliferation, inhibit cancer stem cell proliferation, and/or deplete cancer stem cells. An "individual" according to any of the above embodiments may be a human. In some embodiments, the individual and/or cancer has one or more biomarker. In some embodiments, the one or more biomarkers comprises an RSPO translocation. In some embodiments, the RSPO translocation comprises and RSPO2 and/or RSPO3 translocation. In some embodiments, the individual and/or cancer has increased expression of one or more biomarker. In some embodiments, the one or more biomarker comprises RSPO, *e.g*., RSPO2 and/or RSPO3. In some embodiments, the one or more biomarker comprises a stem cell biomarker. In some embodiments, the stem cell biomarker comprises Myc, Axin2, LGR5, TERT, BIRC5, and/or Ascl2. In some embodiments, the individual and/or cancer has decreased expression of one or more biomarker of differentiation. In some embodiments, the biomarker of differentiation comprises CEACAM7, SLC26A3, CA1, SYT15, CA4, TFF1, and/or KRT20. In some embodiments, treatment with the anti-RSPO antibody reduces expression of one or more stem cell biomarker, *e.g*., Myc, Axin2, LGR5, TERT, BIRC5, and/or Ascl2. In some embodiments, treatment with the anti-RSPO antibody increases expression of one or more biomarker of differentiation, *e.g*., CEACAM7, SLC26A3, CA1, SYTl5, CA4, TFF1, and/or KRT20. In some embodiments, the cancer is adrenal cancer, bladder cancer, brain cancer, breast cancer, cervix cancer, colon cancer, head and neck cancer, kidney cancer, leukemia, liver cancer, lung cancer (*e.g*., NSCLC), lymphoid cancer, ovarian cancer, pancreatic cancer, prostate cancer, rectum cancer, skin cancer *(e.g.,* melanoma), stomach cancer, thyroid cancer, and/or uterine cancer. In some embodiments, the cancer is lung cancer (*e.g*., NSCLC), ovarian cancer, breast cancer, liver cancer, or multiple myeloma.

In a further aspect, the invention provides a method for treating a tumor, cell proliferative disorder, and/or cancer. In one embodiment, the method comprises administering to an individual having such tumor, cell proliferative disorder, and/or cancer an effective amount of an anti-RSPO antibody. In some embodiments, the cancer is colorectal cancer. In one such embodiment, the method further comprises administering to the individual an effective amount of at least one additional therapeutic agent, as described below. An "individual" according to any of the above embodiments may be a human. In some embodiments, the individual and/or cancer has one or more biomarker. In some embodiments, the one or more biomarkers comprises an RSPO translocation. In some embodiments, the RSPO translocation comprises and RSPO2 and/or RSPO3 translocation. In some embodiments, the individual and/or cancer has increased expression of one or more biomarker. In some embodiments, the one or more biomarker comprises RSPO, *e.g*., RSPO2 and/or RSPO3. In some embodiments, the one or more biomarker comprises a stem cell biomarker. In some embodiments, the stem cell biomarker comprises Myc, Axin2, LGR5, TERT, BIRC5, and/or Ascl2. In some embodiments, the individual and/or cancer has decreased expression of one or more biomarker of differentiation. In some embodiments, the biomarker of differentiation comprises CEACAM7, SLC26A3, CA1, SYTl5, CA4, TFF1, and/or KRT20. In some embodiments, treatment with the anti-RSPO antibody reduces expression of one or more stem cell biomarker, *e.g*., Myc, Axin2, LGR5, TERT, BIRC5, and/or Ascl2. In some embodiments, treatment with the anti-RSPO antibody increases expression of one or more biomarker of differentiation, *e.g*., CEACAM7, SLC26A3, CA1, SYTl5, CA4, TFF1, and/or KRT20. In some embodiments, the cancer is adrenal cancer, bladder cancer, brain cancer, breast cancer, cervix cancer, colon cancer, head and neck cancer, kidney cancer, leukemia, liver cancer, lung cancer (*e.g*., NSCLC), lymphoid cancer, ovarian cancer, pancreatic cancer, prostate cancer, rectum cancer, skin cancer (*e.g*., melanoma), stomach cancer, thyroid cancer, and/or uterine cancer. In some embodiments, the cancer is lung cancer (*e.g*., NSCLC), ovarian cancer, breast cancer, liver cancer, or multiple myeloma.

In a further aspect, the invention provides a method inhibiting wnt signaling, inhibiting angiogenesis, inhibiting cell proliferation, inhibiting cancer stem cell proliferation, and/or depleting cancer stem cells in an individual. In one embodiment, the method comprises administering to the individual an effective amount of an anti-RSPO antibody to inhibit wnt signaling, inhibit angiogenesis, inhibit cell proliferation, inhibit cancer stem cell proliferation, and/or deplete cancer stem cells. In one embodiment, an "individual" is a human. In some embodiments, the individual and/or cancer has one or more biomarker. In some embodiments, the one or more biomarkers comprises an RSPO translocation. In some embodiments, the RSPO translocation comprises and RSPO2 and/or RSPO3 translocation. In some embodiments, the individual and/or cancer has increased expression of one or more biomarker. In some embodiments, the one or more biomarker comprises RSPO, *e.g*., RSPO2 and/or RSPO3. In some embodiments, the one or more biomarker comprises a stem cell biomarker. In some embodiments, the stem cell biomarker comprises Myc, Axin2, LGR5, TERT, BIRC5, and/or Ascl2. In some embodiments, the individual and/or cancer has decreased expression of one or more biomarker of differentiation. In some embodiments, the biomarker of differentiation comprises CEACAM7, SLC26A3, CA1, SYTl5, CA4, TFF1, and/or KRT20. In some embodiments, treatment with the anti-RSPO antibody reduces expression of one or more stem cell biomarker, *e.g*., Myc, Axin2, LGR5, TERT, BIRC5, and/or Ascl2. In some embodiments, treatment with the anti-RSPO antibody increases expression of one or more biomarker of differentiation, *e.g*., CEACAM7, SLC26A3, CA1, SYTl5, CA4, TFF1, and/or KRT20. In some embodiments, the cancer is adrenal cancer, bladder cancer, brain cancer, breast cancer, cervix cancer, colon cancer, head and neck cancer, kidney cancer, leukemia, liver cancer, lung cancer (*e.g*., NSCLC), lymphoid cancer, ovarian cancer, pancreatic cancer, prostate cancer, rectum cancer, skin cancer (*e.g*., melanoma), stomach cancer, thyroid cancer, and/or uterine cancer. In some embodiments, the cancer is lung cancer (*e.g*., NSCLC), ovarian cancer, breast cancer, liver cancer, or multiple myeloma. In some embodiments, the cancer is colorectal cancer.

In a further aspect, the invention provides pharmaceutical formulations comprising any of the anti-RSPO antibodies provided herein, *e.g.,* for use in any of the above therapeutic methods. In one embodiment, a pharmaceutical formulation comprises any of the anti-RSPO antibodies provided herein and a pharmaceutically acceptable carrier. In another embodiment, a pharmaceutical formulation comprises any of the anti-RSPO antibodies provided herein and at least one additional therapeutic agent, *e.g.,* as described below. In some embodiments, the RSPO is RSPO2. In some embodiments, the RSPO is RSPO3. In some embodiments, the RSPO is RSPO2 and RSPO3. In some embodiments, the individual and/or cancer has increased expression of one or more biomarker. In some embodiments, the one or more biomarker comprises RSPO, *e.g*., RSPO2 and/or RSPO3. In some embodiments, the one or more biomarker comprises a stem cell biomarker. In some embodiments, the stem cell biomarker comprises Myc, Axin2, LGR5, TERT, BIRC5, and/or Ascl2. In some embodiments, the individual and/or cancer has decreased expression of one or more biomarker of differentiation. In some embodiments, the biomarker of differentiation comprises CEACAM7, SLC26A3, CA1, SYTl5, CA4, TFF1, and/or KRT20. In some embodiments, treatment with the anti-RSPO antibody reduces expression of one or more stem cell biomarker, *e.g*., Myc, Axin2, LGR5, TERT, BIRC5, and/or Ascl2. In some embodiments, treatment with the anti-RSPO antibody increases expression of one or more biomarker of differentiation, *e.g*., CEACAM7, SLC26A3, CA1, SYTl5, CA4, TFF1, and/or KRT20. In some embodiments, the cancer is adrenal cancer, bladder cancer, brain cancer, breast cancer, cervix cancer, colon cancer, head and neck cancer, kidney cancer, leukemia, liver cancer, lung cancer (*e.g.,* NSCLC), lymphoid cancer, ovarian cancer, pancreatic cancer, prostate cancer, rectum cancer, skin cancer (e.g., melanoma), stomach cancer, thyroid cancer, and/or uterine cancer. In some embodiments, the cancer is lung cancer (e.g., NSCLC), ovarian cancer, breast cancer, liver cancer, or multiple myeloma. In some embodiments, the cancer is colorectal cancer.

Antibodies of the invention can be used either alone or in combination with other agents in a therapy. For instance, an antibody of the invention may be co-administered with at least one additional therapeutic agent. In certain embodiments, an additional therapeutic agent is a cytotoxic agent, chemotherapeutic agent, cytostatic agent, anti-hormonal agent, and/or EGFR inhibitor.

Such combination therapies noted above encompass combined administration (where two or more therapeutic agents are included in the same or separate formulations), and separate administration, in which case, administration of the antibody of the invention can occur prior to, simultaneously, and/or following, administration of the additional therapeutic agent or agents. In one embodiment, administration of the anti-RSPO antibody and administration of an additional therapeutic agent occur within about one month, or within about one, two or three weeks, or within about one, two, three, four, five, or six days, of each other. Antibodies of the invention can also be used in combination with radiation therapy.

An antibody of the invention (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

Antibodies of the invention would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The antibody need not be, but is optionally formulated with one or more agents currently used to prevent or treat the disorder in question. The effective amount of such other agents depends on the amount of antibody present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of an antibody of the invention (when used alone or in combination with one or more other additional therapeutic agents) will depend on the type of disease to be treated, the type of antibody, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments. Depending on the type and severity of the disease, about 1 µg/kg to 15 mg/kg (*e.g*. 0.1 mg/kg-10 mg/kg) of antibody can be an initial candidate dosage for administration to the patient, whether, for example, by one or more separate administrations, or by continuous infusion. One typical daily dosage might range from about 1 µg/kg to 100 mg/kg or more, depending on the factors mentioned above. For repeated administrations over several days or longer, depending on the condition, the treatment would generally be sustained until a desired suppression of disease symptoms occurs. One exemplary dosage of the antibody would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, *e.g*. every week or every three weeks (*e.g.* such that the patient receives from about two to about twenty, or *e.g.* about six doses of the antibody). An initial higher loading dose, followed by one or more lower doses may be administered. However, other dosage regimens may be useful. The progress of this therapy is easily monitored by conventional techniques and assays.

It is understood that any of the above formulations or therapeutic methods may be carried out using an immunoconjugate of the invention in place of or in addition to an anti-RSPO antibody.

### H. Articles of Manufacture

In another aspect of the invention, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is an antibody of the invention. The label or package insert indicates that the composition is used for treating the condition of choice. Moreover, the article of manufacture may comprise (a) a first container with a composition contained therein, wherein the composition comprises an antibody of the invention; and (b) a second container with a composition contained therein, wherein the composition comprises a further cytotoxic or otherwise therapeutic agent. The article of manufacture in this embodiment of the invention may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

It is understood that any of the above articles of manufacture may include an immunoconjugate of the invention in place of or in addition to an anti-RSPO antibody.

### III. EXAMPLES

The following are examples of methods and compositions of the invention. It is understood that various other embodiments may be practiced, given the general description provided above.

### METHODS

***Cloning and Purification:*** FLAG-tagged RNF43 was purified by anti-FLAG affinity chromatography (Genentech), followed by size-exclusion chromatography (Superdex 75, GE Healthcare). FLAG-tagged R-spondins (hRSPO2, hRSPO2 L186, cynoRSPO2, hRSPO3, and cynoRSPO3FLAG) were purified by anti-FLAG affinity chromatography, followed by cation exchange chromatography (Mono S, GE Healthcare). Human IgG1 Fc-tagged LGR extracellular domains were purified by affinity chromatography (MabSelect SuRe, GE Healthcare), followed by size-exclusion chromatography (Superdex 200, GE Healthcare).

***WNT reporter assays:*** In 96 well plates, 4,500 293T cells per well were plated in 90 ul of DMEM supplemented with 2.5% fetal bovine serum. Following 16-20 hours of culture, cells were co-transfected with 0.04 ug Topbrite 25 and 0.02 ug SV40 Renilla DNA in 10 ul of transfection mix using Fugene 6 (Promega, Madison, WI). Following an additional 16-20 hours of culture, cells were stimulated with 25 ul of a 5X solution for 6 hours at 37 degrees Celsius. For supernatant screens, cells were stimulated with hybridoma supernatant supplemented with 50 ng/ml rmWNT3a (R&D Systems, Minneapolis, MN) and 250 pM rhRSPO2 or rhRSPO3 (R&D Systems, Minneapolis, MN). For assays using cloned antibodies, DMEM supplemented with 10% fetal bovine serum, 50 ng/ml rmWNT3a, 250 pM or 5X calculated EC50 rRSPO (as indicated) and increasing concentrations of antibody were added. For assays testing conditioned media, media were prepared by transfecting 293T with the indicated genes using Fugene 6 according to manufacturer's instructions (Promega, Madison, WI). Conditioned media were collected 3 days following transfection, supplemented with 50 ng/ml rmWNT3a +/- anti-RSPO antibodies and added to reporter cells. Following stimulation for 6 hours, luciferase activity was detected using the Promega Dual-Glo system (Promega, Madison, WI). Data were analyzed as either a ratio of Firefly/Renilla (RLU WNT reporter), or normalized values in absence of antibodies (RLU with antibody/RLU no antibody). IC50 measurements were determined by stimulating cells with the EC50 of rRSPO with increasing concentrations of antibody. Log transformed data were fit with a four-parameter dose-response equation using GraphPad Prism.

***Generation of RSPO-expressing cell pellets:*** pGCIG is a HIV-based self-inactivating lentiviral vector that was created by replacing the Zeo^{R}-CMVᵢₑ-tGFP-IRES-Puro^{R}-shRNA-WRE content of pGIPZ (Open Biosystems) with a fragment containing the CMVie promoter, a multiple cloning site (MCS), an internal ribosome entry site (IRES) and enhanced green fluorescent protein (eGFP). The human R-spondin 1-4 open reading frames (ORFs) were tagged with an HA epitope (YPYDVPDYA) at the C-terminus by PCR and inserted into the MCS of pGCIG. HEK-293 cells were plated on 15-cm dishes at 15x10⁶ cells/plate in DMEM High Glucose with 10% heat inactivated FBS 24 h prior to transfection. Lentiviral supernatants were prepared by cotransfection using 6 ug of pGCIG-hRSPO, 12 ug of the packaging vector Δ8.9 (Zufferey et al., 1997), 3 ug of the envelope vector pVSV-G (Clontech) and the transfection reagent Genejuice (Novagen). The culture medium was replaced 12 h after transfection and viral supernatant was collected 24 h later, filtered through a 0.45 µm PES filter (Nalgene) and stored at 4 °C until further processing. HEK-293 cells were plated on 10-cm dishes at 1x10⁶ cells/plate in DMEM High Glucose with 10% heat inactivated FBS. The cells were allowed to adhere for 12 h, after which the medium was replaced with 10 ml of viral supernatant. Viral supernatants remained on the cells for 60 h, after which the cells were harvested and analyzed for fluorescent protein expression by FACS. Gates were set to sort out 2x10⁵ low, medium and high eGFP expressing cells for each viral construct. Cell lines were expanded and tested for the absence of replication competent virus (RCV) production using the HIV-1 p24 Antigen ELISA 2.0 kit (ZeptoMetrix Corporation). Expression and secretion of human R-spondins was confirmed by anti-HA Western blotting of concentrated cell culture supernatants and correlated well with the eGFP expression levels.

***IHC reactivity screening:*** Formalin-fixed paraffin-embedded cell pellets were sectioned at 4 um. Slides were pre-treated with citrate-based pH 6.0 buffer (Dako cat no. S1699, Carpinteria, CA) at 99 degrees Celsius for 20 minutes. After a 10% serum block, anti-RSPO sera were used at 1:250 and hybridoma supernatants were run. Pre-immune sera at 1:250 or naive mouse IgG1, 2a, and 2b at a total concentration of 10 ug/ml was used as the negative control. Biotinylated donkey anti-mouse secondary (Jackson Immuno cat no 715-065-151, West Grove, PA) was used at 5ug/ml. VECTASTAIN Elite ABC Kit (Standard*) (Vector Labs cat no PK-6100) was used as detection and signal was visualized with Pierce Metal Enhanced DAB (Thermo cat no 34065, Rockford, IL).

***Epitope Binning:*** Epitope binning of anti-RSPO antibodies was performed using the Octet RED384 instrument (ForteBio). Recombinant RSPO (R&D Systems, Minneapolis, MN) was biotinylated and captured onto Streptavidin biosensors at 10 µg/ml for 120 seconds. Binding of the first antibody to saturation was achieved by adding 10 µg/ml for 600 seconds. The same biosensors were dipped into the competing antibodies at 5 µg/ml and binding was measured for 300 seconds. The failure of the second antibody to bind in the presence of saturating quantities of the first antibody indicated the two antibodies were in the same epitope bin.

***Affinity Measurements:*** Binding affinities of anti-RSPO antibodies were measured by Surface Plasmon Resonance (SPR) using a BIAcore™-2000 instrument. The CM5 biosensor chip was activated with *N*-ethyl-*N*'-(3-dimethylaminopropyl)-carbodiimide hydrochloride (EDC) and *N-*hydroxysuccinimide (NHS) reagents according to the supplier's (GE Healthcare Biosciences, Piscataway, NJ) instructions. RSPO antigens were immobilized onto the biosensor chip to achieve approximately 250 response unit (RU), followed by blocking with 1M ethanolamine.

For kinetic measurements, two-fold serial dilutions of anti-RSPO Fabs were injected in HBS-P buffer (0.01M HEPES pH 7.4, 0.15M NaCl, 0.005% surfactant P20) at 25°C with a flow rate of 30µl/min. Association rates (kₒₙ) and dissociation rates (k_{off}) were calculated using a simple one-to-one Langmuir binding model (BIAcore Evaluation Software version 3.2). The equilibrium dissociation constant (KD) was calculated as the ratio k_{off}/kₒₙ.

***Competitive Binding ELISA:*** To measure the activity of anti-RSPO antibodies in blocking the binding of LGR4 and -5 ECDs to RSPOs, MaxiSorp 384-well microwell plates (Thermo Scientific Nunc, Roskilde, Denmark) were coated with 25 ul/well of 0.5 µg/ml hRSPO2 or hRSPO3 (Genentech) in 50 mM carbonate buffer, pH 9.6, overnight at 4°C. Plates were blocked with at 80 ul/well of 0.5% bovine serum albumin, 15 parts per million Proclin 300 in phosphate buffered saline (PBS), pH 7.4, for 1 hour. Serially diluted anti-RSPO antibodies (0.078-10 ng/ml in 3-fold serial dilution plus buffer blank) containing 0.1 µg/ml LGR4-Fc or 0.015 µg/ml LGR5-Fc in assay buffer (0.5% BSA, 0.05% polysorbate 20, 15 parts per million Proclin 300 in PBS) were added to the plates at 25 ul/well. After a 2-hour incubation, LGR4-Fc and LGR5-Fc bound to the plates were detected using peroxidase labeled goat F(ab')2 anti-human Fc (Jackson ImmunoResearch, West Grove, PA). After a 1 hour incubation, the substrate 3,3',5,5'-tetramethyl benzidine (Moss Inc., Pasadena, Maryland) was added to the plates and the reaction was stopped by adding 1 M phosphoric acid. Plates were washed with PBS, pH 7.4, containing 0.05% tween 20, between steps and all the incubation steps following the coating step were performed at room temperature on an orbital shaker. Absorbance was read at 450 nm on a multiskan Ascent reader (Thermo Scientific, Hudson, NH).

The activities of anti-RSPO antibodies in blocking binding of RNF43 to RSPOs were measured similarly using 0.5 ng/ml biotinylated RNF43-Flag (on RSPO2 coated plates) or 20 ng/ml biotinylated RNF43-Flag (on RSPO3 coated plates). Bound biotinylated RNF43-Flag was detected using peroxidase labeled streptavidin (GE Healthcare, Piscataway, NJ) followed by the substrate as described above.

***Humanization of anti-RSPO3 Antibodies:*** Monoclonal antibody 5D6 was humanized as described below. Residue numbers are according to Kabat et al., Sequences of proteins of immunological interest, 5th Ed., Public Health Service, National Institutes of Health, Bethesda, Md. (1991).

Variants constructed during the humanization of 5D6 were assessed in the form of Fab. The VL and VH domains from murine 5D6 were aligned with the human VL kappa I (VLKI) and human VH subgroup IV (VH4) consensus sequences. Hypervariable regions from the murine antibodies were engineered into VLKI and VHI acceptor frameworks. Specifically, from the mu5D6 VL domain, positions 24-34 (L1), 50-56 (L2) and 89-97 (L3) were grafted into VLKI and from the mu5D6 VH domain; positions 26-35 (H1), 50-65 (H2) and 95-102 (H3) were grafted into VHI. All VL and VH vernier positions from mu5D6 were also grafted to the VLK1 and VH4, respectively. This graft is referred to as v1.

The binding affinity of the antibodies in this section was determined by BIAcore™ T200 Format. Briefly, BIAcore™ research grade CM5 chips were activated with 1-ethyl-3-(3-dimethylaminopropyl) carbodiimide (EDC) and N-hydroxysuccinimide (NHS) reagents according to the supplier's instructions. huRSPO3 was immobilized to achieve approximately 50 response units (RU) in each flow cell. Unreacted coupling groups were blocked with 1M ethanolamine. For kinetics measurements, Four-fold serial dilutions of variant antibody was injected in HBS-P buffer (0.01M HEPES pH7.4, 0.15M NaCl, 0.005% surfactant P20) at 25° C. with a flow rate of 30 µl/min. Association rates (kon) and dissociation rates (koff) were calculated using a 1:1 Langmuir binding model (BIAcore™ T200 Evaluation Software version 2.0). The equilibrium dissociation constant (Kd) was calculated as the ratio koff/kon.

***Crystallography RSPO3 (M33-E210) Purification* :** RSPO3 (M33-E210) containing an N-terminal His-MBP tag was co-expressed with untagged EndoH in SF9 cells grown in medium treated with Kifunensine. Cell supernatants were harvested and passed over a 10 mL Nickel-NTA agarose column that had been pre-equilibrated in Wash Buffer (25 mM Tris-HCl pH 7.5, 500 mM NaCl, 20 mM imidazole, 5% glycerol). The column was then washed with 10 column volumes of Wash Buffer. Protein was eluted from the column using 5 column volumes of Elution Buffer (25 mM Tris-HCl pH 7.5, 500 mM NaCl, 300 mM imidazole, 10% glycerol) and concentrated to less than 30 mL. TEV protease was added and the sample was dialyzed overnight against Dialysis Buffer (25 mM Tris-HCl pH 7.5, 500 mM NaCl, 10 mM imidazole, 10% glycerol) at 4°C. Following dialysis, the sample was passed through a 5 mL HisTrap column that had been pre-equilibrated with Wash Buffer. The sample was then concentrated to less than 2 mL and applied to a Superdex 75 16/60 column that had been pre-equilibrated with Gel Filtration Buffer (25 mM Tris-HCl pH 7.5, 300 mM NaCl, 5% glycerol). Fractions containing RSPO3 (M33-E210) were pooled and concentrated. Aliquots were stored at - 80°C.

***Crystallography Fab Purification:*** Fabs 5D6 and 26E11 were expressed in *E. coli* cells. Cell paste was resuspended in Lysis Buffer (PBS supplemented with 25 mM EDTA and 1 mM PMSF) and cells were lysed by three passages through a microfluidizer. Lysate was then spun at 12,000 rpm for one hour and the cleared lysates were filtered through a 0.8 µm filter. Cleared lysates were applied directly to a 25 mL Protein G column that had been pre-equilibrated with PBS supplemented with 25 mM EDTA. The column was washed with 10 column volumes of PBS and protein was eluted with 0.58% acetic acid. Eluates were then loaded onto a HiTrap SP HP column that had been pre-equilibrated with Buffer A (20 mM MES pH 5.5). The column was washed with 10 column volumes of Buffer A and protein was eluted over a 20 column volume linear gradient from Buffer A to Buffer B (20 mM MES pH 5.5, 500 mM NaCl). Fractions containing Fab were pooled and concentrated to less than 2 mL and applied to a Superdex 75 26/60 column that had been pre-equilibrated with Gel Filtration Buffer. Fractions containing Fab were pooled and concentrated. Aliquots were stored at - 80°C.

***Crystallography RSPO3*/*****Fab Complex Purification:*** To form a complex, a 1.25-fold molar excess of RSPO3 (M33-E210) was added to 150 nmol of either Fab in an 800 µL binding reaction containing Gel Filtration Buffer. Binding reactions were incubated for one hour on ice. Reactions were then spun at 13,000 rpm at 4°C and loaded onto a Superdex 75 16/60 column that had been pre-equilibrated with Gel Filtration Buffer. Fractions containing complex were pooled and concentrated to 20 mg/mL. Aliquots were stored at -80°C.

***Crystallography:*** For RSPO3 (M33-E210)/Fab 5D6, Labcyte Echo liquid handler was used to set several sparse matrix crystal screens using 100 nL sitting drops. Screens were stored at 18°C. Crystals were obtained in a drop containing 100 mM MIB pH 9 and 25% PEG 1500 as the mother liquor. A cryoprotectant solution was made by mixing 1 µL 70% glycerol with 1.8µL reservoir solution. A single crystal was harvested and soaked in cryoprotectant solution for 10 seconds and flash-frozen in liquid nitrogen.

For RSPO3 (M33-E210)/Fab 26E11, a Labcyte Echo liquid handler was used to set several sparse matrix crystal screens using 100 nL sitting drops. Screens were stored at 18°C. Crystals were obtained in a drop containing 200 mM Sodium formate and 20% (w/v) PEG 3,350 as the mother liquor. A cryoprotectant solution was made by mixing 1 µL 70% ethylene glycol with 1.8µL reservoir solution. A single crystal was harvested and soaked in cryoprotectant solution for 10 seconds and flash-frozen in liquid nitrogen.

Both complexes crystallized in a wide range of similar conditions. Nearly all crystals grew in PEG-based conditions, with the most common being 20-25% PEG 3,350. Other successful precipitants included 20% PEG 6,000, 20-25% PEG 4,000, and 25% PEG 1,500. pH ranged from 3.5 - 9, with the majority of crystal growth seen between 7 and 8. Various salts at 200 mM concentration aided crystal growth

***Crystal Structure Determination and Refinement:*** Diffraction data for the two RSPO3/Fab complexes was collected at the synchrotron. The data was indexed, integrated and scaled using XDS and SCALA. The crystal structure of RSPO3/5D6 and RSPO3/26E11 were solved by molecular replacement using the Fab structure as the search model. The phases for the initial molecular replacement solution were improved by solvent flattening and density modification using PHENIX. Iterative round of refinement and rebuilding were used to build the RSPO3 structure. The crystallographic statistics for the RSPO3/5D6 and RSPO/26E11 are below.

**Table 1. Data collection and refinement statistics.**

| | **RSPO3/5D6** | **RSPO3/26E11** |
|---|---|---|
| **Wavelength (Å)** | 1.0 | 0.976 |
| **Resolution range (Å)** | 46.94 - 2.15 (2.22 - 2.15) | 48.0 - 2.51 (2.6 - 2.51) |
| **Space group** | P 1 21 1 | P 1 21 1 |
| **Unit cell** | 87.6 52.1 91.8 90 116.8790 | 95.7 47.7 96.4 90 114.790 |
| **Unique reflections** | 40700 (3821) | 27615 (2713) |
| **Multiplicity** | 3.4 (3.6) | 3.7 (3.7) |
| **Completeness (%)** | 99.40 (95.05) | 99.87 (99.67) |
| **Mean I/sigma(I)** | 10.92 (2.52) | 13.2 (2.8) |
| **Wilson B-factor** | 37.38 | 42.12 |
| **R-merge** | 0.046 (0.4) | 0.083 (0.388) |
| **R/Rfree** | 0.191/0.237 (0.276/0.314) | 0.185/0.241 (0.249/0.296) |
| **Number of non-hydrogen atoms** | 4888 | 4731 |
| **Protein residues** | 596 | 594 |
| **RMS(bonds)** | 0.029 | 0.004 |
| **RMS(angles)** | 1.06 | 0.83 |
| **Ramachandran F/A/D** | 86.2/13.8/0 | 81.6/18.3/0 |

| | | |
|---|---|---|
| Statistics for the highest-resolution shell are shown in parentheses. | | |

***In Vivo Efficacy Experiments:*** RSPO3-fusion positive patient-derived tumors were grown subcutaneously in Balbc/Nude mice. Once tumors reached a size of approximately 200 mm³, mice were treated either with control antibody or anti-RSPO3 antibody (5D6) at 30 mg/kg, twice a week for 3-4 weeks. For experiments in which anti-RSPO3 antibody (5D6) was used in combination with irinotecan, the anti-RSPO3 antibody was dosed as described above, and irinotecan was dosed at 100 mg/kg on day 0 or on day 0 and day 3.

For the serial transplantation study, mice implanted with RSPO3-fusion positive patient-derived tumors were treated with control antibody or anti-RSPO3 antibody as described above. One the growth curves began to separate, tumor fragments were removed and transplanted into naïve Balbc/Nude mice. Mice with transplanted tumor fragments were then treated with either control or anti-RSPO3 antibody as described above.

### RESULTS

### Generation of function blocking and IHC reactive anti-RSPO antibodies

In effort to generate anti-RSPO antibodies, mice and hamsters were immunized with recombinant human RSPO2 and/or human RSP03 and hybridoma cell lines were produced. Supernatents from these cells were first screened for binding to hRSPO1, hRSPO2, hRSPO3 and hRSPO4 by ELISA. Supernatants showing hRSPO2 and/or hRSPO3 binding were then tested for the ability to block hRSPO2 and hRSPO3 stimulation of WNT reporter activity. Candidates were subsequently cloned, expressed and purified. As shown in Figure 1, a subset of the purified clones potently inhibited rhRSPO2 stimulated WNT reporter activity (Figure 1A) and/or rhRSPO3 stimulated WNT reporter activity (Figure IB).

In addition, supernatants were screened to identify anti-RSPO antibodies that could be used as IHC reagents. Formalin-fixed paraffin embedded cell pellets were prepared from 293 cells stably expressing high, medium, or low levels of hRSPO2 or hRSPO3. In addition, cell pellets were prepared from 293 cells and 293 cells stably expressing hRSPO1 or hRSPO4. Hybridoma supernatants and antibody clones were tested for IHC reactivity on the prepared cell pellets. As shown in Figure 2, the antibody 49G5 recognized by IHC reactivity high, medium, and low levels of hRSPO2 expression (D-F) while not recognizing hRSPO3 (A-C), hRSPO1 (G), hRSPO4 (H), or non-hRSPO1-4 (I). A summary of the results for all antibodies tested are shown below in Table 4. Antibodies 4H1, 4D4, 5C2, 5D6, 5E11, 21C2 specifically recognize hRSPO3. Antibodies 1A1, 36D2, 49G5 specifically recognize hRSPO2. Antibodies 6E9 and 26E11 recognize hRSPO2 and hRSPO3.

**Table 4-IHC Reactivity of panel of anti-RSPO antibodies**

| Antibody | RSPO2 Hi | Med | RSPO2 | 293 none | | RSPO3 Med | RSPO3 Low | RSPO1 Hi RSPO1 | RSPO4 Hi |
|---|---|---|---|---|---|---|---|---|---|
| 4H1 | + | - | - | - | ++ 90% | ++ 60% | + 30% | - | - |
| 4D4 | + | - | - | - | ++ > 95% | ++ 70% | ++ 40% | - | - |
| 5C2 | + | - | - | - | ++ > 95% | ++ 60% | ++ 40% | - | - |
| 5D6 | - | - | - | - | ++ > 95% | ++ 70% | ++ 30% | - | - |
| 5E11 | - | - | - | - | +++ 90% | ++ 80% | ++ 20% | - | - |
| 6E9 | ++ 50% | + 10% | - | - | ++ > 95% | ++ 70% | + 50% | - | - |

| Antibody | RSPO2 Hi | RSPO2 Med | RSPO2 Low | 293 none | RSPO3 Hi | RSPO3 Med | RSPO3 Low | RSPO1 Hi | RSPO4 Hi |
|---|---|---|---|---|---|---|---|---|---|
| 21C2 | + | - | - | - | ++ 95% | ++ 60% | + 10% | - | - |
| 26E11 | ++ 70% | + 40% | + 10% | - | +++ 90% | ++ 70% | ++ 50% | - | - |
| 1A1 | ++ 80% | + 40% | + 30% | - | - | - | - | - | - |
| 11F11 | - | - | - | - | - | - | - | - | - |
| 36D2 | ++ > 95% | ++ 50% | + <5% | - | - | - | - | - | - |
| 49G5 | ++ 95% | ++ 60% | + 40% | - | - | - | - | - | - |
| IgG | - | - | - | - | - | - | - | - | - |
| naive mouse IgG2a | - | - | - | - | - | - | - | - | - |

| | | | | | | | | | |
|---|---|---|---|---|---|---|---|---|---|
| -, +, ++, +++ indicates relative intensity with - being not significant staining and +++ being highest level of staining; Percentage indicates relative percentage of cells staining | | | | | | | | | |

### Epitope binning of anti-RSPO antibodies

To further characterize anti-RSPO antibodies, the number of unique epitope bins the antibodies fell into was determined using an OCTET RED assay. Antibodies were first affinity ranked. The antibody with the highest affinity was bound to saturation to a hRSPO2 or hRSPO3 bound biosensor. Binding by a second antibody was then assessed. The anti-RSPO2 antibodies tested fell into two unique epitope bins defined by the ability to compete with either 1A1 or 11F11. The first unique epitope bin included 1A1, 49G5, and 36D2 while the second unique epitope bin included 11F11. The anti-RSPO3 antibodies tested fell into three unique epitope bins defined by the ability to compete with 26E11, 4H1, or 21C2. The first unique epitope bin included 26E11, 5D6, 5E11, and 6E9, the second unique epitope bin included 4H1, and the third unique epitope bin included 5C2 and 21C2.

### Binding specificities and affinities of anti-RSPO antibodies

To further characterize the anti-RSPO antibodies, their function blocking activities were tested against mouse RSPO2 (R&D Systems) and cynomolgus RSPO2 (Genentech). A subset of antibody clones could block hRSPO2, cynoRSPO2, and mRSPO2 stimulation of WNT reporter cells (Figure 3A-C). A polymorphism at position 186 in RSPO2 was identified in the human population. To assess the functional blocking activity of anti-RSPO antibodies to this polymorphism and potential usefulness in this patient population, hRSPO2 L186P protein was first purified and then used to stimulate WNT reporter cells. A subset of anti-RSPO antibodies could block the function hRSPO2 L186P (Figure 3D).

In addition, anti-RSPO antibodies were tested for their ability to block the function of mouse RSPO3 (R&D Systems) and cynomolgus RSPO3 (Genentech). A subset of antibodies could inhibit the WNT reporter cell stimulation of hRSPO3, cynoRSPO3, mRSPO3 (Figure 4A-C). Anti-RSPO antibodies were additionally tested for their ability to inhibit RSPO3-fusion genes recently identified in colorectal tumors (Seshagiri et al., Nature 488:660-664 (2012)). Conditioned media was prepared by transfecting constructs encoding the two PTPRK-RSPO3 fusions genes identified (SEQ ID NO:176 and 178). Conditioned media containing RSPO3 or RSPO3-fusion genes could stimulate WNT reporter activity. An anti-RSPO3 antibody could inhibit RSPO3-fusion gene stimulation of reporter cells (Figure 4D). This result indicates that the anti-RSPO3 antibodies could inhibit RPSO translocation-mediated wnt signaling.

Surface plasmon resonance was used to confirm binding specificities and affinities to human, mouse, and cynomolgus RSPOs. Fab fragments from antibody clones were digested, purified, and then assayed using a BIAcore™-2000 instrument for binding to recombinant proteins. Antibodies fell into three groups: those specific to RSPO2, those specific to RSPO3, and those with some degree of cross-reactivity (Figure 5). Binding affinities were in the sub to low nanomolar range (range 0.073 - 80 nM).

### Binding characteristics of anti-RSPO antibodies

It has previously been shown that RSPO proteins can bind to two different classes of transmembrane proteins: the E3-ligases (RNF43 and ZNRF3) and the LGRs (LGR4 and LGR5) (Hao et al., Nature 485(7397):195-200 (2012)). To test whether the anti-RSPO antibodies could inhibit binding with these two classes of proteins, a competitive binding ELISA assays was developed. Anti-RSPO antibodies fell into three categories when tested for the ability to inhibit LGR4 or LGR5 binding to hRSPO2 and hRSPO3: those that could inhibit, those that did not inhibit, and one that promoted the interaction of LGR4 and LGR5 (Figure 6A-B and data not shown). Likewise, a subset of the panel of anti-RSPO antibodies inhibited RNF43 binding to hRSPO2 or hRSPO3 (Figure 7A-B). A summary of anti-RSPO results is shown below (Table 5).

### Humanization of anti-RSPO antibodies

The binding affinity of humanized 5D6v1(referred to as hu5D6v1) antibody was compared to chimeric 5D6. Murine vernier positions of hu5D6v1were converted back to human residues to evaluate the contribution of murine vernier positions to binding to hRSPO3. Four additional light chains (L1: v1 + Y36(referred to as v2.1), v1+L46 (referred to as v2.2), v1+T69 (referred to as v2.3), v1+F71 (referred to as v2.4)) and four additional heavy chains (v1+V71 (referred to as v2.8), v1+R94(referred to as v2.10), v1+W47+I48+F78(referred to as v3.2), v1+W47+I48+v67+F78(referred to as v3.3)). F36 and T46 on the light chain were the key mouse vernier residues, and V71 and R94 on the heavy chain were determined to be the key mouse vernier residues based on binding affinity evaluation of the variant antibodies described above (data not shown). Chimeric 5D6 bound with a KD of 3.3E-11 M, while v1+T69 (LC) + (W47+I48+V67+F78 (HC) (referred to as hu5D6v4.1), v1+T69 (LC) + (W47+I48+F78 (HC) (referred to as hu5D6v4.3), bound with a KD of 6.3E-11M, and 7.0E-11M, respectively.

The hu5D6v4.1 and the chimeric 5D6 were tested for their ability to bind cyno and mouse RSPO3 as described above except that cyno or murine RSPO3 replaced huRSPO3 in the binding assay. Binding properties for the humanized antibodies are shown below in Table 5.

| **Antibody** | huKD (M) | huka (1/Ms) | hukd (1/s) | cynoKD (M) | cynoka (1/Ms) | cynokd (1/s) | muKD (M) | muka (1/Ms) | mukd (1/s) |
|---|---|---|---|---|---|---|---|---|---|
| 5D6 chimera | 3.31 E-11 | 3.51 E+06 | 1.16 E-04 | 4.53 E-11 | 4.96 E+06 | 2.24 E-04 | 5.78 E-11 | 4.10 E+06 | 2.37 E-04 |
| Hu5D6v4.1 | 6.35 E-11 | 3.60 E+06 | 2.29E-04 | 8.11 E-11 | 5.35 E+06 | 4.34 E-04 | 9.93 E-11 | 4.26 E+06 | 4.23 E-04 |

The humanized antibodies hu5D6v4.1 was tested under thermal stress (40°C, pH 5.5, 2 weeks) and 2,2'-azobis (2-amidinopropane) hydrochloride (AAPH) Analysis. Then sample was thermally stressed to mimic stability over the shelf life of the product. The sample was buffer exchanged into 20mM His Acetate, 240mM sucrose, pH 5.5 and diluted to a concentration of 1 mg/mL. One mL of sample was stressed at 40C for 2 weeks and a second was stored at -70C as a control. Both samples were then digested using trypsin to create peptides that could be analyzed using liquid chromatography(LC) - mass spectrometry(MS) analysis. For each peptide in the sample retention time, from the LC as well as high resolution accurate mass and peptide ion fragmentation information (amino acid sequence information) were acquired in the MS. Extracted ion chromatograms (XIC) were taken for peptides of interest (native and modified peptide ions) from the data sets at a window of +-10 ppm and peaks were integrated to determine area. Relative percentages of modification were calculated for each sample by taking the (area of the modified peptide) divided by (area of the modified peptide plus the area of the native peptide) multiplied by 100.

As determined by the thermal stress test, hu5D6v4.1 has W^{100b} in CDR-H3, which is susceptible to oxidation (11.5% increase in Tryptophan oxidation. From 24.1% in control to 35.6% after AAPH stress). F100b (referred to as hu5D6v5.1) and W100bH (referred to as hu5D6v5.2) variants were constructed to reduce potential oxidation.

### Epitope Mapping By Crystallography

To further characterize the anti-RSPO antibodies, crystals of RSPO3/Fab Complex (5D6 and 26E11) were prepared as described above and the crystal structure determined. *See* Figure 8. Table 6 contains a list of contacts between the heavy chain (HC) and light chain (LC) of 5D6 and RSPO3 (F chain). The cutoff for Table 6 is 4 angstroms. Table 7 contains a list of contacts between the heavy chain (HC) and light chain (LC) of 26E11 and RSPO3 (F chain). The cutoff for Table 7 is 4 angstroms. Most of the contacts of both 5D6 and 26E11 are with the Furin 1 domain of RSPO3.

**Table 5-Summary of anti-RSPO antibody Results.**

| | Inhibit RSPO2 | | Inhibit L186P | Epitope Bin 26E11 | Epitope | Epitope 21C2 | | | RSPO2 | Inhibit LGR4/ RSPO3 | Inhibit LGR5/ RSPO2 | Inhibit LGR5/ RSPO3 | Inhibit RSPO2 | Inhibit RNF43/ |
|---|---|---|---|---|---|---|---|---|---|---|---|---|---|---|
| 4H1 | + | ++ | + | No | Yes | No | ND | ND | - | +++ | - | ++ | - | +++ |
| 4D4 | - | + | ND | ND | ND | ND | ND | ND | ND | ND | ND | ND | ND | ND |
| 5C2 | - | +++ | - | No | No | Yes | ND | ND | + | +++ | - | +++ | - | ++ |
| 5D6 | - | +++ | - | Yes | No | No | ND | ND | + | +++ | - | +++ | - | +++ |
| 5E11 | - | +++ | - | Yes | No | No | ND | ND | + | +++ | - | +++ | - | +++ |
| 6E9 | + | ++ | - | Yes | No | No | ND | ND | +++ | +++ | + | +++ | - | +++ |
| 21C2 | - | ND | ND | No | No | Yes | ND | ND | - | +++ | - | +++ | ND | ND |
| 26E11 | ++ | +++ | ++ | Yes | No | No | ND | ND | +++ | +++ | ++ | +++ | +++ | +++ |
| 1A1 | +++ | - | +++ | ND | ND | ND | Yes | No | (enhanced binding) | - | (enhanced binding) | - | +++ | - |
| 11F11 | ++ | - | ++ | ND | ND | ND | No | Yes | - | - | - | - | - | - |
| 36D2 | +++ | - | +++ | ND | ND | ND | Yes | No | +++ | - | +++ | - | +++ | - |
| 49G5 | - | - | ND | ND | ND | ND | Yes | No | ++ | - | + | - | + | - |

**Table 6-Contact residues of RSPO3 and 5D6 heavy and light chain**

| Antibody Chain | Residue | Atom name | Atom | RSPO3 | Residue | Atom name | Atom | Distance |
|---|---|---|---|---|---|---|---|---|
| /H/ | 316(GLY) | O | O]: | /F/ | 49(SER) | OG | O]: | 3.94 |
| /H/ | 316(GLY) | CA | C]: | /F/ | 54(CYS) | O | O]: | 3.59 |
| | | | | /F/ | 55(LEU) | CA | C]: | 3.9 |
| /H/ | 314(GLY) | CA | C]: | /F/ | 55(LEU) | O | O]: | 3.51 |
| /H/ | 314(GLY) | C | C]: | /F/ | 55(LEU) | O | O]: | 3.56 |
| /H/ | 315(TYR) | N | N]: | /F/ | 55(LEU) | O | O]: | 3.53 |
| /H/ | 316(GLY) | N | N]: | /F/ | 55(LEU) | O | O]: | 3.03 |
| /H/ | 316(GLY) | CA | C]: | /F/ | 55(LEU) | O | O]: | 3.55 |
| /H/ | 317(GLY) | N | N]: | /F/ | 55(LEU) | CD1 | C]: | 3.34 |
| /H/ | 314(GLY) | N | N]: | /F/ | 55(LEU) | CD1 | C]: | 4 |
| /H/ | 316(GLY) | C | C]: | /F/ | 55(LEU) | CD1 | C]: | 3.92 |
| /H/ | 317(GLY) | CA | C]: | /F/ | 55(LEU) | CD1 | C]: | 3.46 |
| /H/ | 313(TYR) | O | O]: | /F/ | 56(SER) | CB | C]: | 3.62 |
| /H/ | 315(TYR) | CD2 | C]: | /F/ | 63(PHE) | CB | C]: | 3.92 |
| /H/ | 315(TYR) | CB | C]: | /F/ | 63(PHE) | CG | C]: | 3.56 |
| | | | | /F/ | 63(PHE) | CD1 | C]: | 3.41 |
| | | | | /F/ | 63(PHE) | CD2 | C]: | 3.89 |
| | | | | /F/ | 63(PHE) | CE1 | C]: | 3.6 |
| /H/ | 316(GLY) | N | N]: | /F/ | 63(PHE) | CE2 | C]: | 3.59 |
| /H/ | 316(GLY) | CA | C]: | /F/ | 63(PHE) | CE2 | C]: | 3.77 |
| /H/ | 315(TYR) | C | C]: | /F/ | 63(PHE) | CZ | C]: | 3.83 |
| /H/ | 315(TYR) | O | O]: | /F/ | 63(PHE) | CZ | C]: | 3.97 |
| /H/ | 315(TYR) | CB | C]: | /F/ | 63(PHE) | CZ | C]: | 3.92 |
| /H/ | 316(GLY) | N | N]: | /F/ | 63(PHE) | CZ | C]: | 3.71 |
| /H/ | 316(GLY) | CA | C]: | /F/ | 63(PHE) | CZ | C]: | 3.76 |
| /H/ | 264(TYR) | OH | O]: | /F/ | 89(TYR) | CB | C]: | 3.79 |
| /H/ | 315(TYR) | OH | O]: | /F/ | 89(TYR) | CD1 | C]: | 3.8 |
| | | | | /F/ | 89(TYR) | CE1 | C]: | 3.95 |
| /H/ | 264(TYR) | OH | O]: | /F/ | 90(PRO) | N | N]: | 3.5 |
| | | | | /F/ | 90(PRO) | CA | C]: | 3.74 |
| | | | | /F/ | 90(PRO) | C | C]: | 3.58 |
| /H/ | 264(TYR) | CE1 | C]: | /F/ | 90(PRO) | O | O]: | 3.62 |
| /H/ | 264(TYR) | CZ | C]: | /F/ | 90(PRO) | O | O]: | 3.61 |
| /H/ | 264(TYR) | OH | O]: | /F/ | 90(PRO) | O | O]: | 2.72 |
| /H/ | 271(THR) | O | O]: | /F/ | 90(PRO) | CB | C]: | 3.81 |
| /H/ | 264(TYR) | OH | O]: | /F/ | 90(PRO) | CB | C]: | 3.5 |
| /H/ | 271(THR) | O | O]: | /F/ | 90(PRO) | CG | C]: | 3.33 |
| /H/ | 264(TYR) | OH | O]: | /F/ | 90(PRO) | CG | C]: | 3.91 |
| | | | | /F/ | 90(PRO) | CD | C]: | 3.55 |
| /H/ | 247(TYR) | OH | O]: | /F/ | 91(ASP) | C | C]: | 3.66 |
| /H/ | 247(TYR) | CE2 | C]: | /F/ | 91(ASP) | O | O]: | 3.55 |
| /H/ | 247(TYR) | CZ | C]: | /F/ | 91(ASP) | O | O]: | 3.52 |
| /H/ | 247(TYR) | OH | O]: | /F/ | 91(ASP) | O | O]: | 2.62 |
| /H/ | 267(TYR) | CE2 | C]: | /F/ | 91(ASP) | O | O]: | 3.7 |
| /H/ | 267(TYR) | OH | O]: | /F/ | 91(ASP) | O | O]: | 3.7 |
| /H/ | 266(SER) | CB | C]: | /F/ | 91(ASP) | CB | C]: | 3.65 |
| /H/ | 266(SER) | OG | O]: | /F/ | 91(ASP) | CB | C]: | 3.56 |
| /H/ | 267(TYR) | CE2 | C]: | /F/ | 91(ASP) | CB | C]: | 3.74 |
| /H/ | 267(TYR) | CZ | C]: | /F/ | 91(ASP) | CB | C]: | 3.93 |
| /H/ | 267(TYR) | OH | O]: | /F/ | 91(ASP) | CB | C]: | 3.88 |
| /H/ | 268(SER) | OG | O]: | /F/ | 91(ASP) | CG | C]: | 3.45 |
| /H/ | 266(SER) | CB | C]: | /F/ | 91(ASP) | CG | C]: | 4 |
| /H/ | 266(SER) | OG | O]: | /F/ | 91(ASP) | CG | C]: | 3.34 |
| /H/ | 270(LYS) | NZ | N]: | /F/ | 91(ASP) | CG | C]: | 3.97 |
| /H/ | 267(TYR) | CE2 | C]: | /F/ | 91(ASP) | CG | C]: | 3.97 |
| /H/ | 267(TYR) | CZ | C]: | /F/ | 91(ASP) | CG | C]: | 3.71 |
| /H/ | 267(TYR) | OH | O]: | /F/ | 91(ASP) | CG | C]: | 3.68 |
| /H/ | 268(SER) | CB | C]: | /F/ | 91(ASP) | OD1 | O]: | 3.47 |
| /H/ | 268(SER) | OG | O]: | /F/ | 91(ASP) | OD1 | O]: | 2.47 |
| /H/ | 270(LYS) | CG | C]: | /F/ | 91(ASP) | OD1 | O]: | 3.83 |
| /H/ | 266(SER) | CB | C]: | /F/ | 91(ASP) | OD1 | O]: | 3.39 |
| /H/ | 266(SER) | OG | O]: | /F/ | 91(ASP) | OD1 | O]: | 2.4 |
| /H/ | 268(SER) | N | N]: | /F/ | 91(ASP) | OD1 | O]: | 3.78 |
| /H/ | 270(LYS) | CB | C]: | /F/ | 91(ASP) | OD1 | O]: | 3.92 |
| /H/ | 270(LYS) | NZ | N]: | /F/ | 91(ASP) | OD1 | O]: | 3.99 |
| /H/ | 267(TYR) | CZ | C]: | /F/ | 91(ASP) | OD1 | O]: | 3.94 |
| /H/ | 268(SER) | OG | O]: | /F/ | 91(ASP) | OD2 | O]: | 3.71 |
| /H/ | 270(LYS) | NZ | N]: | /F/ | 91(ASP) | OD2 | O]: | 3.35 |
| /H/ | 267(TYR) | CZ | C]: | /F/ | 91(ASP) | OD2 | O]: | 3.96 |
| /H/ | 267(TYR) | OH | O]: | /F/ | 91(ASP) | OD2 | O]: | 3.54 |
| /H/ | 270(LYS) | CD | C]: | /F/ | 91(ASP) | OD2 | O]: | 3.95 |
| /H/ | 247(TYR) | CE2 | C]: | /F/ | 92(ILE) | CB | C]: | 3.92 |
| /H/ | 247(TYR) | CZ | C]: | /F/ | 92(ILE) | CB | C]: | 3.94 |
| /H/ | 247(TYR) | OH | O]: | /F/ | 92(ILE) | CB | C]: | 3.57 |
| | | | | /F/ | 92(ILE) | CG1 | C]: | 3.77 |
| /H/ | 315(TYR) | CE2 | C]: | /F/ | 92(ILE) | CG1 | C]: | 3.59 |
| /H/ | 264(TYR) | OH | O]: | /F/ | 92(ILE) | CG2 | C]: | 3.8 |
| /H/ | 247(TYR) | CZ | C]: | /F/ | 92(ILE) | CD1 | C]: | 3.92 |
| /H/ | 247(TYR) | OH | O]: | /F/ | 92(ILE) | CD1 | C]: | 3.86 |
| /H/ | 312(TYR) | CE1 | C]: | /F/ | 92(ILE) | CD1 | C]: | 3.99 |
| /H/ | 315(TYR) | CD2 | C]: | /F/ | 92(ILE) | CD1 | C]: | 3.78 |
| /H/ | 315(TYR) | CE2 | C]: | /F/ | 92(ILE) | CD1 | C]: | 3.51 |
| /H/ | 315(TYR) | CZ | C]: | /F/ | 94(LYS) | CB | C]: | 3.95 |
| /H/ | 315(TYR) | CD2 | C]: | /F/ | 94(LYS) | CB | C]: | 3.96 |
| /H/ | 315(TYR) | CE2 | C]: | /F/ | 94(LYS) | CB | C]: | 3.56 |
| /H/ | 315(TYR) | OH | O]: | /F/ | 94(LYS) | CG | C]: | 3.98 |
| /H/ | 315(TYR) | CE1 | C]: | /F/ | 94(LYS) | CD | C]: | 3.59 |
| /H/ | 315(TYR) | CZ | C]: | /F/ | 94(LYS) | CD | C]: | 3.33 |
| /H/ | 315(TYR) | OH | O]: | /F/ | 94(LYS) | CD | C]: | 3.28 |
| /H/ | 315(TYR) | CE2 | C]: | /F/ | 94(LYS) | CD | C]: | 3.86 |
| /L/ | 53(ARG) | NH2 | N]: | /F/ | 52(ASN) | O | O]: | 3.58 |
| /L/ | 53(ARG) | NE | N]: | /F/ | 52(ASN) | CB | C]: | 3.98 |
| /L/ | 53(ARG) | CZ | C]: | /F/ | 52(ASN) | CB | C]: | 3.29 |
| /L/ | 53(ARG) | NH1 | N]: | /F/ | 52(ASN) | CB | C]: | 3.24 |
| /L/ | 53(ARG) | NH2 | N]: | /F/ | 52(ASN) | CB | C]: | 3.37 |
| /L/ | 53(ARG) | CD | C]: | /F/ | 52(ASN) | CG | C]: | 3.99 |
| /L/ | 53(ARG) | NE | N]: | /F/ | 52(ASN) | CG | C]: | 3.54 |
| /L/ | 53(ARG) | CZ | C]: | /F/ | 52(ASN) | CG | C]: | 3.39 |
| /L/ | 53(ARG) | NH1 | N]: | /F/ | 52(ASN) | CG | C]: | 3.62 |
| /L/ | 53(ARG) | NH2 | N]: | /F/ | 52(ASN) | CG | C]: | 3.75 |
| /L/ | 53(ARG) | CD | C]: | /F/ | 52(ASN) | ND2 | N]: | 3.41 |
| /L/ | 53(ARG) | NE | N]: | /F/ | 52(ASN) | ND2 | N]: | 3.47 |
| /L/ | 53(ARG) | CZ | C]: | /F/ | 52(ASN) | ND2 | N]: | 3.56 |
| /L/ | 53(ARG) | NH1 | N]: | /F/ | 52(ASN) | ND2 | N]: | 3.53 |
| /L/ | 53(ARG) | NE | N]: | /F/ | 52(ASN) | OD1 | O]: | 3.9 |
| /L/ | 53(ARG) | CZ | C]: | /F/ | 52(ASN) | OD1 | O]: | 3.96 |
| /L/ | 32(TYR) | OH | O]: | /F/ | 63(PHE) | CD1 | C]: | 3.43 |
| /L/ | 32(TYR) | CE1 | C]: | /F/ | 63(PHE) | CE1 | C]: | 3.84 |
| /L/ | 32(TYR) | OH | O]: | /F/ | 63(PHE) | CE1 | C]: | 3.49 |
| | | | | /F/ | 65(LEU) | CG | C]: | 3.61 |
| /L/ | 30(ASP) | O | O]: | /F/ | 65(LEU) | CD1 | C]: | 3.64 |
| /L/ | 32(TYR) | CZ | C]: | /F/ | 65(LEU) | CD1 | C]: | 3.98 |
| /L/ | 32(TYR) | OH | O]: | /F/ | 65(LEU) | CD1 | C]: | 3.94 |
| /L/ | 50(LEU) | CD1 | C]: | /F/ | 65(LEU) | CD2 | C]: | 3.81 |
| /L/ | 53(ARG) | NH2 | N]: | /F/ | 72(GLN) | C | C]: | 3.55 |
| /L/ | 53(ARG) | NE | N]: | /F/ | 72(GLN) | O | O]: | 3.85 |
| /L/ | 53(ARG) | CZ | C]: | /F/ | 72(GLN) | O | O]: | 3.57 |
| /L/ | 53(ARG) | NH2 | N]: | /F/ | 72(GLN) | O | O]: | 2.47 |
| /L/ | 31(SER) | CB | C]: | /F/ | 72(GLN) | NE2 | N]: | 3.62 |
| /L/ | 31(SER) | OG | O]: | /F/ | 72(GLN) | NE2 | N]: | 3.34 |
| /L/ | 53(ARG) | NH2 | N]: | /F/ | 73(ILE) | CA | C]: | 3.66 |
| | | | | /F/ | 74(GLY) | N | N]: | 3.93 |
| /L/ | 30(ASP) | OD2 | O]: | /F/ | 84(TYR) | CE2 | C]: | 3.77 |
| /L/ | 94(PHE) | CD2 | C]: | /F/ | 89(TYR) | CG | C]: | 3.72 |
| | | | | /F/ | 89(TYR) | CD1 | C]: | 3.66 |
| | | | | /F/ | 89(TYR) | CD2 | C]: | 3.85 |
| | | | | /F/ | 89(TYR) | CE1 | C]: | 3.74 |
| /L/ | 94(PHE) | CB | C]: | /F/ | 89(TYR) | CE2 | C]: | 3.89 |
| /L/ | 94(PHE) | CD2 | C]: | /F/ | 89(TYR) | CE2 | C]: | 3.93 |
| /L/ | 94(PHE) | N | N]: | /F/ | 89(TYR) | CZ | C]: | 3.57 |
| /L/ | 94(PHE) | CD2 | C]: | /F/ | 89(TYR) | CZ | C]: | 3.87 |
| /L/ | 93(GLU) | C | C]: | /F/ | 89(TYR) | OH | O]: | 3.74 |
| /L/ | 94(PHE) | N | N]: | /F/ | 89(TYR) | OH | O]: | 3.14 |
| /L/ | 93(GLU) | CA | C]: | /F/ | 89(TYR) | OH | O]: | 3.35 |
| /L/ | 93(GLU) | CB | C]: | /F/ | 89(TYR) | OH | O]: | 3.39 |
| /L/ | 92(ASP) | O | O]: | /F/ | 94(LYS) | CD | C]: | 3.68 |
| | | | | /F/ | 94(LYS) | CE | C]: | 3.55 |
| /L/ | 32(TYR) | CE2 | C]: | /F/ | 94(LYS) | CE | C]: | 3.63 |
| /L/ | 32(TYR) | CZ | C]: | /F/ | 94(LYS) | CE | C]: | 3.66 |
| /L/ | 92(ASP) | OD1 | O]: | /F/ | 94(LYS) | CE | C]: | 3.96 |
| /L/ | 92(ASP) | CA | C]: | /F/ | 94(LYS) | NZ | N]: | 3.95 |
| /L/ | 92(ASP) | C | C]: | /F/ | 94(LYS) | NZ | N]: | 3.76 |
| /L/ | 92(ASP) | O | O]: | /F/ | 94(LYS) | NZ | N]: | 2.89 |
| /L/ | 32(TYR) | CE2 | C]: | /F/ | 94(LYS) | NZ | N]: | 3.74 |
| /L/ | 92(ASP) | CG | C]: | /F/ | 94(LYS) | NZ | N]: | 3.85 |
| /L/ | 92(ASP) | OD1 | O]: | /F/ | 94(LYS) | NZ | N]: | 2.81 |
| /L/ | 28(ASP) | OD2 | O]: | /F/ | 97(LYS) | CD | C]: | 3.7 |
| | | | | /F/ | 97(LYS) | CE | C]: | 3.96 |
| /L/ | 30(ASP) | OD1 | O]: | /F/ | 97(LYS) | CE | C]: | 4 |
| /L/ | 28(ASP) | CG | C]: | /F/ | 97(LYS) | NZ | N]: | 3.82 |
| /L/ | 28(ASP) | OD1 | O]: | /F/ | 97(LYS) | NZ | N]: | 3.85 |
| /L/ | 28(ASP) | OD2 | O]: | /F/ | 97(LYS) | NZ | N]: | 3.11 |
| /L/ | 27(GLN) | NE2 | N]: | /F/ | 108(LYS) | NZ | N]: | 3.9 |

**Table 7-Contact residues of RSPO3 and 26E11 heavy and light chain**

| Antibody Chain | Residue name | Atom name | Atom | RSPO3 | Residue name | Atom name | Atom | Distance |
|---|---|---|---|---|---|---|---|---|
| /H/ | 313(HIS) | CE1 | C | /F/ | 47(THR) | CB | C | 3.72 |
| /H/ | 313(HIS) | NE2 | N | /F/ | 47(THR) | CB | C | 3.73 |
| /H/ | 313(HIS) | CE1 | C | /F/ | 47(THR) | CG2 | C | 3.82 |
| /H/ | 313(HIS) | NE2 | N | /F/ | 47(THR) | CG2 | C | 3.3 |
| /H/ | 313(HIS) | CE1 | C | /F/ | 47(THR) | OG1 | O | 2.92 |
| /H/ | 313(HIS) | NE2 | N | /F/ | 47(THR) | OG1 | O | 3.25 |
| /H/ | 316(GLY) | CA | C | /F/ | 54(CYS) | O | O | 3.93 |
| /H/ | 316(GLY) | CA | C | /F/ | 55(LEU) | CA | C | 3.89 |
| /H/ | 316(GLY) | C | C | /F/ | 55(LEU) | CA | C | 3.86 |
| /H/ | 316(GLY) | N | N | /F/ | 55(LEU) | C | C | 3.92 |
| /H/ | 316(GLY) | CA | C | /F/ | 55(LEU) | C | C | 4 |
| /H/ | 314(GLY) | CA | C | /F/ | 55(LEU) | O | O | 3.72 |
| /H/ | 314(GLY) | C | C | /F/ | 55(LEU) | O | O | 3.63 |
| /H/ | 315(TYR) | N | N | /F/ | 55(LEU) | O | O | 3.66 |
| /H/ | 316(GLY) | N | N | /F/ | 55(LEU) | O | O | 2.92 |
| /H/ | 316(GLY) | CA | C | /F/ | 55(LEU) | O | O | 3.34 |
| /H/ | 316(GLY) | C | C | /F/ | 55(LEU) | O | O | 3.7 |
| /H/ | 317(GLY) | N | N | /F/ | 55(LEU) | O | O | 3.63 |
| /H/ | 313(HIS) | ND1 | N | /F/ | 55(LEU) | CB | C | 3.96 |
| /H/ | 313(HIS) | CE1 | C | /F/ | 55(LEU) | CB | C | 3.45 |
| /H/ | 316(GLY) | C | C | /F/ | 55(LEU) | CD2 | C | 3.96 |
| /H/ | 316(GLY) | O | O | /F/ | 55(LEU) | CD2 | C | 3.29 |
| /H/ | 313(HIS) | CE1 | C | /F/ | 56(SER) | CB | C | 3.92 |
| /H/ | 313(HIS) | CE1 | C | /F/ | 56(SER) | OG | O | 3.74 |
| /H/ | 313(HTS) | NE2 | N | /F/ | 56(SER) | OG | O | 3.86 |
| /H/ | 315(TYR) | CB | C | /F/ | 63(PHE) | CG | C | 3.69 |
| /H/ | 315(TYR) | CB | C | /F/ | 63(PHE) | CD2 | C | 3.48 |
| /H/ | 315(TYR) | O | O | /F/ | 63(PHE) | CE2 | C | 3.86 |
| /H/ | 315(TYR) | CB | C | /F/ | 63(PHE) | CE2 | C | 3.89 |
| /H/ | 316(GLY) | N | N | /F/ | 63(PHE) | CE1 | C | 3.9 |
| /H/ | 316(GLY) | CA | C | /F/ | 63(PHE) | CE1 | C | 3.82 |
| /H/ | 315(TYR) | O | O | /F/ | 63(PHE) | CZ | c | 3.95 |
| /H/ | 315(TYR) | C | C | /F/ | 63(PHE) | CZ | c | 3.98 |
| /H/ | 316(GLY) | N | N | /F/ | 63(PHE) | CZ | c | 3.87 |
| /H/ | 316(GLY) | CA | C | /F/ | 63(PHE) | CZ | c | 3.67 |
| /H/ | 264(TYR) | OH | O | /F/ | 89(TYR) | CB | C | 3.67 |
| /H/ | 315(TYR) | OH | O | /F/ | 89(TYR) | CD2 | C | 3.53 |
| /H/ | 315(TYR) | OH | O | /F/ | 89(TYR) | CE2 | C | 3.65 |
| /H/ | 264(TYR) | OH | O | /F/ | 90(PRO) | N | N | 3.57 |
| /H/ | 264(TYR) | OH | O | /F/ | 90(PRO) | CA | C | 3.96 |
| /H/ | 264(TYR) | OH | O | /F/ | 90(PRO) | C | C | 3.76 |
| /H/ | 264(TYR) | CE1 | C | /F/ | 90(PRO) | O | O | 3.73 |
| /H/ | 264(TYR) | CZ | c | /F/ | 90(PRO) | O | O | 3.74 |
| /H/ | 264(TYR) | OH | O | /F/ | 90(PRO) | O | O | 2.86 |
| /H/ | 264(TYR) | OH | O | /F/ | 90(PRO) | CB | C | 3.87 |
| /H/ | 271(THR) | O | O | /F/ | 90(PRO) | CG | C | 3.48 |
| /H/ | 264(TYR) | OH | O | /F/ | 90(PRO) | CD | C | 3.51 |
| /H/ | 247(TYR) | CE2 | C | /F/ | 91(ASP) | C | c | 3.95 |
| /H/ | 247(TYR) | OH | O | /F/ | 91(ASP) | C | c | 3.46 |
| /H/ | 247(TYR) | CE2 | C | /F/ | 91(ASP) | O | O | 3.35 |
| /H/ | 247(TYR) | CZ | C | /F/ | 91(ASP) | O | O | 3.28 |
| /H/ | 247(TYR) | OH | O | /F/ | 91(ASP) | O | O | 2.37 |
| /H/ | 267(PHE) | CE2 | C | /F/ | 91(ASP) | O | O | 3.8 |
| /H/ | 266(SER) | CB | C | /F/ | 91(ASP) | CB | C | 3.71 |
| /H/ | 266(SER) | OG | O | /F/ | 91(ASP) | CB | C | 3.73 |
| /H/ | 267(PHE) | CE2 | C | /F/ | 91(ASP) | CB | C | 3.83 |
| /H/ | 267(PHE) | CZ | C | /F/ | 91(ASP) | CB | C | 3.81 |
| /H/ | 266(SER) | CB | C | /F/ | 91(ASP) | CG | C | 3.91 |
| /H/ | 266(SER) | OG | O | /F/ | 91(ASP) | CG | C | 3.36 |
| /H/ | 270(LYS) | CD | C | /F/ | 91(ASP) | CG | C | 3.75 |
| /H/ | 268(SER) | OG | O | /F/ | 91(ASP) | CG | C | 3.4 |
| /H/ | 267(PHE) | CZ | C | /F/ | 91(ASP) | CG | C | 3.84 |
| /H/ | 266(SER) | CB | C | /F/ | 91(ASP) | OD2 | O | 3.35 |
| /H/ | 266(SER) | OG | O | /F/ | 91(ASP) | OD2 | O | 2.48 |
| /H/ | 270(LYS) | CB | C | /F/ | 91(ASP) | OD2 | O | 3.25 |
| /H/ | 270(LYS) | CG | C | /F/ | 91(ASP) | OD2 | O | 3.29 |
| /H/ | 270(L YS) | CD | C | /F/ | 91(ASP) | OD2 | O | 3.16 |
| /H/ | 268(SER) | OG | O | /F/ | 91(ASP) | OD2 | O | 2.77 |
| /H/ | 270(LYS) | NZ | N | /F/ | 91(ASP) | OD2 | O | 3.91 |
| /H/ | 270(LYS) | CD | C | /F/ | 91(ASP) | OD1 | O | 3.97 |
| /H/ | 267(PHE) | CE1 | C | /F/ | 91(ASP) | OD1 | O | 3.83 |
| /H/ | 268(SER) | OG | O | /F/ | 91(ASP) | OD1 | O | 3.41 |
| /H/ | 270(LYS) | NZ | N | /F/ | 91(ASP) | OD1 | O | 3.97 |
| /H/ | 267(PHE) | CZ | C | /F/ | 91(ASP) | OD1 | O | 3.61 |
| /H/ | 247(TYR) | OH | O | /F/ | 92(ILE) | CA | C | 3.82 |
| /H/ | 247(TYR) | CE2 | C | /F/ | 92(ILE) | CB | C | 3.92 |
| /H/ | 247(TYR) | CZ | C | /F/ | 92(ILE) | CB | C | 3.72 |
| /H/ | 247(TYR) | OH | O | /F/ | 92(ILE) | CB | C | 3.26 |
| /H/ | 315(TYR) | CD2 | C | /F/ | 92(ILE) | CG1 | C | 3.8 |
| /H/ | 315(TYR) | CE2 | C | /F/ | 92(ILE) | CG1 | C | 3.61 |
| /H/ | 247(TYR) | OH | O | /F/ | 92(ILE) | CG1 | C | 3.48 |
| /H/ | 264(TYR) | OH | O | /F/ | 92(ILE) | CG2 | C | 3.72 |
| /H/ | 315(TYR) | CD2 | C | /F/ | 92(ILE) | CD1 | C | 3.65 |
| /H/ | 315(TYR) | CE2 | C | /F/ | 92(ILE) | CD1 | C | 3.62 |
| /H/ | 247(TYR) | CE1 | C | /F/ | 92(ILE) | CD1 | C | 3.88 |
| /H/ | 247(TYR) | CZ | C | /F/ | 92(ILE) | CD1 | C | 3.81 |
| /H/ | 247(TYR) | OH | O | /F/ | 92(ILE) | CD1 | C | 3.73 |
| /H/ | 315(TYR) | CD2 | C | /F/ | 93(ASN) | O | O | 3.79 |
| /H/ | 315(TYR) | CE2 | C | /F/ | 93(ASN) | O | O | 3.86 |
| /H/ | 315(TYR) | CD2 | C | /F/ | 94(LYS) | CB | C | 3.57 |
| /H/ | 315(TYR) | CE2 | C | /F/ | 94(LYS) | CB | C | 3.46 |
| /H/ | 315(TYR) | CE2 | C | /F/ | 94(LYS) | CG | C | 3.73 |
| /H/ | 315(TYR) | CZ | C | /F/ | 94(LYS) | CG | C | 3.84 |
| /H/ | 315(TYR) | CD1 | C | /F/ | 94(LYS) | CD | C | 3.83 |
| /H/ | 315(TYR) | CE1 | C | /F/ | 94(LYS) | CD | C | 3.45 |
| /H/ | 315(TYR) | CD2 | C | /F/ | 94(LYS) | CD | C | 3.99 |
| /H/ | 315(TYR) | CE2 | C | /F/ | 94(LYS) | CD | C | 3.62 |
| /H/ | 315(TYR) | CZ | C | /F/ | 94(LYS) | CD | C | 3.33 |
| /H/ | 315(TYR) | OH | O | /F/ | 94(LYS) | CD | C | 3.73 |
| /L/ | 53(ARG) | NH2 | N | /F/ | 52(ASN) | O | O | 3.44 |
| /L/ | 53(ARG) | CZ | C | /F/ | 52(ASN) | CB | C | 3.48 |
| /L/ | 53(ARG) | NH1 | N | /F/ | 52(ASN) | CB | C | 3.31 |
| /L/ | 53(ARG) | NH2 | N | /F/ | 52(ASN) | CB | C | 3.72 |
| /L/ | 53(ARG) | CD | C | /F/ | 52(ASN) | CG | C | 3.8 |
| /L/ | 53(ARG) | CZ | C | /F/ | 52(ASN) | CG | C | 3.44 |
| /L/ | 53(ARG) | NH1 | N | /F/ | 52(ASN) | CG | C | 3.54 |
| /L/ | 53(ARG) | NH2 | N | /F/ | 52(ASN) | CG | C | 3.96 |
| /L/ | 53(ARG) | NE | N | /F/ | 52(ASN) | CG | C | 3.53 |
| /L/ | 53(ARG) | CD | C | /F/ | 52(ASN) | ND2 | N | 3.82 |
| /L/ | 53(ARG) | NH1 | N | /F/ | 52(ASN) | ND2 | N | 3.99 |
| /L/ | 53(ARG) | CD | C | /F/ | 52(ASN) | OD1 | O | 3.72 |
| /L/ | 53(ARG) | CZ | C | /F/ | 52(ASN) | OD1 | O | 3.42 |
| /L/ | 53(ARG) | NH1 | N | /F/ | 52(ASN) | OD1 | O | 3.96 |
| /L/ | 53(ARG) | NH2 | N | /F/ | 52(ASN) | OD1 | O | 3.76 |
| /L/ | 53(ARG) | NE | N | /F/ | 52(ASN) | OD1 | O | 3.25 |
| /L/ | 32(TYR) | OH | O | /F/ | 63(PHE) | CD2 | C | 3.3 |
| /L/ | 32(TYR) | CE1 | C | /F/ | 63(PHE) | CE2 | C | 3.9 |
| /L/ | 32(TYR) | OH | O | /F/ | 63(PHE) | CE2 | C | 3.47 |
| /L/ | 32(TYR) | OH | O | /F/ | 65(LEU) | CG | C | 3.91 |
| /L/ | 30(ASP) | O | O | /F/ | 65(LEU) | CD1 | C | 3.62 |
| /L/ | 53(ARG) | NH2 | N | /F/ | 72(GLN) | O | O | 2.93 |
| /L/ | 31(SER) | OG | O | /F/ | 72(GLN) | CD | C | 3.94 |
| /L/ | 31(SER) | OG | O | /F/ | 72(GLN) | NE2 | N | 3.81 |
| /L/ | 31(SER) | CB | C | /F/ | 72(GLN) | OE1 | O | 3.48 |
| /L/ | 31(SER) | OG | O | /F/ | 72(GLN) | OE1 | O | 3.24 |
| /L/ | 30(ASP) | OD2 | O | /F/ | 84(TYR) | CE2 | C | 3.77 |
| /L/ | 94(PHE) | CD2 | C | /F/ | 89(TYR) | CG | C | 3.57 |
| /L/ | 94(PHE) | CE2 | C | /F/ | 89(TYR) | CG | C | 4 |
| /L/ | 94(PHE) | CD2 | C | /F/ | 89(TYR) | CD1 | C | 3.69 |
| /L/ | 94(PHE) | CE2 | C | /F/ | 89(TYR) | CD2 | C | 3.49 |
| /L/ | 94(PHE) | CE2 | C | /F/ | 89(TYR) | CD2 | C | 3.83 |
| /L/ | 94(PHE) | CB | C | /F/ | 89(TYR) | CE1 | C | 3.79 |
| /L/ | 94(PHE) | CD2 | C | /F/ | 89(TYR) | CE1 | C | 3.74 |
| /L/ | 94(PHE) | CD2 | C | /F/ | 89(TYR) | CE2 | C | 3.54 |
| /L/ | 94(PHE) | CB | C | /F/ | 89(TYR) | CZ | C | 3.96 |
| /L/ | 94(PHE) | N | N | /F/ | 89(TYR) | CZ | C | 3.59 |
| /L/ | 94(PHE) | CD2 | C | /F/ | 89(TYR) | CZ | C | 3.66 |
| /L/ | 94(PHE) | O | O | /F/ | 89(TYR) | OH | O | 3.79 |
| /L/ | 93(GLU) | CA | C | /F/ | 89(TYR) | OH | O | 3.67 |
| /L/ | 93(GLU) | C | C | /F/ | 89(TYR) | OH | O | 3.85 |
| /L/ | 94(PHE) | N | N | /F/ | 89(TYR) | OH | O | 3.05 |
| /L/ | 93(GLU) | CB | C | /F/ | 89(TYR) | OH | O | 3.56 |
| /L/ | 92(ASP) | O | O | /F/ | 94(LYS) | CD | C | 3.91 |
| /L/ | 92(ASP) | O | O | /F/ | 94(LYS) | CE C | | 3.31 |
| /L/ | 32(TYR) | CD2 | C | /F/ | 94(LYS) | CE | C | 3.85 |
| /L/ | 32(TYR) | CE2 | C | /F/ | 94(LYS) | CE | C | 3.35 |
| /L/ | 32(TYR) | CZ | C | /F/ | 94(LYS) | CE | C | 3.57 |
| /L/ | 32(TYR) | OH | O | /F/ | 94(LYS) | CE | C | 3.85 |
| /L/ | 92(ASP) | OD1 | O | /F/ | 94(LYS) | CE | C | 3.54 |
| /L/ | 92(ASP) | C | C | /F/ | 94(LYS) | NZ | N | 3.72 |
| /L/ | 92(ASP) | O | O | /F/ | 94(LYS) | NZ | N | 2.61 |
| /L/ | 92(ASP) | OD1 | O | /F/ | 94(LYS) | NZ | N | 3.14 |
| /L/ | 30(ASP) | CG | C | /F/ | 97(LYS) | CG | C | 3.96 |
| /L/ | 30(ASP) | OD2 | O | /F/ | 97(LYS) | CG | C | 3.81 |
| /L/ | 28(ASP) | CG | C | /F/ | 97(LYS) | CD | C | 3.72 |
| /L/ | 28(ASP) | OD1 | O | /F/ | 97(LYS) | CD | C | 3.81 |
| /L/ | 28(ASP) | OD2 | O | /F/ | 97(LYS) | CD | C | 3.53 |
| /L/ | 28(ASP) | OD2 | O | /F/ | 97(LYS) | CE | C | 3.96 |
| /L/ | 30(ASP) | CG | C | /F/ | 97(LYS) | CE | C | 3.71 |
| /L/ | 30(ASP) | OD1 | O | /F/ | 97(LYS) | CE | C | 3.47 |
| /L/ | 28(ASP) | CG | C | /F/ | 97(LYS) | NZ | N | 3.74 |
| /L/ | 28(ASP) | OD1 | O | /F/ | 97(LYS) | NZ | N | 3.59 |
| /L/ | 28(ASP) | OD2 | O | /F/ | 97(LYS) | NZ | N | 3.2 |
| /L/ | 27(GLN) | NE2 | N | /F/ | 108(LYS) | CD | C | 4 |

### In Vivo Efficacy

Efficacy of anti-RSPO3 antibodies were tested in colorectal cancer PTPRK-RSPO fusion patient derived tumor models. In PTPRK-RSPO fusion patient derived tumor models and/or NSCLC tissue, anti-RSPO3 antibody (5D6) significantly reduced gene expression of markers of intestinal stem cell markers: Myc, Axin2, LGR5, TERT, BIRC5, and/or Ascl2, whereas gene expression of markers of differentiation were increased, e.g., CEACAM7, SLC26A3, CA1, SYTl5, CA4, TFF1, and KRT20 compared to expression levels prior to treatment with the anti-RSPO3 antibody (data not shown). While not wanting to be bound by any particular theory, these results suggest that the anti-RSPO3 antibody (5D6) is capable of promoting a transition, as determined by gene expression markers, from a stem cell-like marker profile to a differentiation marker profile.

The effect on tumor volume over time (e.g., tumor growth inhibition) was also tested in the colorectal cancer PTPRK-RSPO fusion patient derived tumor models upon treatment with the anti-RSPO3 antibody (5D6) is shown in Figures 11A-D. Treatment of the models with anti-RSPO3 antibody (5D6) showed significant reduction in tumor growth or stasis of tumor growth. In the models, the onset of regression and/or stasis was not immediate upon treatment with the anti-RSPO3 antibody (5D6); there was a delay in the onset of regression or stasis after initiation of treatment. Further, when the colorectal cancer patient derived model tumors treated with anti-Ragweed antibody or anti-RSPO3 antibody (5D6) were stained with H&E stain and Alcian Blue stain, there was a striking difference in histopathology as shown in Figure 12A-D. In anti-RSPO3 (5D6)-treated tumors, there was a significant reduction in the number of tumor cells. The histology of most of the remaining cells was consistent with differentiated, mature non-proliferating goblet cells. In addition, there was a significant increase in mucous as indicated by Alcian Blue staining compared to the anti-Ragweed antibody control. Accordingly, the measured tumor volume may actually have been occupied in significant party by mucous, and not by tumor cells, and therefore, the effect on tumor growth inhibition may actually have been underestimated. While not wanting to be bound by any particular theory, these efficacy data are consistent with a hierarchical organization of RSPO3 fusion positive tumors: the proliferation of the cancer stem cells is dependent upon RSPO proteins, and upon treatment with anti-RSPO3 antibody (5D6), the cancer stem cells die or differentiate into transit-amplifying (TA) cell. In the absence of a stem cell source to ensure their replenishment, the latter undergo a limited number of cell divisions, after which they terminally differentiate, leading to their exhaustion. Therefore, the kinetics and the overall size of the TA cell population may determine the onset of tumor growth inhibition.

Again while not wanting to be bound by any particular theory, based on the theory of hierarchical organization of RSPO3 fusion positive tumors described, combination treatment with a chemotherapeutic agent should reduce the delay in onset of regression and/or stasis by killing the TA cell population and increase efficacy compared to treatment with the chemotherapeutic agent alone in the PTPRK-RSPO fusion patient derived tumor models. Consistent with this theory and as shown in Figures 11D and 13A, the anti-RSPO3 antibody (5D6) in combination with Irinotecan significantly reduced the delay in onset of regression and/or stasis and a decreased tumor growth when compared to treatment with irinotecan alone in CRCD and CRCC colorectal cancer PTPRK-RSPO fusion patient derived tumor models. By administering an anti-RSPO3 antibody in combination with chemotherapy, both cancer stem cells and TA cells are targeted for earlier regression or stasis of tumor growth.

Further, while not wanting to be bound by any particular theory, based on the theory of hierarchical organization of RSPO3 fusion positive tumors described above as well as the idea that the stem cell compartment is responsible to tumor initiation as measured by a tumor transplantation assay, transplanted PTPRK-RSPO fusion patient derived tumor models treated with an anti-RSPO3 antibody should have a reduced cancer stem cell population, which should reduce the establishment and tumor growth of serial PTPRK-RSPO fusion patient derived tumors. Again, consistently with this theory and as shown Figure 13B-C, in serial transplant experiments, treatment with anti-RSPO3 antibodies (5D6) results in fewer tumors being established and growing from anti-RSPO3 treated fragments following serial transplantation.

Although the foregoing invention has been described in some detail by way of illustration and example for purposes of clarity of understanding, the descriptions and examples should not be construed as limiting the scope of the invention.

| NAME | SEQUENCE | SEQ ID NO |
|---|---|---|
| 4H1-HVR L1 | RSSQSIVHSNGNTYLE | 5 |
| 4H1-HVR L2 | RISNRFS | 6 |
| 4H1-HVR L3 | FQGSHVPYT | 7 |
| 4H1-HVR H1 | NFAMS | 8 |
| 4H1-HVR H2 | EINNGGNYAYYQDTVTG | 9 |
| 4H1-HVR H3 | EDYVNYEAYFAY | 10 |
| 4D4-HVR L1 | RSSQSIVHSNGNTYLE | 11 |
| 4D4-HVR L2 | RISNRFS | 12 |
| 4D4-HVR L3 | FQGSHVPYT | 13 |
| 4D4-HVR H1 | NFAMS | 14 |
| 4D4-HVR H2 | EINNGGNYAYYQDTVTG | 15 |
| 4D4-HVR H3 | EDYVNYEAYFAY | 16 |
| 5C2-HVR L1 | RASQDISNYLN | 17 |
| 5C2-HVR L2 | YTSRLHS | 18 |
| 5C2-HVR L3 | QQGDTLPPT | 19 |
| 5C2-HVR H1 | SYGVH | 20 |
| 5C2-HVR H2 | VIWTGGSTNYNSALMS | 21 |
| 5C2-HVR H3 | VDGYYYFDY | 22 |
| 5D6-HVR L1 | KASQDIDSYLS | 23 |
| 5D6-HVR L2 | LTNRLVD | 24 |
| 5D6-HVR L3 | LHYDEFPLT | 25 |
| 5D6-HVR H1 | SGYWN | 26 |
| 5D6-HVR H2 | YISYSGKTYQNPSLKS | 27 |
| 5D6-HVR H3 | YYGYGGPWFAY | 28 |
| 5E11-HVR L1 | RASQDISNYLN | 29 |
| 5E11-HVR L2 | YTSRLHS | 30 |
| 5E11-HVR L3 | QHGDTLPPT | 31 |
| 5E11-HVR H1 | SYAVH | 32 |
| 5E11-HVR H2 | VIWSGGSTDYNAAFIS | 33 |
| 5E11-HVR H3 | NDGYYYFDY | 34 |
| 6E9-HVR L1 | RASQDISNYLN | 35 |
| 6E9-HVR L2 | YTSRLHS | 36 |
| 6E9-HVR L3 | QQGDTLPPA | 37 |
| 6E9-HVR H1 | SYGVH | 38 |
| 6E9-HVR H2 | VIWSGGSTDYNAAFIS | 39 |
| 6E9-HVR H3 | NDGYYYFDY | 40 |
| 21C2-HVR L1 | RASESVDSYGNTFMH | 41 |
| 21C2-HVR L2 | LASNLES | 42 |
| 21C2-HVR L3 | QQNNEDPYT | 43 |
| 21C2-HVR H1 | DYVIH | 44 |
| 21C2-HVR H2 | VITTYYGDASYNQKFKG | 45 |
| 21C2-HVR H3 | GAYGNSPSYWYFDV | 46 |
| 26E11-HVR L1 | KASQDIDSYLS | 47 |
| 26E11-HVR L2 | LTNRLID | 48 |
| 26E11-HVR L3 | LQYDEFPVT | 49 |
| 26E11-HVR H1 | SGYWS | 50 |
| 26E11-HVR H2 | YISFSGKTYYIPSLKS | 51 |
| 26E11-HVR H3 | YHGYGGPWFAY | 52 |
| 1A1-HVR L1 | TLSSQHSTNYIE | 53 |
| 1A1-HVR L2 | VRDGSHSKGD | 54 |
| 1A1-HVR L3 | GLSDVSLYL | 55 |
| 1A1-HVR H1 | DYFMS | 56 |
| 1A1-HVR H2 | HIYTKTYNYATYYSGSVKG | 57 |
| 1A1-HVR H3 | DEDWYFDF | 58 |
| 11F11-HVR L1 | TLSSQHSSYGIT | 59 |
| 11F11-HVR L2 | LRSDGSHSKGD | 60 |
| 11F11-HVR L3 | VTYDSTVGV | 61 |
| 11F11-HVR H1 | EYYVT | 62 |
| 11F11-HVR H2 | DIDPENGDTDYNQKFQG | 63 |
| 11F11-HVR H3 | GYDYAFDS | 64 |
| 36D2-HVR L1 | TRSSGNIGSNYVS | 65 |
| 36D2-HVR L2 | KFDQRPS | 66 |
| 36D2-HVR L3 | LSGYDKYV | 67 |
| 36D2-HVR H1 | SSDWS | 68 |
| 36D2-HVR H2 | YMNYGGGTYYNPSLEN | 69 |
| 36D2-HVR H3 | ERPHPYAYFDV | 70 |
| 49G5-HVR L1 | TLSSQYNTYYIE | 71 |
| 49G5-HVR L2 | LSDGSHSKGD | 72 |
| 49G5-HVR L3 | GVSDVSLYV | 73 |
| 49G5-HVR H1 | SYNIH | 74 |
| 49G5-HVR H2 | AVWRGGGTYYNSNLKS | 75 |
| 49G5-HVR H3 | EELRYVYFDV | 76 |
| COMP1-HVR L1 | KASQDIDSYLS | 77 |
| COMP1-HVR L2 | LTNRLX₁D wherein X₁ is V or I | 78 |
| COMP1-HVR L3 | LX₁YDEFPX₂T wherein X₁ is H or Q and X₂ is L or V | 79 |
| COMP1-HVR H1 | SGYWX₁ wherein X₁ is N or S | 80 |
| COMP1-HVR H2 | YISX₁SGKTYX₂X₃PSLKS wherein X₁ is Y or F, X₂ is Q or Y, and X₃ is N or I | 81 |
| COMF1-HVR H3 | YX₁GYGGPWFAY wherein X₁ is Y or H | 82 |
| COMP2-HVR L1 | RASQDISNYLN | 83 |
| COMP2-HVR L2 | YTSRLHS | 84 |
| COMP2-HVR L3 | QX₁GDTLPPX₂ wherein X₁ is Q or H and X₂ is T or A | 85 |
| COMP2-HVR H1 | SYX₁VH wherein X₁ is A or G | 86 |
| COMP2-HVR H2 | VIWX₁GGSTX₂YNX₃AX₄X₅S wherein X₁ is S or T, X₂ is D or N, X₃ is A or S, X₄ is L or F, X₅ is M or I | 87 |
| COMP2-HVR H3 | X₁DGYYYFDY wherein X₁ is N or V | 88 |
| 4H1 V_{L} | | 89 |
| 4H1 V_{H} | | 90 |
| 4D4 V_{L} | | 91 |
| 4D4 V_{H} | | 92 |
| 5C2 V_{L} | | 93 |
| 5C2 V_{H} | | 94 |
| 5D6 V_{L} | | 95 |
| 5D6 V_{H} | | 96 |
| 5E11 V_{L} | | 97 |
| 5E11 V_{H} | | 98 |
| 6E9 V_{L} | | 99 |
| 6E9 V_{H} | | 100 |
| 21C2 V_{L} | | 101 |
| 21C2 V_{H} | | 102 |
| 26E11 V_{L} | | 103 |
| 26E11 V_{H} | | 104 |
| 1A1 V_{L} | | 105 |
| | | |
| 1A1 V_{H} | | 106 |
| 11F11 V_{L} | | 107 |
| 11F11 V_{H} | | 108 |
| 36D2 V_{L} | | 109 |
| 36D2 V_{H} | | 110 |
| 49G5 V_{L} | | 111 |
| 49G5 V_{H} | | 112 |
| 5D6v5.1-HVR-H3 | YYGYGGPFFAY | 188 |
| 5D6v5.2-HVR-H3 | YYGYGGPHFAY | 189 |
| 5D6v1 V_{L} | | 190 |
| 5D6v1 V_{H} | | 191 |
| 5D6v2.1 V_{L} | | 192 |
| 5D6v2.1 V_{H} | | 193 |
| 5D6v2.2 V_{L} | | 194 |
| 5D6v2.2 V_{H} | | 195 |
| 5D6v2.3 V_{L} | | 196 |
| 5D6v2.3 V_{H} | | 197 |
| 5D6v2.4 V_{L} | | 198 |
| 5D6v2.4 V_{H} | | 199 |
| 5D6v2.8 V_{L} | | 200 |
| 5D6v2.8 V_{H} | | 201 |
| | | |
| 5D6v2.10 V_{L} | | 202 |
| 5D6v2.10 V_{H} | | 203 |
| 5D6v3.2 V_{L} | | 204 |
| 5D6v3.2 V_{H} | | 205 |
| 5D6v3.3 V_{L} | | 206 |
| 5D6v3.3 V_{H} | | 207 |
| 5D6v4.1 V_{L} | | 208 |
| 5D6v4.1 V_{H} | | 209 |
| 5D6v4.3 V_{L} | | 210 |
| 5D6v4.3 V_{H} | | 211 |
| 5D6v5.1 V_{L} | | 212 |
| 5D6v5.1 V_{H} | | 213 |
| 5D6v5.2 V_{L} | | 214 |
| 5D6v5.2 V_{H} | | 215 |
| COMP1-HVR H3 | YX₁GYGGPX₂FAY wherein X₁ is Y or H and X₂ is W, F, or H | 216 |

EIF3E(e1)-RSPO2(e2) translocation fusion polynucleotide (SEQ ID NO:173)
EIF3E(e1)-RSPO2(e2) translocation fusion polypeptide sequence (SEQ ID NO:174)
PTPRK(e1)-RSPO3(e2) translocation fusion polynucleotide sequence (SEQ ID NO:175)
PTPRK(e1)-RSPO3(e2) translocation fusion polypeptide sequence (SEQ ID NO:176)
PTPRK(e7)-RSPO3(e2) translocation fusion polynucleotide sequence (SEQ ID NO:177)
PTPRK(e7)-RSPO3(e2) translocation fusion polypeptide sequence (SEQ ID NO:178)

## Claims

1. An isolated antibody that binds to RSPO3, wherein the antibody comprises:
(a) VL comprising (i) HVR-L1 comprising the amino acid sequence of SEQ ID NO:5, (ii) HVR-L2 comprising the amino acid sequence of SEQ ID NO:6, and (iii) HVR-L3 comprising the amino acid sequence of SEQ ID NO:7; and a VH comprising (i) HVR-H1 comprising the amino acid sequence of SEQ ID NO:8, (ii) HVR-H2 comprising the amino acid sequence of SEQ ID NO:9, and (iii) HVR-H3 comprising the amino acid sequence of SEQ ID NO:10;
(b) a VL comprising (i) HVR-L1 comprising the amino acid sequence of SEQ ID NO:11, (ii) HVR-L2 comprising the amino acid sequence of SEQ ID NO:12, and (iii) HVR-L3 comprising the amino acid sequence of SEQ ID NO:13; and a VH comprising (i) HVR-H1 comprising the amino acid sequence of SEQ ID NO:14, (ii) HVR-H2 comprising the amino acid sequence of SEQ ID NO:15, and (iii) HVR-H3 comprising the amino acid sequence of SEQ ID NO:16;
(c) a VL comprising (i) HVR-L1 comprising the amino acid sequence of SEQ ID NO:17, (ii) HVR-L2 comprising the amino acid sequence of SEQ ID NO:18, and (iii) HVR-L3 comprising the amino acid sequence of SEQ ID NO:19; and a VH comprising (i) HVR-H1 comprising the amino acid sequence of SEQ ID NO:20, (ii) HVR-H2 comprising the amino acid sequence of SEQ ID NO:21, and (iii) HVR-H3 comprising the amino acid sequence of SEQ ID NO:22;
(d) a VL comprising (i) HVR-L1 comprising the amino acid sequence of SEQ ID NO:23, (ii) HVR-L2 comprising the amino acid sequence of SEQ ID NO:24, and (iii) HVR-L3 comprising the amino acid sequence of SEQ ID NO:25; and a VH comprising (i) HVR-H1 comprising the amino acid sequence of SEQ ID NO:26, (ii) HVR-H2 comprising the amino acid sequence of SEQ ID NO:27, and (iii) HVR-H3 comprising the amino acid sequence of SEQ ID NO:28;
(e) a VL comprising (i) HVR-L1 comprising the amino acid sequence of SEQ ID NO:29, (ii) HVR-L2 comprising the amino acid sequence of SEQ ID NO:30, and (iii) HVR-L3 comprising the amino acid sequence of SEQ ID NO:31; and a VH comprising (i) HVR-H1 comprising the amino acid sequence of SEQ ID NO:32, (ii) HVR-H2 comprising the amino acid sequence of SEQ ID NO:33, and (iii) HVR-H3 comprising the amino acid sequence of SEQ ID NO:34;
(f) a VL comprising (i) HVR-L1 comprising the amino acid sequence of SEQ ID NO:35, (ii) HVR-L2 comprising the amino acid sequence of SEQ ID NO:36, and (iii) HVR-L3 comprising the amino acid sequence of SEQ ID NO:37; and a VH comprising (i) HVR-H1 comprising the amino acid sequence of SEQ ID NO:38, (ii) HVR-H2 comprising the amino acid sequence of SEQ ID NO:39, and (iii) HVR-H3 comprising the amino acid sequence of SEQ ID NO:40;
(g) a VL comprising (i) HVR-L1 comprising the amino acid sequence of SEQ ID NO:41, (ii) HVR-L2 comprising the amino acid sequence of SEQ ID NO:42, and (iii) HVR-L3 comprising the amino acid sequence of SEQ ID NO:43; and a VH comprising (i) HVR-H1 comprising the amino acid sequence of SEQ ID NO:44, (ii) HVR-H2 comprising the amino acid sequence of SEQ ID NO:45, and (iii) HVR-H3 comprising the amino acid sequence of SEQ ID NO:46
(h) a VL comprising (i) HVR-L1 comprising the amino acid sequence of SEQ ID NO:23, (ii) HVR-L2 comprising the amino acid sequence of SEQ ID NO:24, and (iii) HVR-L3 comprising the amino acid sequence of SEQ ID NO:25; and a VH comprising (i) HVR-H1 comprising the amino acid sequence of SEQ ID NO:26, (ii) HVR-H2 comprising the amino acid sequence of SEQ ID NO:27, and (iii) HVR-H3 comprising the amino acid sequence of SEQ ID NO:188;
(i) a VL comprising (i) HVR-L1 comprising the amino acid sequence of SEQ ID NO:23, (ii) HVR-L2 comprising the amino acid sequence of SEQ ID NO:24, and (iii) HVR-L3 comprising the amino acid sequence of SEQ ID NO:25; and a VH comprising (i) HVR-H1 comprising the amino acid sequence of SEQ ID NO:26, (ii) HVR-H2 comprising the amino acid sequence of SEQ ID NO:27, and (iii) HVR-H3 comprising the amino acid sequence of SEQ ID NO:189;
(j) a VL comprising (i) HVR-L1 comprising the amino acid sequence of SEQ ID NO:77, (ii) HVR-L2 comprising the amino acid sequence of SEQ ID NO:78, and (iii) HVR-L3 comprising the amino acid sequence of SEQ ID NO:79; and a VH comprising (i) HVR-H1 comprising the amino acid sequence of SEQ ID NO:80, (ii) HVR-H2 comprising the amino acid sequence of SEQ ID NO:81, and (iii) HVR-H3 comprising the amino acid sequence of SEQ ID NO:82;
(k) a VL comprising (i) HVR-L1 comprising the amino acid sequence of SEQ ID NO:83, (ii) HVR-L2 comprising the amino acid sequence of SEQ ID NO:84, and (iii) HVR-L3 comprising the amino acid sequence of SEQ ID NO:85; and a VH comprising (i) HVR-H1 comprising the amino acid sequence of SEQ ID NO:86, (ii) HVR-H2 comprising the amino acid sequence of SEQ ID NO:87, and (iii) HVR-H3 comprising the amino acid sequence of SEQ ID NO:88; or
(l) a VL comprising (i) HVR-L1 comprising the amino acid sequence of SEQ ID NO:77, (ii) HVR-L2 comprising the amino acid sequence of SEQ ID NO:78, and (iii) HVR-L3 comprising the amino acid sequence of SEQ ID NO:79; and a VH comprising (i) HVR-H1 comprising the amino acid sequence of SEQ ID NO:80, (ii) HVR-H2 comprising the amino acid sequence of SEQ ID NO:81, and (iii) HVR-H3 comprising the amino acid sequence of SEQ ID NO:216.

2. The isolated antibody of claim 1, wherein the antibody comprises
(a) a VL sequence of SEQ ID NO:89 and a VH sequence of SEQ ID NO:90;
(b) a VL sequence of SEQ ID NO:91 and a VH sequence of SEQ ID NO:92;
(c) a VL sequence of SEQ ID NO:93 and a VH sequence of SEQ ID NO:94;
(d) a VL sequence of SEQ ID NO:95 and a VH sequence of SEQ ID NO:96;
(e) a VL sequence of SEQ ID NO:97 and a VH sequence of SEQ ID NO:98;
(f) a VL sequence of SEQ ID NO:99 and a VH sequence of SEQ ID NO:100;
(g) a VL sequence of SEQ ID NO:101 and a VH sequence of SEQ ID NO:102;
(h) a VL sequence of SEQ ID NO:190 and a VH sequence of SEQ ID NO:191;
(i) a VL sequence of SEQ ID NO:192 and a VH sequence of SEQ ID NO:193;
(j) a VL sequence of SEQ ID NO:194 and a VH sequence of SEQ ID NO:195;
(k) a VL sequence of SEQ ID NO:196 and a VH sequence of SEQ ID NO:197;
(l) a VL sequence of SEQ ID NO:198 and a VH sequence of SEQ ID NO:199;
(m) a VL sequence of SEQ ID NO:200 and a VH sequence of SEQ ID NO:201;
(n) a VL sequence of SEQ ID NO:202 and a VH sequence of SEQ ID NO:203;
(o) a VL sequence of SEQ ID NO:204 and a VH sequence of SEQ ID NO:205;
(p) a VL sequence of SEQ ID NO:206 and a VH sequence of SEQ ID NO:207;
(q) a VL sequence of SEQ ID NO:208 and a VH sequence of SEQ ID NO:209;
(r) a VL sequence of SEQ ID NO:210 and a VH sequence of SEQ ID NO:211;
(w) a VL sequence of SEQ ID NO:212 and a VH sequence of SEQ ID NO:213; or
(x) a VL sequence of SEQ ID NO:214 and a VH sequence of SEQ ID NO:215.

3. The antibody of claim 1 or 2, wherein the antibody inhibits the interaction of RSPO3 with a transmembrane E3 ubiquitinase.

4. The isolated antibody of claim 3, wherein the transmembrane E3 ubiquitinase is ZNRF3 and/or RNF43.

5. The isolated antibody of any one of claims 1-4, wherein the antibody inhibits RSPO3 mediated wnt signaling.

6. The isolated antibody of any one of claims 1-5, which is an antibody fragment that binds RSPO3.

7. The antibody of any one of claims 1-6, which is a monoclonal antibody.

8. The antibody of any one of claims 1-7, which is a humanized, or chimeric antibody.

9. The antibody of any one of claims 1-8, which is a full length IgG1 or IgG2a antibody.

10. Isolated nucleic acid encoding the antibody of any one of claims 1-9.

11. A host cell comprising the nucleic acid of claim 10.

12. A method of producing an antibody comprising culturing the host cell of claim 11 so that the antibody is produced.

13. The method of claim 12, further comprising recovering the antibody from the host cell.

14. An immunoconjugate comprising the antibody of any one of claims 1-9 and a cytotoxic agent.

15. A pharmaceutical formulation comprising the antibody of any one of claims 1-9 and a pharmaceutically acceptable carrier.

16. The pharmaceutical formulation of claim 15, further comprising an additional therapeutic agent.

17. The antibody of any one of claims 1-9 for use as a medicament.

18. The antibody of any one of claims 1-9 for use in treating cancer.

19. The antibody of claim 18, wherein the cancer is gastrointestinal cancer, stomach cancer, colon cancer, colorectal cancer, lung cancer, or rectal cancer.

20. The antibody of any one of claims 1-9 for use, when occurring in pathological conditions, in inhibiting wnt signaling, inhibiting angiogenesis and/or vasculogenesis, and/or inhibiting cell proliferation.

21. The antibody of any one of claims 18-19, wherein the cancer is **characterized by** a RSPO translocation.

## Patentansprüche

1. Isolierter Antikörper, der an RSPO3 bindet, wobei der Antikörper Folgendes umfasst:
(a) eine VL, umfassend (i) HVR-L1, umfassend die Aminosäuresequenz von SEQ ID NO:5, (ii) HVR-L2, umfassend die Aminosäuresequenz von SEQ ID NO:6, und (iii) HVR-L3, umfassend die Aminosäuresequenz von SEQ ID NO:7; und eine VH, umfassend (i) HVR-H1, umfassend die Aminosäuresequenz von SEQ ID NO:8, (ii) HVR-H2, umfassend die Aminosäuresequenz von SEQ ID NO:9, und (iii) HVR-H3, umfassend die Aminosäuresequenz von SEQ ID NO:10;
(b) eine VL, umfassend (i) HVR-L1, umfassend die Aminosäuresequenz von SEQ ID NO:11, (ii) HVR-L2, umfassend die Aminosäuresequenz von SEQ ID NO:12, und (iii) HVR-L3, umfassend die Aminosäuresequenz von SEQ ID NO:13; und eine VH, umfassend (i) HVR-H1, umfassend die Aminosäuresequenz von SEQ ID NO:14, (ii) HVR-H2, umfassend die Aminosäuresequenz von SEQ ID NO:15, und (iii) HVR-H3, umfassend die Aminosäuresequenz von SEQ ID NO:16;
(c) eine VL, umfassend (i) HVR-L1, umfassend die Aminosäuresequenz von SEQ ID NO:17, (ii) HVR-L2, umfassend die Aminosäuresequenz von SEQ ID NO:18, und (iii) HVR-L3, umfassend die Aminosäuresequenz von SEQ ID NO:19; und eine VH, umfassend (i) HVR-H1, umfassend die Aminosäuresequenz von SEQ ID NO:20, (ii) HVR-H2, umfassend die Aminosäuresequenz von SEQ ID NO:21, und (iii) HVR-H3, umfassend die Aminosäuresequenz von SEQ ID NO:22;
(d) eine VL, umfassend (i) HVR-L1, umfassend die Aminosäuresequenz von SEQ ID NO:23, (ii) HVR-L2, umfassend die Aminosäuresequenz von SEQ ID NO:24, und (iii) HVR-L3, umfassend die Aminosäuresequenz von SEQ ID NO:25; und eine VH, umfassend (i) HVR-H1, umfassend die Aminosäuresequenz von SEQ ID NO:26, (ii) HVR-H2, umfassend die Aminosäuresequenz von SEQ ID NO:27, und (iii) HVR-H3, umfassend die Aminosäuresequenz von SEQ ID NO:28;
(e) eine VL, umfassend (i) HVR-L1, umfassend die Aminosäuresequenz von SEQ ID NO:29, (ii) HVR-L2, umfassend die Aminosäuresequenz von SEQ ID NO:30, und (iii) HVR-L3, umfassend die Aminosäuresequenz von SEQ ID NO:31; und eine VH, umfassend (i) HVR-H1, umfassend die Aminosäuresequenz von SEQ ID NO:32, (ii) HVR-H2, umfassend die Aminosäuresequenz von SEQ ID NO:33, und (iii) HVR-H3, umfassend die Aminosäuresequenz von SEQ ID NO:34;
(f) eine VL, umfassend (i) HVR-L1, umfassend die Aminosäuresequenz von SEQ ID NO:35, (ii) HVR-L2, umfassend die Aminosäuresequenz von SEQ ID NO:36, und (iii) HVR-L3, umfassend die Aminosäuresequenz von SEQ ID NO:37; und eine VH, umfassend (i) HVR-H1, umfassend die Aminosäuresequenz von SEQ ID NO:38, (ii) HVR-H2, umfassend die Aminosäuresequenz von SEQ ID NO:39, und (iii) HVR-H3, umfassend die Aminosäuresequenz von SEQ ID NO:40;
(g) eine VL, umfassend (i) HVR-L1, umfassend die Aminosäuresequenz von SEQ ID NO:41, (ii) HVR-L2, umfassend die Aminosäuresequenz von SEQ ID NO:42, und (iii) HVR-L3, umfassend die Aminosäuresequenz von SEQ ID NO:43; und eine VH, umfassend (i) HVR-H1, umfassend die Aminosäuresequenz von SEQ ID NO:44, (ii) HVR-H2, umfassend die Aminosäuresequenz von SEQ ID NO:45, und (iii) HVR-H3, umfassend die Aminosäuresequenz von SEQ ID NO:46;
(h) eine VL, umfassend (i) HVR-L1, umfassend die Aminosäuresequenz von SEQ ID NO:23, (ii) HVR-L2, umfassend die Aminosäuresequenz von SEQ ID NO:24, und (iii) HVR-L3, umfassend die Aminosäuresequenz von SEQ ID NO:25; und eine VH, umfassend (i) HVR-H1, umfassend die Aminosäuresequenz von SEQ ID NO:26, (ii) HVR-H2, umfassend die Aminosäuresequenz von SEQ ID NO:27, und (iii) HVR-H3, umfassend die Aminosäuresequenz von SEQ ID NO:188;
(i) eine VL, umfassend (i) HVR-L1, umfassend die Aminosäuresequenz von SEQ ID NO:23, (ii) HVR-L2, umfassend die Aminosäuresequenz von SEQ ID NO:24, und (iii) HVR-L3, umfassend die Aminosäuresequenz von SEQ ID NO:25; und eine VH, umfassend (i) HVR-H1, umfassend die Aminosäuresequenz von SEQ ID NO:26, (ii) HVR-H2, umfassend die Aminosäuresequenz von SEQ ID NO:27, und (iii) HVR-H3, umfassend die Aminosäuresequenz von SEQ ID NO:189;
(j) eine VL, umfassend (i) HVR-L1, umfassend die Aminosäuresequenz von SEQ ID NO:77, (ii) HVR-L2, umfassend die Aminosäuresequenz von SEQ ID NO:78, und (iii) HVR-L3, umfassend die Aminosäuresequenz von SEQ ID NO:79; und eine VH, umfassend (i) HVR-H1, umfassend die Aminosäuresequenz von SEQ ID NO:80, (ii) HVR-H2, umfassend die Aminosäuresequenz von SEQ ID NO:81, und (iii) HVR-H3, umfassend die Aminosäuresequenz von SEQ ID NO:82;
(k) eine VL, umfassend (i) HVR-L1, umfassend die Aminosäuresequenz von SEQ ID NO:83, (ii) HVR-L2, umfassend die Aminosäuresequenz von SEQ ID NO:84, und (iii) HVR-L3, umfassend die Aminosäuresequenz von SEQ ID NO:85; und eine VH, umfassend (i) HVR-H1, umfassend die Aminosäuresequenz von SEQ ID NO:86, (ii) HVR-H2, umfassend die Aminosäuresequenz von SEQ ID NO:87, und (iii) HVR-H3, umfassend die Aminosäuresequenz von SEQ ID NO:88 oder
(l) eine VL, umfassend (i) HVR-L1, umfassend die Aminosäuresequenz von SEQ ID NO:77, (ii) HVR-L2, umfassend die Aminosäuresequenz von SEQ ID NO:78, und (iii) HVR-L3, umfassend die Aminosäuresequenz von SEQ ID NO:79; und eine VH, umfassend (i) HVR-H1, umfassend die Aminosäuresequenz von SEQ ID NO:80, (ii) HVR-H2, umfassend die Aminosäuresequenz von SEQ ID NO:81, und (iii) HVR-H3, umfassend die Aminosäuresequenz von SEQ ID NO:216.

2. Isolierter Antikörper nach Anspruch 1, wobei der Antikörper Folgendes umfasst
(a) eine VL-Sequenz von SEQ ID NO:89 und eine VH-Sequenz von SEQ ID NO:90;
(b) eine VL-Sequenz von SEQ ID NO:91 und eine VH-Sequenz von SEQ ID NO:92;
(c) eine VL-Sequenz von SEQ ID NO:93 und eine VH-Sequenz von SEQ ID NO:94;
(d) eine VL-Sequenz von SEQ ID NO:95 und eine VH-Sequenz von SEQ ID NO:96;
(e) eine VL-Sequenz von SEQ ID NO:97 und eine VH-Sequenz von SEQ ID NO:98;
(f) eine VL-Sequenz von SEQ ID NO:99 und eine VH-Sequenz von SEQ ID NO:100;
(g) eine VL-Sequenz von SEQ ID NO:101 und eine VH-Sequenz von SEQ ID NO:102;
(h) eine VL-Sequenz von SEQ ID NO:190 und eine VH-Sequenz von SEQ ID NO:191;
(i) eine VL-Sequenz von SEQ ID NO:192 und eine VH-Sequenz von SEQ ID NO:193;
(j) eine VL-Sequenz von SEQ ID NO:194 und eine VH-Sequenz von SEQ ID NO:195;
(k) eine VL-Sequenz von SEQ ID NO:196 und eine VH-Sequenz von SEQ ID NO:197;
(l) eine VL-Sequenz von SEQ ID NO:198 und eine VH-Sequenz von SEQ ID NO:199;
(m) eine VL-Sequenz von SEQ ID NO:200 und eine VH-Sequenz von SEQ ID NO:201;
(n) eine VL-Sequenz von SEQ ID NO:202 und eine VH-Sequenz von SEQ ID NO:203;
(o) eine VL-Sequenz von SEQ ID NO:204 und eine VH-Sequenz von SEQ ID NO:205;
(p) eine VL-Sequenz von SEQ ID NO:206 und eine VH-Sequenz von SEQ ID NO:207;
(q) eine VL-Sequenz von SEQ ID NO:208 und eine VH-Sequenz von SEQ ID NO:209;
(r) eine VL-Sequenz von SEQ ID NO:210 und eine VH-Sequenz von SEQ ID NO:211;
(w) eine VL-Sequenz von SEQ ID NO:212 und eine VH-Sequenz von SEQ ID NO:213 oder
(x) eine VL-Sequenz von SEQ ID NO:214 und eine VH-Sequenz von SEQ ID NO:215.

3. Antikörper nach Anspruch 1 oder 2, wobei der Antikörper die Wechselwirkung von RSPO3 mit einer Transmembran-E3-Ubiquitinase inhibiert.

4. Isolierter Antikörper nach Anspruch 3, wobei die Transmembran-E3-Ubiquitinase ZNRF3 und/oder RNF43 ist.

5. Isolierter Antikörper nach einem der Ansprüche 1-4, wobei der Antikörper RSPO3-vermittelte Wnt-Signalisierung inhibiert.

6. Isolierter Antikörper nach einem der Ansprüche 1-5, der ein Antikörperfragment ist, das RSPO3 bindet.

7. Antikörper nach einem der Ansprüche 1-6, der ein monoklonaler Antikörper ist.

8. Antikörper nach einem der Ansprüche 1-7, der ein humanisierter oder chimärer Antikörper ist.

9. Antikörper nach einem der Ansprüche 1-8, der ein IgG1- oder IgG2a-Antikörper voller Länge ist.

10. Isolierte Nukleinsäure, die für den Antikörper nach einem der Ansprüche 1-9 kodiert.

11. Wirtszelle, umfassend die Nukleinsäure nach Anspruch 10.

12. Verfahren zum Produzieren eines Antikörpers, umfassend das Kultivieren der Wirtszelle nach Anspruch 11, so dass der Antikörper produziert wird.

13. Verfahren nach Anspruch 12, ferner umfassend das Gewinnen des Antikörpers aus der Wirtszelle.

14. Immunkonjugat, umfassend den Antikörper nach einem der Ansprüche 1-9 und ein zytotoxisches Mittel.

15. Pharmazeutische Formulierung, umfassend den Antikörper nach einem der Ansprüche 1-9 und einen pharmazeutisch unbedenklichen Träger.

16. Pharmazeutische Formulierung nach Anspruch 15, ferner umfassend ein zusätzliches Therapeutikum.

17. Antikörper nach einem der Ansprüche 1-9 zur Verwendung als ein Medikament.

18. Antikörper nach einem der Ansprüche 1-9 zur Verwendung bei der Behandlung von Krebs.

19. Antikörper nach Anspruch 18, wobei der Krebs Magen-Darm-Krebs, Magenkrebs, Kolonkrebs, Dickdarmkrebs, Lungenkrebs oder Rektumkrebs ist.

20. Antikörper nach einem der Ansprüche 1-9 zur Verwendung beim Inhibieren von Wnt-Signalisierung, Inhibieren von Angiogenese und/oder Gefäßneubildung und/oder Inhibieren von Zellproliferation, sofern diese in pathologischen Zuständen auftreten.

21. Antikörper nach einem der Ansprüche 18-19, wobei der Krebs durch eine RSPO-Translokation gekennzeichnet ist.

## Revendications

1. Anticorps isolé qui se lie à la RSPO3, dans lequel l'anticorps comprend :
(a) un VL comprenant (i) une HVR-L1 comprenant la séquence d'acides aminés de SEQ ID NO : 5, (ii) une HVR-L2 comprenant la séquence d'acides aminés de SEQ ID NO : 6 et (iii) une HVR-L3 comprenant la séquence d'acides aminés de SEQ ID NO : 7 ; et un VH comprenant (i) une HVR-H1 comprenant la séquence d'acides aminés de SEQ ID NO : 8, (ii) une HVR-H2 comprenant la séquence d'acides aminés de SEQ ID NO : 9 et (iii) une HVR-H3 comprenant la séquence d'acides aminés de SEQ ID NO : 10 ;
(b) un VL comprenant (i) une HVR-L1 comprenant la séquence d'acides aminés de SEQ ID NO : 11, (ii) une HVR-L2 comprenant la séquence d'acides aminés de SEQ ID NO : 12 et (iii) une HVR-L3 comprenant la séquence d'acides aminés de SEQ ID NO : 13 ; et un VH comprenant (i) une HVR-H1 comprenant la séquence d'acides aminés de SEQ ID NO : 14, (ii) une HVR-H2 comprenant la séquence d'acides aminés de SEQ ID NO : 15 et (iii) une HVR-H3 comprenant la séquence d'acides aminés de SEQ ID NO : 16 ;
(c) un VL comprenant (i) une HVR-L1 comprenant la séquence d'acides aminés de SEQ ID NO : 17, (ii) une HVR-L2 comprenant la séquence d'acides aminés de SEQ ID NO : 18 et (iii) une HVR-L3 comprenant la séquence d'acides aminés de SEQ ID NO : 19 ; et un VH comprenant (i) une HVR-H1 comprenant la séquence d'acides aminés de SEQ ID NO : 20, (ii) une HVR-H2 comprenant la séquence d'acides aminés de SEQ ID NO : 21 et (iii) une HVR-H3 comprenant la séquence d'acides aminés de SEQ ID NO : 22 ;
(d) un VL comprenant (i) une HVR-L1 comprenant la séquence d'acides aminés de SEQ ID NO : 23, (ii) une HVR-L2 comprenant la séquence d'acides aminés de SEQ ID NO : 24 et (iii) une HVR-L3 comprenant la séquence d'acides aminés de SEQ ID NO : 25 ; et un VH comprenant (i) une HVR-H1 comprenant la séquence d'acides aminés de SEQ ID NO : 26, (ii) une HVR-H2 comprenant la séquence d'acides aminés de SEQ ID NO : 27 et (iii) une HVR-H3 comprenant la séquence d'acides aminés de SEQ ID NO : 28 ;
(e) un VL comprenant (i) une HVR-L1 comprenant la séquence d'acides aminés de SEQ ID NO : 29, (ii) une HVR-L2 comprenant la séquence d'acides aminés de SEQ ID NO : 30 et (iii) une HVR-L3 comprenant la séquence d'acides aminés de SEQ ID NO : 31 ; et un VH comprenant (i) une HVR-H1 comprenant la séquence d'acides aminés de SEQ ID NO : 32, (ii) une HVR-H2 comprenant la séquence d'acides aminés de SEQ ID NO : 33 et (iii) une HVR-H3 comprenant la séquence d'acides aminés de SEQ ID NO : 34 ;
(f) un VL comprenant (i) une HVR-L1 comprenant la séquence d'acides aminés de SEQ ID NO : 35, (ii) une HVR-L2 comprenant la séquence d'acides aminés de SEQ ID NO : 36 et (iii) une HVR-L3 comprenant la séquence d'acides aminés de SEQ ID NO : 37 ; et un VH comprenant (i) une HVR-H1 comprenant la séquence d'acides aminés de SEQ ID NO : 38, (ii) une HVR-H2 comprenant la séquence d'acides aminés de SEQ ID NO : 39 et (iii) une HVR-H3 comprenant la séquence d'acides aminés de SEQ ID NO : 40 ;
(g) un VL comprenant (i) une HVR-L1 comprenant la séquence d'acides aminés de SEQ ID NO : 41, (ii) une HVR-L2 comprenant la séquence d'acides aminés de SEQ ID NO : 42 et (iii) une HVR-L3 comprenant la séquence d'acides aminés de SEQ ID NO : 43 ; et un VH comprenant (i) une HVR-H1 comprenant la séquence d'acides aminés de SEQ ID NO : 44, (ii) une HVR-H2 comprenant la séquence d'acides aminés de SEQ ID NO : 45 et (iii) une HVR-H3 comprenant la séquence d'acides aminés de SEQ ID NO : 46 ;
(h) un VL comprenant (i) une HVR-L1 comprenant la séquence d'acides aminés de SEQ ID NO : 23, (ii) une HVR-L2 comprenant la séquence d'acides aminés de SEQ ID NO : 24 et (iii) une HVR-L3 comprenant la séquence d'acides aminés de SEQ ID NO : 25 ; et un VH comprenant (i) une HVR-H1 comprenant la séquence d'acides aminés de SEQ ID NO : 26, (ii) une HVR-H2 comprenant la séquence d'acides aminés de SEQ ID NO : 27 et (iii) une HVR-H3 comprenant la séquence d'acides aminés de SEQ ID NO : 188 ;
(i) un VL comprenant (i) une HVR-L1 comprenant la séquence d'acides aminés de SEQ ID NO : 23, (ii) une HVR-L2 comprenant la séquence d'acides aminés de SEQ ID NO : 24 et (iii) une HVR-L3 comprenant la séquence d'acides aminés de SEQ ID NO : 25 ; et un VH comprenant (i) une HVR-H1 comprenant la séquence d'acides aminés de SEQ ID NO : 26, (ii) une HVR-H2 comprenant la séquence d'acides aminés de SEQ ID NO : 27 et (iii) une HVR-H3 comprenant la séquence d'acides aminés de SEQ ID NO : 189 ;
(j) un VL comprenant (i) une HVR-L1 comprenant la séquence d'acides aminés de SEQ ID NO : 77, (ii) une HVR-L2 comprenant la séquence d'acides aminés de SEQ ID NO : 78 et (iii) une HVR-L3 comprenant la séquence d'acides aminés de SEQ ID NO : 79 ; et un VH comprenant (i) une HVR-H1 comprenant la séquence d'acides aminés de SEQ ID NO : 80, (ii) une HVR-H2 comprenant la séquence d'acides aminés de SEQ ID NO : 81 et (iii) une HVR-H3 comprenant la séquence d'acides aminés de SEQ ID NO : 82 ;
(k) un VL comprenant (i) une HVR-L1 comprenant la séquence d'acides aminés de SEQ ID NO : 83, (ii) une HVR-L2 comprenant la séquence d'acides aminés de SEQ ID NO : 84 et (iii) une HVR-L3 comprenant la séquence d'acides aminés de SEQ ID NO : 85 ; et un VH comprenant (i) une HVR-H1 comprenant la séquence d'acides aminés de SEQ ID NO : 86, (ii) une HVR-H2 comprenant la séquence d'acides aminés de SEQ ID NO : 87 et (iii) une HVR-H3 comprenant la séquence d'acides aminés de SEQ ID NO : 88 ; ou
(l) un VL comprenant (i) une HVR-L1 comprenant la séquence d'acides aminés de SEQ ID NO : 77, (ii) une HVR-L2 comprenant la séquence d'acides aminés de SEQ ID NO : 78 et (iii) une HVR-L3 comprenant la séquence d'acides aminés de SEQ ID NO : 79 ; et un VH comprenant (i) une HVR-H1 comprenant la séquence d'acides aminés de SEQ ID NO : 80, (ii) une HVR-H2 comprenant la séquence d'acides aminés de SEQ ID NO : 81 et (iii) une HVR-H3 comprenant la séquence d'acides aminés de SEQ ID NO : 216.

2. Anticorps isolé selon la revendication 1, dans lequel l'anticorps comprend
(a) une séquence VL de SEQ ID NO : 89 et une séquence VH de SEQ ID NO : 90 ;
(b) une séquence VL de SEQ ID NO : 91 et une séquence VH de SEQ ID NO : 92 ;
(c) une séquence VL de SEQ ID NO : 93 et une séquence VH de SEQ ID NO : 94 ;
(d) une séquence VL de SEQ ID NO : 95 et une séquence VH de SEQ ID NO : 96 ;
(e) une séquence VL de SEQ ID NO : 97 et une séquence VH de SEQ ID NO : 98 ;
(f) une séquence VL de SEQ ID NO : 99 et une séquence VH de SEQ ID NO : 100 ;
(g) une séquence VL de SEQ ID NO : 101 et une séquence VH de SEQ ID NO : 102 ;
(h) une séquence VL de SEQ ID NO : 190 et une séquence VH de SEQ ID NO : 191 ;
(i) une séquence VL de SEQ ID NO : 192 et une séquence VH de SEQ ID NO : 193 ;
(j) une séquence VL de SEQ ID NO : 194 et une séquence VH de SEQ ID NO : 195 ;
(k) une séquence VL de SEQ ID NO : 196 et une séquence VH de SEQ ID NO : 197 ;
(l) une séquence VL de SEQ ID NO : 198 et une séquence VH de SEQ ID NO : 199 ;
(m) une séquence VL de SEQ ID NO : 200 et une séquence VH de SEQ ID NO : 201 ;
(n) une séquence VL de SEQ ID NO : 202 et une séquence VH de SEQ ID NO : 203 ;
(o) une séquence VL de SEQ ID NO : 204 et une séquence VH de SEQ ID NO : 205 ;
(p) une séquence VL de SEQ ID NO : 206 et une séquence VH de SEQ ID NO : 207 ;
(q) une séquence VL de SEQ ID NO : 208 et une séquence VH de SEQ ID NO : 209 ;
(r) une séquence VL de SEQ ID NO : 210 et une séquence VH de SEQ ID NO : 211 ;
(w) une séquence VL de SEQ ID NO : 212 et une séquence VH de SEQ ID NO : 213 ; ou
(x) une séquence VL de SEQ ID NO : 214 et une séquence VH de SEQ ID NO : 215.

3. Anticorps selon la revendication 1 ou 2, dans lequel l'anticorps inhibe l'interaction de la RSPO3 avec une ubiquitinase E3 transmembranaire.

4. Anticorps isolé selon la revendication 3, dans lequel l'ubiquitinase E3 transmembranaire est ZNRF3 et/ou RNF43.

5. Anticorps isolé selon l'une quelconque des revendications 1 à 4, dans lequel l'anticorps inhibe la signalisation wnt médiée par la RSPO3.

6. Anticorps isolé selon l'une quelconque des revendications 1 à 5, qui est un fragment d'anticorps qui se lie à la RSPO3.

7. Anticorps selon l'une quelconque des revendications 1 à 6, qui est un anticorps monoclonal.

8. Anticorps selon l'une quelconque des revendications 1 à 7, qui est un anticorps humanisé ou chimérique.

9. Anticorps selon l'une quelconque des revendications 1 à 8, qui est un anticorps IgG1 ou IgG2a pleine longueur.

10. Acide nucléique isolé codant pour l'anticorps selon l'une quelconque des revendications 1 à 9.

11. Cellule hôte comprenant l'acide nucléique selon la revendication 10.

12. Procédé de production d'un anticorps comprenant la culture de la cellule hôte selon la revendication 11 de manière à ce que l'anticorps soit produit.

13. Procédé selon la revendication 12, comprenant en outre la récupération de l'anticorps de la cellule hôte.

14. Immunoconjugué comprenant l'anticorps selon l'une quelconque des revendications 1 à 9 et un agent cytotoxique.

15. Formulation pharmaceutique comprenant l'anticorps selon l'une quelconque des revendications 1 à 9 et un véhicule pharmaceutiquement acceptable.

16. Formulation pharmaceutique selon la revendication 15, comprenant en outre un agent thérapeutique supplémentaire.

17. Anticorps selon l'une quelconque des revendications 1 à 9 pour une utilisation comme médicament.

18. Anticorps selon l'une quelconque des revendications 1 à 9 pour une utilisation dans le traitement d'un cancer.

19. Anticorps selon la revendication 18, dans lequel le cancer est un cancer gastrointestinal, un cancer de l'estomac, un cancer du côlon, un cancer colorectal, un cancer du poumon ou un cancer rectal.

20. Anticorps selon l'une quelconque des revendications 1 à 9 pour une utilisation, lorsqu'elles se manifestent dans des états pathologiques, dans l'inhibition de la signalisation wnt, l'inhibition de l'angiogenèse et/ou de la vasculogenèse, et/ou l'inhibition de la prolifération cellulaire.

21. Anticorps selon l'une quelconque des revendications 18 à 19, dans lequel le cancer est **caractérisé par** une translocation de la RSPO.
